(19) Europäisches Patentamt
European Patent Office
Office européen des brevets

(11) EP 4 570 799 A1

(12) EUROPEAN PATENT APPLICATION
published in accordance with Art. 153(4) EPC

(43) Date of publication:
**18.06.2025 Bulletin 2025/25**

(21) Application number: **23851722.1**

(22) Date of filing: **04.08.2023**

(51) International Patent Classification (IPC):
***C07D 417/14*** (2006.01) ***C07D 413/14*** (2006.01)
***C07D 271/113*** (2006.01) ***A61K 31/4178*** (2006.01)
***A61K 31/445*** (2006.01) ***A61P 25/28*** (2006.01)
***A61P 25/00*** (2006.01)

(52) Cooperative Patent Classification (CPC):
**A61K 31/4178; A61K 31/445; A61P 25/00; A61P 25/28; C07D 271/113; C07D 413/14; C07D 417/14**

(86) International application number:
**PCT/CN2023/111229**

(87) International publication number:
**WO 2024/032501 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.08.2022 CN 202210945768**
**16.06.2023 CN 202310729419**

(71) Applicant: **Shenzhen Zhongge Biological Technology Co., Ltd.**
**Futian District**
**Shenzhen, Guangdong 518017 (CN)**

(72) Inventors:
- **CAO, Bin**
  **Shenzhen, Guangdong 518017 (CN)**
- **ZHAO, Liyu**
  **Shenzhen, Guangdong 518017 (CN)**
- **DOU, Guosheng**
  **Shenzhen, Guangdong 518017 (CN)**
- **LU, Sizhu**
  **Shenzhen, Guangdong 518017 (CN)**
- **YANG, Jun**
  **Shenzhen, Guangdong 518017 (CN)**
- **CHEN, Zhaoqiang**
  **Shenzhen, Guangdong 518017 (CN)**
- **ZHANG, Qihua**
  **Shenzhen, Guangdong 518017 (CN)**
- **WANG, Shaohui**
  **Shenzhen, Guangdong 518017 (CN)**
- **CHEN, Bin**
  **Shenzhen, Guangdong 518017 (CN)**
- **ZHANG, Peiyu**
  **Shenzhen, Guangdong 518017 (CN)**

(74) Representative: **Marks & Clerk LLP**
**15 Fetter Lane**
**London EC4A 1BW (GB)**

# (54) COMPOUND, PHARMACEUTICAL COMPOSITION COMPRISING SAME, AND USE THEREOF

(57) Provided are a compound of formula 0, a stereoisomer thereof, a tautomer thereof, a geometric isomer thereof, an enantiomer thereof, a diastereomer thereof, a racemate thereof, a polymorph thereof, a solvate thereof, a hydrate thereof, an N-oxide thereof, an isotopically labeled compound thereof, a metabolite thereof, an ester thereof, a prodrug thereof, and a pharmaceutically acceptable salt thereof. In formula 0, ring B is a 5- to 10-membered spiro ring or a 5- to 10-membered spiro heterocyclic ring. The 5- to 10-membered spiro ring and the 5- to 10-membered spiro heterocyclic ring are optionally substituted with 1-5 $R^{1F}$. The compound can significantly weaken the integrated stress response (ISR) of cells and activate the activity of elF2B, so that proteins in the cells tend to be synthesized normally.

EP 4 570 799 A1

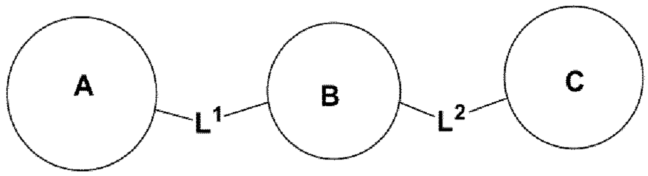

Formula ()

## Description

The present application claims the priority to Chinese patent application CN202210945768.0 filed on August 8, 2022 and Chinese patent application CN202310729419.X filed on June 16, 2023. The Chinese patent applications are incorporated herein by reference in their entirety.

### Technical Field

The present disclosure relates to a compound, and a pharmaceutical composition comprising same and the use thereof.

### Background Art

Amyotrophic Lateral Sclerosis (ALS), also known as motor neuron disease (MND) and Lou Gehrig's disease, is an irreversible, fatal motor neuron disease. The main symptoms of amyotrophic lateral sclerosis is progressive muscle weakness and atrophy of muscles of the limbs and trunk, and gradual loss of motor function, as if being "frozen", so it is called "jian dong ren", meaning "gradually frozen men". Most ALS patients die from respiratory failure, usually within three to five years after the first appearance of symptoms. Currently, there is no cure for ALS and no effective treatment methods that can stop or reverse the progression of the disease. The core pathological finding in ALS is motor neuron death in the motor cortex and spinal cord. Degeneration of corticospinal axons leads to thinning and scarring (sclerosis) of the lateral aspects of the spinal cord.

Loss of protein-folding homeostasis is a hallmark of many of the most prevalent neurodegenerative diseases. As a mechanism to cope with folding stress in the endoplasmic reticulum (ER), the unfolded protein response (UPR) comprises a set of signaling mechanisms that initiate gene expression programs to restore protein homeostasis or, when the stress is chronic or overloaded, promote neuron death. This function of the UPR has been proposed to play a key role in ALS.

The integrated stress response (ISR) is an evolutionarily conserved intracellular signaling network that helps the cell, tissue and organism to adapt to a variable environment and maintain health. In response to various changes, the ISR restores balance by reprogramming gene expression. In the brain, long-term memory formation requires new protein synthesis, and therefore inhibition of the ISR can enhance long-term memory formation, whereas ISR activation prevents it. Furthermore, age-related cognitive disorders are commonly associated with ISR activation.

As a central regulator of protein homeostasis, the ISR is activated in a wide range of diseases of the brain. This activation is evidenced by detection of eIF2-P and phosphorylation of PKR, PERK and GCN2 in brains, including those from patient samples and animal model samples of neurodegenerative diseases, such as Alzheimer's disease, Parkinson's disease, Huntington's disease, traumatic brain injury, Down syndrome, and Charcot-Marie-Tooth disease. Notably, ISR activation causes cognitive defects in mouse models of traumatic brain injury, aging and Alzheimer's disease.

eIF2B (eukaryotic translation initiation factor 2B) is a key enzyme in the regulation of protein synthesis and is a guanine nucleotide exchange factor (GEF) specific for translation initiation factor 2. As an eIF2B activator, ISRIB can restore protein translation, revert UPR transcription to basal levels, and alleviate the integrated stress response (ISR).

In addition, the eIF2B activators ABBV-CLS-7262 (AbbVie/Calico) and DNL-343 (Denali Therapeutics) both are indicated for ALS and have entered Phase 1 clinical trials, wherein DNL-343 has demonstrated its safety and tolerability by the Phase 1 data in healthy participants which is already published.

A large number of animal experiments have confirmed that the eIF2B activator ISRIB can improve long-term memory in mouse models. After oral administration of the eIF2B activator ABBV-CLS-7262 for three days, the brain function of model animals can be restored to youthful levels. It means that ABBV-CLS-7262 may be capable of suppressing some neurodegenerative diseases in the later stages and has the potential to treat diseases such as Alzheimer's disease and Parkinson's disease.

### Summary of the Invention

The present disclosure provides a compound of formula 0, or a stereoisomer thereof, a tautomer thereof, a geometric isomer thereof, an enantiomer thereof, a diastereomer thereof, a racemate thereof, a polymorph thereof, a solvate thereof, a hydrate thereof, an N-oxide thereof, an isotopically labeled compound thereof, a metabolite thereof, an ester thereof, a prodrug thereof or a pharmaceutically acceptable salt thereof. The compound can significantly weaken the integrated stress response (ISR) of cells and activate the activity of eIF2B, so that proteins in the cells tend to be synthesized normally.

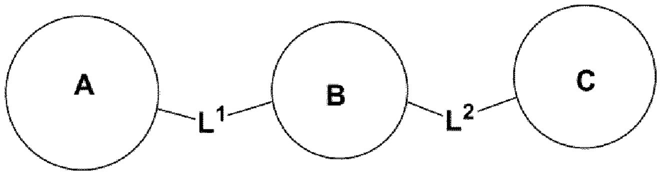

Formula 0

In the formula, ring A and ring C are each independently C1-C6 alkyl, C3-C9 cycloalkyl, 3-to 9-membered heterocyclyl, 6- to 10-membered aryl, 5- to 10-membered heteroaryl, -6- to 10-membered aryl-D-C1-C6 alkyl, 5- to 10-membered heteroaryl-D-C1-C6 alkyl, C3-C9 cycloalkyl-D-C1-C6 alkyl or 3- to 9-membered heterocyclyl-D-C1-C6 alkyl, wherein the alkyl is optionally substituted with halogen or C1-C6 alkyl; the cycloalkyl, heterocyclyl, aryl and heteroaryl can each be optionally substituted with 1-5 $R^1$;

each of the $R^1$ is independently selected from the group consisting of hydrogen, deuterium, halogen, hydroxyl, phenyl, cyano, C1-C10 alkyl, hydroxyl-substituted C1-C6 alkyl, halogen-substituted C1-C6 alkyl, C1-C6 alkoxy, halogen-substituted C1-C6 alkoxy, halogen-substituted C1-C6 alkoxy-C1-C6 alkenyl, amino-substituted C1-C6 alkyl, cyano-substituted C1-C6 alkyl, C1-C3 alkoxy-C1-C6 alkyl, C3-C6 cycloalkyl, C1-C3 alkoxy-C3-C6 cycloalkyl, C1-C3 alkoxy-3- to 6-membered heterocyclyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-SR^{1E}$, $-S(O)R^{1D}$, $-S(O)_2R^{1D}$, $-G^1$, $-O-G^1$ and $-NR^{1B}-G^1$; or 2 $R^1$ groups on adjacent atoms taken together with the atoms to which they are attached can form C3-C7 cycloalkyl, 3- to 7-membered heterocyclyl, 6- to 10-membered aryl or 5- to 6-membered heteroaryl; the C3-C7 cycloalkyl, 3- to 7-membered heterocyclyl, 6- to 10-membered aryl or 5- to 6-membered heteroaryl is each optionally substituted with 1-5 $R^{1F}$; with regard to options for the $R^1$, the phenyl, C1-C10 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen;

each of the $R^{1A}$, $R^{1B}$, $R^{1C}$, $R^{1D}$ and $R^{1E}$ is independently selected from the group consisting of hydrogen, deuterium, C1-C6 alkyl, C3-C9 cycloalkyl, 3- to 9-membered heterocyclyl, halogen-substituted C1-C6 alkyl, halogen-substituted C3-C9 cycloalkyl, halogen-substituted 3- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;

each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F}$;

$L^1$ is null, -NH-,

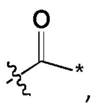, 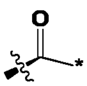, 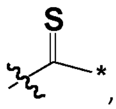, 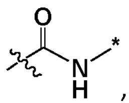, 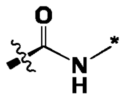, 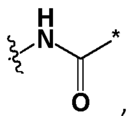, 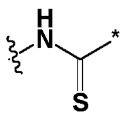

or

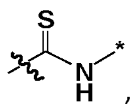,

wherein the end is connected to ring A, and the * end is connected to ring B;

preferably, $L^1$ is null, -NH-,

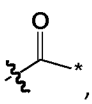, 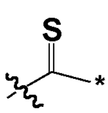, 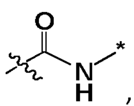, 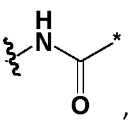, 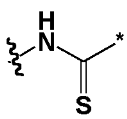 or 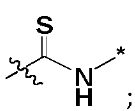;

preferably, $L^1$ is null,

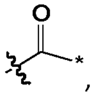, 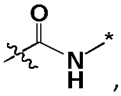, 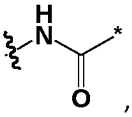, 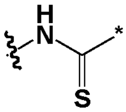 or 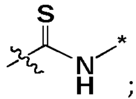;

preferably, the $L^1$ is null,

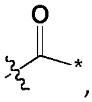, 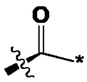, 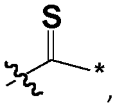, 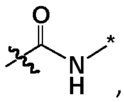, 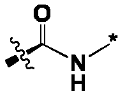, 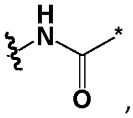, 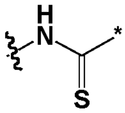

or 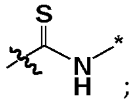;

preferably, the $L^1$ is null,

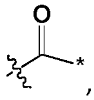, 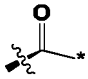, 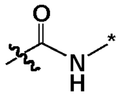 or 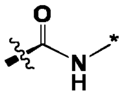;

preferably, the $L^1$ is null,

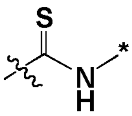 or 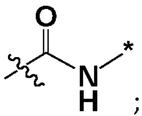;

ring B is a 5- to 10-membered spiro ring or a 5- to 10-membered spiro heterocyclic ring, wherein the 5- to 10-membered spiro ring and the 5- to 10-membered spiro heterocyclic ring are optionally substituted with 1-5 $R^{1F}$;
each of the $R^{1F}$ is independently selected from the group consisting of hydrogen, deuterium, halogen, hydroxyl, amino, cyano, carboxyl, C1-C6 alkyl, C3-C9 cycloalkyl, hydroxyl-substituted C1-C6 alkyl, halogen-substituted C1-C6 alkyl, halogen-substituted C1-C6 alkoxy, halogen-substituted C3-C9 cycloalkyl, halogen-substituted C1-C6 alkenyl, halogen-substituted C1-C6 alkoxy-C1-C6 alkenyl, amino-substituted C1-C6 alkyl, cyano-substituted C1-C6 alkyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-P(O)R^{1B}R^{1C}$ $-SR^{1E}$, $-S(O)R^{1D}$ and $-S(O)_2R^{1D}$.
$L^2$ is null, -NH-,

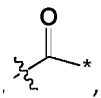, 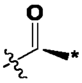, 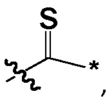, 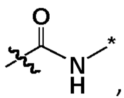, 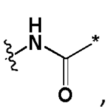, 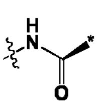, 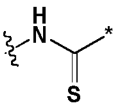 or 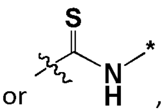,

wherein the end is connected to ring B, and the * end is connected to ring C;
preferably, $L^2$ is null, -NH-,

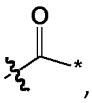, 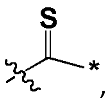, 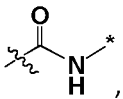, 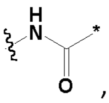, 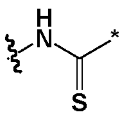 or 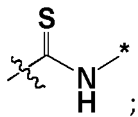;

preferably, $L^2$ is null,

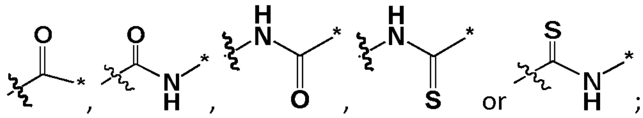 , , , , or ;

preferably, the $L^2$ is null,

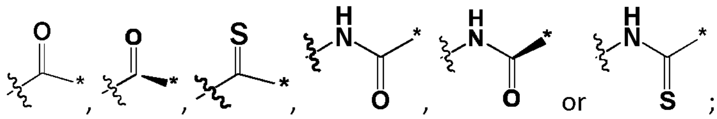 , , , , , or ;

preferably, the $L^2$ is null,

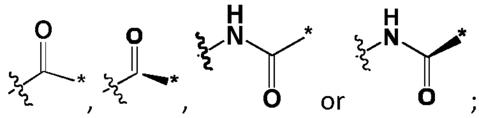 , , , or ;

preferably, the $L^2$ is null or

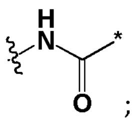 ;

each of the D is independently a linking group containing a heteroatom; preferably, each of the D is independently O or $NR^d$ or S, wherein each $R^d$ is independently selected from hydrogen, deuterium, C1-C6 alkyl and halogen-substituted C1-C6 alkyl; more preferably, each of the D is independently O or NH. Alternatively, each of the D is independently a heteroatom, preferably O or N or S, and more preferably O or N.

In some embodiments, the above-mentioned ring A is C1-C6 alkyl, C3-C9 cycloalkyl, 3- to 9-membered heterocyclyl, 6- to 10-membered aryl, 5- to 10-membered heteroaryl, 6- to 10-membered aryl-D-C1-C6 alkyl, 5- to 10-membered heteroaryl-D-C1-C6 alkyl or 3- to 9-membered heterocyclyl-D-C1-C6 alkyl, wherein the alkyl is optionally substituted with halogen or C1-C6 alkyl; the cycloalkyl, heterocyclyl, aryl and heteroaryl can each be optionally substituted with 1-5 $R^1$;

each of the $R^1$ is independently selected from the group consisting of hydrogen, halogen, cyano, C1-C10 alkyl, hydroxyl-substituted C1-C6 alkyl, halogen-substituted C1-C6 alkyl, C1-C6 alkoxy, halogen-substituted C1-C6 alkoxy, halogen-substituted C1-C6 alkoxy-C1-C6 alkenyl, amino-substituted C1-C6 alkyl, cyano-substituted C1-C6 alkyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-SR^{1E}$, $-S(O)R^{1D}$, $-S(O)_2R^{1D}$, $-G^1$, $-O-G^1$ and $-NR^{1B}-G^1$; or 2 $R^1$ groups on adjacent atoms taken together with the atoms to which they are attached can form C3-C7 cycloalkyl, 3- to 7-membered heterocyclyl, 6- to 10-membered aryl or 5- to 6-membered heteroaryl; the C3-C7 cycloalkyl, 3- to 7-membered heterocyclyl, 6- to 10-membered aryl or 5-to 6-membered heteroaryl is each optionally substituted with 1-5 $R^{1F}$;

each of the $R^{1A}$, $R^{1B}$, $R^{1C}$, $R^{1D}$ and $R^{1E}$ is independently selected from the group consisting of hydrogen, C1-C6 alkyl, C3-C9 cycloalkyl, 3- to 9-membered heterocyclyl, halogen-substituted C1-C6 alkyl, halogen-substituted C3-C9 cycloalkyl, halogen-substituted 3- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;

each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F}$;

each of the $R^{1F}$ is independently selected from the group consisting of hydrogen, halogen, hydroxyl, amino, cyano, carboxyl, C1-C6 alkyl, C3-C9 cycloalkyl, hydroxyl-substituted C1-C6 alkyl, halogen-substituted C1-C6 alkyl, halogen-substituted C1-C6 alkoxy, halogen-substituted C3-C9 cycloalkyl, halogen-substituted C1-C6 alkenyl, halogen-substituted C1-C6 alkoxy-C1-C6 alkenyl, amino-substituted C1-C6 alkyl, cyano-substituted C1-C6 alkyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-P(O)R^{1B}R^{1C}$, $-SR^{1E}$, $-S(O)R^{1D}$ and $-S(O)_2R^{1D}$;

the ring C is 6- to 10-membered aryl-E-C1-C6 alkyl, C3-C9 cycloalkyl-E-C1-C6 alkyl, 3- to 9-membered heterocyclyl-E-C1-C6 alkyl, 3- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, deuterium, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen; or 2 $R^2$ groups on adjacent atoms taken together with the atoms to which they are attached can form C3-C7 cycloalkyl, 3- to 7-membered heterocyclyl, 5- to 6-membered aryl (such as phenyl) or 5- to 6-membered heteroaryl; the C3-C7 cycloalkyl, 3- to 7-membered heterocyclyl, 5- to 6-membered aryl (such as phenyl) or 5- to 6-membered heteroaryl is each optionally substituted with 1-5 $R^{1F}$;

each of the D or E is independently a linking group containing a heteroatom; preferably, each of the D or E is independently O or $NR^d$ or S, wherein each $R^d$ is independently selected from hydrogen, deuterium and C1-C3 alkyl; more preferably, each of the D or E is independently O or NH.

In some embodiments, ring A is 3- to 9-membered heterocyclyl, 6- to 10-membered aryl, 5- to 10-membered heteroaryl, -6- to 10-membered aryl-D-C1-C6 alkyl or 3- to 9-membered heterocyclyl-D-C1-C6 alkyl, wherein the heterocyclyl, aryl and heteroaryl can each be optionally substituted with 1-5 $R^1$;

each of the $R^1$ is independently selected from the group consisting of hydrogen, halogen, halogen-substituted C1-C6 alkyl, halogen-substituted C1-C6 alkoxy, $-OR^{1A}$, $-NR^{1B} R^{1C}$, $- NR^{1B}C(O)R^{1D}$, $-C(O)NR^{1B}R^{1C}$, $-C(O)R^{1D}$, $-G^1$, $-O-G^1$ and $-NR^{1B}-G^1$; or 2 $R^1$ groups on adjacent atoms taken together with the atoms to which they are attached can form 6- to 10-membered aryl or 5- to 6-membered heteroaryl; the 6- to 10-membered aryl or 5- to 6-membered heteroaryl is each optionally substituted with 1-5 $R^{1F}$;

each of the $R^{1A}$, $R^{1B}$, $R^{1C}$ and $R^{1D}$ is independently selected from the group consisting of hydrogen, C1-C6 alkyl, C3-C9 cycloalkyl, 3- to 9-membered heterocyclyl, halogen-substituted C1-C6 alkyl, halogen-substituted C3-C9 cycloalkyl, halogen-substituted 3- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;

each of the $G^1$ is independently selected from C3-C9 cycloalkyl, 3- to 9-membered heterocyclyl and 6- to 10-membered aryl; each of the $G^1$ is optionally substituted with 1-3 $R^{1F}$; when $G^1$ is phenyl, the phenyl and 3- to 9-membered heterocyclyl or 5- to 10-membered heteroaryl optionally share two carbon atoms to form a fused ring;

each of the $R^{1F}$ is independently selected from the group consisting of hydrogen, halogen, C1-C6 alkyl, C3-C9 cycloalkyl, halogen-substituted C1-C6 alkyl, halogen-substituted C1-C6 alkoxy, halogen-substituted C3-C9 cycloalkyl, halogen-substituted C1-C6 alkenyl, oxo, $-OR^{1A}$, $-NR^{1B} R^{1C}$, $- NR^{1B}C(O)R^{1D}$, $-C(O)NR^{1B}R^{1C}$, $-C(O)R^{1D}$, $-P(O) R^{1B}R^{1C}$ and $-S(O)_2R^{1D}$.

In some embodiments, ring A is 4- to 9-membered heterocyclyl, 5- to 7-membered heteroaryl, phenyl-D-C1-C6 alkyl or 4- to 9-membered heterocyclyl-D-C1-C6 alkyl, wherein the heterocyclyl, heteroaryl and phenyl can each be optionally substituted with 1-5 $R^1$;

each of the $R^1$ is independently selected from the group consisting of hydrogen, halogen, halogen-substituted C1-C6 alkyl, halogen-substituted C1-C6 alkoxy, $-OR^{1A}$, $-NR^{1B} R^{1C}$, $- NR^{1B}C(O)R^{1D}$, $-C(O)NR^{1B}R^{1C}$, $-G^1$, $O-G^1$ and $-NR^{1B}-G^1$;

each of the $R^{1A}$, $R^{1B}$, $R^{1C}$ and $R^{1D}$ is independently selected from the group consisting of hydrogen, C1-C6 alkyl, C4-C9 cycloalkyl, 4- to 9-membered heterocyclyl, halogen-substituted C1-C6 alkyl, halogen-substituted C4-C9 cycloalkyl, halogen-substituted 4- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;

each of the $G^1$ is independently selected from C3-C9 cycloalkyl, 3- to 9-membered heterocyclyl and phenyl; each of the $G^1$ is optionally substituted with 1-3 $R^{1F}$; when $G^1$ is phenyl, the phenyl and 3- to 9-membered heterocyclyl or 5- to 7-membered heteroaryl optionally share two carbon atoms to form a fused ring;

each of the $R^{1F}$ is independently selected from the group consisting of hydrogen, halogen, C1-C6 alkyl, C3-C9 cycloalkyl, halogen-substituted C1-C6 alkyl, halogen-substituted C1-C6 alkoxy, halogen-substituted C3-C9 cycloalkyl, halogen-substituted C1-C6 alkenyl, oxo, $-OR^{1A}$, $-NR^{1B} R^{1C}$, $- P(O) R^{1B}R^{1C}$ and $-S(O)_2R^{1D}$.

In some embodiments, ring A is 4- to 8-membered heterocyclyl, 5- to 6-membered heteroaryl, phenyl-D-C1-C3

alkyl or 4- to 8-membered heterocyclyl-D-C1-C3 alkyl, wherein the heterocyclyl, heteroaryl and phenyl can each be optionally substituted with 1-2 $R^1$;

each of the $R^1$ is independently selected from the group consisting of hydrogen, halogen, halogen-substituted C1-C3 alkyl, halogen-substituted C1-C3 alkoxy, $-OR^{1A}$, $-NR^{1B}R^{1C}$, $-NR\ ^{B}C(O)R^{1D}$, $-C(O)NR^{1B}R^{1C}$, $-G^1$, $O-G^1$ and $-NR^{1B}-G^1$;

each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl, wherein preferably, the C3-C6 cycloalkyl is cyclobutyl or cyclopentyl, and the 3- to 6-membered heterocyclyl is oxetanyl, azetidinyl, oxocyclopentyl or azacyclopentyl; each of the $G^1$ is optionally substituted with 1-3 $R^{1F}$; when $G^1$ is phenyl, the phenyl and 5- to 6-membered heterocyclyl or 5- to 7-membered heteroaryl optionally share two carbon atoms to form a fused ring;

each of the $R^{1F}$ is independently selected from the group consisting of hydrogen, halogen, C1-C3 alkyl, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, $-P(O)R^{1B}R^{1C}$ and $-S(O)_2R^{1D}$.

In some embodiments, ring A is 5- to 6-membered heterocyclyl, preferably

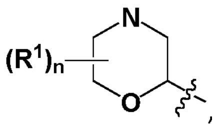,

, or preferably

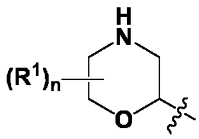 or 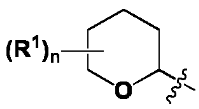.

Preferably, the number n of $R^1$ is 1, 2 or 3;

preferably, each of the $R^1$ is independently selected from the group consisting of hydrogen, halogen, hydroxyl, C1-C3 alkyl and phenyl; and when $R^1$ is phenyl, the phenyl and the 3- to 6-membered heterocyclyl share two carbon atoms to form a fused ring, preferably the phenyl and

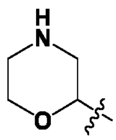 or 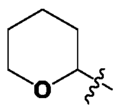

share two carbon atoms to form a fused ring, and the phenyl is optionally substituted with halogen, C1-C3 alkyl, C1-C3 haloalkyl or C1-C3 haloalkoxy;

further preferably, the fusion mode of the phenyl and

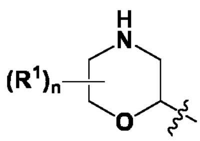 or 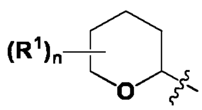 is

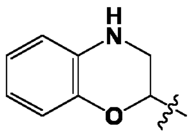 or 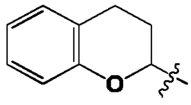,

and the phenyl is optionally substituted with halogen.

In some embodiments, ring A is 5- to 6-membered heteroaryl, preferably any one of the following groups:

(1) 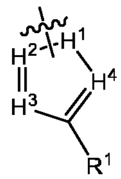,

wherein $H^1$, $H^2$, $H^3$ and $H^4$ are each independently C or a heteroatom, with at least one being a heteroatom, and preferably, the heteroatom is selected from any one of N, O and S; preferably, the $R^1$ is selected from the group consisting of hydrogen, $-OR^{1A}$, $-NR^{1B}R^{1C}$, $-G^1$, $-O-G^1$ and $-NR^{1B}-G^1$, wherein each of the $R^{1A}$, $R^{1B}$ and $R^{1D}$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from 3- to 6-membered cycloalkyl, 3- to 6-membered heterocyclyl and phenyl; each of the $G^1$ is optionally substituted with 1-3 $R^{1F}$;

each of the $R^{1F}$ is independently selected from the group consisting of hydrogen, halogen, C1-C3 alkyl, C3-C6 cycloalkyl, C1-C3 alkoxy, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)\ R^{1B}R^{1C}$ and $-S(O)_2R^{1D}$,

wherein the $R^{1D}$ is selected from the group consisting of hydrogen, C1-C3 alkyl, halogen-substituted C1-C3 alkyl and halogen-substituted C1-C3 alkoxy;

or

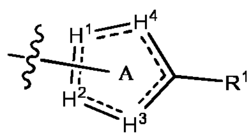,

wherein the ring A is 5-membered heteroaryl, $H^1$, $H^2$, $H^3$ and $H^4$ are each independently selected from CH, N, O and S, and ------ is a single bond or a double bond;

preferably, each of the $R^1$ is independently selected from the group consisting of $-OR^{1A}$, $-NR^{1B}R^{1C}$, $-G^1$, $-O-G^1$ and $-NR^{1B}-G^1$; each of the $R^{1A}$, $R^{1B}$ and $R^{1C}$ is independently selected from the group consisting of hydrogen, C1-C3 alkyl, C1-C3 alkoxy-C1-C6 alkyl, C3-C6 cycloalkyl, 4- to 6-membered heterocyclyl, halogen-substituted C1-C3 alkyl, halogen-substituted C1-C3 alkoxy-C1-C6 alkyl, halogen-substituted C3-C6 cycloalkyl, halogen-substituted 4- to 6-membered heterocyclyl and halogen-substituted C1-C3 alkoxy;

each of the $G^1$ is independently selected from C4-C6 cycloalkyl and 4- to 9-membered heterocyclyl; each of the $G^1$ is optionally substituted with 1-3 $R^{1F}$;

each of the $R^{1F}$ is independently selected from the group consisting of hydrogen, halogen, C1-C3 alkyl, C3-C6 cycloalkyl, C1-C3 alkoxy, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)R^{1B}R^{1C}$ and $-S(O)_2R^{1D}$,

wherein the $R^{1D}$ is selected from the group consisting of hydrogen, C1-C3 alkyl, halogen-substituted C1-C3 alkyl and halogen-substituted C1-C3 alkoxy;

preferably, the ring A is

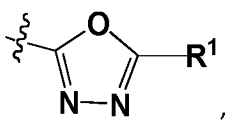, 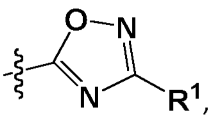, 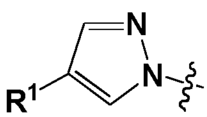,

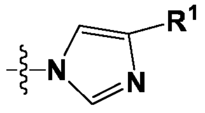 or 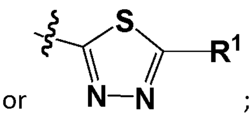 ;

further preferably, the $L^1$ is null;

preferably, the $R^1$ is selected from $-OR^{1A}$, $-G^1$, $-O-G^1$ and $-NR^{1B}-G^1$; $R^{1A}$ is selected from halogen-substituted C1-C3 alkoxy-C1-C3 alkyl; $R^{1B}$ is selected from H and C1-C3 alkyl; each of the $R^{1F}$ is independently selected from the group consisting of halogen, C1-C3 alkyl, C3-C6 cycloalkyl, C1-C3 alkoxy, halogen-substituted C1-C3 alkyl, halogen-substituted C1-C3 alkoxy, halogen-substituted C1-C3 alkenyl, oxo, $-P(O)R^{1B}R^{1C}$ and $-S(O)_2R^{1D}$; $R^{1B}$, $R^{1C}$ and $R^{1D}$ are each independently selected from the group consisting of hydrogen and C1-C3 alkyl;

(2) 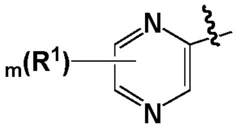 or 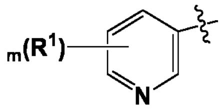 ;

preferably, each $R^1$ is independently selected from any one of the following groups: hydrogen, halogen, C1-C3 alkyl, C1-C3 haloalkyl, C1-C3 haloalkoxy and phenyl, and when $R^1$ is phenyl, the phenyl and

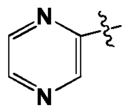 or 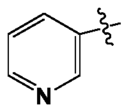

share two carbon atoms to form a fused ring, and the phenyl is optionally substituted with halogen, C1-C3 alkyl, C1-C3 haloalkyl and C1-C3 haloalkoxy; further preferably, the phenyl is optionally substituted with halogen; the number m of $R^1$ is 1 or 2;

(3) 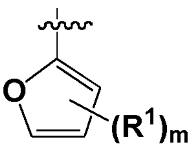 ;

preferably, the $R^1$ is hydrogen, halogen or phenyl, wherein the phenyl is optionally substituted with 1 or more halogens, and the number m of $R^1$ is 1 or 2; when $R^1$ is phenyl, the phenyl and

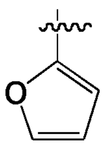

optionally share two carbon atoms to form a fused ring;

further preferably, the $R^1$ is phenyl, m is 1, the fusion mode of the phenyl and

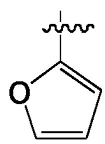 is

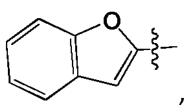 ,

and the phenyl is optionally substituted with 1 or more halogens.

In some embodiments, ring A is phenyl-O-C1-C3 alkyl, wherein the phenyl is optionally substituted with 1 or 2 $R^1$; each of the $R^1$ is independently selected from the group consisting of hydrogen, halogen, C1-C3 alkyl, halogen-substituted C1-C3 alkyl and halogen-substituted C1-C3 alkoxy.

Preferably, the ring A is phenyl-O-methyl, and $R^1$ is H or halogen.

In some embodiments, ring A is 4- to 8-membered heterocyclyl-O-C1-C3 alkyl, wherein the heterocyclyl is optionally substituted with 1-3 $R^1$; each of the $R^1$ is independently selected from the group consisting of hydrogen, halogen, C1-C3 alkyl, halogen-substituted C1-C3 alkyl and halogen-substituted C1-C3 alkoxy.

Preferably, the ring A is 4- to 7-membered heterocyclyl-O-methyl, and $R^1$ is halogen-substituted C1-C3 alkyl or halogen-substituted C1-C3 alkoxy.

In some embodiments, the ring A is C3-C7 cycloalkyl-O-C1-C3 alkyl, wherein the C3-C7 cycloalkyl is optionally substituted with 1-3 $R^1$; each of the $R^1$ is independently selected from the group consisting of hydrogen, halogen, C1-C3 alkyl, halogen-substituted C1-C3 alkyl and halogen-substituted C1-C3 alkoxy.

Preferably, the ring A is C4-C6 cycloalkyl-O-methyl, and $R^1$ is halogen-substituted C1-C3 alkyl or halogen-substituted C1-C3 alkoxy.

In some embodiments, the ring A is phenyl, wherein the phenyl is substituted with a group selected from halogen, C1-C6 alkyl, C1-C6 alkoxy, C1-C6 haloalkyl and C1-C6 haloalkoxy.

Preferably, the phenyl is substituted with a group selected from halogen, C1-C3 alkyl, C1-C3 alkoxy, C1-C3 haloalkyl and C1-C3 haloalkoxy; further preferably, the phenyl is substituted with C1-C3 haloalkyl.

In some embodiments, ring A is selected from any one of the following groups:

(1) 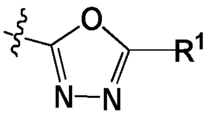 ;

preferably, the $R^1$ is selected from the group consisting of $-G^1$, $O-G^1$ and $-NR^{1B}-G^1$, wherein each of the $G^1$ is optionally substituted with 1 or 2 $R^{1F}$; the $R^{1B}$ is selected from the group consisting of hydrogen and C1-C3 alkyl; each of the $G^1$ is independently selected from C3-C6 cycloalkyl and 4-to 9-membered heterocyclyl;

further preferably, the C3-C6 cycloalkyl is cyclobutyl or cyclopentyl; the 4- to 9-membered heterocyclyl is preferably oxocycloalkyl or azacycloalkyl, and further preferably oxetanyl, azetidinyl, oxocyclopentyl, azacyclopentyl,

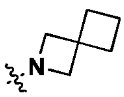 or 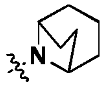 ,

further preferably oxetanyl, azetidinyl, oxocyclopentyl or azacyclopentyl, or further preferably

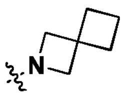 or 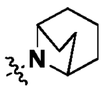 ;

each of the $R^{1F}$ is independently selected from the group consisting of oxo, C1-C3 alkyl, C1-C3 alkoxy, halogen-substituted C1-C3 alkyl and halogen-substituted C1-C3 alkoxy; moreover, the $L^1$ is preferably null;

(2) 4- to 8-membered heterocyclyl-O-C1-C3 alkyl, wherein the heterocyclyl is optionally substituted with 1 or 2 $R^1$; each of the $R^1$ is independently selected from the group consisting of halogen-substituted C1-C3 alkyl and halogen-substituted C1-C3 alkoxy;

(3) C4-C6 cycloalkyl-O-C1-C3 alkyl, wherein the C4-C6 cycloalkyl is optionally substituted with 1 or 2 $R^1$; each of the $R^1$ is independently selected from halogen-substituted C1-C3 alkyl and halogen-substituted C1-C3 alkoxy.

In some embodiments, ring A is selected from any one of the following groups:

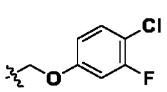 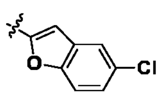 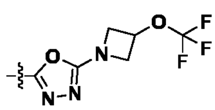 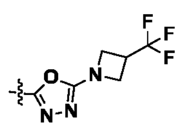

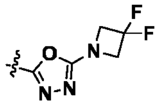 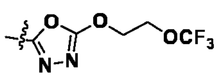 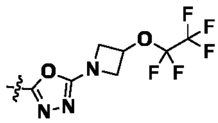 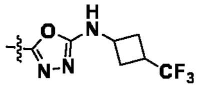

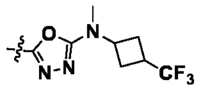 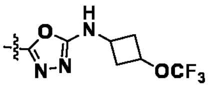 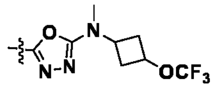 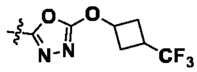

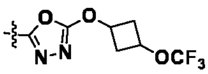 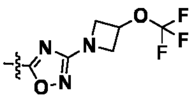 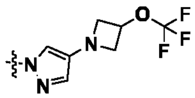 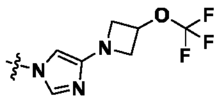

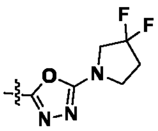 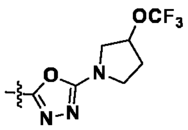 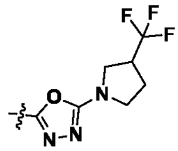 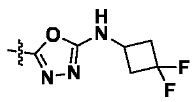

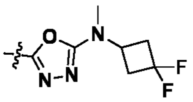 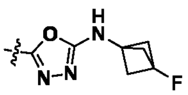 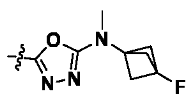 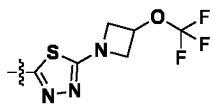

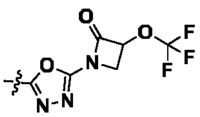 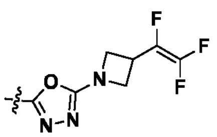 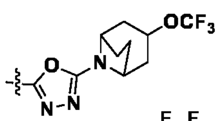 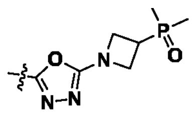

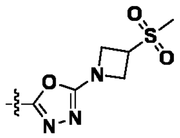 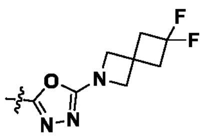 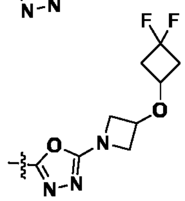 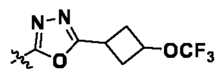

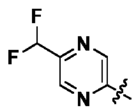 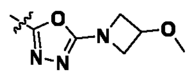 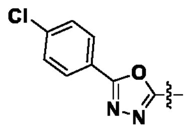 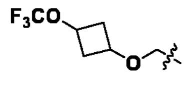

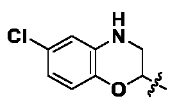 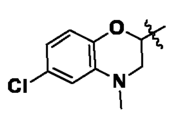 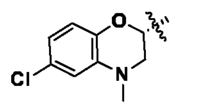 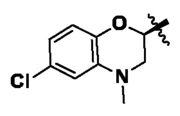

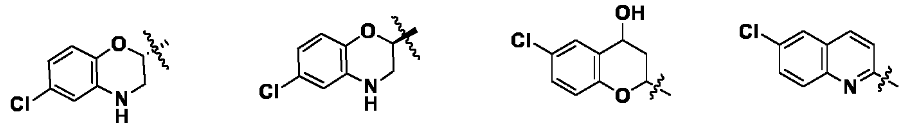

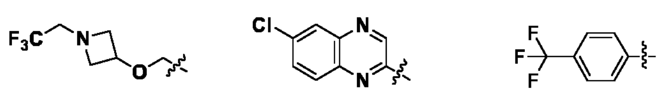

Preferably, ring A is selected from any one of the following groups:

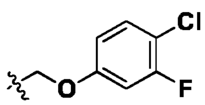, 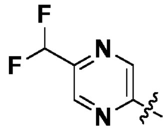, 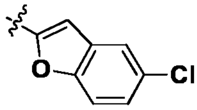, 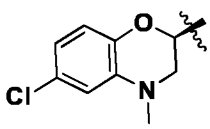, 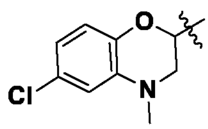,

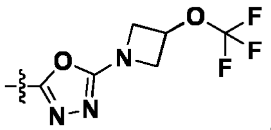, 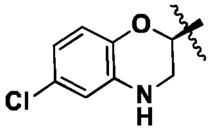, 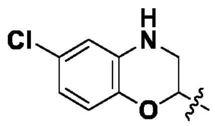, 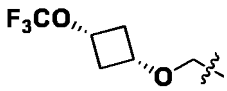,

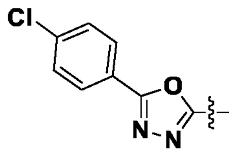.

Preferably, ring A is selected from any one of the following groups:

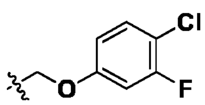, 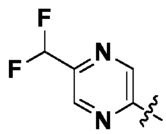, 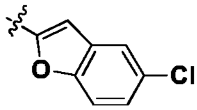, 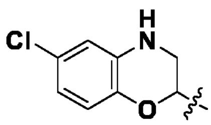, 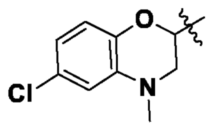,

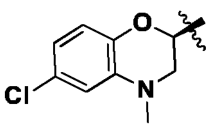, 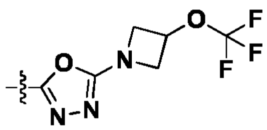, 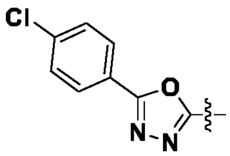, 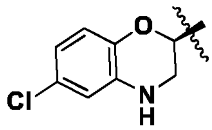.

Preferably, ring A is selected from any one of the following groups:

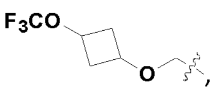, 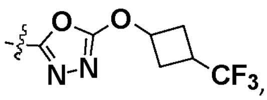, 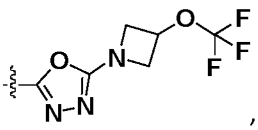, 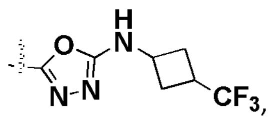,

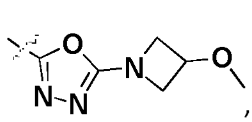, 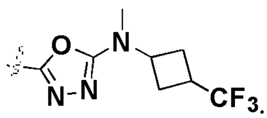.

In some embodiments, the spiro ring is selected from any one of the following groups: a spiro[2,5]octyl ring, a spiro[3,4]octyl ring, a spiro[3,3]heptyl ring, a spiro[3,5]nonyl ring, a spiro[4,5]decyl ring, a spiro[2,2]pentyl ring, a spiro[2,3]hexyl ring, a spiro[2,4]heptyl ring, a spiro[2,6]nonyl ring, a spiro[2,7]nonyl ring, a spiro[3,6]nonyl ring and a spiro[4,4]nonyl ring; and the spiro heterocyclic ring is a group formed by substituting any one or more carbon atoms in the spiro ring with a heteroatom(s), wherein the number of heteroatoms in the spiro heterocyclic ring is 1, 2 or 3.

Preferably, the heteroatom in the spiro heterocyclic ring is nitrogen, or a combination of nitrogen and oxygen.

Preferably, the substituent $R^{1F}$ of the spiro ring or the spiro heterocyclic ring is hydrogen, halogen, hydroxyl, amino, cyano, C1-C6 alkyl, C3-C9 cycloalkyl, hydroxyl-substituted C1-C6 alkyl, halogen-substituted C1-C6 alkyl or halogen-substituted C1-C6 alkoxy.

In some embodiments, the number of the substituent $R^{1F}$ of the spiro ring or the spiro heterocyclic ring is preferably 1 or 2, and preferably 1.

More preferably, the substituent $R^{1F}$ of the spiro ring or the spiro heterocyclic ring is halogen, hydroxyl or amino.

Most preferably, the substituent $R^{1F}$ of the spiro ring or the spiro heterocyclic ring is hydroxyl.

In some embodiments, ring B is a 7- to 9-membered spiro ring or a 7- to 9-membered spiro heterocyclic ring.

Preferably, the spiro ring is selected from any one of the following groups: a spiro[2,5]octyl ring, a spiro[3,4]octyl ring, a spiro[3,3]heptyl ring and a spiro[3,5]nonyl ring; the 7- to 9-membered spiro heterocyclic ring is a group formed by substituting any one or more carbon atoms in the 7- to 9-membered spiro ring with a heteroatom(s), wherein the number of heteroatoms in the spiro heterocyclic ring is 1, 2 or 3.

Preferably, the heteroatom in the spiro heterocyclic ring is nitrogen, or a combination of nitrogen and oxygen.

In some embodiments, ring B is selected from any one of the following groups:

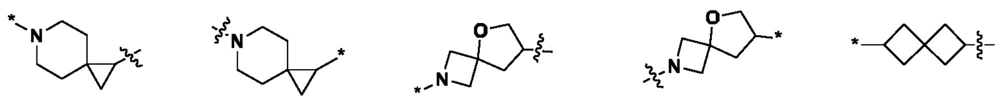

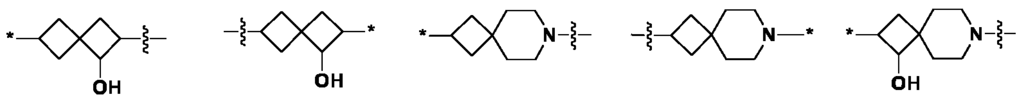

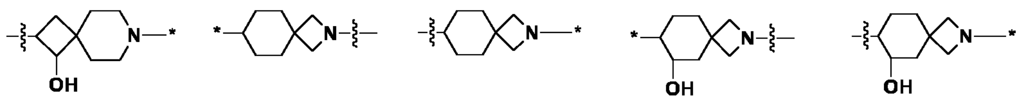

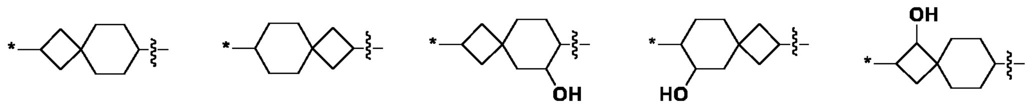

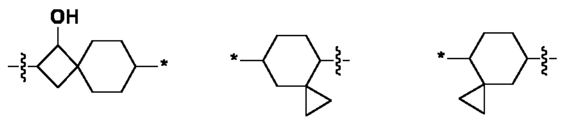

,

wherein the ⌇ end is connected to $L^1$, and the * end is connected to $L^2$.

In some embodiments, ring B is selected from any one of the following groups:

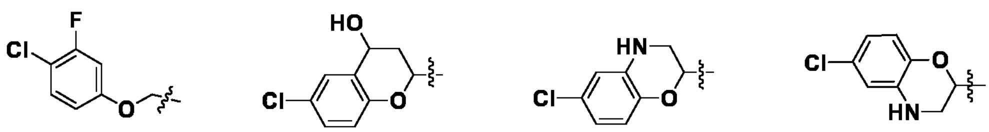

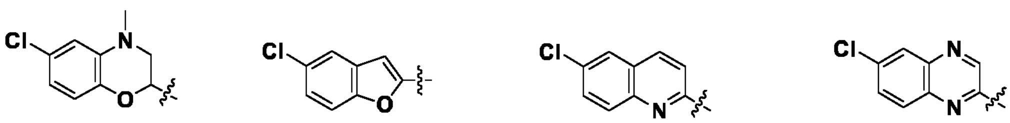

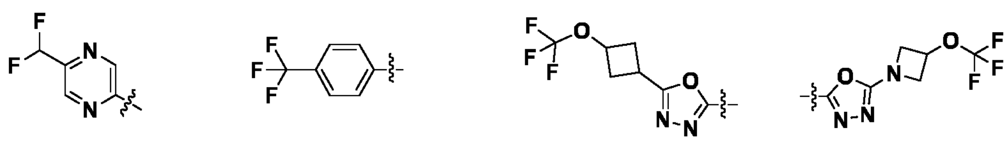

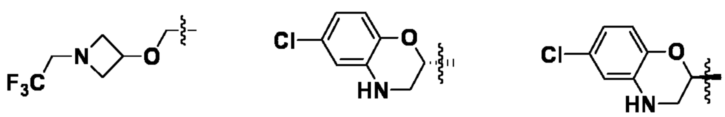

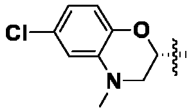 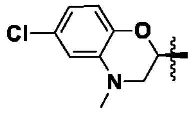,

wherein the end is connected to $L^1$, and the * end is connected to $L^2$.

In some embodiments, the ring B is selected from any one of the following groups:

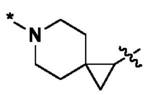 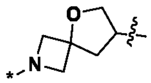 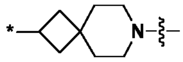 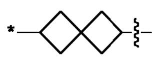 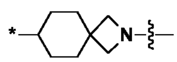

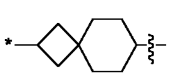 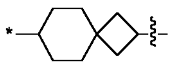 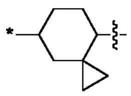 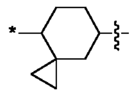,

wherein the end is connected to $L^1$, and the * end is connected to $L^2$.

In some embodiments, the ring B is selected from any one of the following groups:

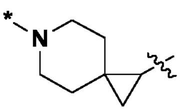, 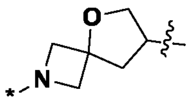, 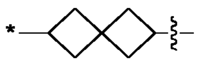, 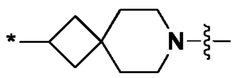, 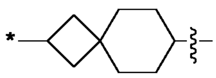,

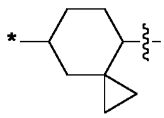,

wherein the end is connected to $L^1$, and the * end is connected to $L^2$.

In some embodiments, the ring C is 6- to 10-membered aryl-E-C1-C3 alkyl, C4-C9 cycloalkyl-E-C1-C3 alkyl, 4- to 9-membered heterocyclyl-E-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F}$; when $R^2$ is phenyl, the phenyl and 4- to 9-membered heterocyclyl or 5- to 10-membered heteroaryl optionally share two carbon atoms to form a fused ring;

each of the E is independently a linking group containing a heteroatom.

Preferably, each of the E is independently O or $NR^d$ or S, wherein each $R^d$ is independently selected from hydrogen, deuterium and C1-C3 alkyl; more preferably, each of the E is independently O or NH.

In some embodiments, the ring C is phenyl-E-C1-C3 alkyl, wherein the phenyl is substituted with 1, 2 or 3 $R^2$, and the E is a linking group containing a heteroatom(s).

Preferably, the E is O or $NR^d$ or S, and $R^d$ is selected from hydrogen, deuterium and C1-C3 alkyl.

Preferably, the E is O or NH.

Preferably, each of the $R^2$ is independently halogen.

In some embodiments, ring C is C4-C9 cycloalkyl-E-C1-C3 alkyl or 4- to 9-membered heterocyclyl-E-C1-C3 alkyl, wherein the cycloalkyl or heterocyclyl is substituted with $R^2$; the E is O or $NR^d$ or S, and $R^d$ is selected from hydrogen, deuterium and C1-C3 alkyl; the $R^2$ is hydrogen, halogen, C1-C3 alkyl, C1-C3 haloalkyl, C1-C3 alkoxy or C1-C3 haloalkoxy;

preferably, the E is O or NH;
preferably, the cycloalkyl is cyclopropyl, cyclobutyl or cyclopentyl;
preferably, the heterocyclyl is oxiranyl, oxetanyl, oxocyclopentyl, aziridinyl, azetidinyl or azacyclopentyl.

In some embodiments, ring C is 4- to 9-membered heterocyclyl, preferably 5- to 6-membered heterocyclyl.

Preferably, the ring C is

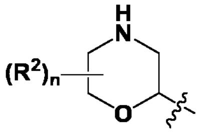 or 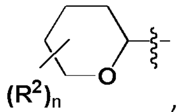 ,

wherein the number n of the $R^2$ is 1 or 2 or 3; preferably, each of the $R^2$ is independently selected from the group consisting of hydrogen, halogen, C1-C3 alkyl, phenyl, halophenyl and hydroxyl; when the $R^2$ is phenyl or halophenyl, the phenyl or halophenyl and

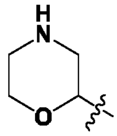 or 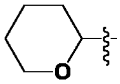

optionally share two carbon atoms to form a fused ring, wherein the fusion mode is preferably

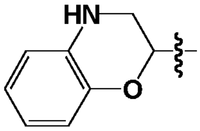 or 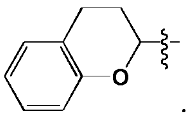 .

In some embodiments, the ring C is

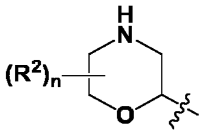 ,

wherein the number n of the $R^2$ is 1 or 2 or 3.

Preferably, each of the $R^2$ is independently selected from the group consisting of hydrogen, halogen, C1-C3 alkyl, phenyl and halophenyl; when the $R^2$ is a substituent on an N atom, the $R^2$ is selected from the group consisting of hydrogen, halogen and C1-C3 alkyl; when the $R^2$ is phenyl or halophenyl, the phenyl or the halophenyl and morpholinyl optionally share two carbon atoms to form a fused ring, wherein the fusion mode is preferably

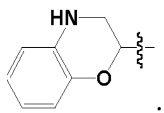 .

In some embodiments, the ring C is

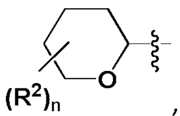 ,

wherein the number n of the $R^2$ is 1 or 2 or 3.

Preferably, each of the $R^2$ is independently selected from the group consisting of halogen, phenyl, halophenyl and hydroxyl; when the $R^2$ is phenyl or halophenyl, the phenyl or the halophenyl and

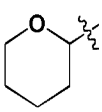

optionally share two carbon atoms to form a fused ring, wherein the fusion mode is preferably

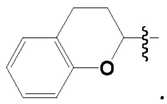

.

In some embodiments, ring C is 5- to 10-membered heteroaryl.

Preferably, the ring C is any one of the following 5- to 10-membered heteroaryl groups:

(1) 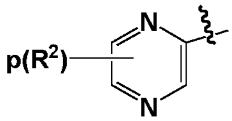 or 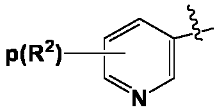 ;

preferably, each $R^2$ is independently selected from any one of the following groups: hydrogen, halogen, C1-C3 alkyl, C1-C3 haloalkyl, phenyl and halophenyl, wherein the number p of the $R^2$ is 1 or 2; and when the $R^2$ is phenyl or halophenyl, the phenyl or halophenyl and

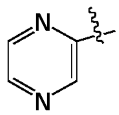

or

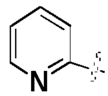

share two carbon atoms to form a fused ring, wherein the fusion mode is preferably

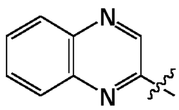 or 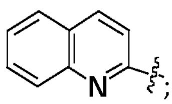 ;

(2) 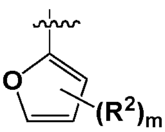 ;

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

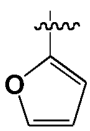

optionally share two carbon atoms to form a fused ring, wherein the fusion mode is preferably

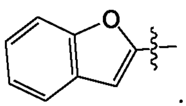

.

In some embodiments, ring C is phenyl; the phenyl is substituted with $R^2$; preferably, the $R^2$ is selected from the

group consisting of C1-C3 haloalkyl and C1-C3 haloalkoxy.

In some embodiments, ring C is selected from any one of the following groups:

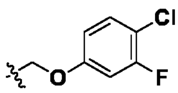
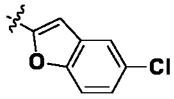
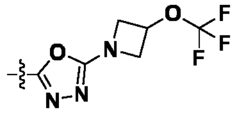
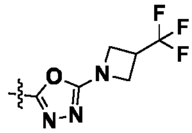
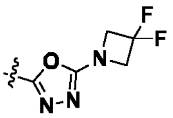
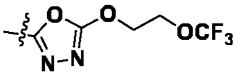
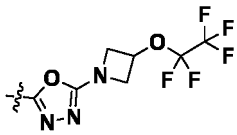
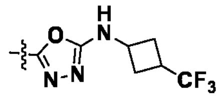
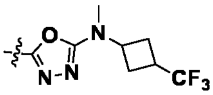
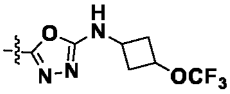
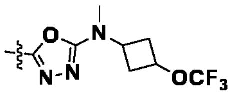
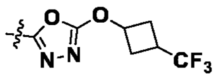
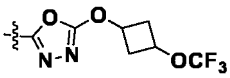
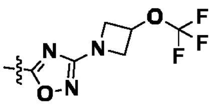
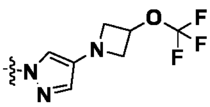
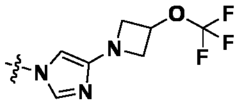
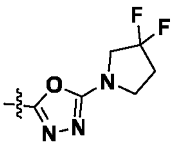
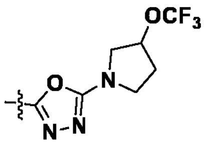
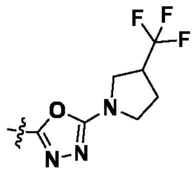
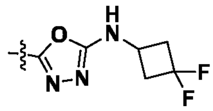
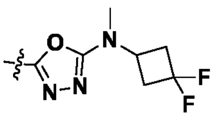
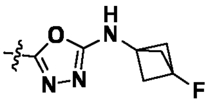
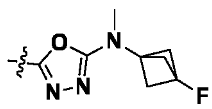
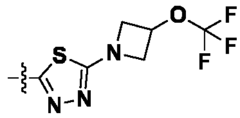
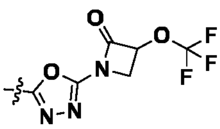
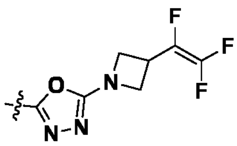
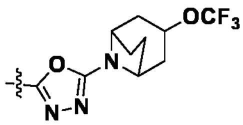
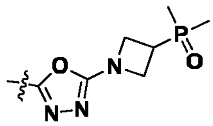
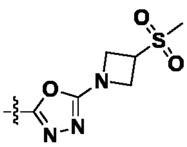
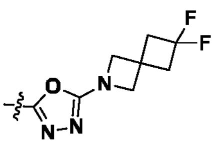
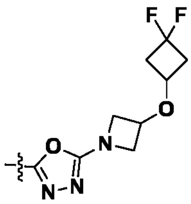
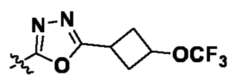
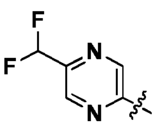
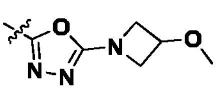
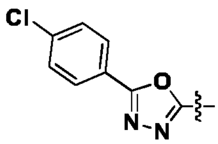
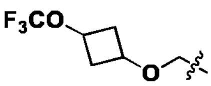

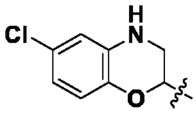 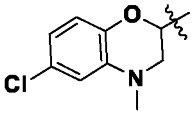 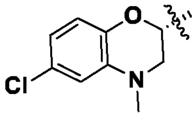 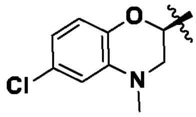

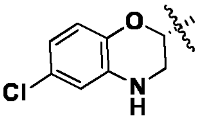 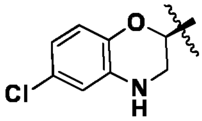 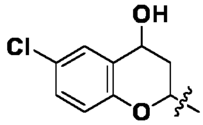 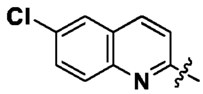

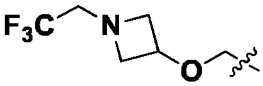 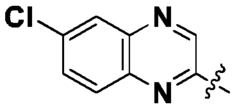 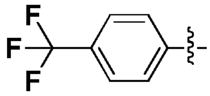 ;

preferably, the ring C is selected from any one of the following groups:

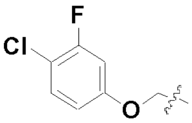 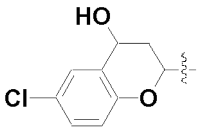 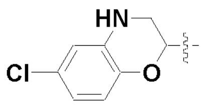 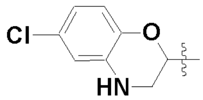

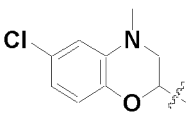 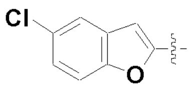 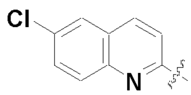 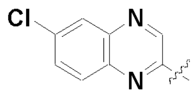

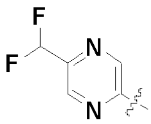 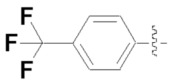 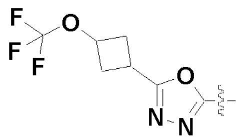 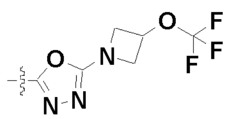

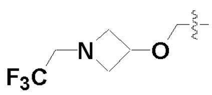 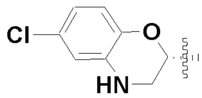 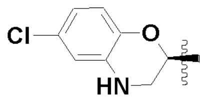

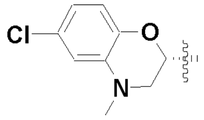 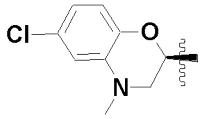 ;

preferably, the ring C is selected from any one of the following groups:

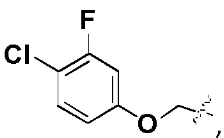, 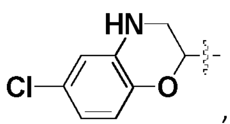, 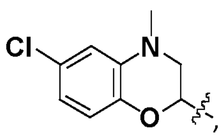, 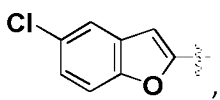, 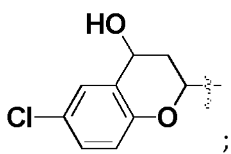 ;

preferably, the ring C is selected from any one of the following groups:

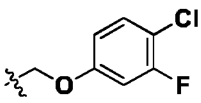, 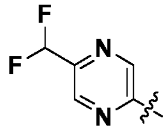, 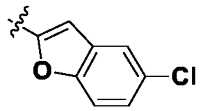, 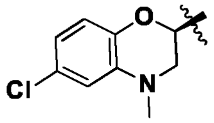, 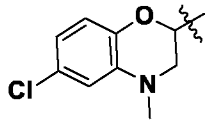,

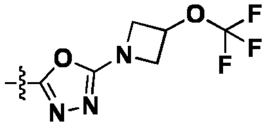, 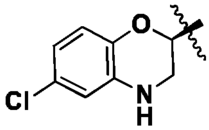, 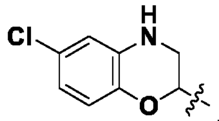, 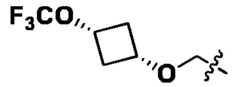,

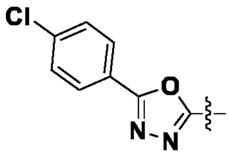;

preferably, the ring C is selected from any one of the following groups:

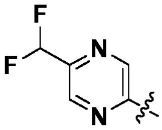, 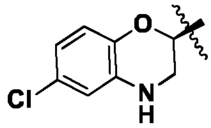, 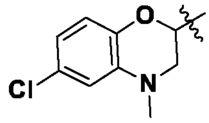, 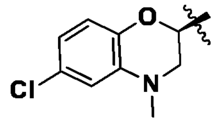, 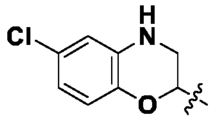,

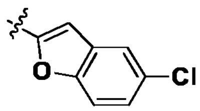, 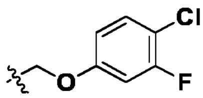, 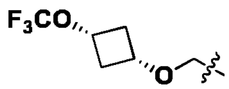, 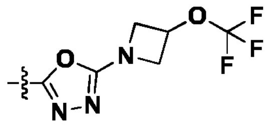.

In some embodiments, the compound has a structure as shown in formula I:

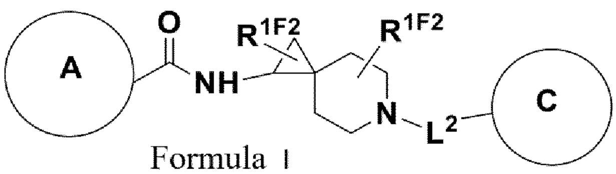

Formula I

.

Preferably,

ring A is 3- to 9-membered heterocyclyl, 5- to 10-membered heteroaryl, 6- to 10-membered aryl-D-C1-C6 alkyl or 5- to 10-membered heteroaryl-D-C1-C6 alkyl, wherein the heterocyclyl, aryl and heteroaryl can each be optionally substituted with 1-5 $R^1$;

each of the $R^1$ is independently selected from the group consisting of halogen, C1-C10 alkyl, hydroxyl-substituted C1-C6 alkyl, halogen-substituted C1-C6 alkyl, halogen-substituted C1-C6 alkoxy, $-G^1$ and $-O-G^1$; or 2 $R^1$ groups on adjacent atoms taken together with the atoms to which they are attached can form C3-C7 cycloalkyl, 3- to 7-membered heterocyclyl, 6- to 10-membered aryl or 5- to 6-membered heteroaryl; the C3-C7 cycloalkyl, 3- to 7-membered heterocyclyl, 6- to 10-membered aryl or 5- to 6-membered heteroaryl is each optionally substituted with 1-5 $R^{1F}$;

each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F1}$;

each of the $R^{1F1}$ and $R^{1F2}$ is independently selected from the group consisting of hydrogen, halogen, hydroxyl, amino, cyano, carboxyl, C1-C6 alkyl, C3-C9 cycloalkyl, hydroxyl-substituted C1-C6 alkyl, halogen-substituted C1-C6 alkyl and halogen-substituted C1-C6 alkoxy; preferably, each of the $R^{1F2}$ is independently selected from hydrogen, halogen, hydroxyl and amino;

$L^2$ is null, -NH-

-, 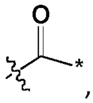, 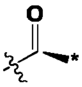, 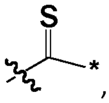, 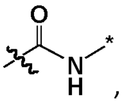, 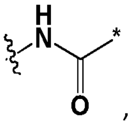, 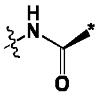, 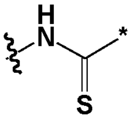

or

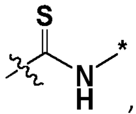,

wherein the end is connected to ring B, and the * end is connected to ring C;

preferably, the end is connected to the N atom in

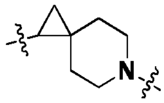

in formula I;

preferably, the $L^2$ is null, -NH-,

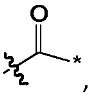, 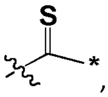, 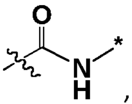, 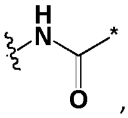, 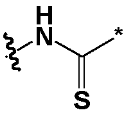 or 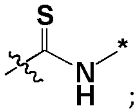;

preferably, the $L^2$ is null,

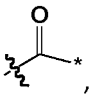, 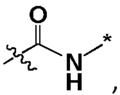, 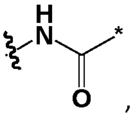, 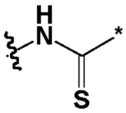 or 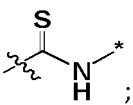;

preferably, the $L^2$ is null,

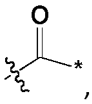, 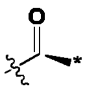, 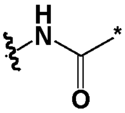 or 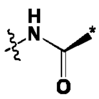;

preferably, the $L^2$ is

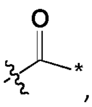, 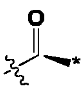, 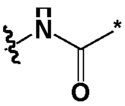 or 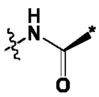;

preferably, the $L^2$ is null,

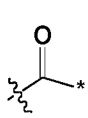 or 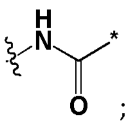;

ring C is 6- to 10-membered aryl-E-C1-C6 alkyl, 3- to 9-membered heterocyclyl, 5- to 10-membered heteroaryl or 6- to

10-membered aryl; the aryl, heteroaryl and heterocyclyl can each be optionally substituted with 1-3 $R^2$; each of the $R^2$ is independently selected from the group consisting of halogen, hydroxyl, phenyl, C1-C6 alkyl, C1-C6 alkoxy and C1-C3 alkoxy-C1-C6 alkyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl and C1-C3 alkoxy in the C1-C3 alkoxy-C3-C6 cycloalkyl are each optionally substituted with halogen; when $R^2$ is phenyl, the phenyl and 3- to 9-membered heterocyclyl or 6- to 10-membered heteroaryl optionally share two carbon atoms to form a fused ring;
each of the D or E is independently a linking group containing a heteroatom;
preferably, each of the D or E is independently O or $NR^d$ or S, wherein each $R^d$ is independently selected from hydrogen, deuterium, C1-C6 alkyl and halogen-substituted C1-C6 alkyl;
more preferably, each of the D or E is independently O or NH.

Preferably, ring A is 5- to 10-membered heteroaryl or 6- to 10-membered aryl-D-C1-C6 alkyl.
Preferably, each of the $R^1$ is independently selected from C1-C10 alkyl, hydroxyl-substituted C1-C6 alkyl, halogen-substituted C1-C6 alkyl, halogen-substituted C1-C6 alkoxy, $-G^1$ and $-O-G^1$.
Preferably, ring C is 6- to 10-membered aryl. The aryl can be optionally substituted with 1-3 $R^2$; each of the $R^2$ is independently selected from: halogen and C1-C6 alkyl.
In some embodiments, the compound has a structure as shown in formula I-1:

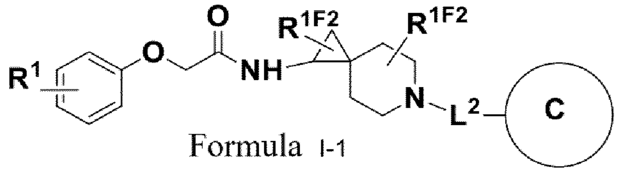
Formula I-1
,

wherein the number of the $R^1$ is 1-5, and each of the $R^1$ is independently selected from the group consisting of halogen, C1-C10 alkyl, hydroxyl-substituted C1-C6 alkyl, halogen-substituted C1-C6 alkyl and halogen-substituted C1-C6 alkoxy;
preferably, the number of the $R^1$ is 1, 2 or 3, and each of the $R^1$ is independently selected from the group consisting of halogen, C1-C10 alkyl, halogen-substituted C1-C6 alkyl and halogen-substituted C1-C6 alkoxy;
further preferably, the number of the $R^1$ is 1 or 2, and each of the $R^1$ is independently selected from any one of halogens;
each of the $R^{1F2}$ is independently selected from the group consisting of hydrogen, halogen, hydroxyl, amino, cyano, carboxyl, C1-C6 alkyl, C3-C9 cycloalkyl, hydroxyl-substituted C1-C6 alkyl, halogen-substituted C1-C6 alkyl and halogen-substituted C1-C6 alkoxy;
preferably, each of the $R^{1F2}$ is independently selected from the group consisting of hydrogen, halogen and hydroxyl;
the $L^2$ is

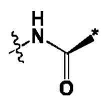 or 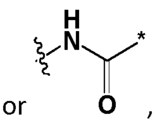 ,

wherein the end is connected to ring B, and the * end is connected to ring C; preferably, the end is connected to the N atom in

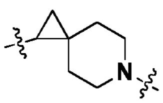 in

formula I-1;
the ring C is 6- to 10-membered aryl-O-C1-C6 alkyl, 3- to 9-membered heterocyclyl or 5- to 10-membered heteroaryl; the aryl, heteroaryl and heterocyclyl can each be optionally substituted with 1-3 $R^2$; each of the $R^2$ is independently selected from the group consisting of halogen, hydroxyl, phenyl, C1-C6 alkyl, C1-C6 alkoxy and C1-C3 alkoxy-C1-C6 alkyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl and C1-C3 alkoxy in the C1-C3 alkoxy-C3-C6 cycloalkyl are each optionally substituted with halogen; when $R^2$ is phenyl, the phenyl and 3- to 9-membered heterocyclyl or 6- to 10-membered

heteroaryl optionally share two carbon atoms to form a fused ring.

In some embodiments, preferably, the ring C is any one of the following groups: 1) 4- to 9-membered heterocyclyl; preferably, the ring C is

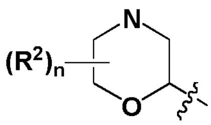 or 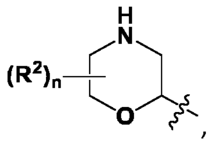 ,

wherein the number n of the $R^2$ is 1 or 2 or 3;
preferably, each of the $R^2$ is independently selected from the group consisting of hydrogen, halogen, C1-C3 alkyl, phenyl, halophenyl and hydroxyl; when $R^2$ is phenyl or halophenyl, the phenyl or halophenyl and 4- to 9-membered heterocyclyl optionally share two carbon atoms to form a fused ring, and the phenyl or halophenyl and

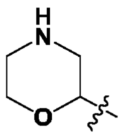

optionally share two carbon atoms to form a fused ring, wherein the fusion mode is preferably

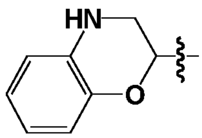
;

2) 5- to 10-membered heteroaryl;
preferably, the ring C is any one of the following 5- to 10-membered heteroaryl groups:

(1) 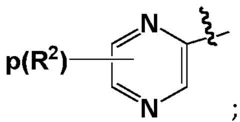 ;

preferably, each $R^2$ is independently selected from any one of the following groups: hydrogen, halogen, C1-C3 alkyl, C1-C3 haloalkyl, phenyl and halophenyl, wherein the number p of the $R^2$ is 1 or 2; and when the $R^2$ is phenyl or halophenyl, the phenyl or halophenyl and

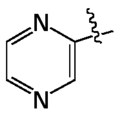

share two carbon atoms to form a fused ring, wherein the fusion mode is preferably

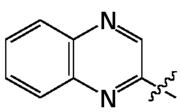;

(2) 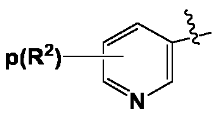 ;

preferably, each $R^2$ is independently selected from any one of the following groups: hydrogen, halogen, C1-C3 alkyl, C1-C3 haloalkyl, phenyl and halophenyl, wherein the number p of the $R^2$ is 1 or 2; and when the $R^2$ is phenyl or halophenyl, the phenyl or halophenyl and

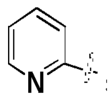

share two carbon atoms to form a fused ring, wherein the fusion mode is preferably

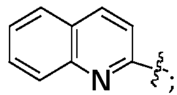
;

(3) 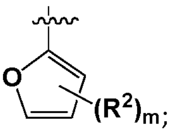

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

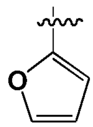

optionally share two carbon atoms to form a fused ring, wherein the fusion mode is preferably

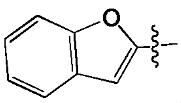
;

3) phenyl-O-C1-C3 alkyl, wherein the phenyl is substituted with 1, 2 or 3 $R^2$; preferably, each of the $R^2$ is independently halogen;

4) phenyl, wherein the phenyl is substituted with $R^2$;

preferably, the $R^2$ is selected from the group consisting of C1-C3 haloalkyl and C1-C3 haloalkoxy.

In some embodiments, the compound has a structure as shown in formula I-2:

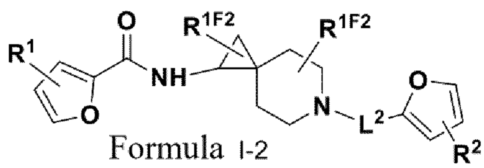
Formula I-2

,

wherein each of the $R^{1F2}$ is independently selected from the group consisting of hydrogen, halogen, hydroxyl, amino, cyano, carboxyl, C1-C6 alkyl, C3-C9 cycloalkyl, hydroxyl-substituted C1-C6 alkyl, halogen-substituted C1-C6 alkyl and halogen-substituted C1-C6 alkoxy;

preferably, each of the $R^{1F2}$ is independently selected from the group consisting of hydrogen, halogen and hydroxyl; the $L^2$ is

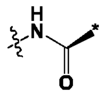 or 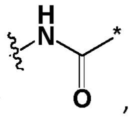 ,

wherein the end is connected to ring B, and the * end is connected to ring C; preferably, the end is connected to the N atom in

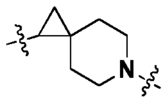 in

formula I-2;

the number of the $R^1$ and the $R^2$ is independently 1-3, and each of the $R^1$ and each of the $R^2$ are independently selected from the group consisting of halogen, C1-C10 alkyl, hydroxyl-substituted C1-C6 alkyl, halogen-substituted C1-C6 alkyl, halogen-substituted C1-C6 alkoxy, $-G^1$ and $-O-G^1$;

each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F1}$; each of the $R^{1F1}$ is independently selected from the group consisting of hydrogen, halogen, hydroxyl, amino, cyano and carboxyl.

Preferably, the number of the $R^1$ and the $R^2$ is 1, and the $R^1$ and the $R^2$ are selected from the group consisting of 6- to 10-membered aryl and 5- to 10-membered heteroaryl; the 6- to 10-membered aryl and the 5- to 10-membered heteroaryl are each optionally substituted with 1, 2 or 3 $R^{1F1}$ wherein each of the $R^{1F1}$ is independently selected from the group consisting of hydrogen and halogen.

Further preferably, the number of the $R^1$ and the $R^2$ is 1, and the $R^1$ and the $R^2$ are phenyl; each of the phenyl is optionally substituted with 1, 2 or 3 $R^{1F1}$ and each of the $R^{1F1}$ is independently selected from the group consisting of hydrogen and halogen.

In some embodiments, the compound has a structure as shown in formula II or formula III:

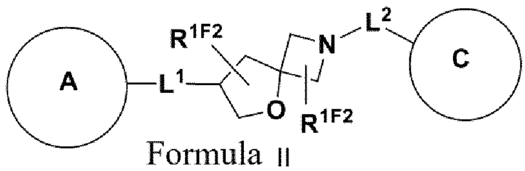

Formula II

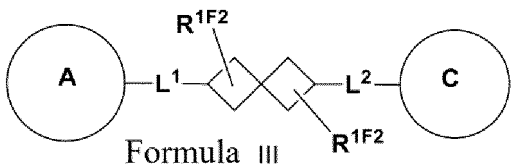

Formula III

,

wherein each of the ring A is independently 5- to 7-membered heteroaryl, phenyl-D-C1-C6 alkyl or 3- to 9-membered heterocyclyl, wherein the heteroaryl and phenyl can each be optionally substituted with 1 or 2 $R^1$;

each of the $R^1$ is independently selected from the group consisting of halogen, halogen-substituted C1-C6 alkyl, halogen-substituted C1-C6 alkoxy, $-OR^{1A}$, $-NR^{1B}R^{1C}$, $-G^1$, $O-G^1$ and $-NR^{1B}-G^1$;

each of the $R^{1A}$, $R^{1B}$, $R^{1C}$ and $R^{1D}$ is independently selected from the group consisting of hydrogen, C1-C6 alkyl, C4-C9 cycloalkyl, 4- to 9-membered heterocyclyl, halogen-substituted C1-C6 alkyl, halogen-substituted C4-C9 cycloalkyl, halogen-substituted 4- to 9-membered heterocyclyl and halogen-substituted C1-C6 alkoxy;

each of the $G^1$ is independently selected from C3-C9 cycloalkyl, 3- to 9-membered heterocyclyl and phenyl; each of the $G^1$ is optionally substituted with 1-3 $R^{1F1}$; when $G^1$ is phenyl, the phenyl and 3- to 9-membered heterocyclyl or 5- to 7-membered heteroaryl optionally share two carbon atoms to form a fused ring;

each of the $R^{1F1}$ is independently selected from the group consisting of halogen, C1-C6 alkyl, C3-C9 cycloalkyl, halogen-substituted C1-C6 alkyl, halogen-substituted C3-C9 cycloalkyl, halogen-substituted C1-C3 alkoxy, halogen-substituted C1-C6 alkenyl, oxo, $-OR^{1A}$, $-NR^{1B}R^{1C}$, $-P(O)R^{1B}R^{1C}$ and $-S(O)_2R^{1D}$;

$L^1$ is null, -NH-,

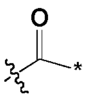, 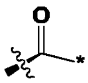, 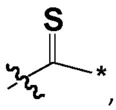, 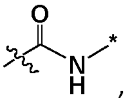, 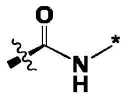, 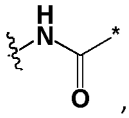, 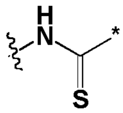 or

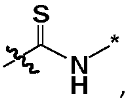,

wherein the  end is connected to ring A, and the * end is connected to ring B; preferably, the * end is connected to the &C atom in

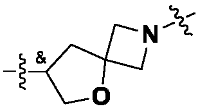

in formula II or the &C atom in

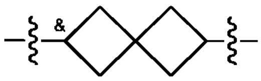

in formula III;
preferably, the $L^1$ is null, -NH-,

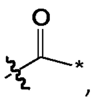, 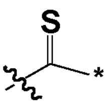, 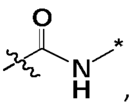, 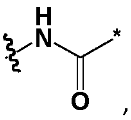, 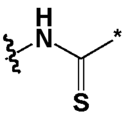 or 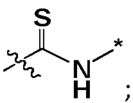;

preferably, the $L^1$ is null,

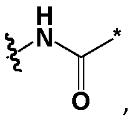, 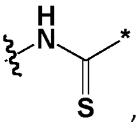, 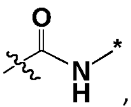, 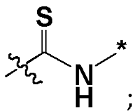;

preferably, the $L^1$ is null,

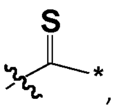, 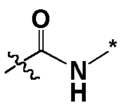 or 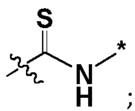;

further preferably, the $L^1$ is null or

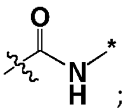;

$L^2$ is null, -NH-,

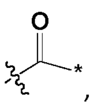, 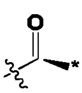, 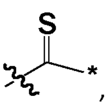, 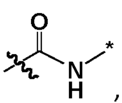, 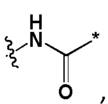, 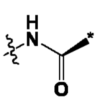, 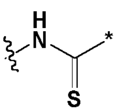 or 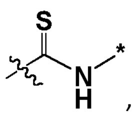,

wherein the end is connected to ring B, and the * end is connected to ring C; preferably, the end is connected to the N atom in

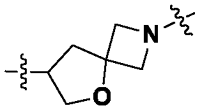

in formula II or the @C atom in

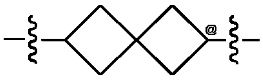

in formula III;
preferably, the $L^2$ is null, -NH-,

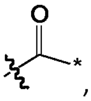, 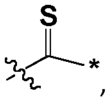, 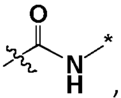, 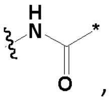, 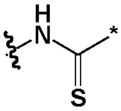 or 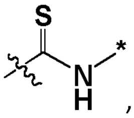,

preferably, the $L^2$ is null,

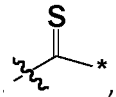, 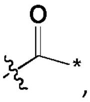, 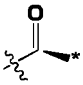, 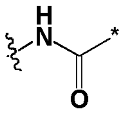 or 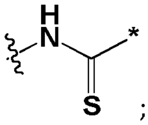;

preferably, in the formula II, the $L^2$ is

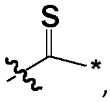, 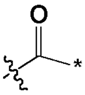 or 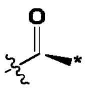,

more preferably

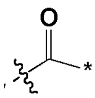

or

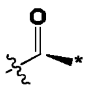;

preferably, in the formula III, the $L^2$ is null,

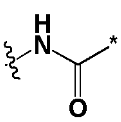 or 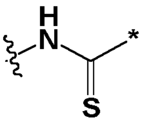

, more preferably is null or

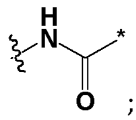 ;

each of the $R^{1F2}$ is independently selected from hydrogen, halogen, hydroxyl and amino;
preferably, each of the $R^{1F2}$ is independently selected from hydrogen and hydroxyl;
the ring C is selected from 6- to 10-membered aryl-E-C1-C3 alkyl, C4-C9 cycloalkyl-E-C1-C3 alkyl, 4- to 9-membered heterocyclyl-E-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F}$;
when $R^2$ is phenyl, the phenyl and 4- to 9-membered heterocyclyl or 5- to 10-membered heteroaryl optionally share two carbon atoms to form a fused ring;
each of the E is independently a linking group containing a heteroatom;
preferably, each of the E is independently O or $NR^d$ or S, wherein each $R^d$ is independently selected from hydrogen, deuterium and C1-C3 alkyl;
more preferably, each of the E is independently O or NH.

In some embodiments, the compound has a structure as shown in formula II-1, II-1', III-1 or III-1':

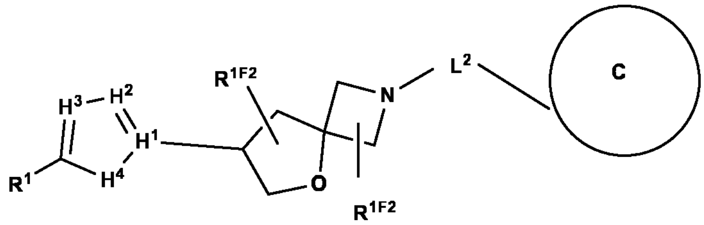

formula II-1

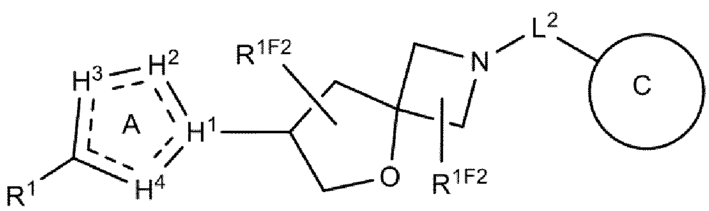

Formula II-1'

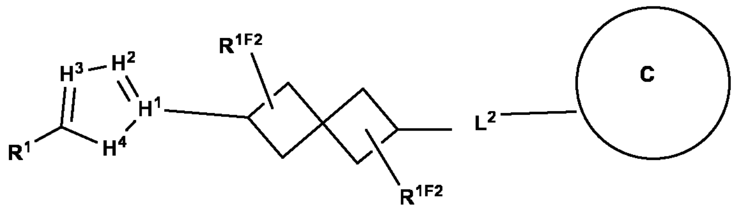

formula III-1

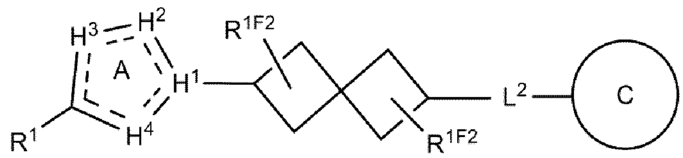

Formula III-1'

,

wherein ring A is 5-membered heteroaryl;

$H^1$, $H^2$, $H^3$ and $H^4$ are each independently selected from CH, N, O and S;

------ is a single bond or a double bond;

each of the $R^{1F2}$ is independently hydrogen, halogen, hydroxyl or amino, and preferably hydrogen or hydroxyl;

preferably, the

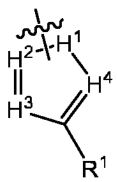 or 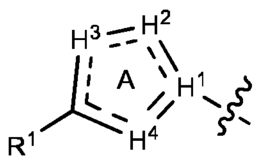 is 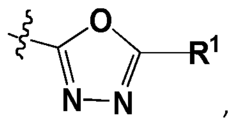, 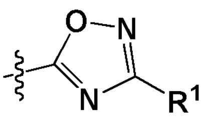,

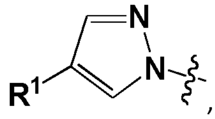, 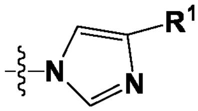 or 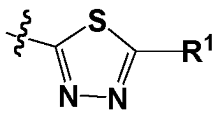;

preferably, the $R^1$ is selected from the group consisting of $-OR^{1A}$, $-NR^{1B}R^{1C}$, $-G^1$, $O-G^1$ and $-NR^{1B}-G^1$, wherein each of the $R^{1A}$, $R^{1B}$ and $R^1$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl; each of the $G^1$ is optionally substituted with 1-3 $R^{1F1}$;

each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)\,R^{1B}R^{1C}$ and $-S(O)_2R^{1D}$, wherein the $R^{1D}$ is selected from the group consisting of hydrogen, C1-C3 alkyl, halogen-substituted C1-C3 alkyl and halogen-substituted C1-C3 alkoxy.

In some embodiments, the

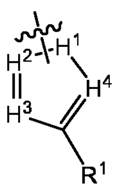 or 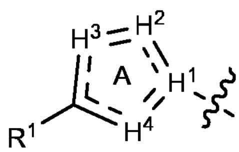

is any one of the following groups:

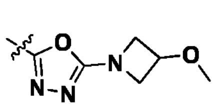 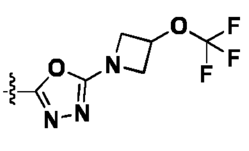 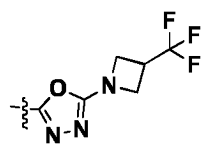 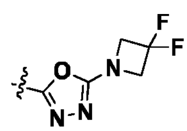

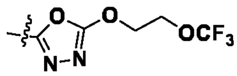
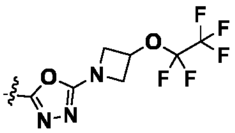
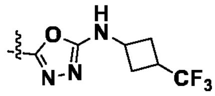
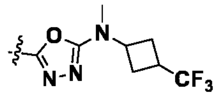
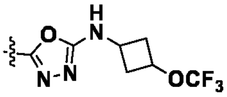
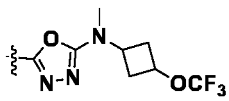
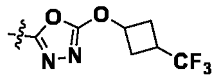
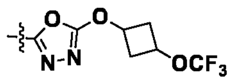
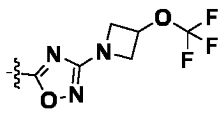
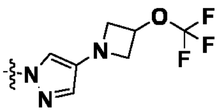
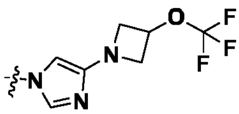
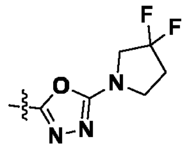
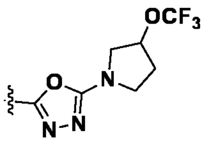
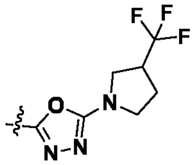
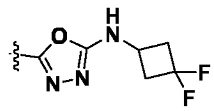
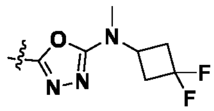
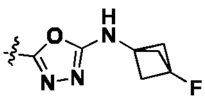
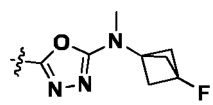
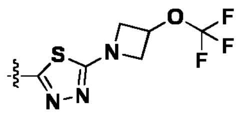
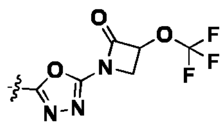
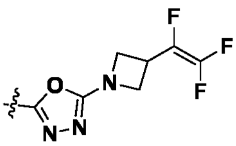
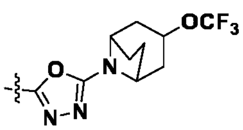
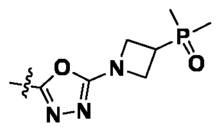
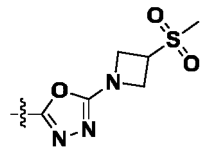
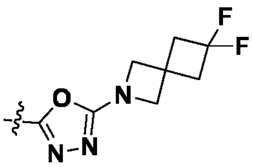
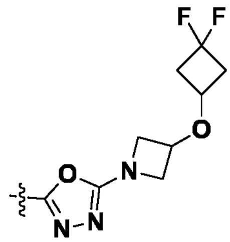
.

In some embodiments, the ring C is 6- to 10-membered aryl-O-C1-C6 alkyl, 3- to 9-membered heterocyclyl-O-C1-C3 alkyl, 3- to 9-membered heterocyclyl, 5- to 10-membered heteroaryl or 6- to 10-membered aryl; and the aryl, heteroaryl and heterocyclyl can each be optionally substituted with 1-3 $R^2$; each of the $R^2$ is independently selected from the group consisting of hydrogen, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen.

In some embodiments, the ring C is 4- to 8-membered heterocyclyl-O-C1-C3 alkyl, wherein the heterocyclyl is substituted with 1 $R^2$; preferably, each of the $R^2$ is independently haloalkyl.

In some embodiments, the ring C is phenyl-O-C1-C3 alkyl, wherein the phenyl is substituted with 1, 2 or 3 $R^2$; preferably, each of the $R^2$ is independently halogen.

In some embodiments, the ring C is 4- to 9-membered heterocyclyl, and preferably the ring C is

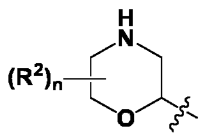 or 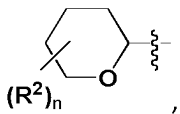 ,

wherein the number n of the $R^2$ is 1 or 2 or 3; preferably, each of the $R^2$ is independently selected from the group consisting of hydrogen, halogen, C1-C3 alkyl, phenyl, halophenyl and hydroxyl; when $R^2$ is phenyl or halophenyl, the phenyl or halophenyl and

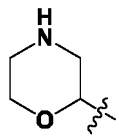 or 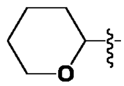

optionally share two carbon atoms to form a fused ring, wherein the fusion mode is preferably

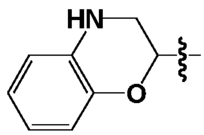 or 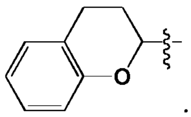 .

In some embodiments, the ring C is any one of the following 5- to 10-membered heteroaryl groups:

1) 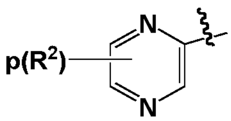 or 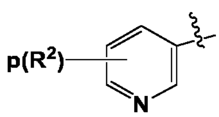 ;

preferably, each $R^2$ is independently selected from any one of the following groups: hydrogen, halogen, C1-C3 alkyl, C1-C3 haloalkyl, phenyl and halophenyl, wherein the number p of the $R^2$ is 1 or 2; and when the $R^2$ is phenyl or halophenyl, the phenyl or halophenyl and

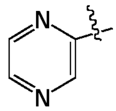

or

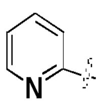

share two carbon atoms to form a fused ring, wherein the fusion mode is preferably

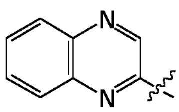 or 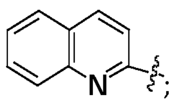;

2) 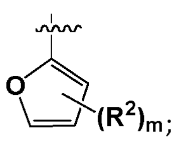;

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

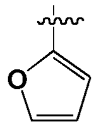

optionally share two carbon atoms to form a fused ring, wherein the fusion mode is preferably

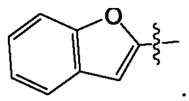

In some embodiments, the ring C is phenyl; the phenyl is substituted with $R^2$; preferably, the $R^2$ is C1-C3 haloalkyl;
preferably, the ring C is selected from any one of the following groups:

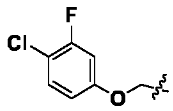
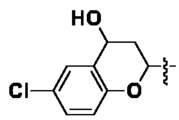
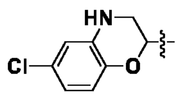
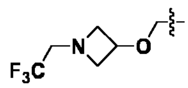
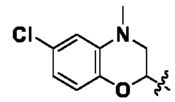

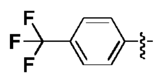
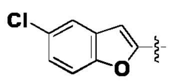
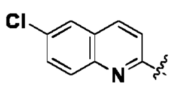
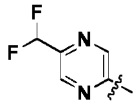
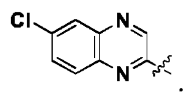

In some embodiments, the compound has a structure as shown in formula II-2, II-2', formula III-2' or formula III-2:

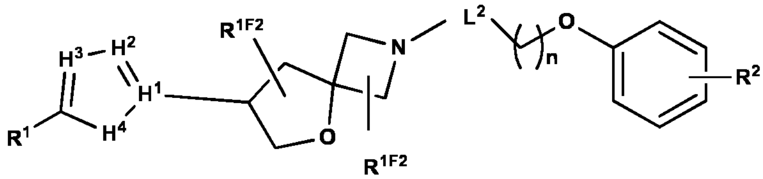

formula II-2

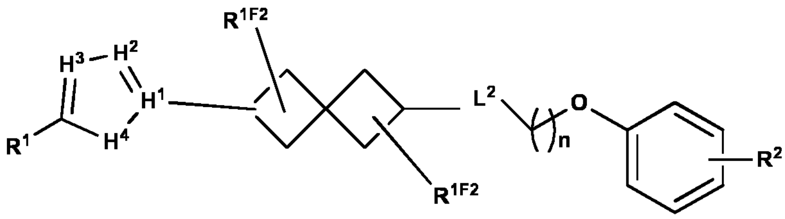

formula III-2

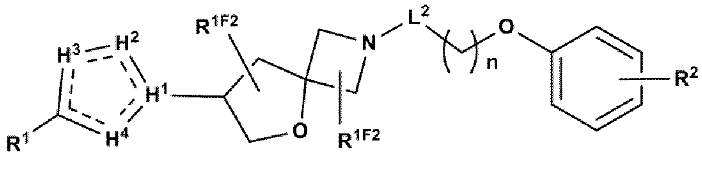

Formula II-2'

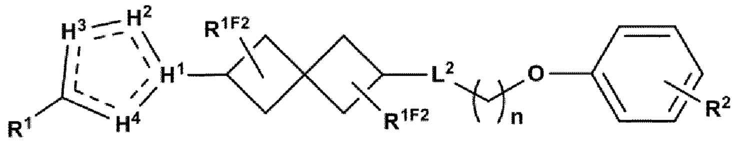

Formula III-2'

,

wherein the $L^2$ is

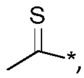, 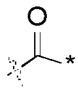 or 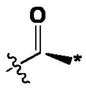,

wherein the end is connected to the N atom in

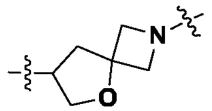

in formula II-2 or the @C atom in

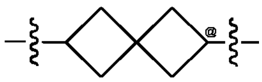

in formula III-2; n is independently 1-3, the number of the $R^2$ is 1, 2 or 3, and each of the $R^2$ is independently selected from the group consisting of hydrogen, halogen and hydroxyl.

In some embodiments, the compound has a structure as shown in formula IV:

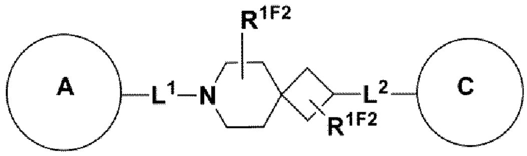

Formula IV

Preferably, the ring A is 4- to 8-membered heterocyclyl, 5- to 7-membered heteroaryl, phenyl-O-C1-C6 alkyl or C4-C8 cycloalkyl-O-C1-C6 alkyl, wherein the heterocyclyl, heteroaryl, phenyl and cycloalkyl can each be optionally substituted with 1-2 $R^1$;

each of the $R^1$ is independently selected from the group consisting of halogen, halogen-substituted C1-C3 alkyl, halogen-substituted C1-C3 alkoxy, $-OR^{1A}$, $-NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C6 alkyl, halogen-substituted C3-C6 cycloalkyl, halogen-substituted 4- to 6-membered heterocyclyl and halogen-substituted C1-C3 alkoxy;
each of the $G^1$ is independently selected from C3-C6 cycloalkyl, 3- to 6-membered heterocyclyl and phenyl; each of the $G^1$ is optionally substituted with 1-3 $R^{1F1}$; when $G^1$ is phenyl, the phenyl and 5- to 6-membered heterocyclyl or 5- to 7-membered heteroaryl optionally share two carbon atoms to form a fused ring;
each of the $R^{1F1}$ is independently selected from the group consisting of halogen, C1-C3 alkyl, C1-C3 alkoxy, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, $-P(O)R^{1B}R^{1C}$ and $-S(O)_2R^{1D}$;
each of the $R^{1F2}$ is independently selected from hydrogen, hydroxyl, amino, carboxyl or halogen;
$L^1$ is null, -NH-,

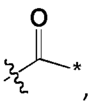, 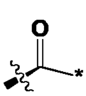, 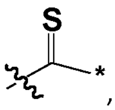, 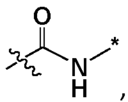, 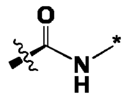, 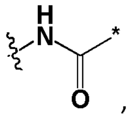, 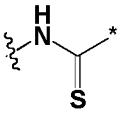

or

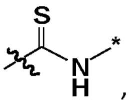,

wherein the end is connected to ring A, and the * end is connected to ring B; preferably, the * end is connected to the N atom in

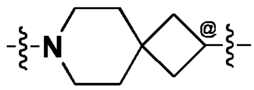

in formula IV; preferably, the $L^1$ is null, -NH-,

, 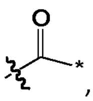, 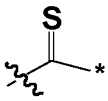, 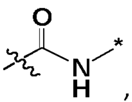, 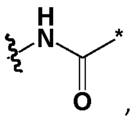, 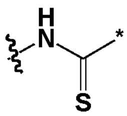 or 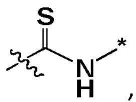,

preferably, the $L^1$ is null,

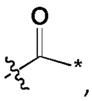, 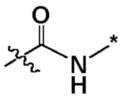 or 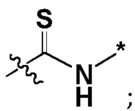;

preferably, the $L^1$ is null,

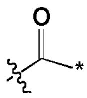 or 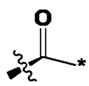;

$L^2$ is null, -NH-,

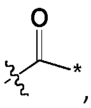, 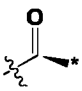, 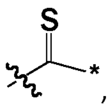, 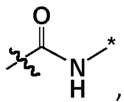, 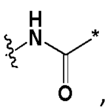, 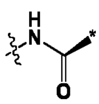, 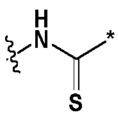 or 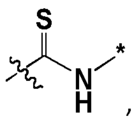,

wherein the end is connected to ring B, and the * end is connected to ring C; preferably, the end is connected to the @C atom in

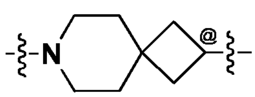

in formula IV; preferably, the $L^2$ is null, -NH-

, 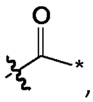, 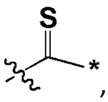, 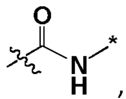, 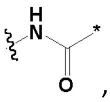, 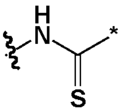 or 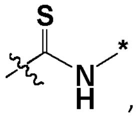,

preferably, the $L^2$ is null,

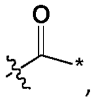, 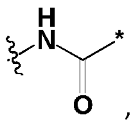, 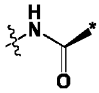 or 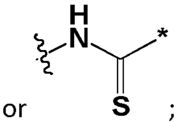;

further preferably, the $L^2$ is

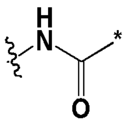 or 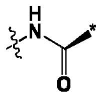;

the ring C is 6- to 10-membered aryl-O-C1-C3 alkyl, C4-C9 cycloalkyl-O-C1-C3 alkyl, 4- to 9-membered heterocyclyl-O-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F1}$; when $R^2$ is phenyl, the phenyl and 4- to 9-membered heterocyclyl or 5- to 10-membered heteroaryl optionally share two carbon atoms to form a fused ring.

In some embodiments, the compound has a structure as shown in formula IV-1 or formula IV-1':

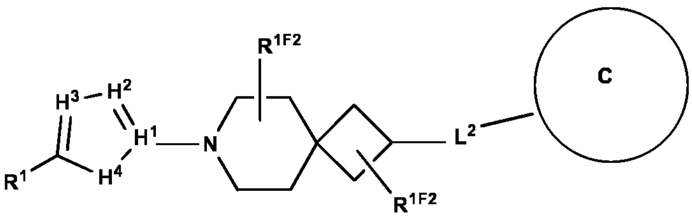

formula IV-1

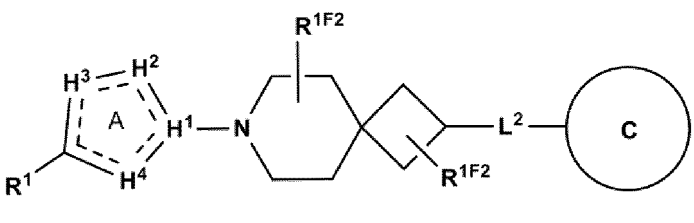

Formula IV-1'

,

wherein each of the $R^{1F2}$ is independently hydrogen, halogen, hydroxyl and amino;
$H^1$, $H^2$, $H^3$ and $H^4$ are each independently C or a chemically acceptable heteroatom, with at least one being a heteroatom; ------ is a single bond or a double bond;
preferably, the heteroatom is selected from any one of N, O and S;
preferably, the

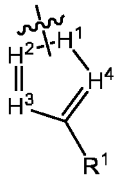 or 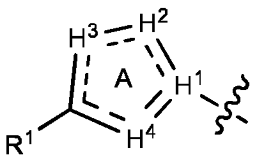 is 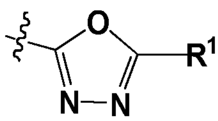, 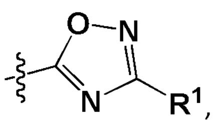,

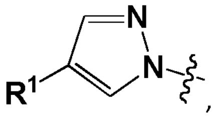, 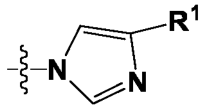 or 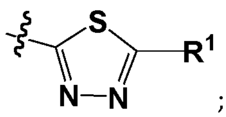;

preferably, the $R^1$ is selected from the group consisting of $-OR^{1A}$, $-NR^{1B}R^{1C}$, $-G^1$, $O-G^1$ and $-NR^{1B}-G^1$, wherein each of the $R^{1A}$, $R^{1B}$ and $R^{1C}$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl; each of the $G^1$ is optionally substituted with 1-3 $R^{1F1}$;

each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)R^{1B}R^{1C}$ and $-S(O)_2R^{1D}$, wherein the $R^{1D}$ is selected from the group consisting of hydrogen, C1-C3 alkyl, halogen-substituted C1-C3 alkyl and halogen-substituted C1-C3 alkoxy.

In some embodiments, the

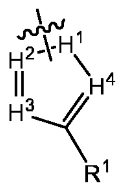 or 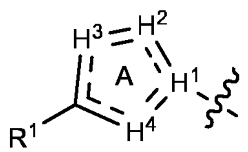

is any one of the following groups:

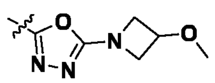 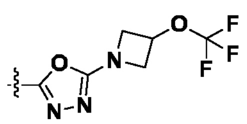 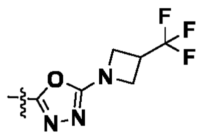 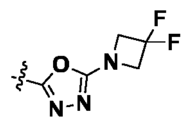

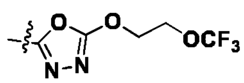 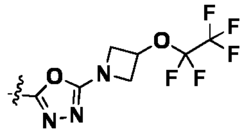 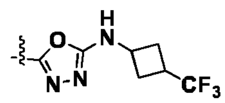 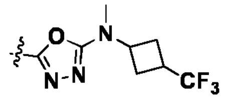

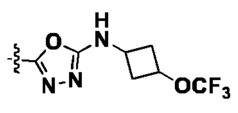 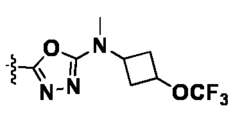 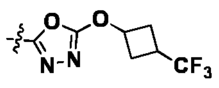 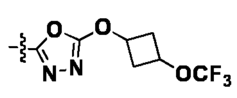

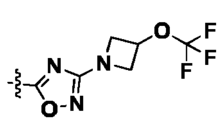 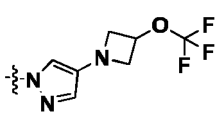 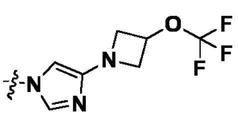 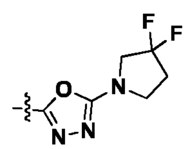

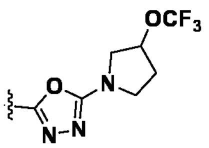 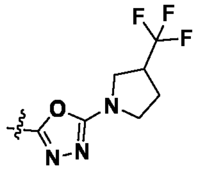 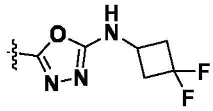 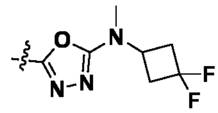

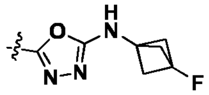 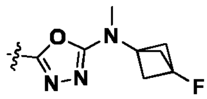 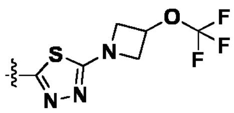 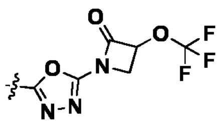

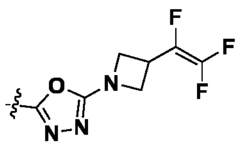 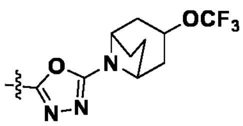 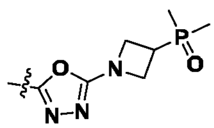 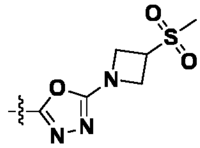

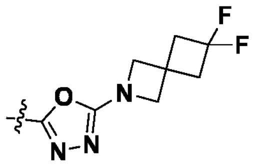 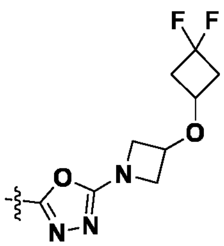

.

In some embodiments, the ring C is 6- to 10-membered aryl-O-C1-C6 alkyl, 3- to 9-membered heterocyclyl-O-C1-C3 alkyl, 3- to 9-membered heterocyclyl, 5- to 10-membered heteroaryl or 6- to 10-membered aryl; and the aryl, heteroaryl and heterocyclyl can each be optionally substituted with 1-3 $R^2$; each of the $R^2$ is independently selected from the group consisting of hydrogen, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen.

In some embodiments, the ring C is 4- to 8-membered heterocyclyl-O-C1-C3 alkyl, wherein the heterocyclyl is substituted with 1 $R^2$, and preferably, the $R^2$ is haloalkyl.

In some embodiments, the ring C is phenyl-O-C1-C3 alkyl, wherein the phenyl is substituted with 1, 2 or 3 $R^2$, and preferably, each of the $R^2$ is independently halogen.

In some embodiments, the ring C is 4- to 9-membered heterocyclyl, and preferably the ring C is

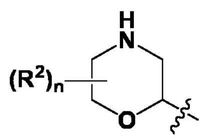 or 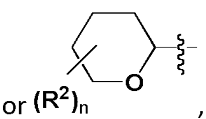 ,

wherein the number n of the $R^2$ is 1 or 2 or 3; preferably, each of the $R^2$ is independently selected from the group consisting of hydrogen, halogen, C1-C3 alkyl, phenyl, halophenyl and hydroxyl; when $R^2$ is phenyl or halophenyl, the phenyl or halophenyl and

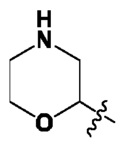 or 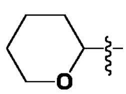

optionally share two carbon atoms to form a fused ring, wherein the fusion mode is preferably

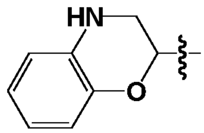, or 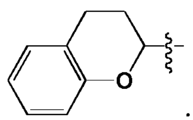.

In some embodiments, the ring C is any one of the following 5- to 10-membered heteroaryl groups:

1) 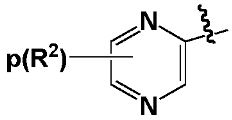 or 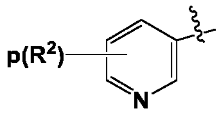;

preferably, the $R^2$ is any one of the following groups: hydrogen, halogen, C1-C3 alkyl, C1-C3 haloalkyl, phenyl and halophenyl, wherein the number p of the $R^2$ is 1 or 2, and when the $R^2$ is phenyl or halophenyl, the phenyl or halophenyl and

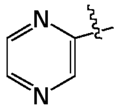 or 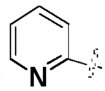

share two carbon atoms to form a fused ring, wherein the fusion mode is preferably

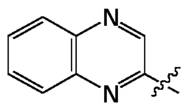 or

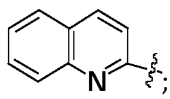;

2) 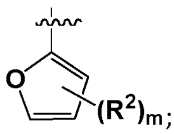;

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

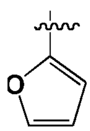

optionally share two carbon atoms to form a fused ring, wherein the fusion mode is preferably

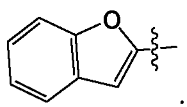.

In some embodiments, the ring C is phenyl; the phenyl is substituted with $R^2$; preferably, the $R^2$ is C1-C3 haloalkyl.

In some embodiments, the ring C is selected from any one of the following groups:

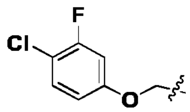 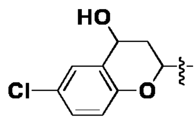 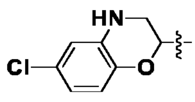 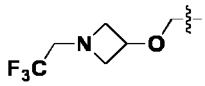 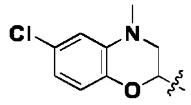

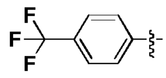 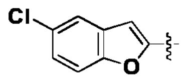 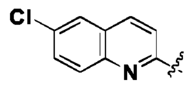 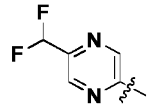 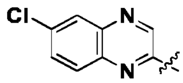 ;

the

$L^2$ is 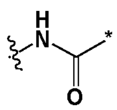 or 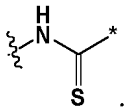 .

In some embodiments, the compound has a structure as shown in formula IV-2 or formula IV-2':

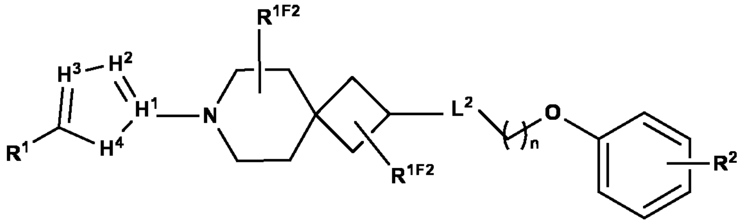

formula IV-2

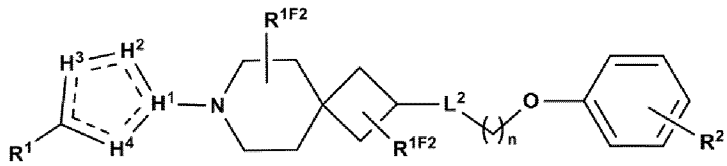

Formula IV-2'

the $L^2$ is

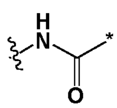 or 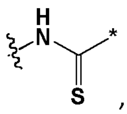 ,

wherein the end is connected to the @C atom in

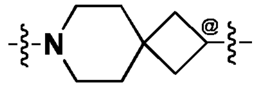

in formula IV-2; n is 1-3; the number of the $R^2$ is 1, 2 or 3, and each of the $R^2$ is independently selected from the group consisting of hydrogen, halogen and hydroxyl.

In some embodiments, the compound preferably has a structure as shown in formula IV-3:

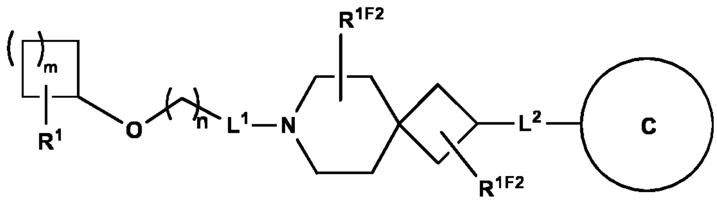

IV-3

each of the $R^{1F2}$ is independently hydrogen, halogen, hydroxyl or amino, and preferably hydrogen or hydroxyl; m is 1, 2, 3, 4 or 5, n is 1 to 3, the number of $R^1$ is 1, 2 or 3, and each of the $R^1$ is independently selected from the group consisting of hydrogen, halogen, hydroxyl, phenyl, cyano, C1-C10 alkyl, hydroxyl-substituted C1-C6 alkyl, halogen-substituted C1-C6 alkyl, C1-C6 alkoxy and halogen-substituted C1-C6 alkoxy;
the ring C is 6- to 10-membered aryl-O-C1-C6 alkyl, 3- to 9-membered heterocyclyl-O-C1-C3 alkyl, 3- to 9-membered heterocyclyl, 5- to 10-membered heteroaryl or 6- to 10-membered aryl; and the aryl, heteroaryl and heterocyclyl can each be optionally substituted with 1-3 $R^2$; each of the $R^2$ is independently selected from the group consisting of hydrogen, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen.

In some embodiments, the ring C is

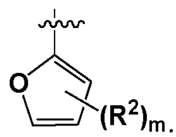

Preferably, the $R^2$ is selected the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

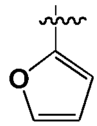

optionally share two carbon atoms to form a fused ring, wherein the fusion mode is preferably

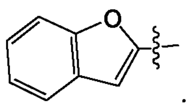

In some embodiments, the ring C is phenyl; the phenyl is substituted with $R^2$.
Preferably, the $R^2$ is C1-C3 haloalkyl.
In some embodiments, the ring C is

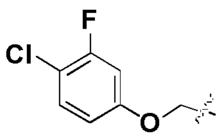
or
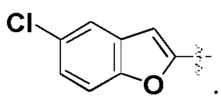

In some embodiments, the $L^2$ is

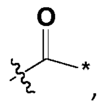

wherein the end is connected to the @C atom in

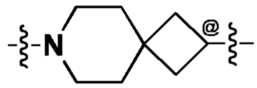

in formula IV-3.

In some embodiments, the compound has a structure as shown in formula V:

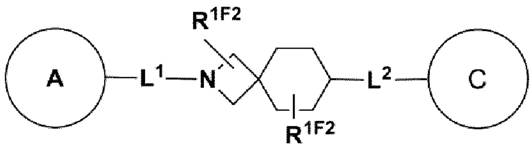

Formula V

Preferably, the ring A is 4- to 8-membered heterocyclyl, 5- to 7-membered heteroaryl or phenyl-O-C1-C6 alkyl, wherein the heterocyclyl, heteroaryl and phenyl can each be optionally substituted with 1-2 $R^1$;

each of the $R^1$ is independently selected from the group consisting of halogen, halogen-substituted C1-C3 alkyl, halogen-substituted C1-C3 alkoxy, $-OR^{1A}$, $-NR^{1B}R^{1C}$, $-G^1$, $O-G^1$ and $-NR^{1B}-G^1$;

each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C6 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl; each of the $G^1$ is optionally substituted with 1-3 $R^{1F1}$; when $G^1$ is phenyl, the phenyl and 5- to 6-membered heterocyclyl or 5- to 7-membered heteroaryl optionally share two carbon atoms to form a fused ring;

each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, $-P(O) R^{1B}R^{1C}$ and $-S(O)_2R^{1D}$.

$L^1$ is null, -NH-,

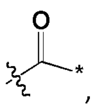, 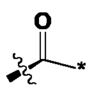, 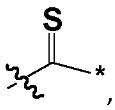, 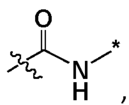, 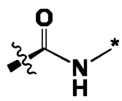, 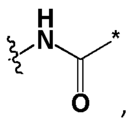, 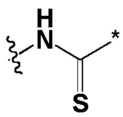

or

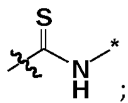;

preferably, the $L^1$ is null, -NH-,

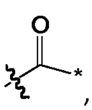, 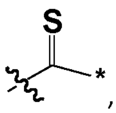, 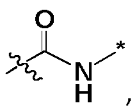, 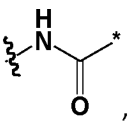, 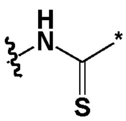 or 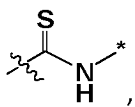,

preferably, the $L^1$ is null,

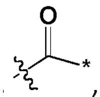, 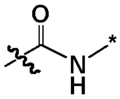 or 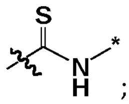;

preferably, the $L^1$ is

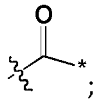;

each $R^{1F2}$ is independently selected from hydrogen, halogen, hydroxyl and amino;
$L^2$ is null, -NH-,

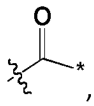, 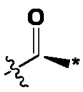, 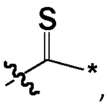, 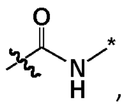, 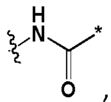, 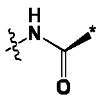, 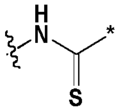 or 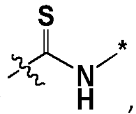,

wherein the  end is connected to the @C atom in

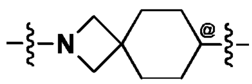

in formula V, and the * end is connected to ring C;
preferably, the $L^2$ is null, -NH-,

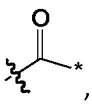, 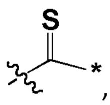, 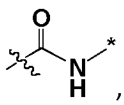, 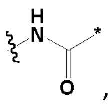, 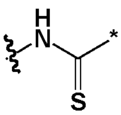 or 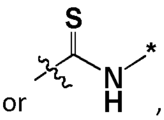,

preferably, the $L^2$ is null,

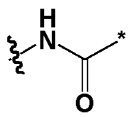 or 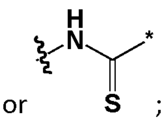;

preferably, the $L^2$ is

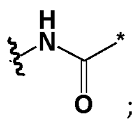;

the ring C is 6- to 10-membered aryl-O-C1-C3 alkyl, 4- to 9-membered heterocyclyl-O-C1-C3 alkyl, 4- to 9-membered heterocyclyl, 5- to 10-membered heteroaryl or 6- to 10-membered aryl, the aryl, heteroaryl, cycloalkyl and heterocyclyl can each be optionally substituted with 1-3 $R^2$; each of the $R^2$ is independently selected from the group consisting of halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F1}$; when $R^2$ is phenyl, the phenyl and 4- to 9-membered heterocyclyl or 5- to 10-membered heteroaryl optionally share two carbon atoms to form a fused ring.

In some embodiments, the compound has a structure as shown in formula V-1 or formula V-1':

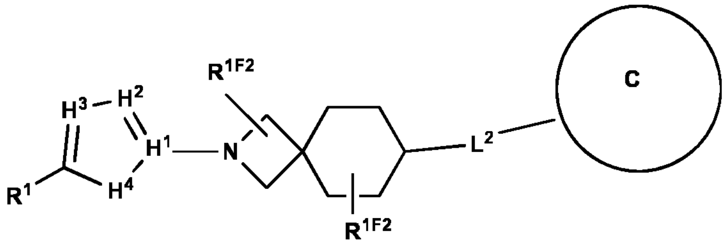

formula V-1

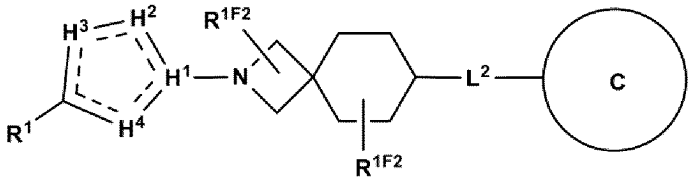

Formula V-1'

,

wherein each of the $R^{1F2}$ is independently selected from hydrogen, hydroxyl, amino and halogen, and preferably hydrogen or hydroxyl;

$H^1$, $H^2$, $H^3$ and $H^4$ are each independently C or a chemically acceptable heteroatom, with at least one being a heteroatom;

- - - - - - is a single bond or a double bond;

preferably, the heteroatom is selected from any one of N, O and S;

preferably, the

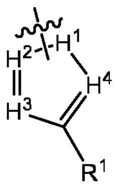 or 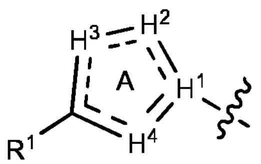 is 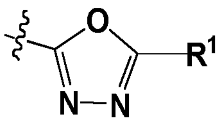 , 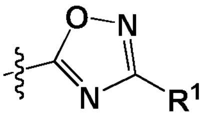

or

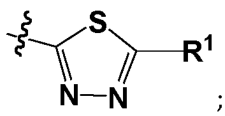 ;

preferably, the $R^1$ is selected from the group consisting of $-OR^{1A}$, $-NR^{1B}R^{1C}$, $-G^1$, $O-G^1$ and $-NR^{1B}-G^1$, wherein each of the $R^{1A}$, $R^{1B}$ and $R^{1C}$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl; each of the $G^1$ is optionally substituted with 1-3 $R^{1F1}$;

each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)\,R^{1B}R^{1C}$ and $-S(O)_2R^{1D}$, wherein the $R^{1D}$ is selected from the group consisting of hydrogen, C1-C3 alkyl, halogen-substituted C1-C3 alkyl and halogen-substituted C1-C3 alkoxy.

In some embodiments, the

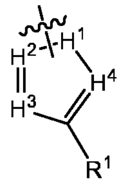 or 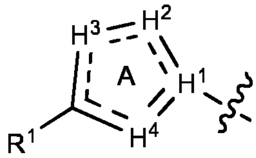

is any one of the following groups:

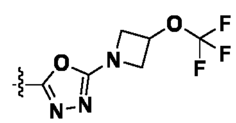 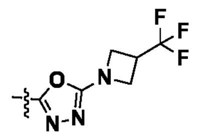 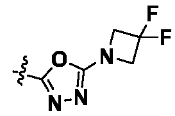 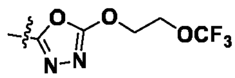

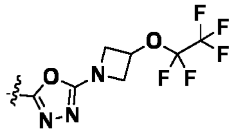 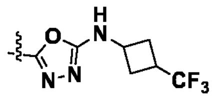 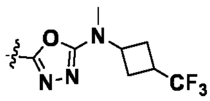 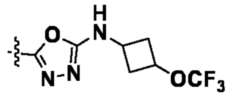

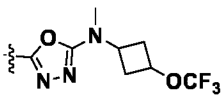 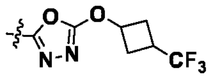 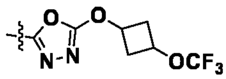 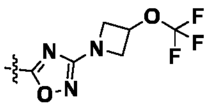

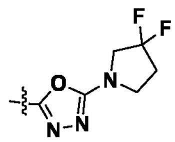 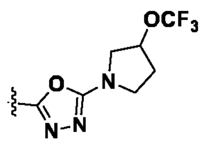 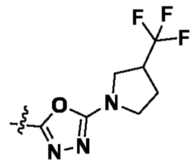 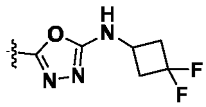

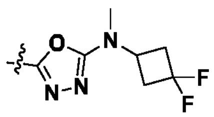 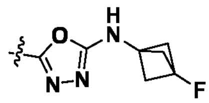 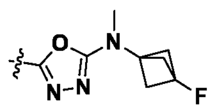 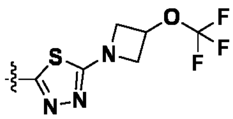

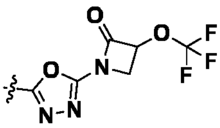 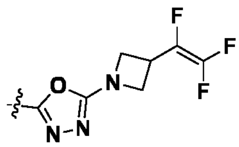 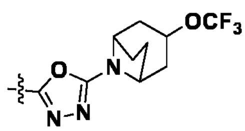 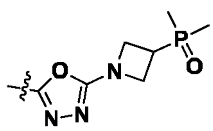

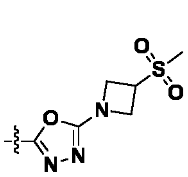 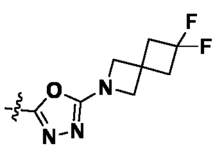 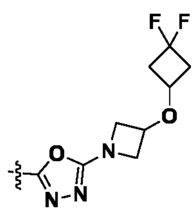 .

In some embodiments, the ring C is 6- to 10-membered aryl-O-C1-C6 alkyl, 3- to 9-membered heterocyclyl-O-C1-C3 alkyl, 3- to 9-membered heterocyclyl, 5- to 10-membered heteroaryl or 6- to 10-membered aryl; and the aryl, heteroaryl and heterocyclyl can each be optionally substituted with 1-3 $R^2$; each of the $R^2$ is independently selected from the group consisting of hydrogen, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6

cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen.

In some embodiments, the ring C is 4- to 8-membered heterocyclyl-O-C1-C3 alkyl, wherein the heterocyclyl is substituted with 1 $R^2$, and preferably, the $R^2$ is haloalkyl.

In some embodiments, the ring C is phenyl-O-C1-C3 alkyl, wherein the phenyl is substituted with 1, 2 or 3 $R^2$, and preferably, each of the $R^2$ is independently halogen.

In some embodiments, the ring C is 4- to 9-membered heterocyclyl, and preferably the ring C is

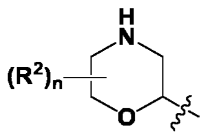 or 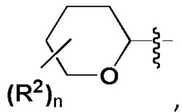 ,

wherein the number n of the $R^2$ is 1 or 2 or 3; preferably, each of the $R^2$ is independently selected from the group consisting of hydrogen, halogen, C1-C3 alkyl, phenyl, halophenyl and hydroxyl; when $R^2$ is phenyl or halophenyl, the phenyl or halophenyl and

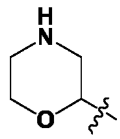 or 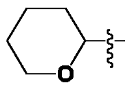

optionally share two carbon atoms to form a fused ring, wherein the fusion mode is preferably

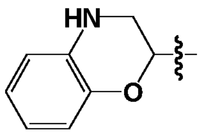 or 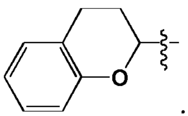 .

In some embodiments, the ring C is any one of the following 5- to 10-membered heteroaryl groups:

1) 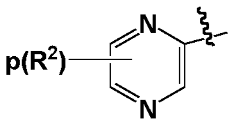 or 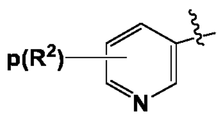 ;

preferably, the $R^2$ is any one of the following groups: hydrogen, halogen, C1-C3 alkyl, C1-C3 haloalkyl, phenyl and halophenyl, wherein the number p of the $R^2$ is 1 or 2, and when the $R^2$ is phenyl or halophenyl, the phenyl or halophenyl and

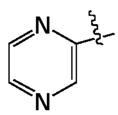 or 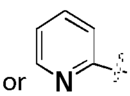

share two carbon atoms to form a fused ring, wherein the fusion mode is preferably

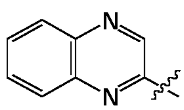

or

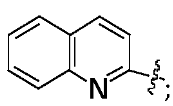 ;

2) 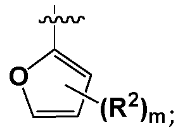;

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

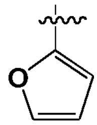

optionally share two carbon atoms to form a fused ring, wherein the fusion mode is preferably

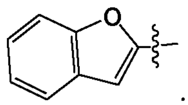.

In some embodiments, the ring C is phenyl; the phenyl is substituted with $R^2$; preferably, the $R^2$ is C1-C3 haloalkyl.

In some embodiments, the ring C is selected from any one of the following groups:

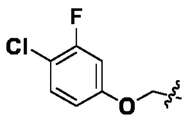
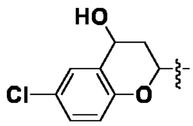
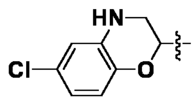
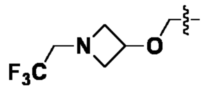
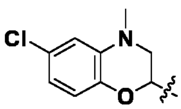
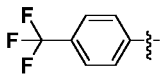
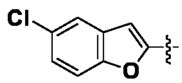
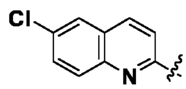
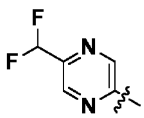
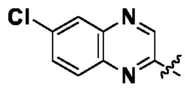
;

the $L^2$ is

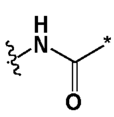 or 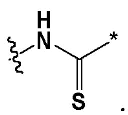.

In some embodiments, the compound has a structure as shown in formula V-2 or formula V-2':

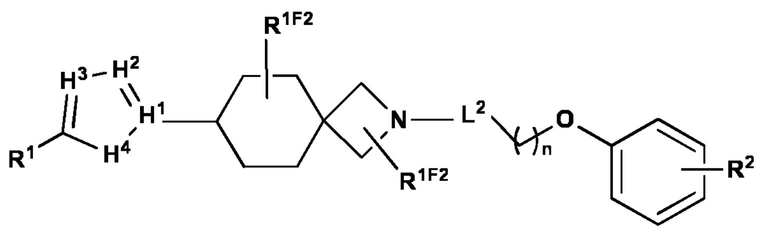

formula V-2

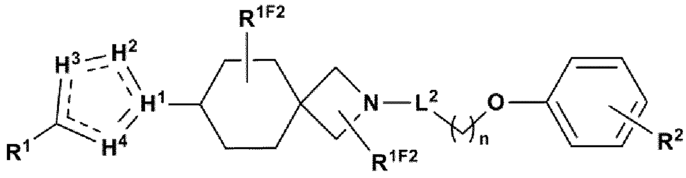

Formula V-2'

.

The $L^2$ is

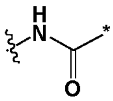 or 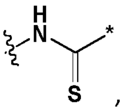 ,

wherein, the end is connected to the N atom in

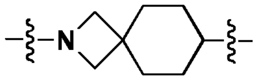

in formula V-2; n is 1-3; the number of the $R^2$ is 1, 2 or 3, and each of the $R^2$ is independently selected from the group consisting of hydrogen, halogen and hydroxyl.

In some embodiments, the compound has a structure as shown in formula VI, formula VII, formula VIII or formula IX:

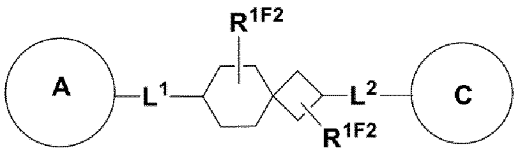

Formula VI

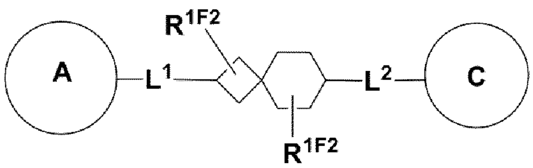

Formula VII

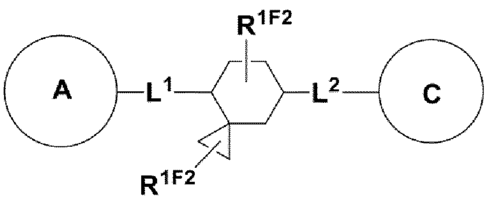

Formula VIII

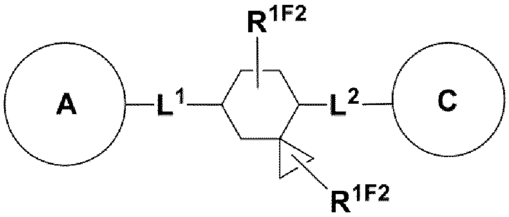

Formula IX

the ring A is 5- to 7-membered heteroaryl, wherein the heteroaryl can be substituted with 1-2 $R^1$;
each of the $R^1$ is independently selected from the group consisting of halogen, halogen-substituted C1-C3 alkyl, halogen-substituted C1-C3 alkoxy, $-OR^{1A}$, $-NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C6 alkyl, halogen-substituted C3-C6 cycloalkyl, halogen-substituted 4- to 6-membered heterocyclyl and halogen-substituted C1-C3 alkoxy;
each of the $G^1$ is independently selected from C3-C6 cycloalkyl, 3- to 6-membered heterocyclyl and phenyl; each of the $G^1$ is optionally substituted with 1-3 $R^{1F1}$. when $G^1$ is phenyl, the phenyl and 5- to 6-membered heterocyclyl or 5- to 7-membered heteroaryl optionally share two carbon atoms to form a fused ring;
each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, $-P(O)\ R^{1B}R^{1C}$ and $-S(O)_2R^{1D}$;
$L^1$ is null, -NH-,

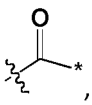 , 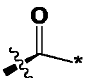 , 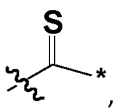 , 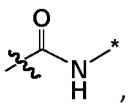 , 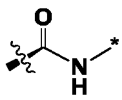 , 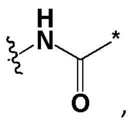 , 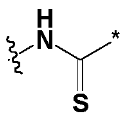

or

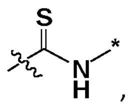 ,

wherein the end is connected to ring A, and the * end is connected to ring B;
preferably, the * end is connected to the &C atom in

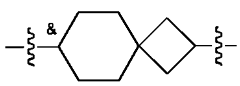

in formula VI, the &C atom in

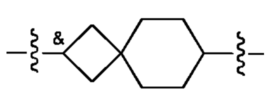

in formula VII, the &C atom in

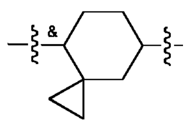

in formula VIII or the &C atom in

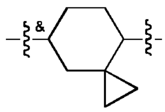

in formula IX;
preferably, the $L^1$ is null, -NH-,

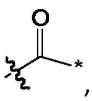, 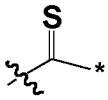, 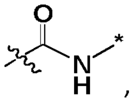, 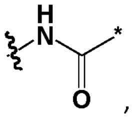, 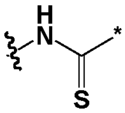 or 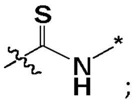;

preferably, the $L^1$ is null,

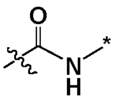 or 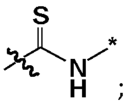;

$L^2$ is null, -NH-,

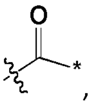, 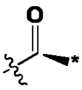, 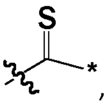, 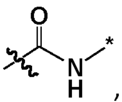, 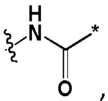, 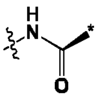, 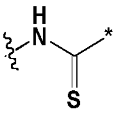 or 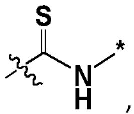,

wherein the end is connected to ring B, and the * end is connected to ring C; preferably, the end is connected to the @C atom in

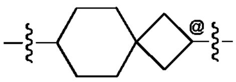

in formula VI, the @C atom in

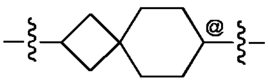

in formula VII, the @C atom in

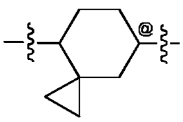

in formula VIII or the @C atom in

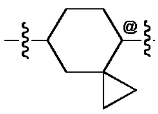

in formula IX;
preferably, the $L^2$ is null, -NH-,

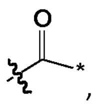, 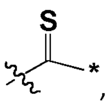, 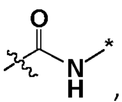, 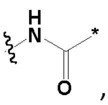, 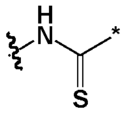 or 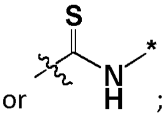;

preferably, the $L^2$ is null,

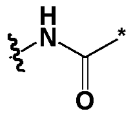 or 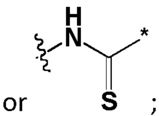;

each of the $R^{1F2}$ is independently selected from hydrogen, hydroxyl, amino, carboxyl or halogen.

In some embodiments, the compound preferably has a structure as shown in formula VI-1, formula VII-1, formula VIII-1, formula IX-1, formula VI-1', formula VII-1', formula VIII-1' or formula IX-1':

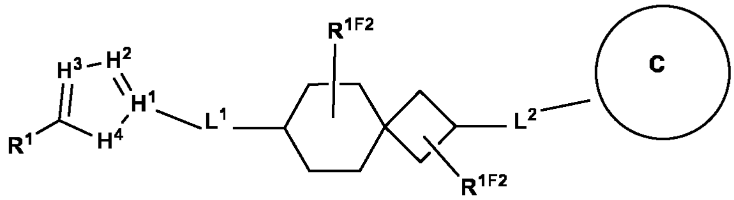

formula VI-1

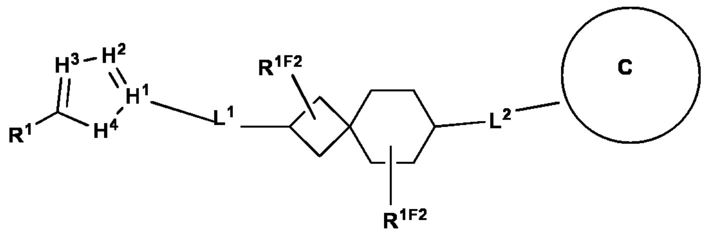

formula VII-1

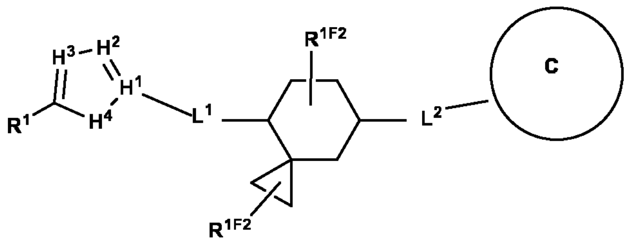

formula VIII-1

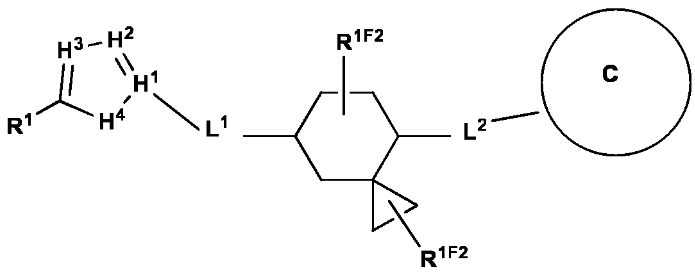

formula IX-1

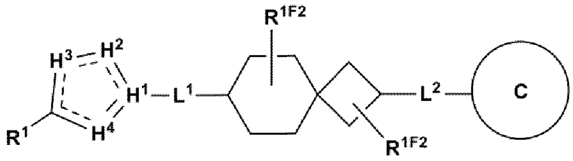

Formula VI-1'

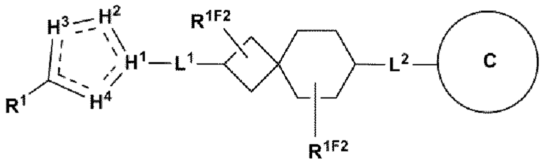

Formula VII-1'

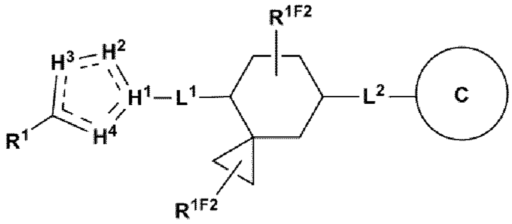

Formula VIII-1'

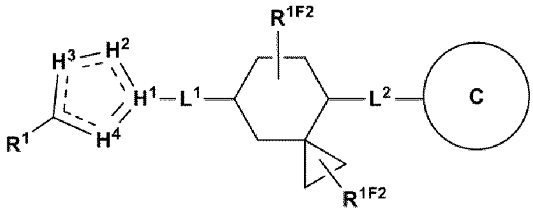

Formula IX-1'

.

In each formula, each of the $R^{1F2}$ is independently hydrogen, hydroxyl, amino, carboxyl or halogen, and preferably hydrogen or hydroxyl;

$H^1$, $H^2$, $H^3$ and $H^4$ are each independently C or a chemically acceptable heteroatom, with at least one being a heteroatom;
------ is a single bond or a double bond;
preferably, the heteroatom is selected from any one of N, O and S;
preferably, the

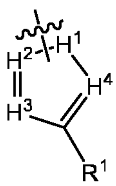 or 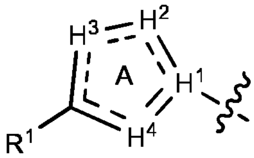 is 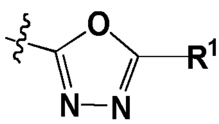, 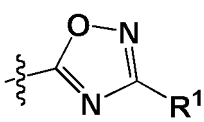,

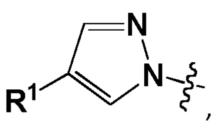, 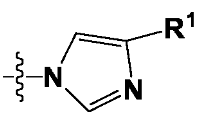 or 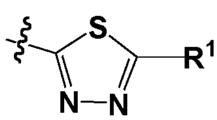;

preferably, the $R^1$ is selected from the group consisting of $-OR^{1A}$, $-NR^{1B}R^{1C}$, $-G^1$, $O-G^1$ and $-NR^{1B}-G^1$, wherein each of the $R^{1A}$, $R^{1B}$ and $R^{1C}$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl; each of the $G^1$ is optionally substituted with 1-3 $R^{1F1}$;

each of the $R^{1F1}$ is independently selected from the group consisting of hydrogen, halogen, C1-C3 alkyl, C3-C6 cycloalkyl, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)\ R^{1B}R^{1C}$ and $-S(O)_2R^{1D}$, wherein the $R^{1D}$ is selected from the group consisting of hydrogen, C1-C3 alkyl, halogen-substituted C1-C3 alkyl and halogen-substituted C1-C3 alkoxy.

In some embodiments, the

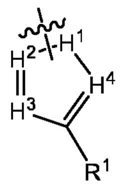 or 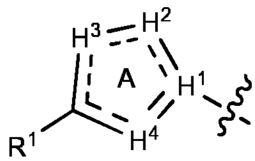

is any one of the following groups:

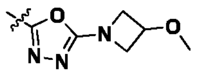 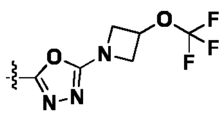 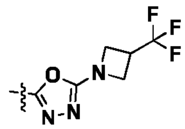 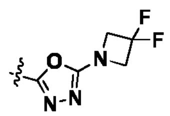

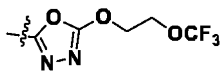 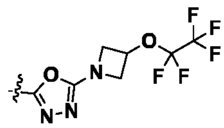 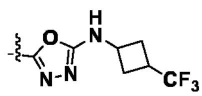 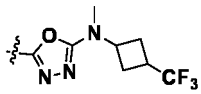

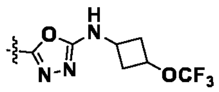 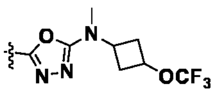 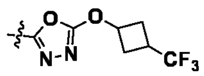 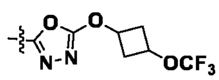

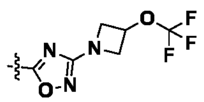 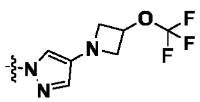 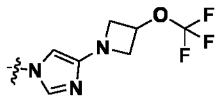 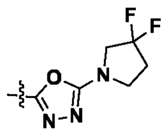

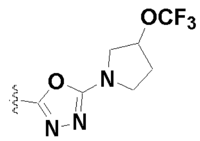 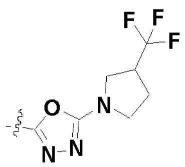 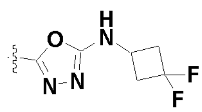 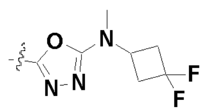

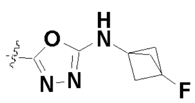 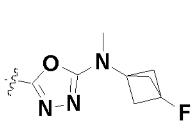 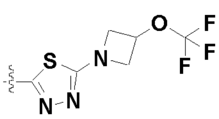 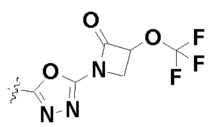

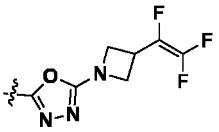 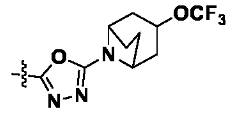 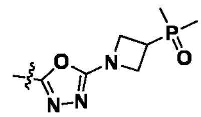 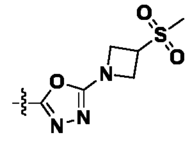

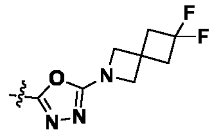 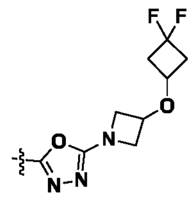

In some embodiments, the ring C is 6- to 10-membered aryl-O-C1-C6 alkyl, 3- to 9-membered heterocyclyl-O-C1-C3 alkyl, 3- to 9-membered heterocyclyl, 5- to 10-membered heteroaryl or 6- to 10-membered aryl; and the aryl, heteroaryl and heterocyclyl can each be optionally substituted with 1-3 $R^2$; each of the $R^2$ is independently selected from the group consisting of hydrogen, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen.

In some embodiments, the ring C is 4- to 8-membered heterocyclyl-O-C1-C3 alkyl, wherein the heterocyclyl is substituted with 1 $R^2$, and preferably, the $R^2$ is haloalkyl.

In some embodiments, the ring C is phenyl-O-C1-C3 alkyl, wherein the phenyl is substituted with 1, 2 or 3 $R^2$, and preferably, each of the $R^2$ is independently halogen.

In some embodiments, the ring C is 4- to 9-membered heterocyclyl.

Preferably, the ring C is

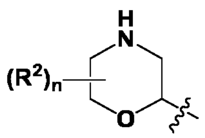 or 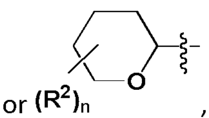 ,

wherein the number n of the $R^2$ is 1 or 2 or 3; preferably, each of the $R^2$ is independently selected from the group consisting of hydrogen, halogen, C1-C3 alkyl, phenyl, halophenyl and hydroxyl; when $R^2$ is phenyl or halophenyl, the phenyl or halophenyl and

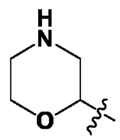 or 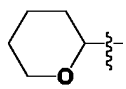

optionally share two carbon atoms to form a fused ring, wherein the fusion mode is preferably

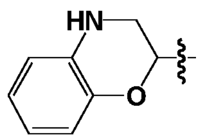

or

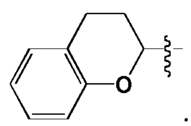 .

In some embodiments, the ring C is any one of the following 5- to 10-membered heteroaryl groups:

1) 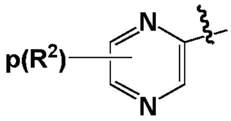 or 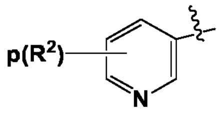 ;

preferably, the $R^2$ is any one of the following groups: hydrogen, halogen, C1-C3 alkyl, C1-C3 haloalkyl, phenyl and halophenyl, wherein the number p of the $R^2$ is 1 or 2, and when the $R^2$ is phenyl or halophenyl, the phenyl or halophenyl and

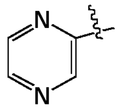 or 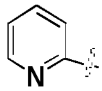

share two carbon atoms to form a fused ring, wherein the fusion mode is preferably

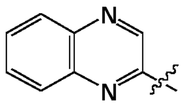

or

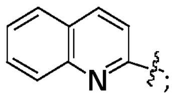

2) 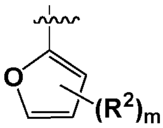 ;

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

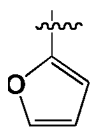

optionally share two carbon atoms to form a fused ring, wherein the fusion mode is preferably

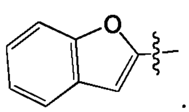

.

In some embodiments, the ring C is phenyl; the phenyl is substituted with $R^2$; preferably, the $R^2$ is C1-C3 haloalkyl.

In some embodiments, the ring C is selected from any one of the following groups:

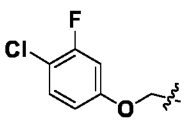 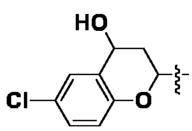 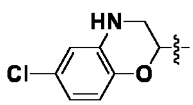 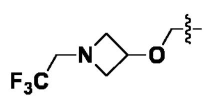

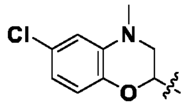 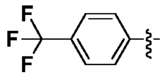 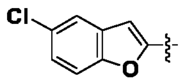 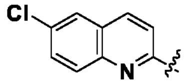

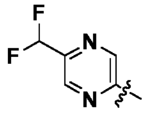 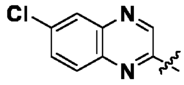 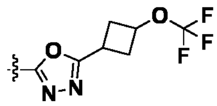 ;

the $L^2$ is

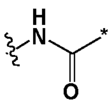 or 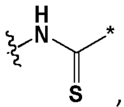 ,

wherein the end is connected to the @C atom in

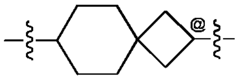

in formula VI-1, the @C atom in

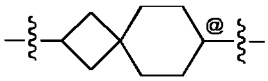

in formula VII-1, the @C atom in

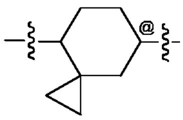

in formula VIII-1 or the @C atom in

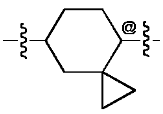

in formula IX-1.

In some embodiments, the compound has a structure as shown in formula VI-2, formula VII-2, formula VIII-2, formula IX-2, formula VI-2', formula VII-2', formula VIII-2' or formula IX-2':

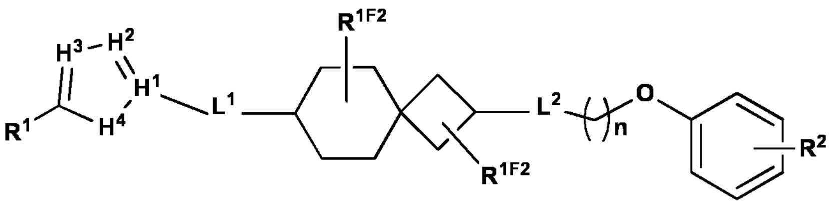

formula VI-2

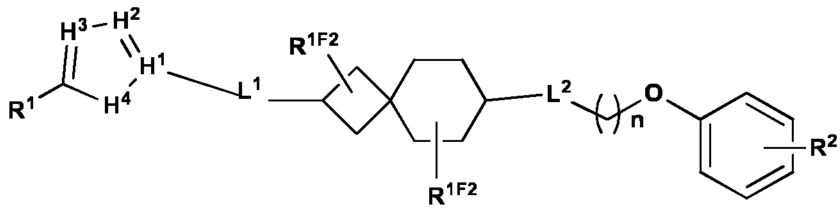

formula VII-2

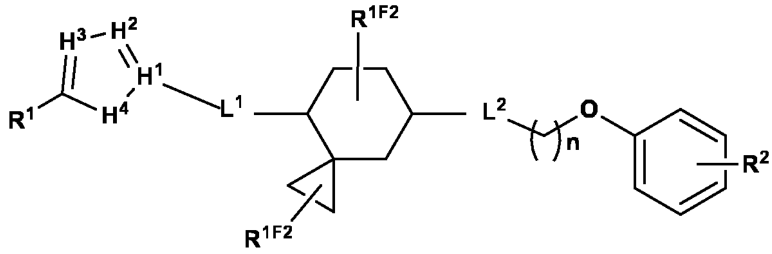

formula VIII-2

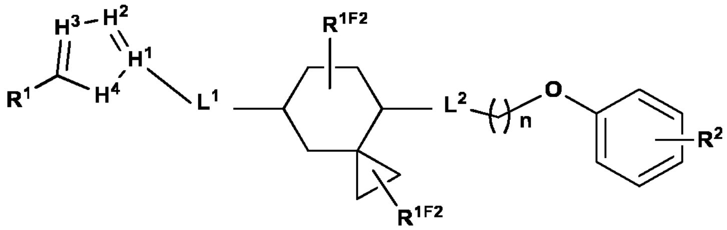

formula IX-2

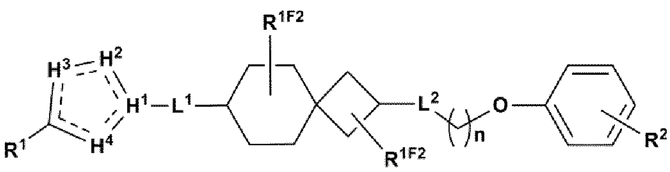

Formula VI-2'

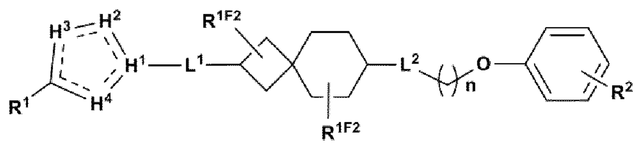

Formula VII-2'

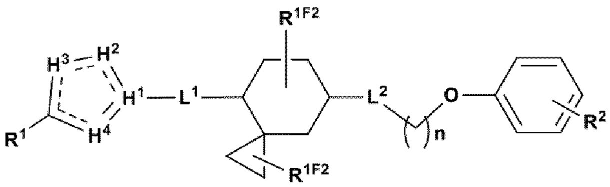

Formula VIII-2'

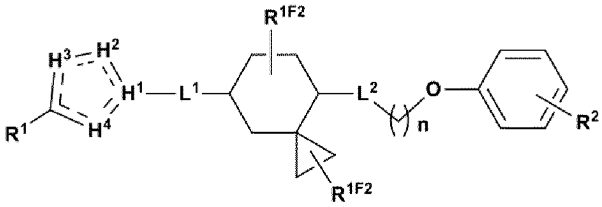

Formula IX-2'

each of the $L^2$ is

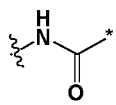 or 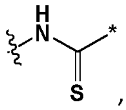 ,

wherein the  end is connected to the @C atom in

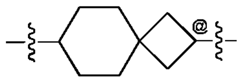

in formula VI-2, the @C atom in

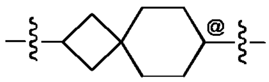

in formula VII-2, the @C atom in

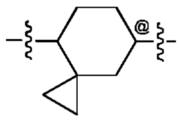

in formula VIII-2 or the @C atom in

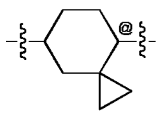

in formula IX-2; n is 1-3; the number of the $R^2$ is 1, 2 or 3, and each of the $R^2$ is independently selected from the group consisting of hydrogen, halogen and hydroxyl.

In some embodiments, the compound has a structure as shown in any one of the following formulas:

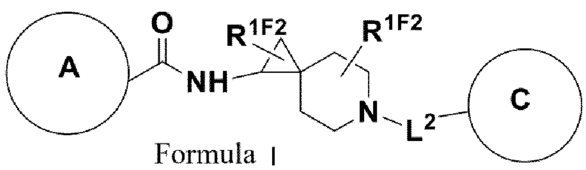

Formula I

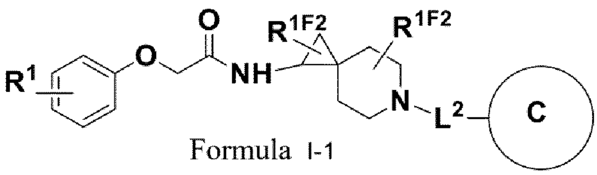

Formula I-1

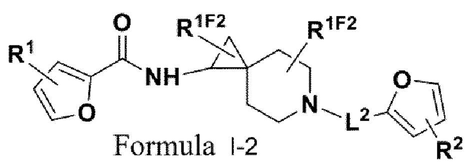

Formula I-2

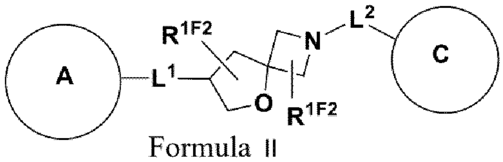

Formula II

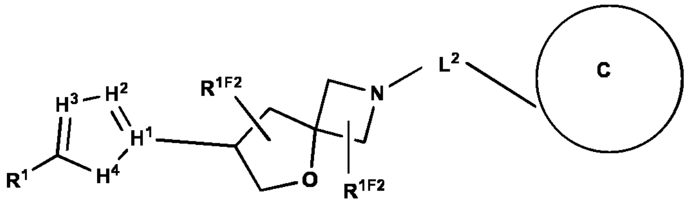

formula II-1

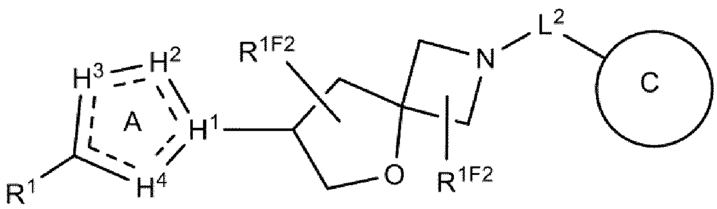

Formula II-1'

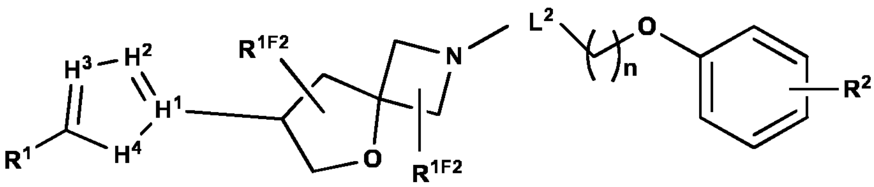

formula II-2

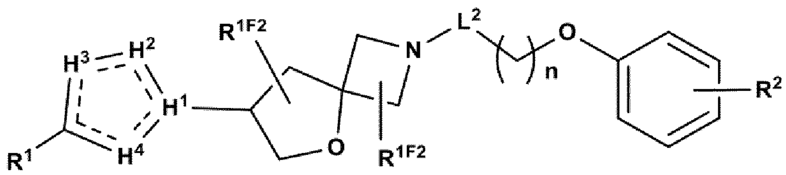

Formula II-2'

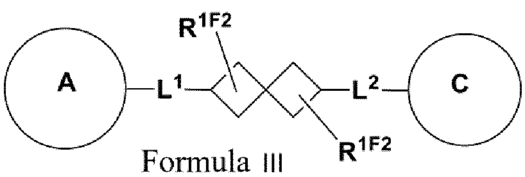

Formula III

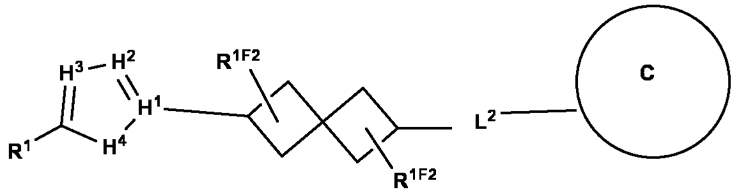

formula III-1

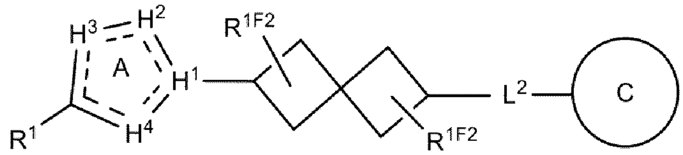

Formula III-1'

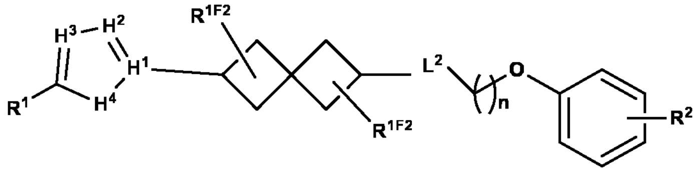

formula III-2

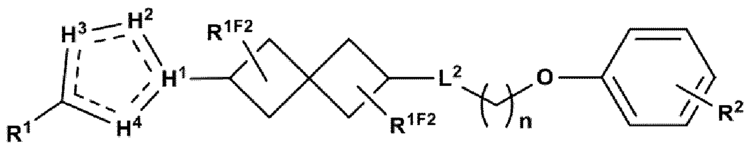

Formula III-2'

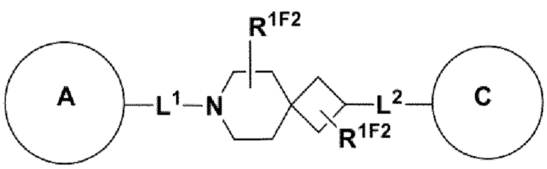

Formula IV

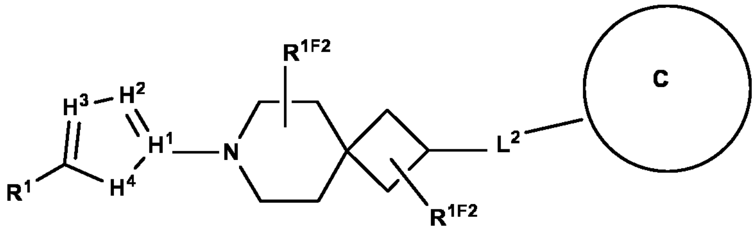

formula IV-1

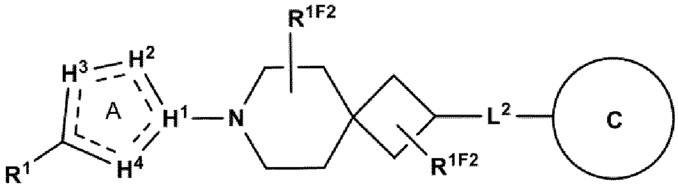

Formula IV-1'

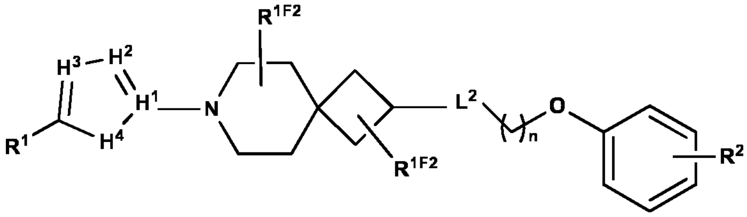

formula IV-2

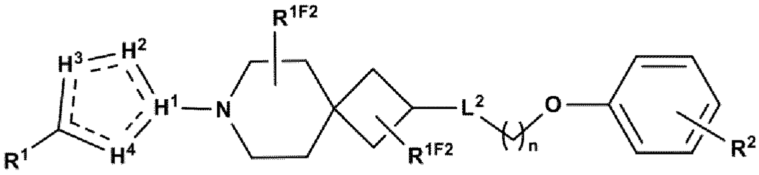

Formula IV-2'

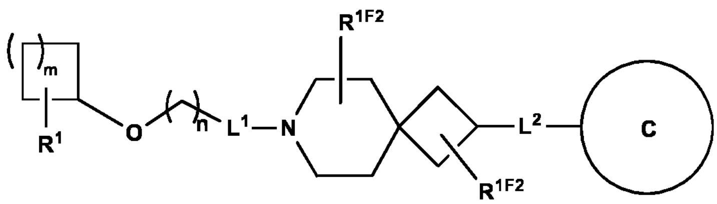

IV-3

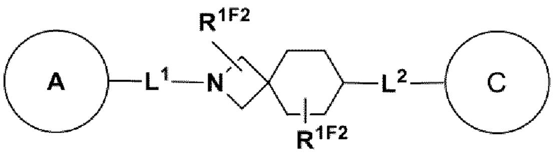

Formula V

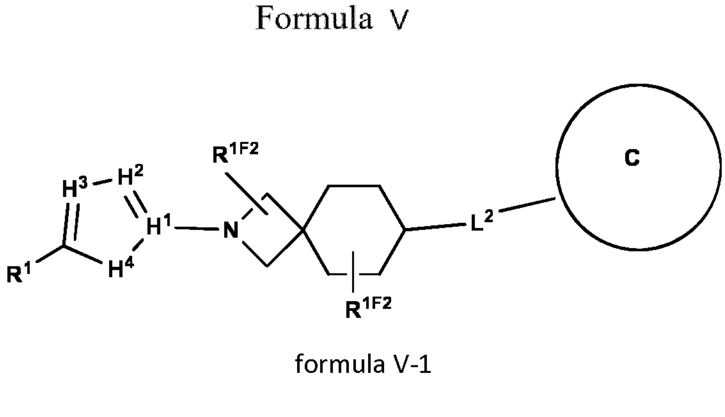

formula V-1

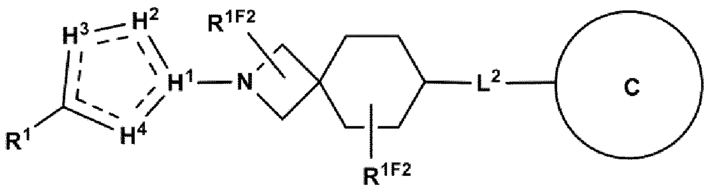

Formula V-1'

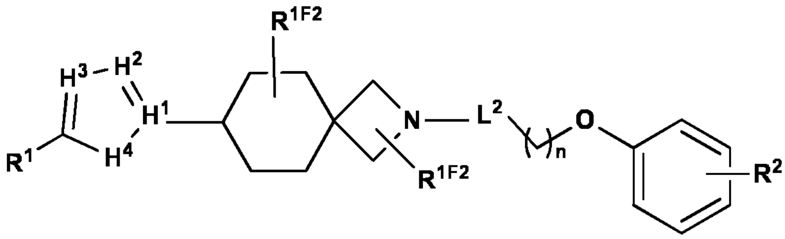

formula V-2

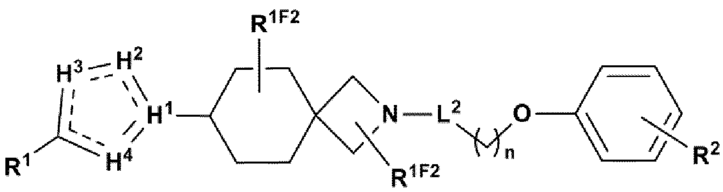

Formula V-2'

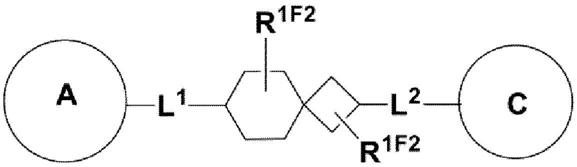

Formula VI

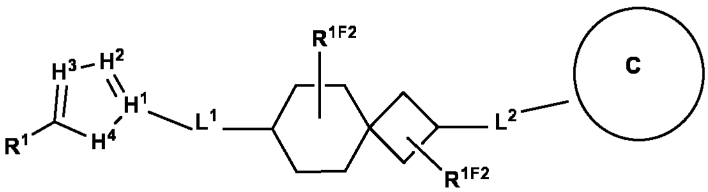

formula VI-1

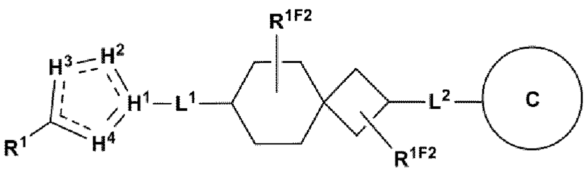

Formula VI-1'

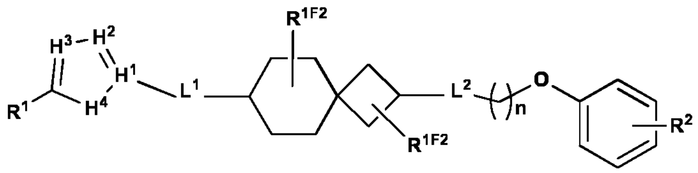

formula VI-2

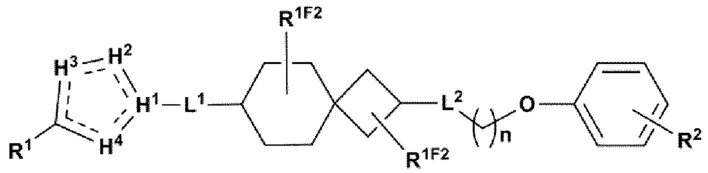

Formula VI-2'

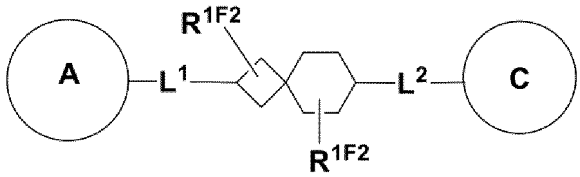

Formula VII

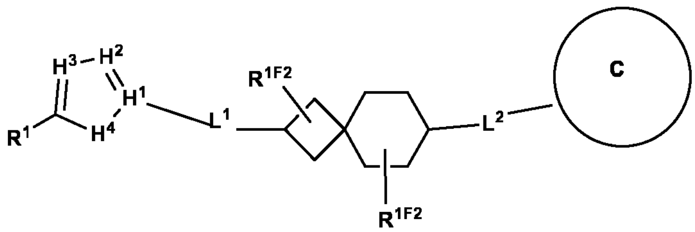

formula VII-1

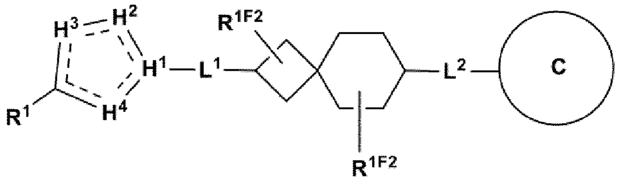

Formula VII-1'

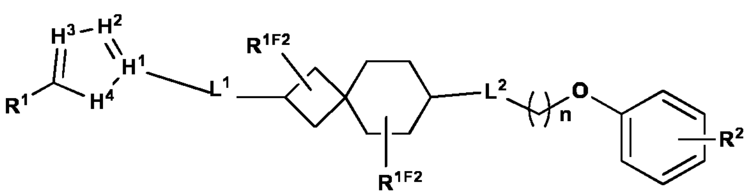

formula VII-2

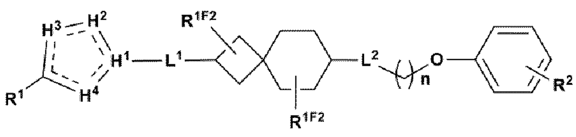

Formula VII-2'

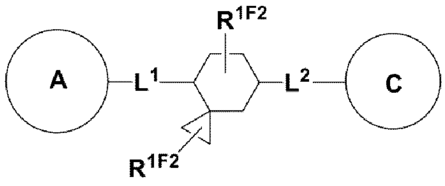

Formula VIII

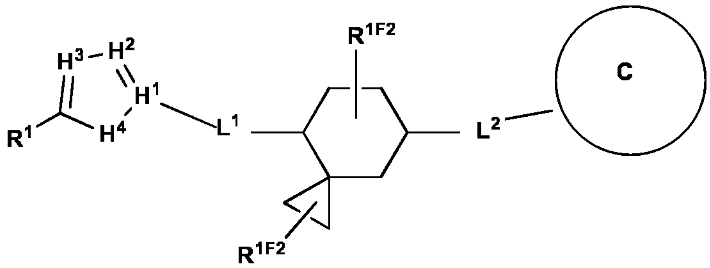

formula VIII-1

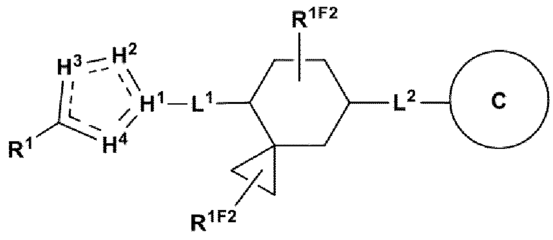

Formula VIII-1'

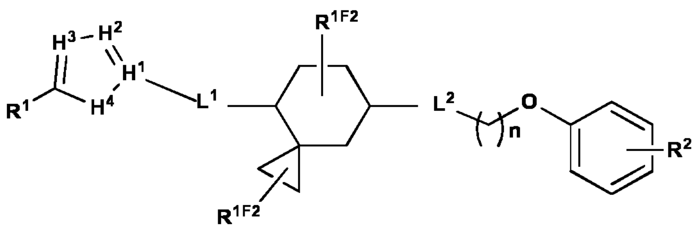

formula VIII-2

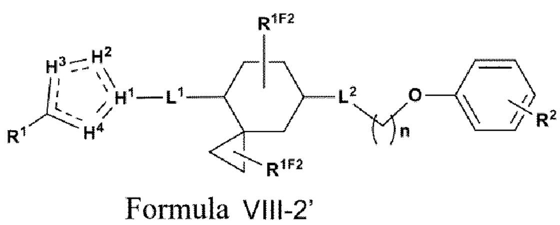

Formula VIII-2'

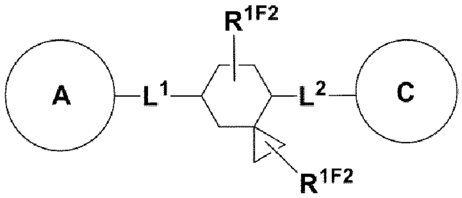

Formula IX

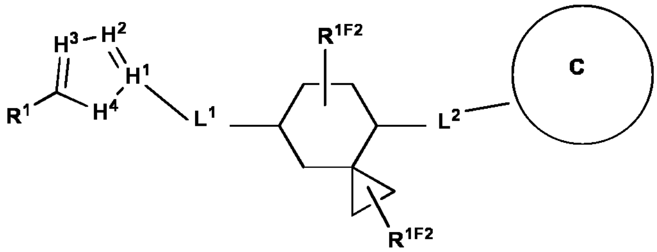

formula IX-1

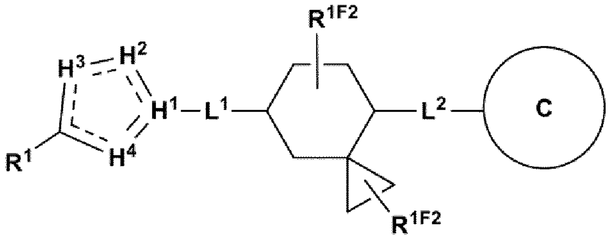

Formula IX-1'

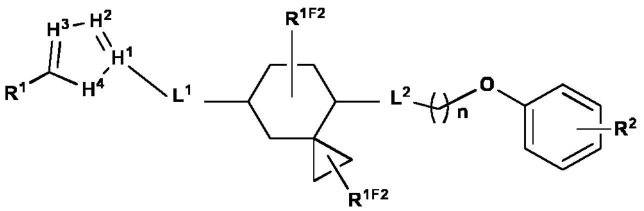

formula IX-2

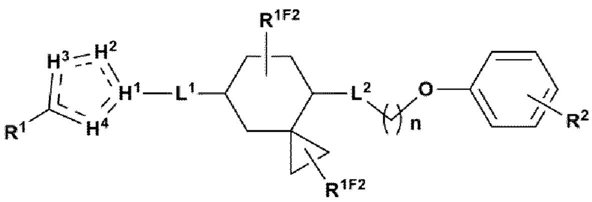

Formula IX-2'

;

the ring A, $L^1$, ring B, $L^2$, ring C, $R^1$, $R^{1F2}$ $H^1$, $H^2$, $H^3$, $H^4$, $R^2$, n and m are as defined in any of the previous schemes.

In some embodiments, the compound is specifically:

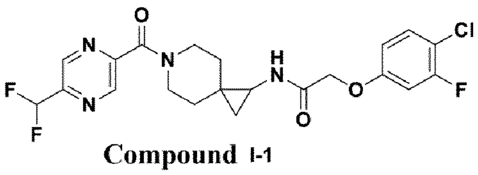

**Compound I-1**

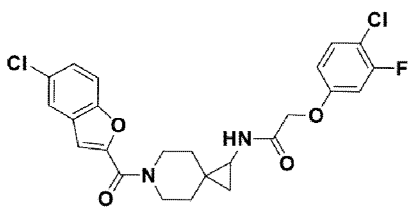

**Compound I-2**

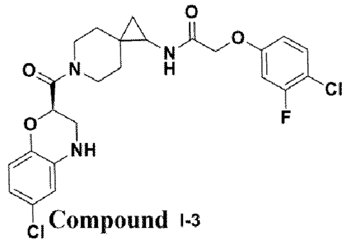
Compound I-3

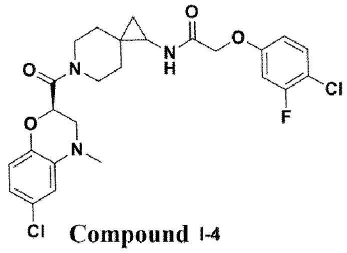
Compound I-4

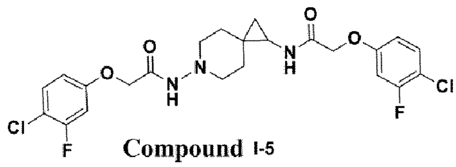
Compound I-5

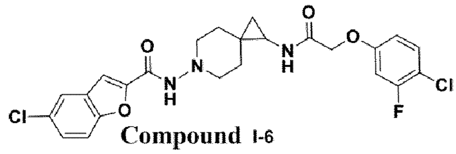
Compound I-6

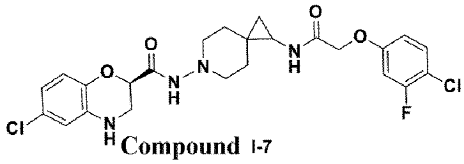
Compound I-7

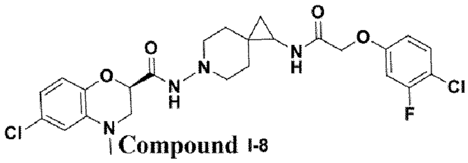
Compound I-8

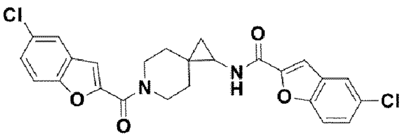
Compound I-9

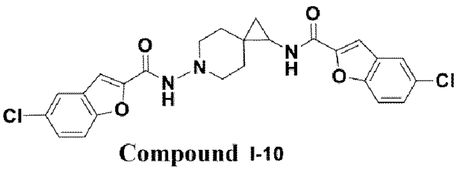
Compound I-10

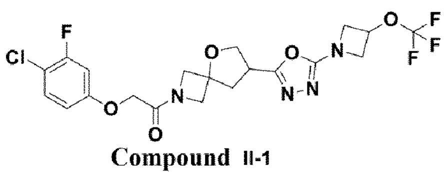
Compound II-1

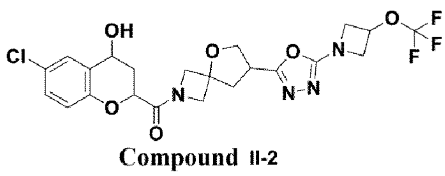
Compound II-2

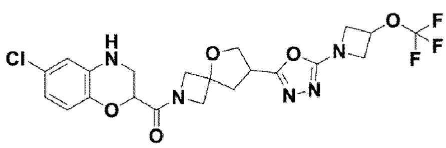
Compound II-3

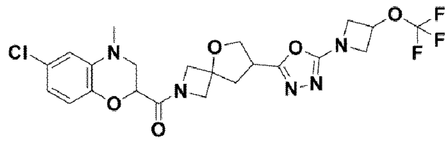
Compound II-4

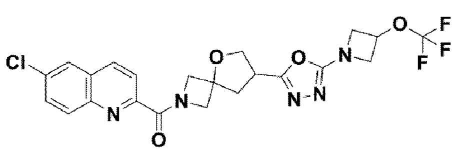
Compound II-5

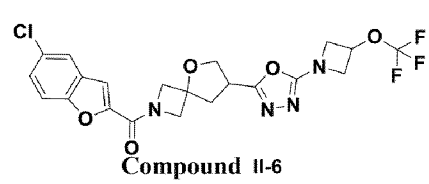
Compound II-6

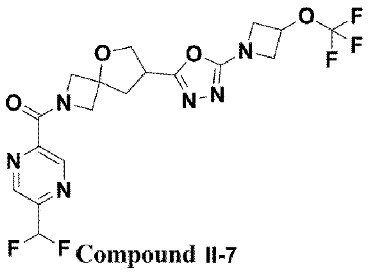

Compound II-7

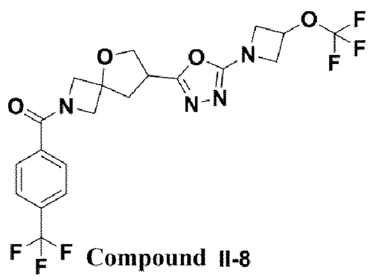

Compound II-8

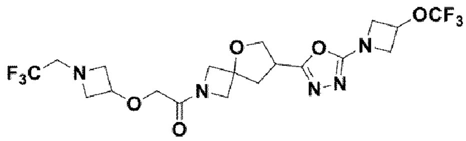

Compound II-9

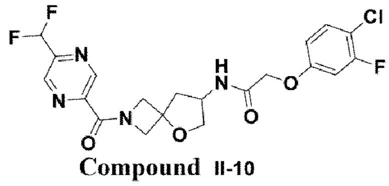

Compound II-10

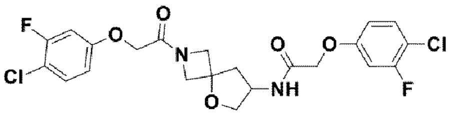

Compound II-11

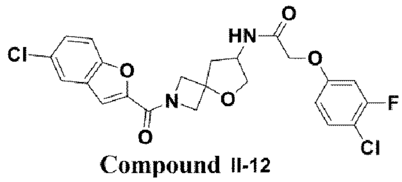

Compound II-12

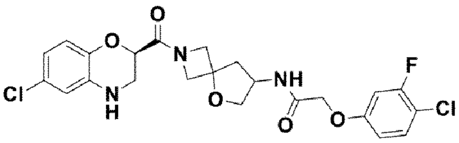

Compound II-13

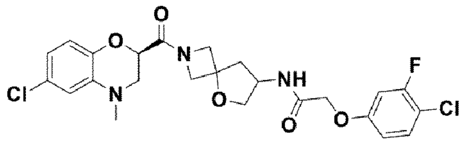

Compound II-14

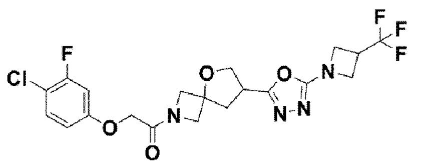

Compound II-15

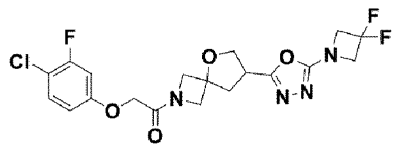

Compound II-16

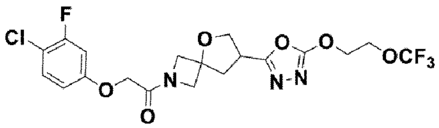

Compound II-17

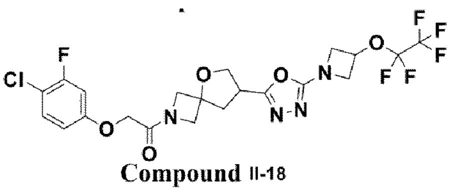

Compound II-18

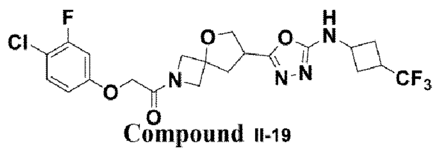

Compound II-19

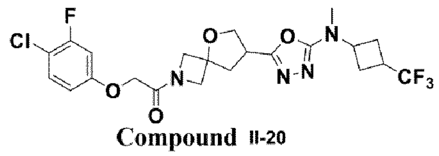

Compound II-20

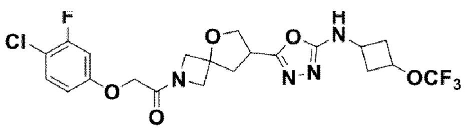

Compound II-21

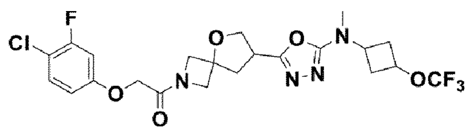

Compound II-22

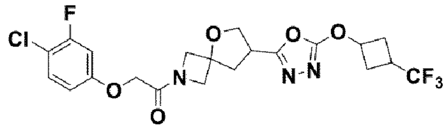

Compound II-23

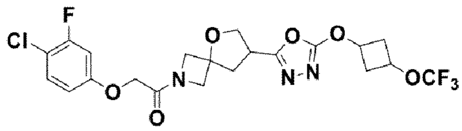

Compound II-24

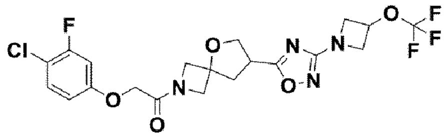

Compound II-25

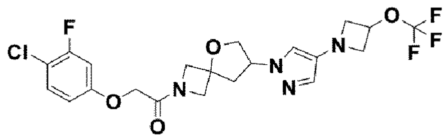

Compound II-26

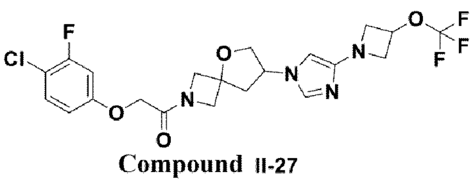

Compound II-27

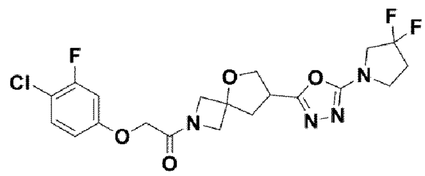

Compound II-28

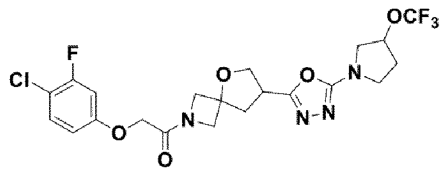

Compound II-29

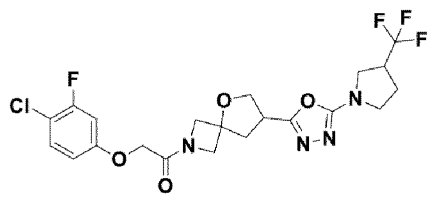

Compound II-30

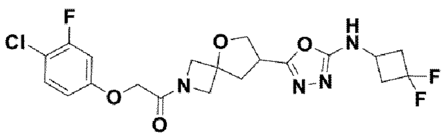

Compound II-31

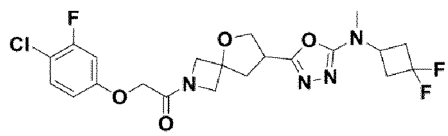

Compound II-32

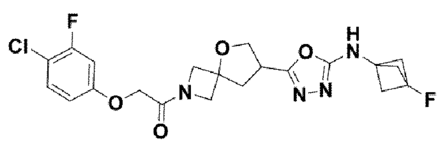

Compound II-33

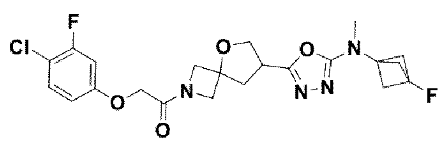

Compound II-34

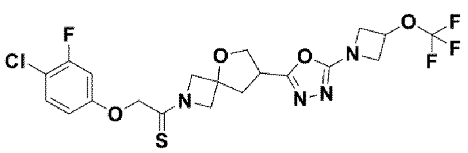

Compound II-35

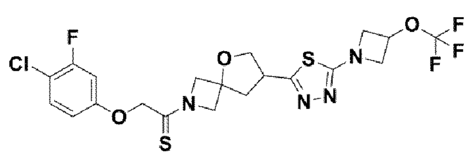

Compound II-36

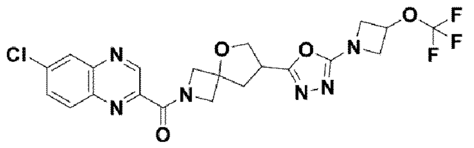
Compound II-37

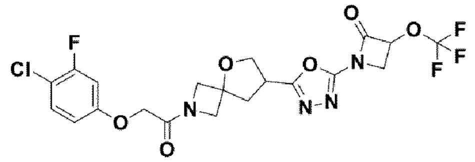
Compound II-38

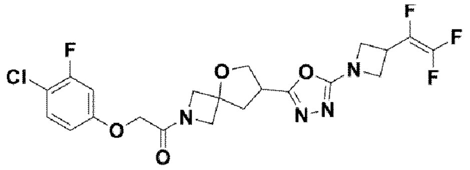
Compound II-39

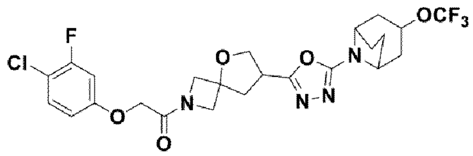
Compound II-40

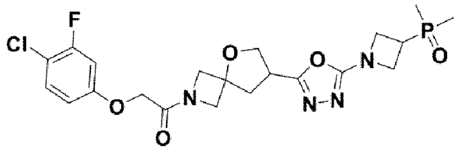
Compound II-41

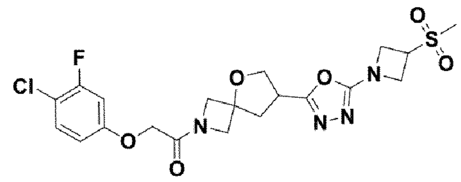
Compound II-42

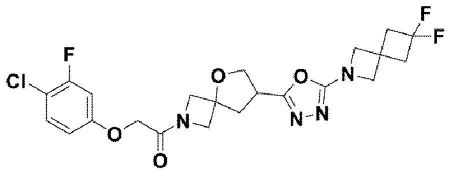
Compound II-43

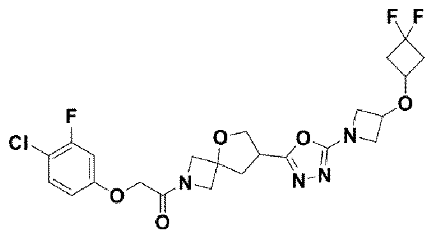
Compound II-44

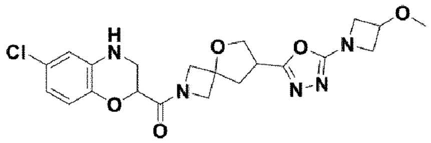
Compound II-45

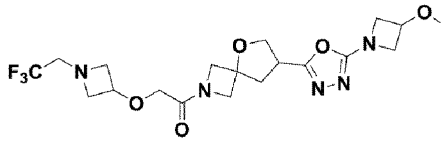
Compound II-46

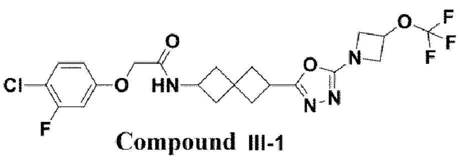
Compound III-1

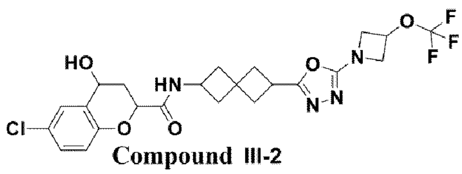
Compound III-2

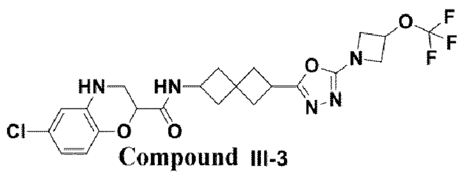
Compound III-3

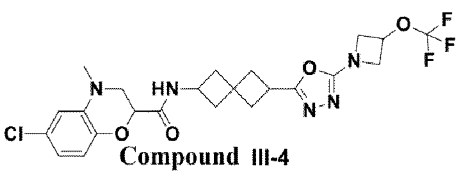
Compound III-4

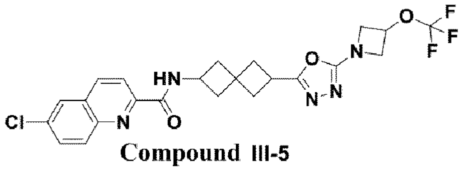

**Compound III-5**

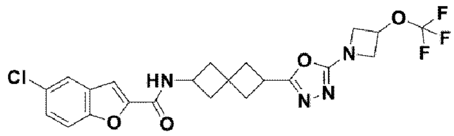

**Compound III-6**

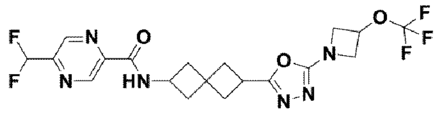

**Compound III-7**

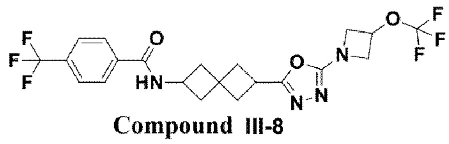

**Compound III-8**

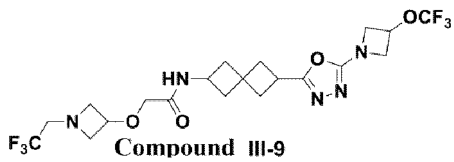

**Compound III-9**

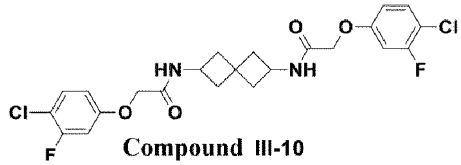

**Compound III-10**

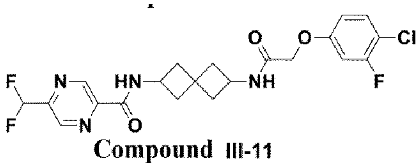

**Compound III-11**

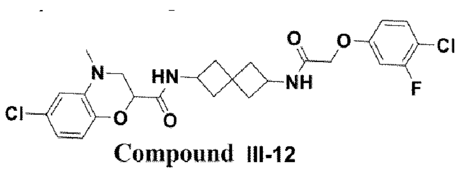

**Compound III-12**

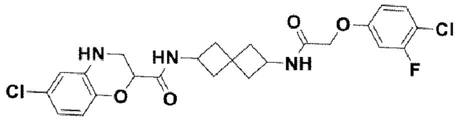

**Compound III-13**

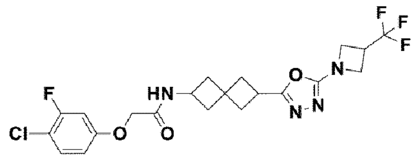

**Compound III-14**

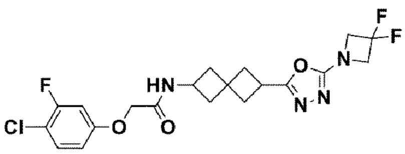

**Compound III-15**

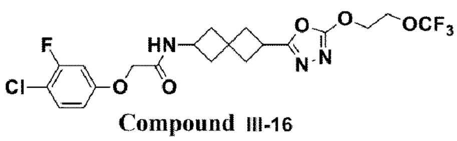

**Compound III-16**

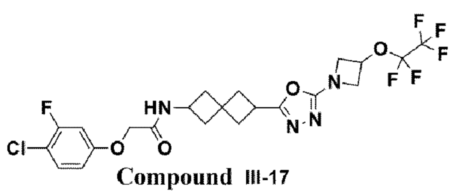

**Compound III-17**

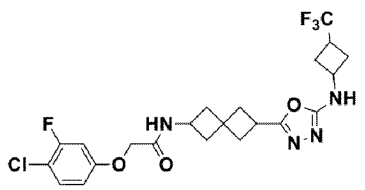

**Compound III-18**

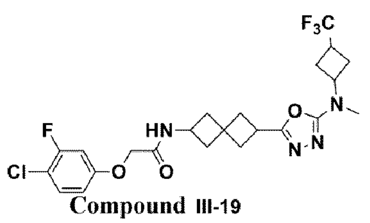

**Compound III-19**

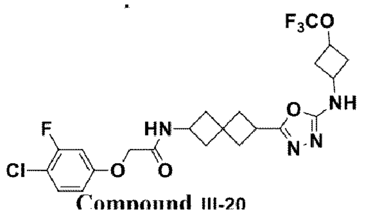

**Compound III-20**

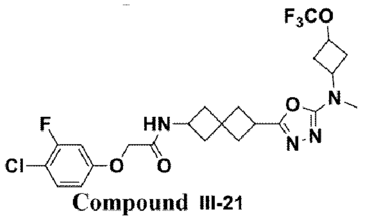
Compound III-21

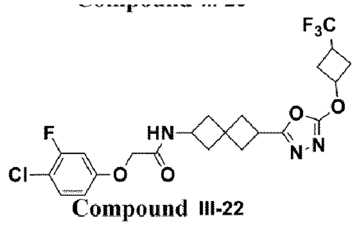
Compound III-22

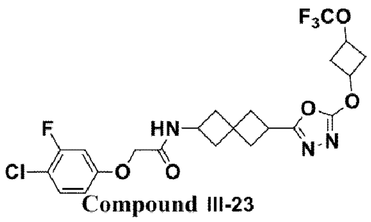
Compound III-23

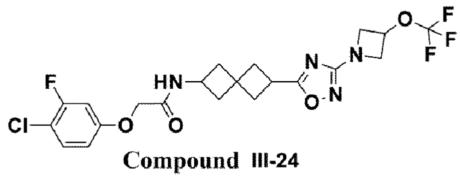
Compound III-24

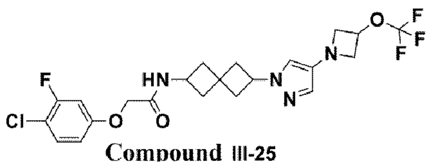
Compound III-25

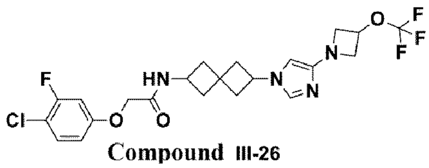
Compound III-26

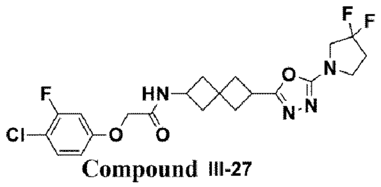
Compound III-27

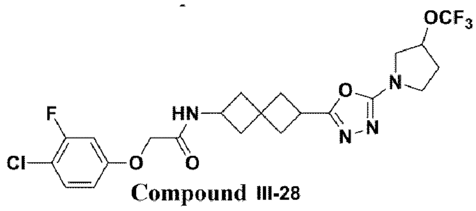
Compound III-28

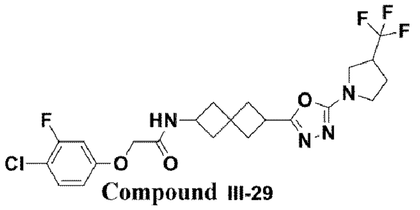
Compound III-29

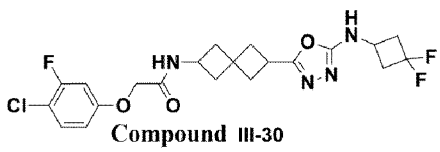
Compound III-30

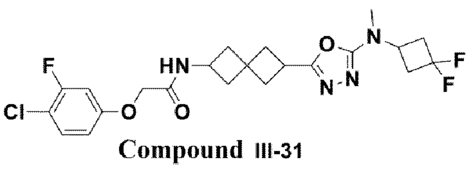
Compound III-31

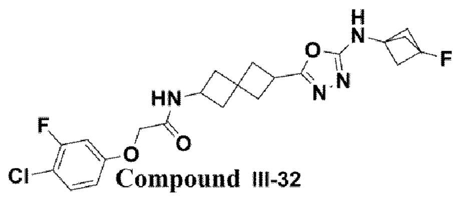
Compound III-32

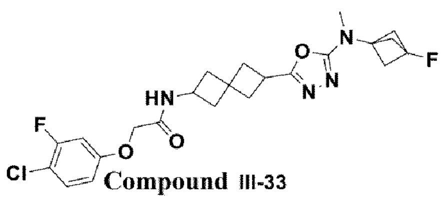
Compound III-33

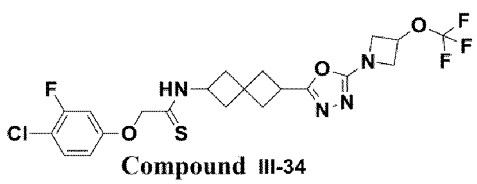
Compound III-34

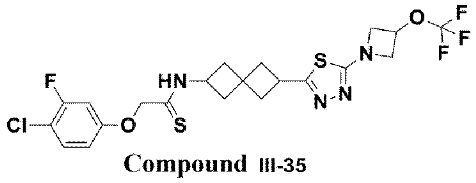
Compound III-35

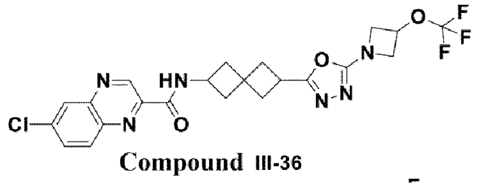
Compound III-36

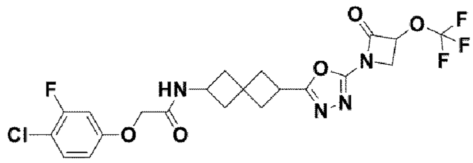
Compound III-37

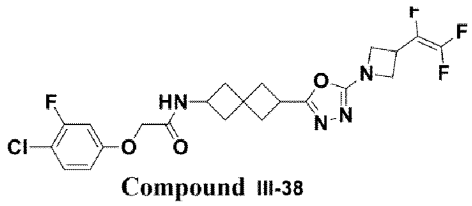
Compound III-38

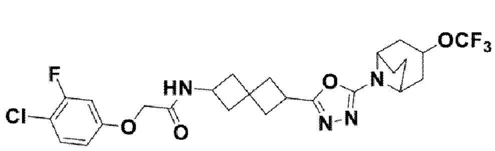
Compound III-39

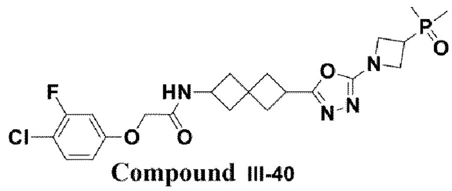
Compound III-40

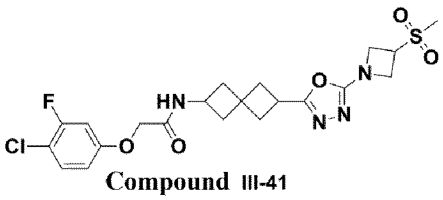
Compound III-41

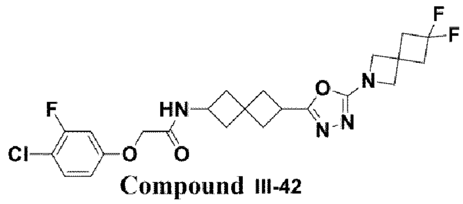
Compound III-42

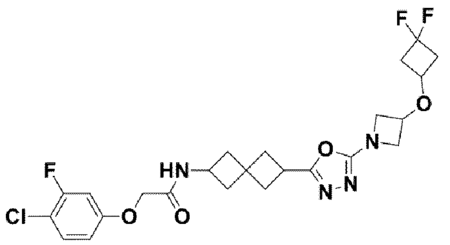
Compound III-43

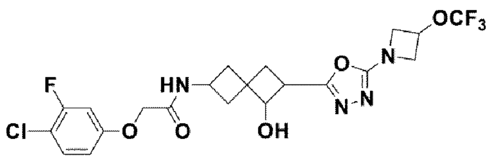
Compound III-44

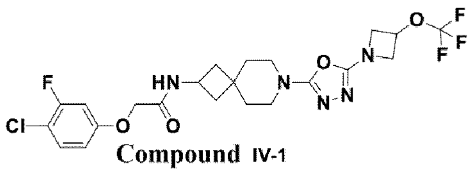
Compound IV-1

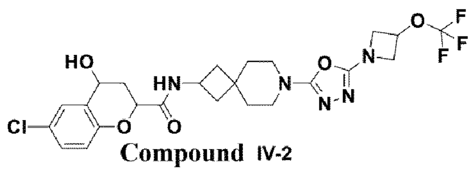
Compound IV-2

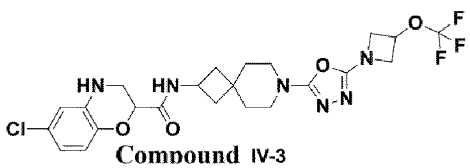
Compound IV-3

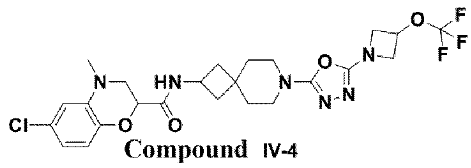
Compound IV-4

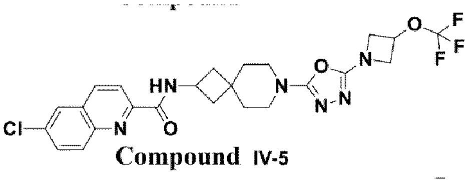

Compound IV-5

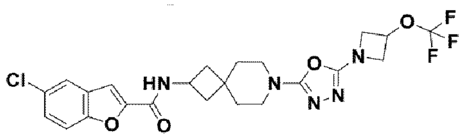

Compound IV-6

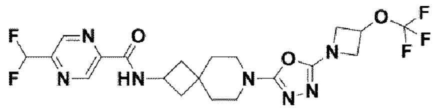

Compound IV-7

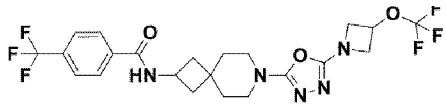

Compound IV-8

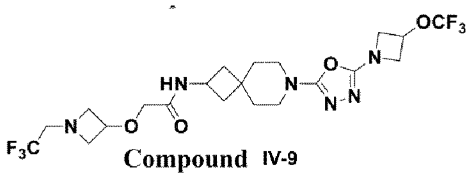

Compound IV-9

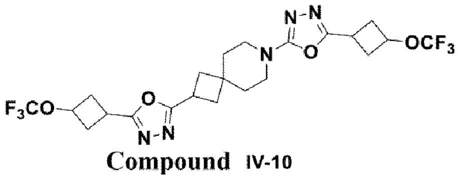

Compound IV-10

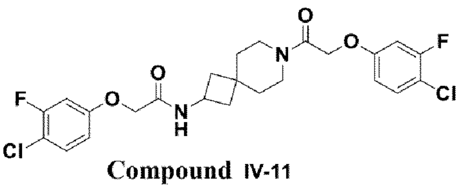

Compound IV-11

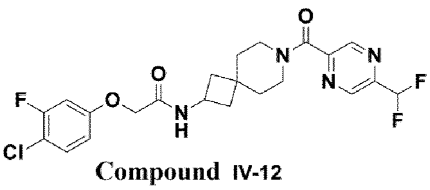

Compound IV-12

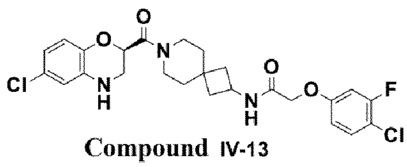

Compound IV-13

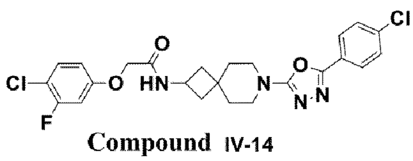

Compound IV-14

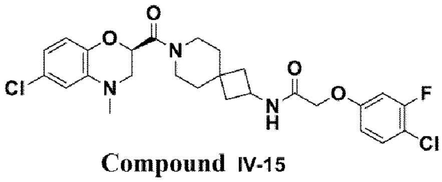

Compound IV-15

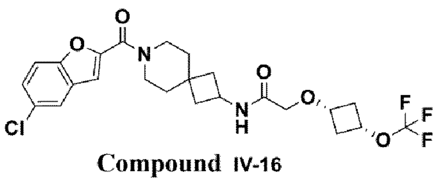

Compound IV-16

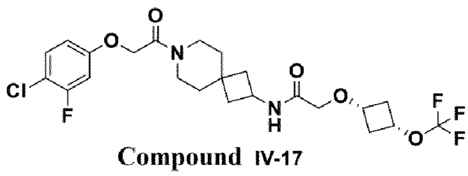

Compound IV-17

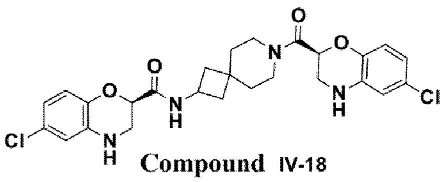

Compound IV-18

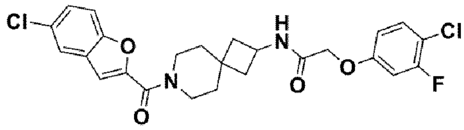

Compound IV-19

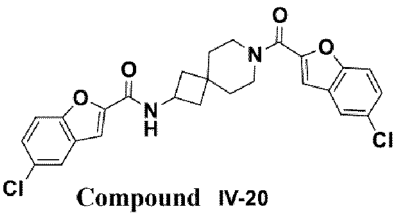

Compound IV-20

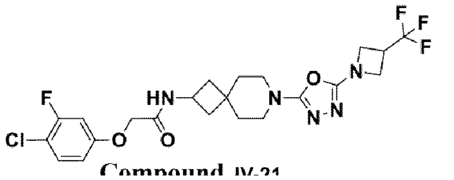

Compound IV-21

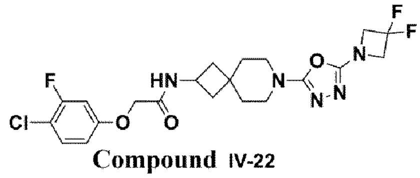

Compound IV-22

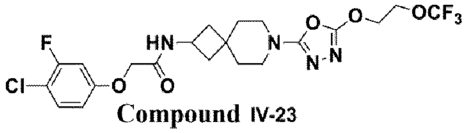

Compound IV-23

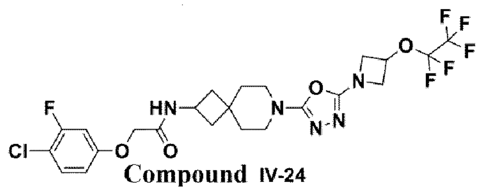

Compound IV-24

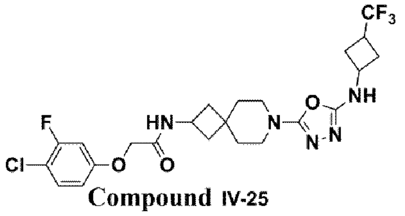

Compound IV-25

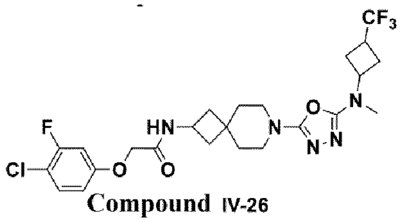

Compound IV-26

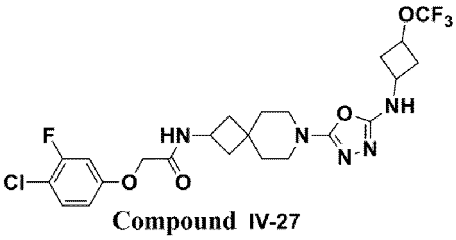

Compound IV-27

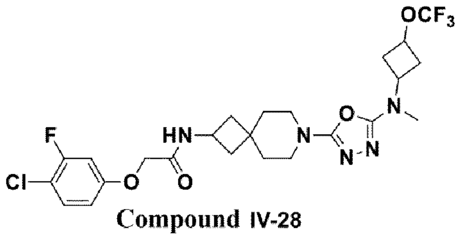

Compound IV-28

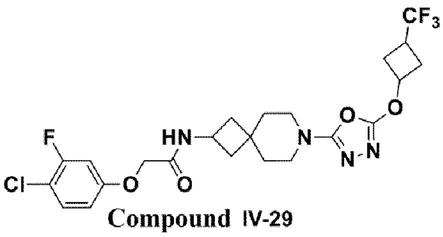

Compound IV-29

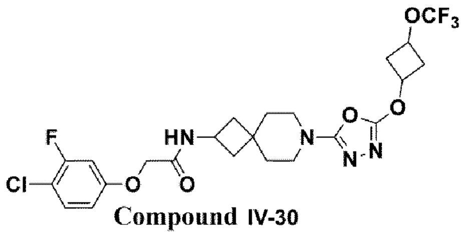

Compound IV-30

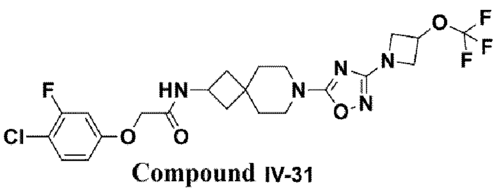

Compound IV-31

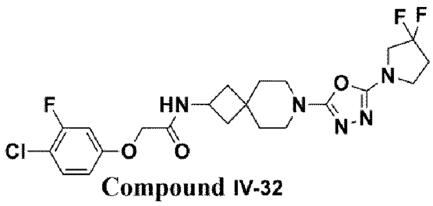

Compound IV-32

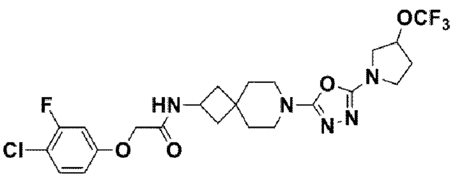

Compound IV-33

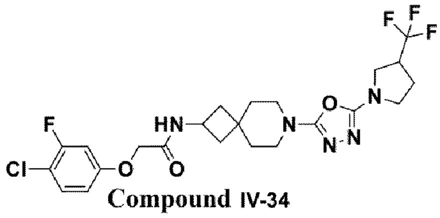

Compound IV-34

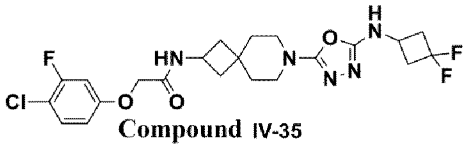
Compound IV-35

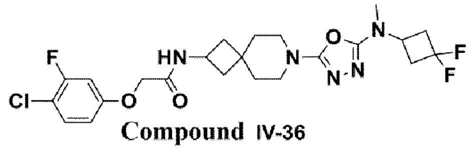
Compound IV-36

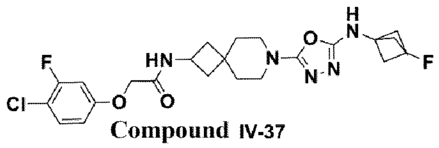
Compound IV-37

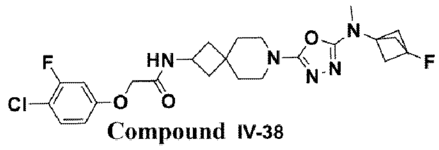
Compound IV-38

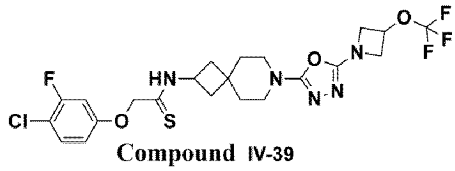
Compound IV-39

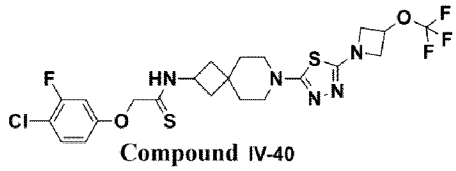
Compound IV-40

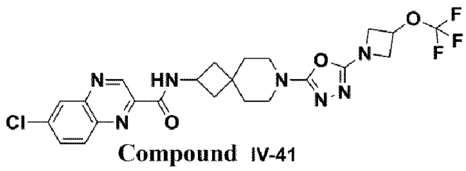
Compound IV-41

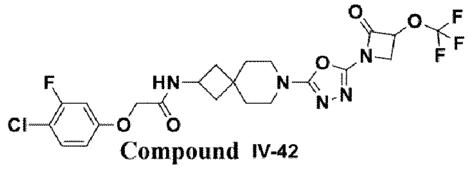
Compound IV-42

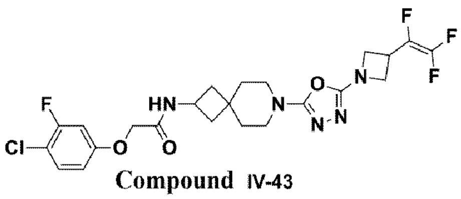
Compound IV-43

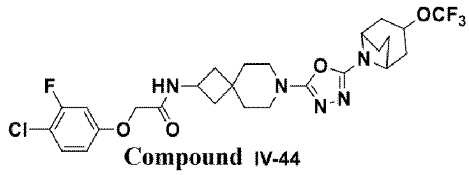
Compound IV-44

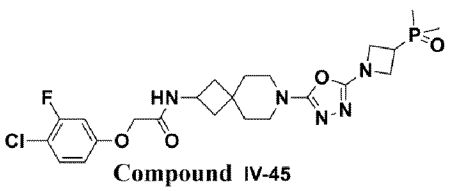
Compound IV-45

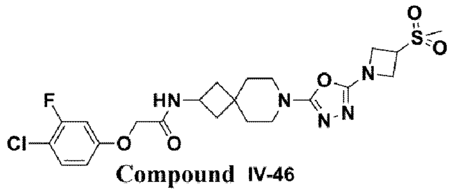
Compound IV-46

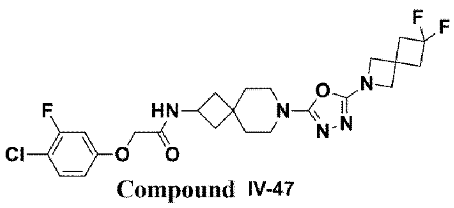
Compound IV-47

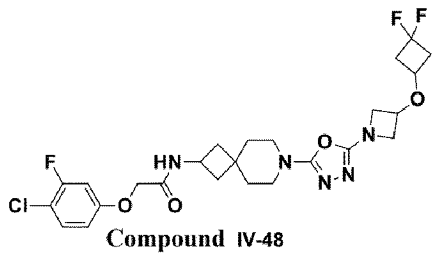
Compound IV-48

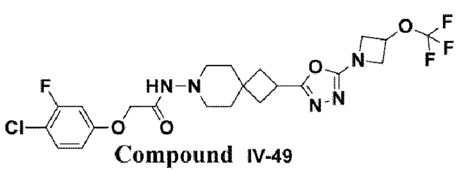
Compound IV-49

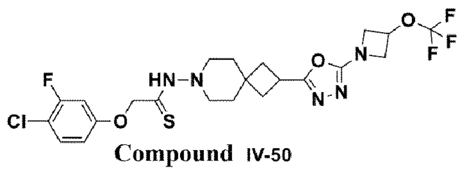
Compound IV-50

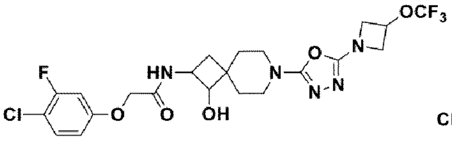
Compound IV-51

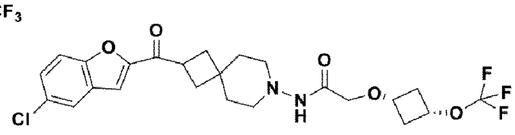
Compound IV-52

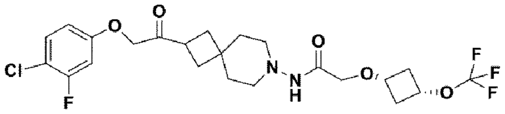
Compound IV-53

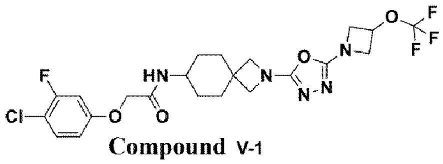
Compound V-1

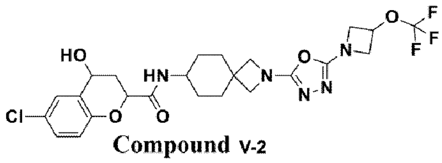
Compound V-2

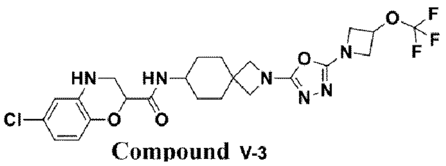
Compound V-3

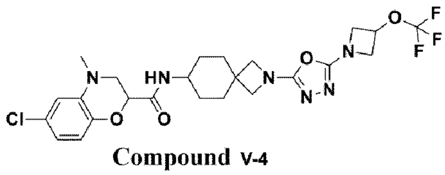
Compound V-4

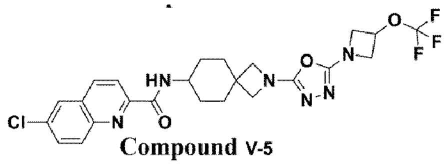
Compound V-5

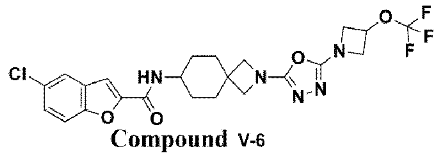
Compound V-6

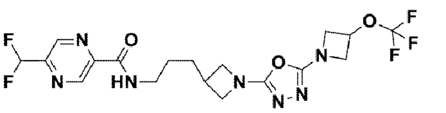
Compound V-7

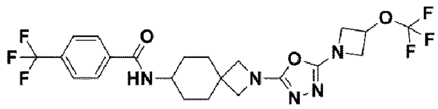
Compound V-8

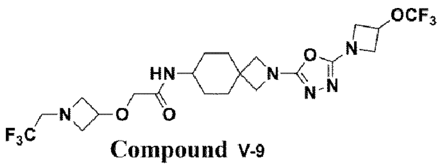
Compound V-9

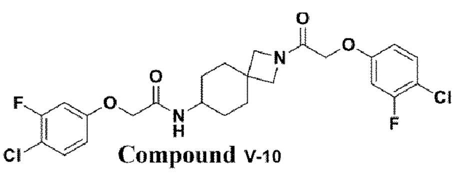
Compound V-10

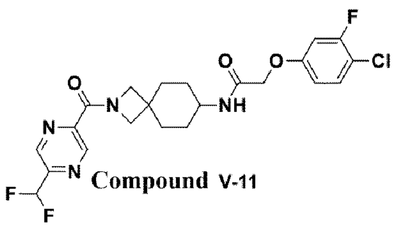
Compound V-11

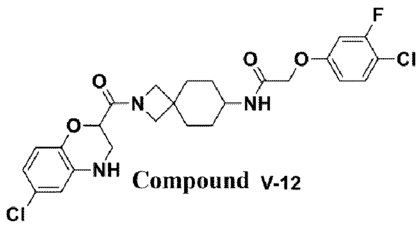
Compound V-12

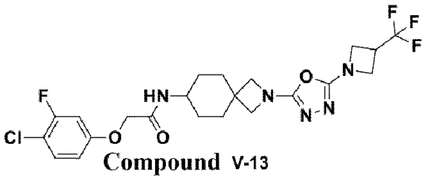

Compound V-13

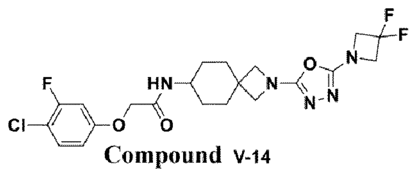

Compound V-14

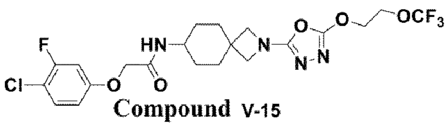

Compound V-15

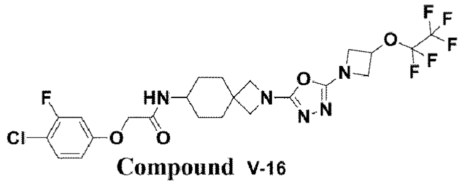

Compound V-16

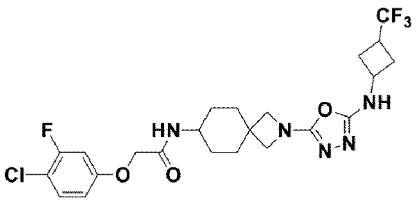

Compound V-17

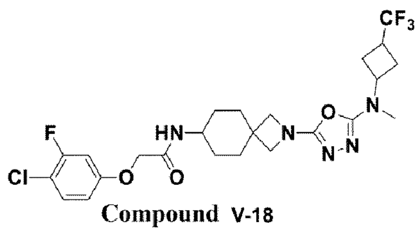

Compound V-18

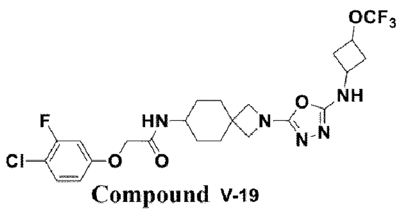

Compound V-19

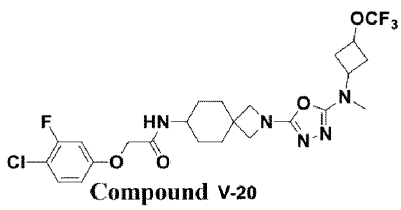

Compound V-20

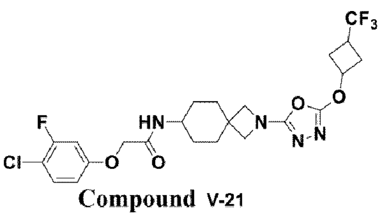

Compound V-21

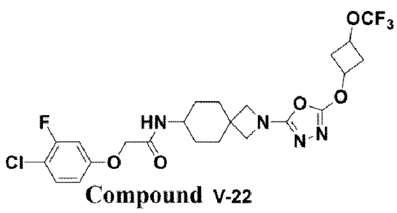

Compound V-22

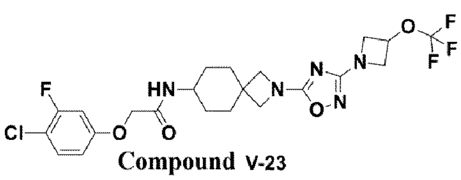

Compound V-23

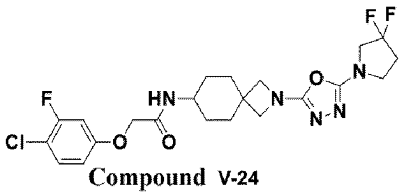

Compound V-24

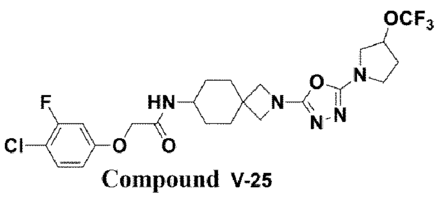

Compound V-25

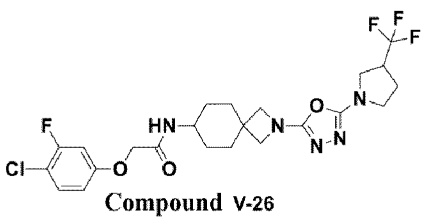

Compound V-26

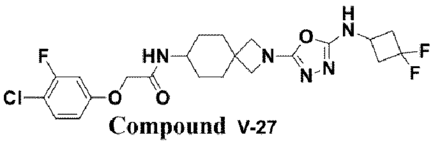
Compound V-27

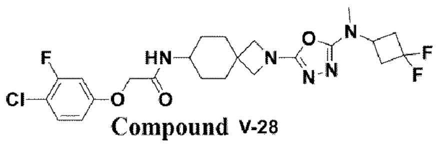
Compound V-28

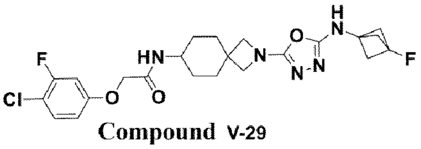
Compound V-29

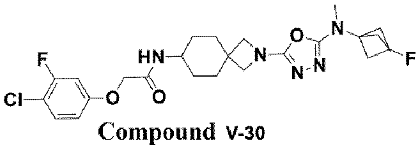
Compound V-30

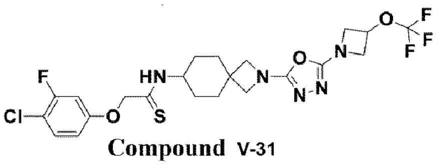
Compound V-31

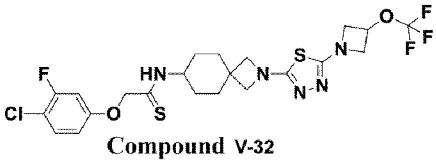
Compound V-32

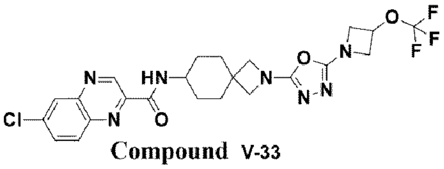
Compound V-33

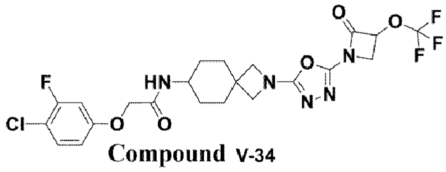
Compound V-34

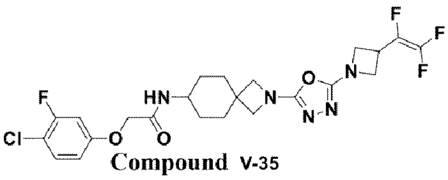
Compound V-35

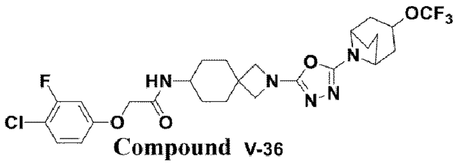
Compound V-36

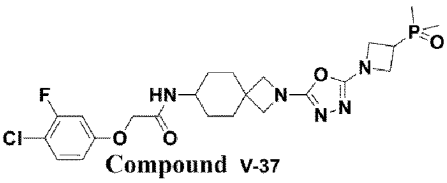
Compound V-37

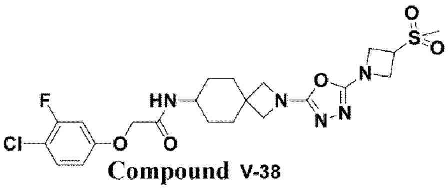
Compound V-38

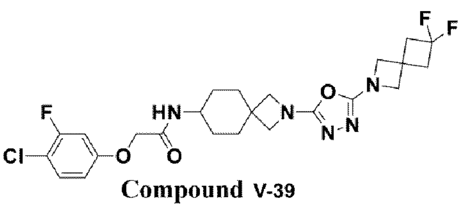
Compound V-39

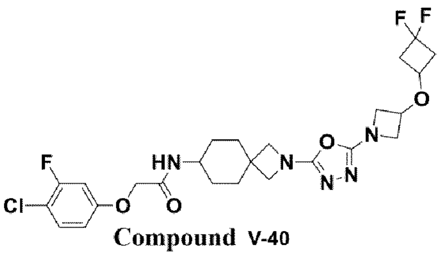
Compound V-40

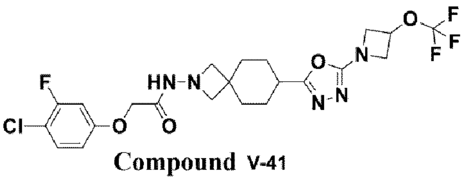
Compound V-41

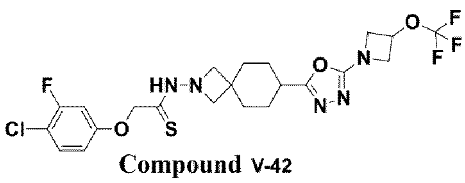
Compound V-42

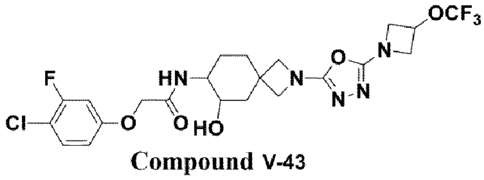
Compound V-43

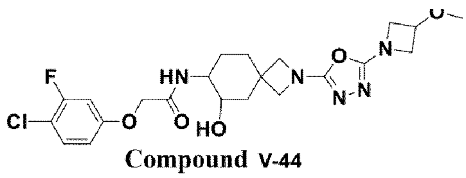
Compound V-44

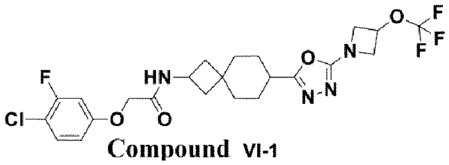
Compound VI-1

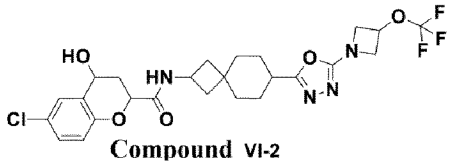
Compound VI-2

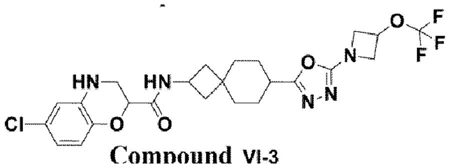
Compound VI-3

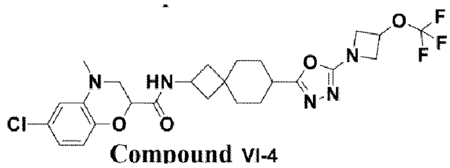
Compound VI-4

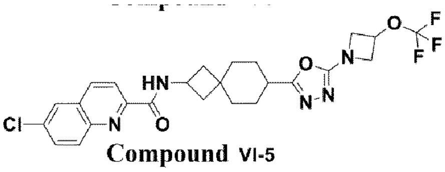
Compound VI-5

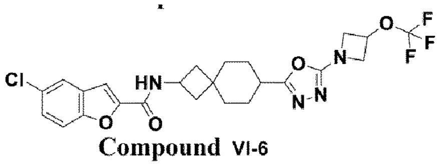
Compound VI-6

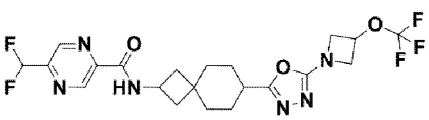
Compound VI-7

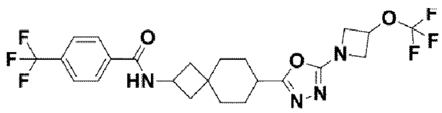
Compound VI-8

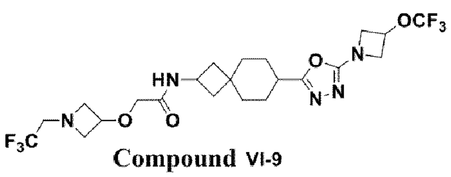
Compound VI-9

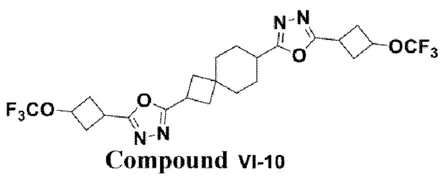
Compound VI-10

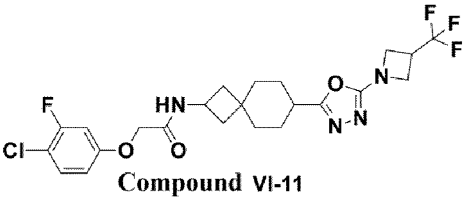
Compound VI-11

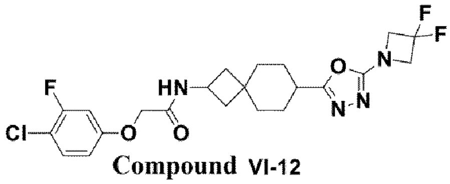
Compound VI-12

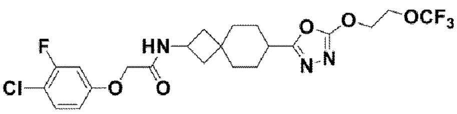
Compound VI-13

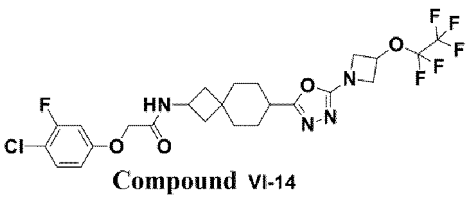
Compound VI-14

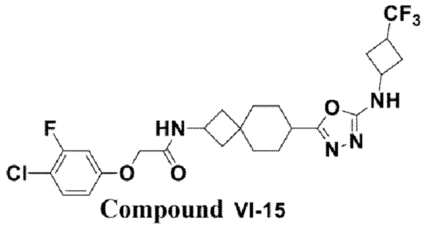

Compound VI-15

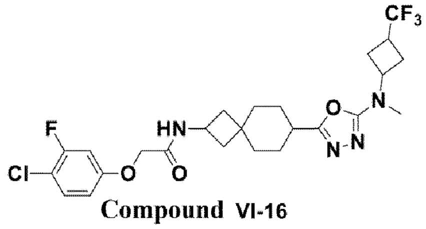

Compound VI-16

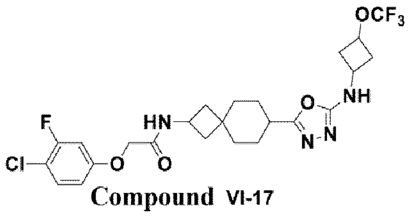

Compound VI-17

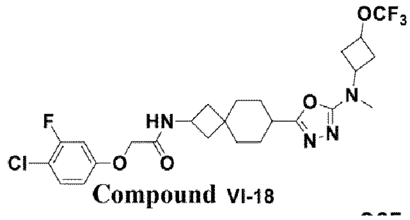

Compound VI-18

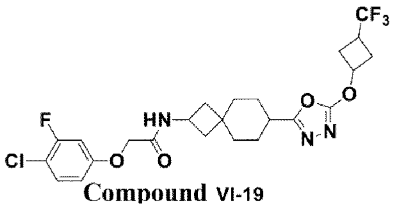

Compound VI-19

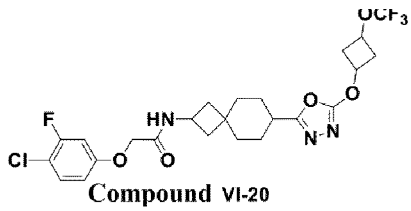

Compound VI-20

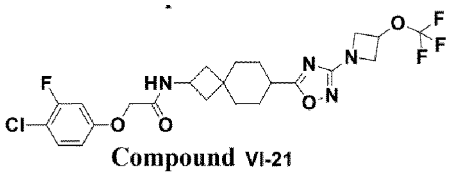

Compound VI-21

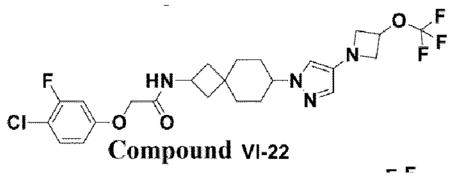

Compound VI-22

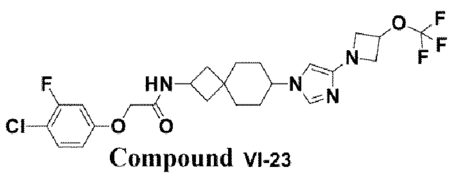

Compound VI-23

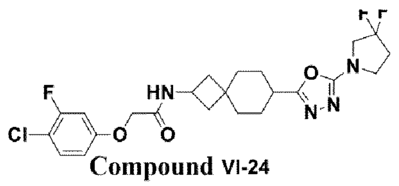

Compound VI-24

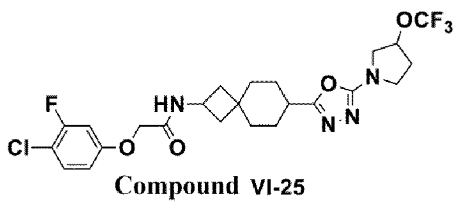

Compound VI-25

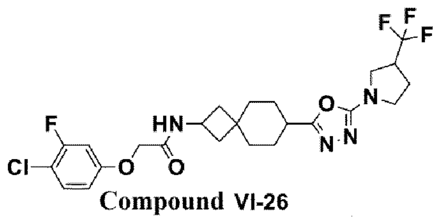

Compound VI-26

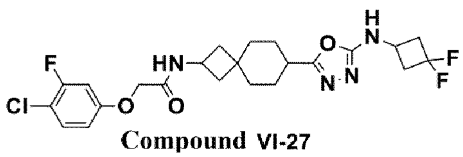

Compound VI-27

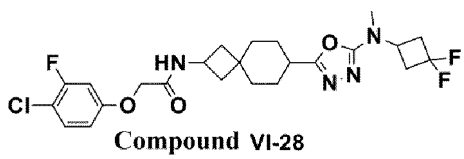

Compound VI-28

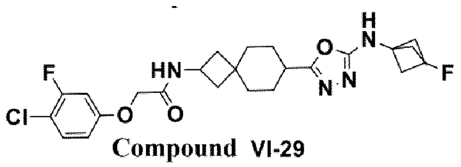
Compound VI-29

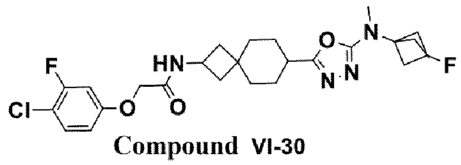
Compound VI-30

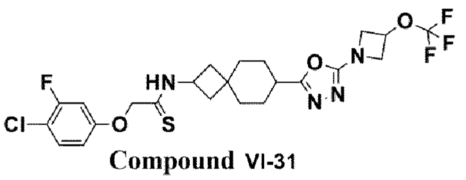
Compound VI-31

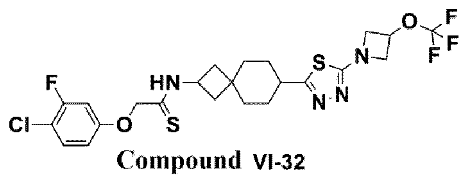
Compound VI-32

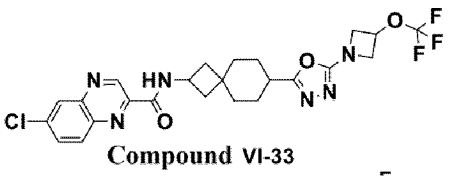
Compound VI-33

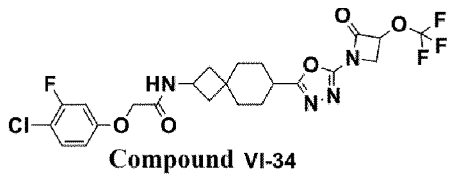
Compound VI-34

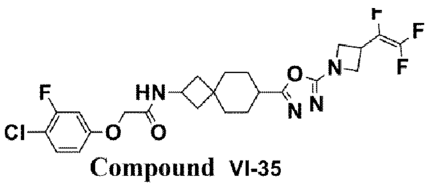
Compound VI-35

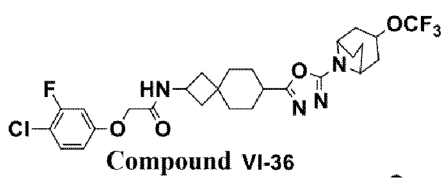
Compound VI-36

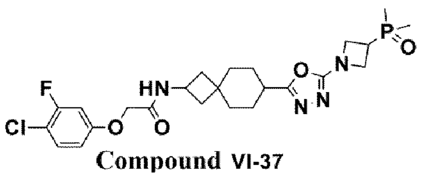
Compound VI-37

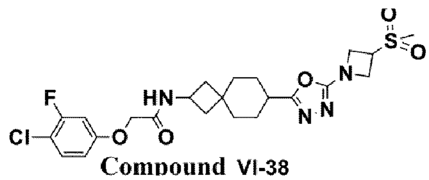
Compound VI-38

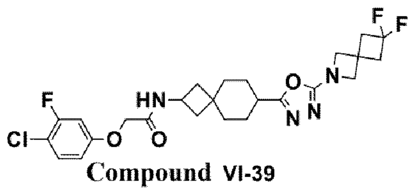
Compound VI-39

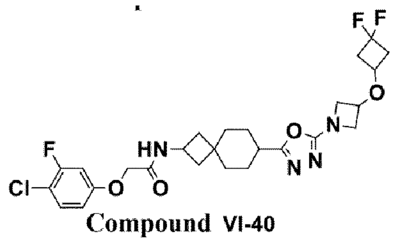
Compound VI-40

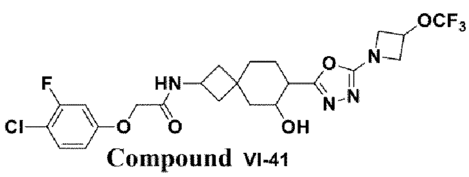
Compound VI-41

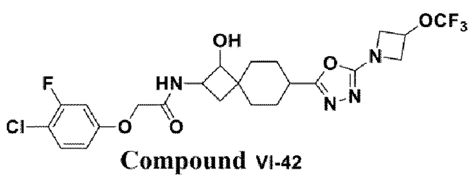
Compound VI-42

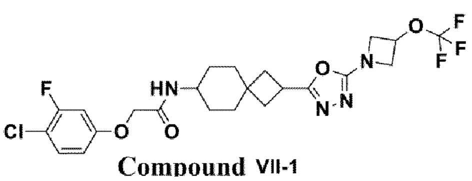
Compound VII-1

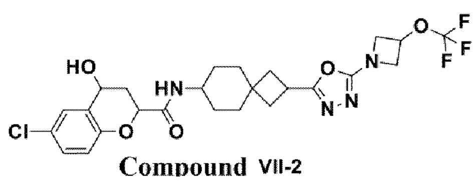
Compound VII-2

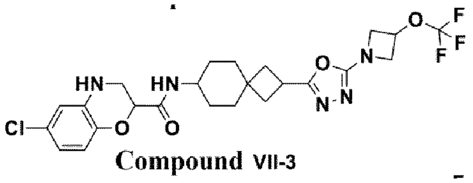
Compound VII-3

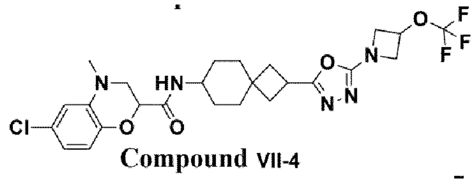
Compound VII-4

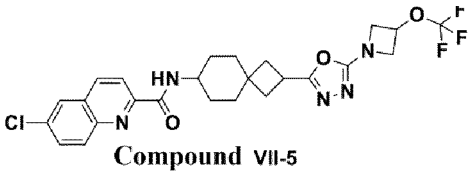
Compound VII-5

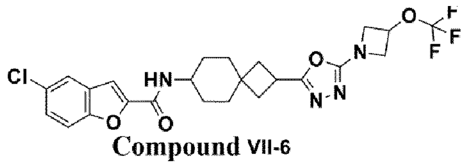
Compound VII-6

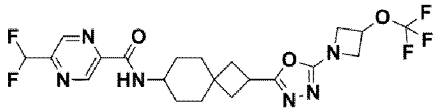
Compound VII-7

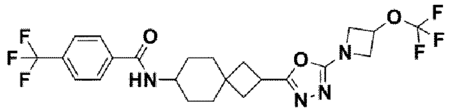
Compound VII-8

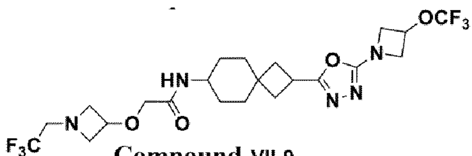
Compound VII-9

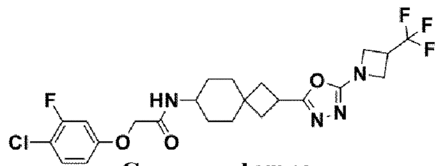
Compound VII-10

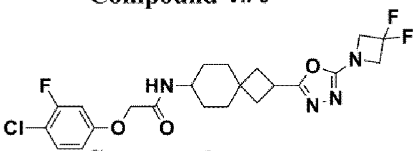
Compound VII-11

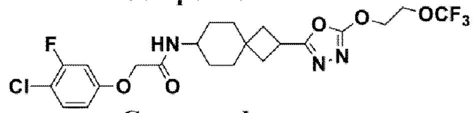
Compound VII-12

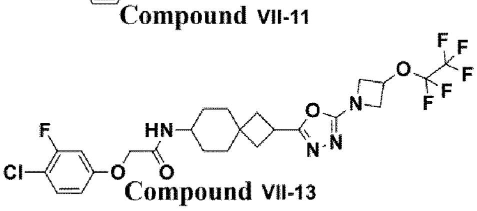
Compound VII-13

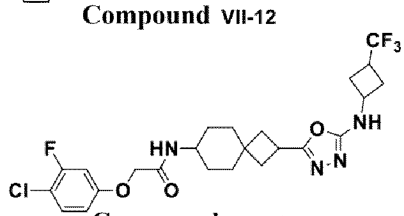
Compound VII-14

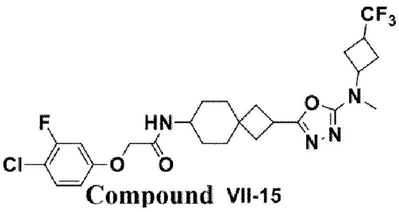
Compound VII-15

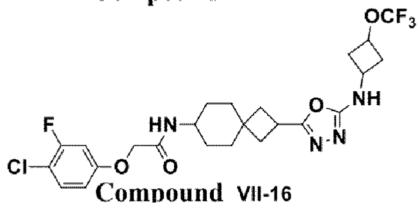
Compound VII-16

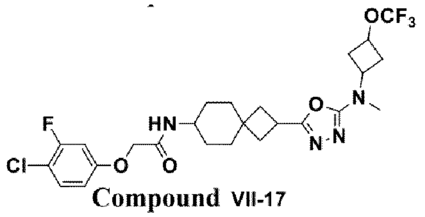
Compound VII-17

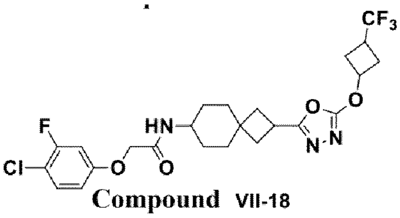
Compound VII-18

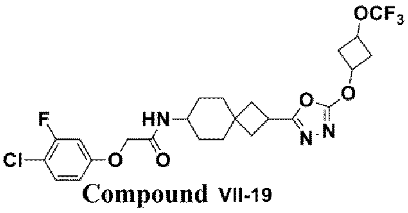

Compound VII-19

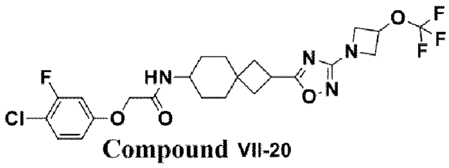

Compound VII-20

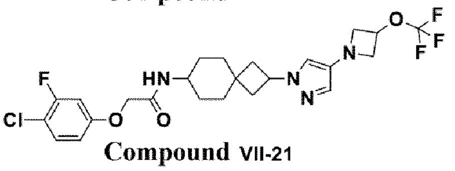

Compound VII-21

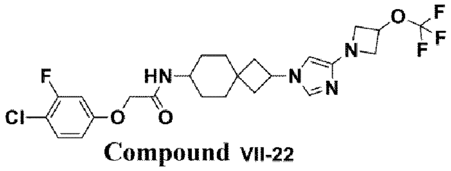

Compound VII-22

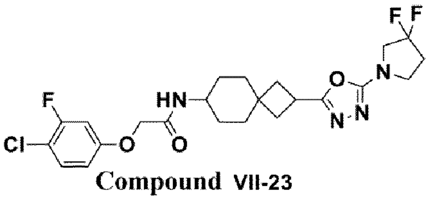

Compound VII-23

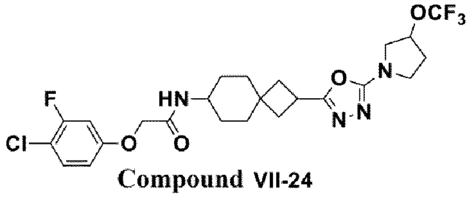

Compound VII-24

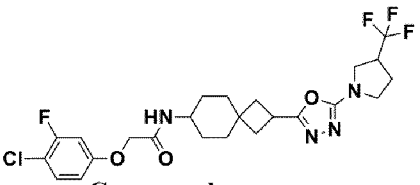

Compound VII-25

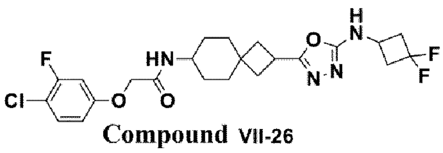

Compound VII-26

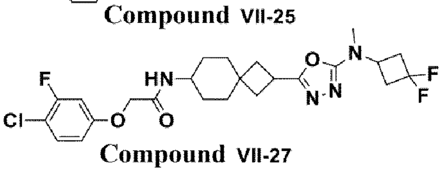

Compound VII-27

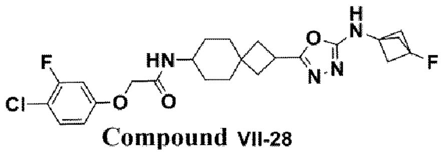

Compound VII-28

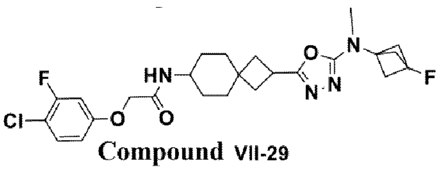

Compound VII-29

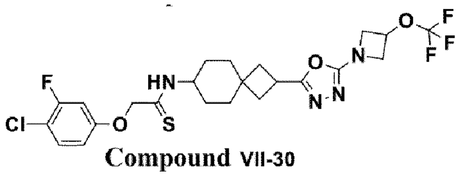

Compound VII-30

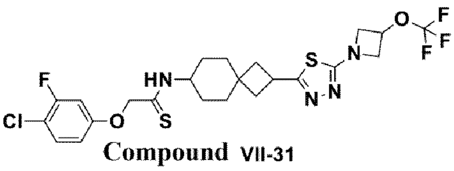

Compound VII-31

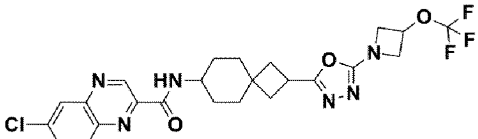

Compound VII-32

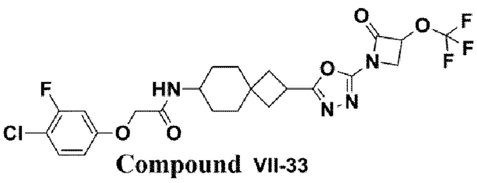

Compound VII-33

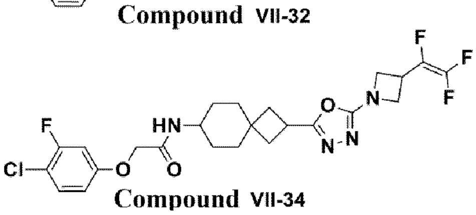

Compound VII-34

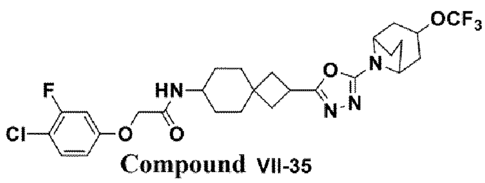

Compound VII-35

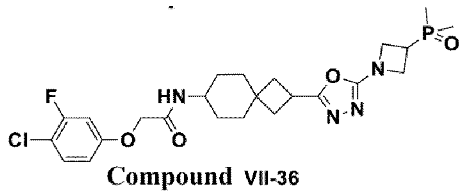

Compound VII-36

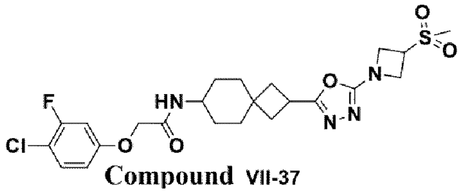

Compound VII-37

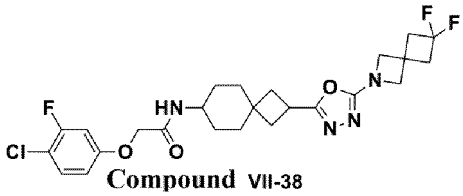

Compound VII-38

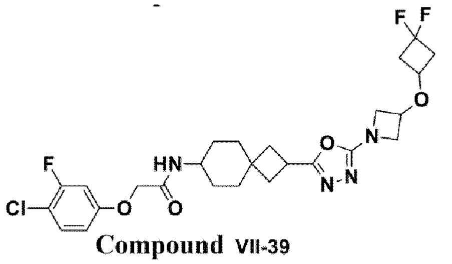

Compound VII-39

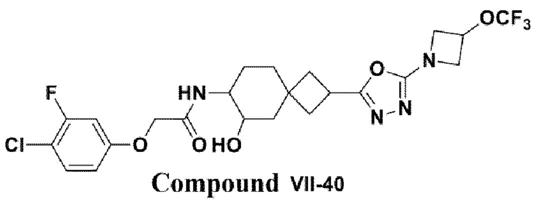

Compound VII-40

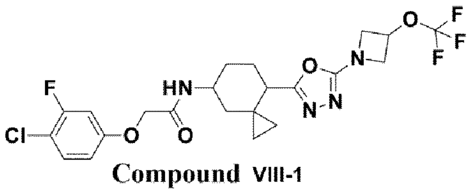

Compound VIII-1

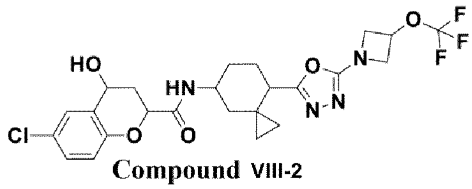

Compound VIII-2

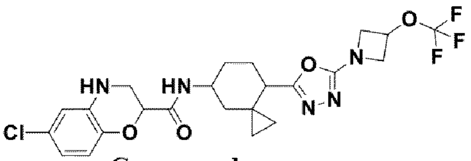

Compound VIII-3

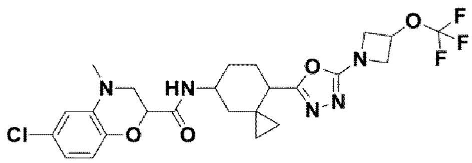

Compound VIII-4

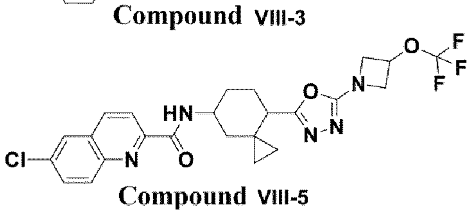

Compound VIII-5

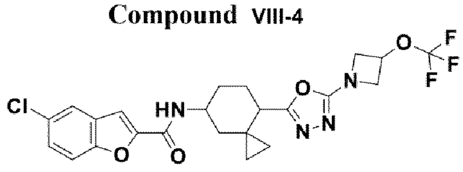

Compound VIII-6

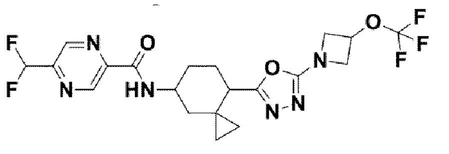

Compound VIII-7

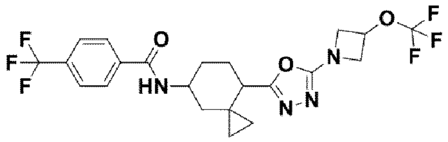

Compound VIII-8

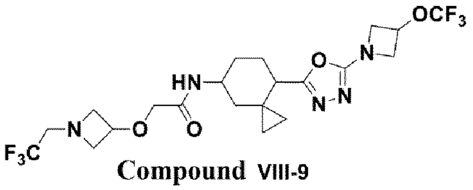

Compound VIII-9

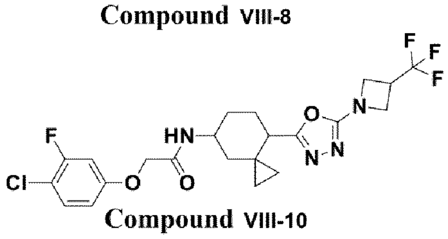

Compound VIII-10

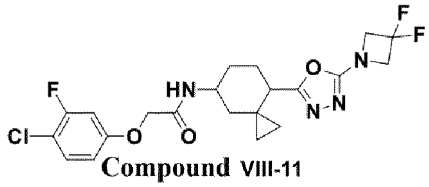

Compound VIII-11

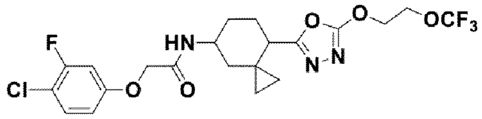

Compound VIII-12

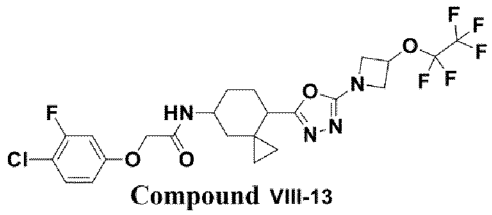

Compound VIII-13

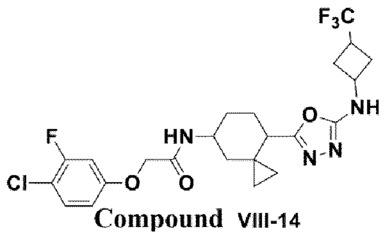

Compound VIII-14

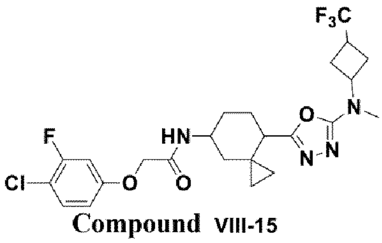

Compound VIII-15

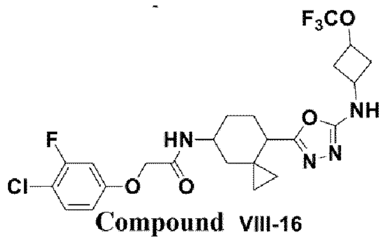

Compound VIII-16

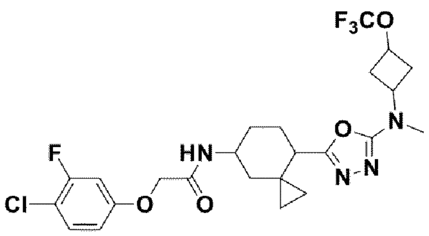

Compound VIII-17

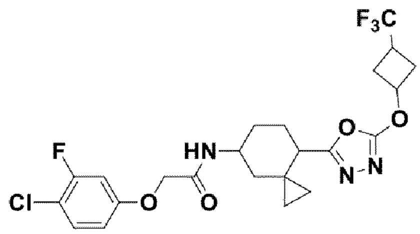

Compound VIII-18

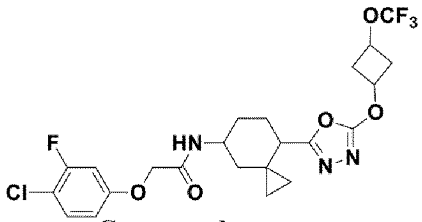
Compound VIII-19

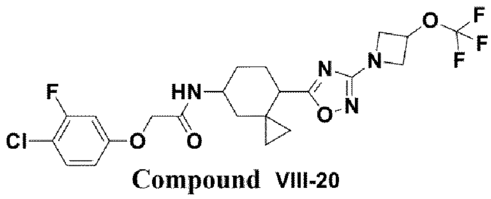
Compound VIII-20

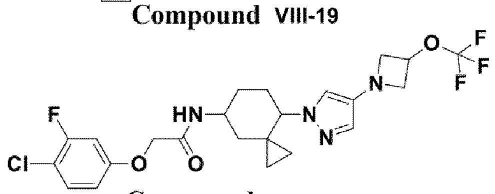
Compound VIII-21

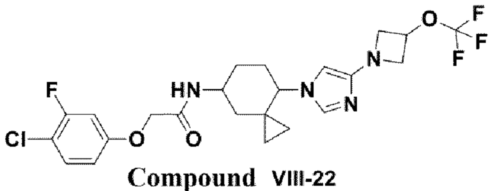
Compound VIII-22

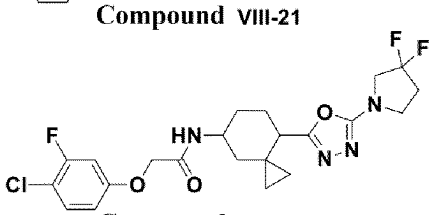
Compound VIII-23

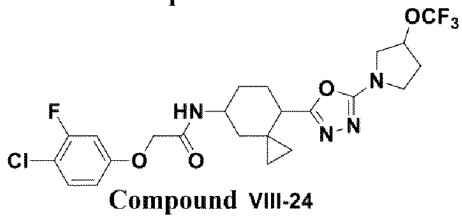
Compound VIII-24

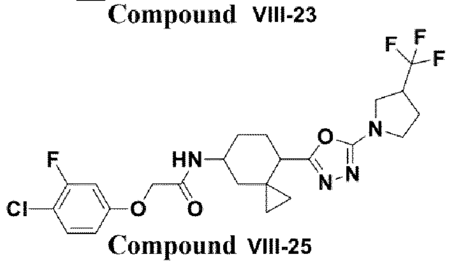
Compound VIII-25

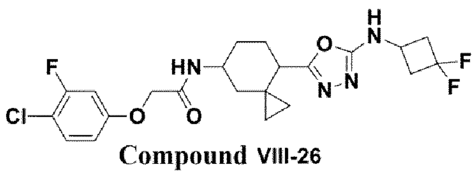
Compound VIII-26

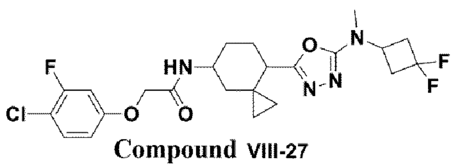
Compound VIII-27

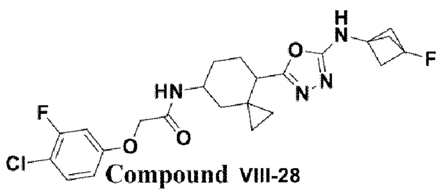
Compound VIII-28

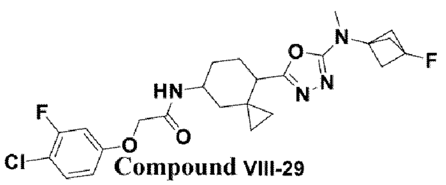
Compound VIII-29

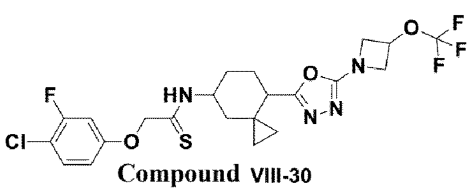
Compound VIII-30

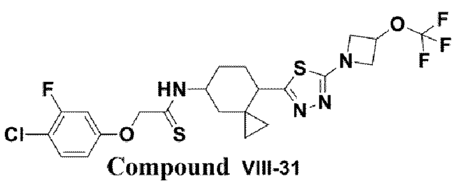
Compound VIII-31

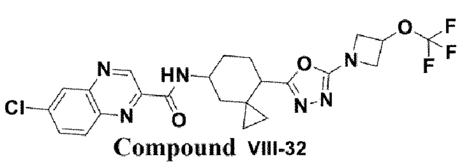
Compound VIII-32

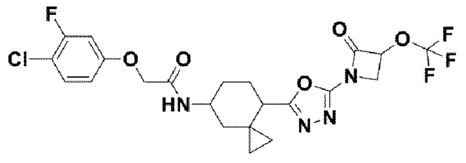
Compound VIII-33

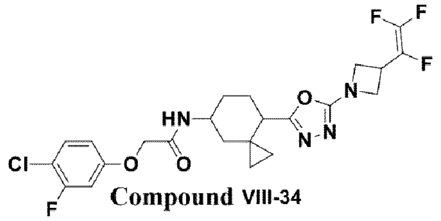
Compound VIII-34

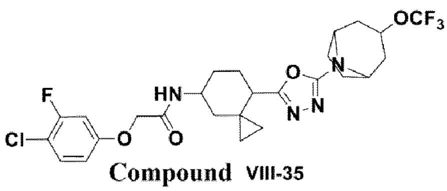
Compound VIII-35

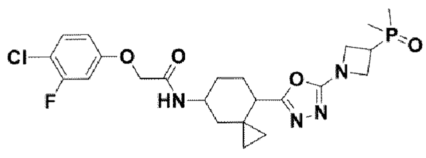
Compound VIII-36

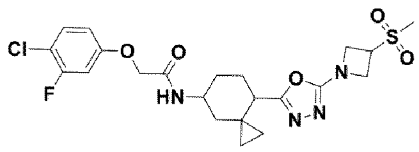
Compound VIII-37

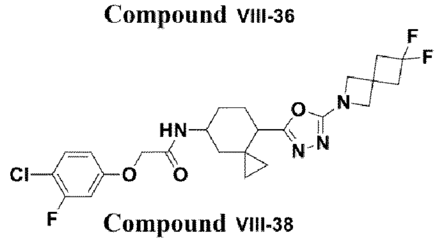
Compound VIII-38

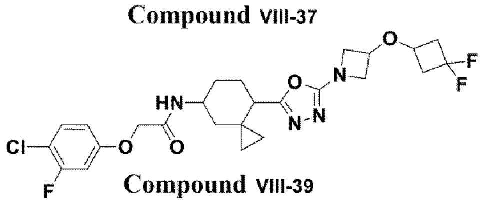
Compound VIII-39

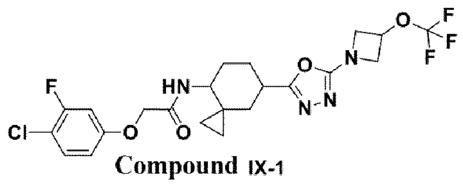
Compound IX-1

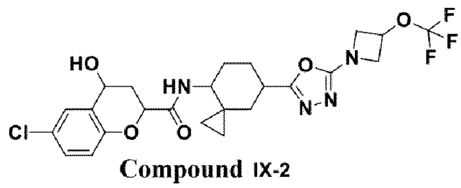
Compound IX-2

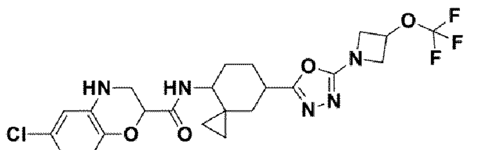
Compound IX-3

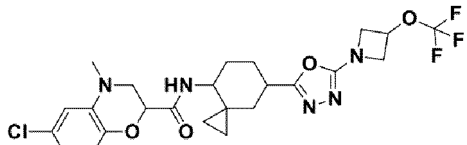
Compound IX-4

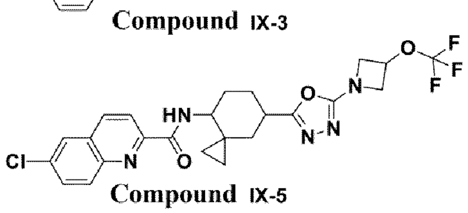
Compound IX-5

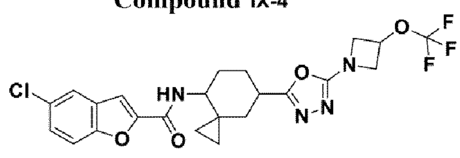
Compound IX-6

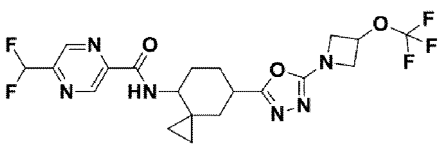
Compound IX-7

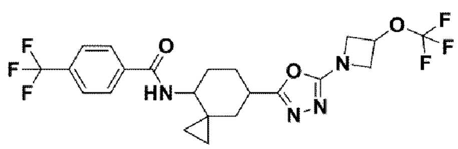
Compound IX-8

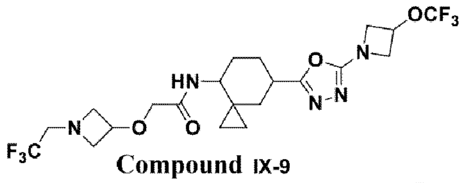
Compound IX-9

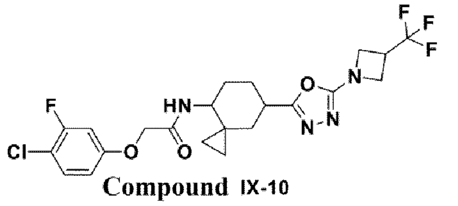
Compound IX-10

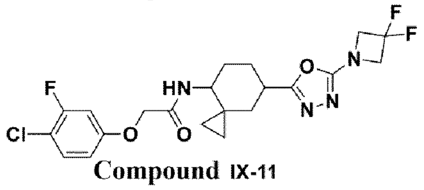
Compound IX-11

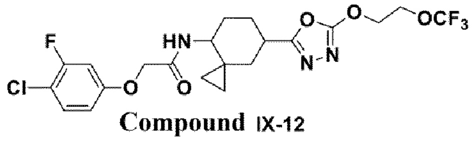
Compound IX-12

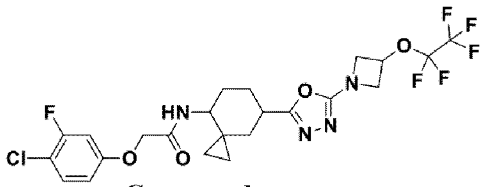
Compound IX-13

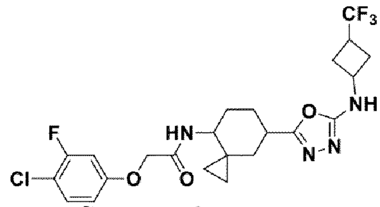
Compound IX-14

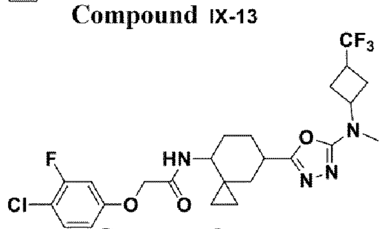
Compound IX-15

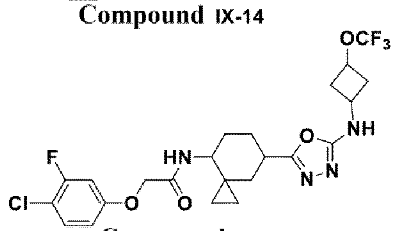
Compound IX-16

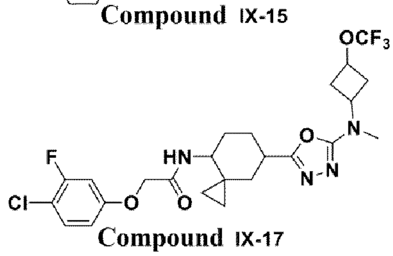
Compound IX-17

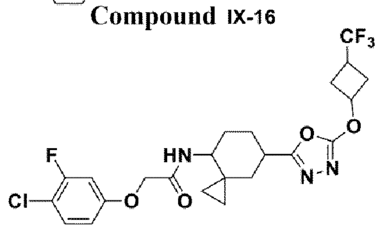
Compound IX-18

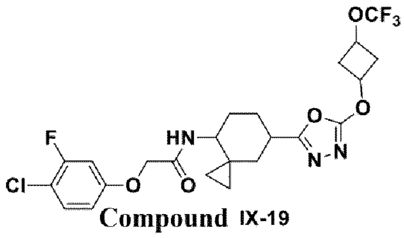
Compound IX-19

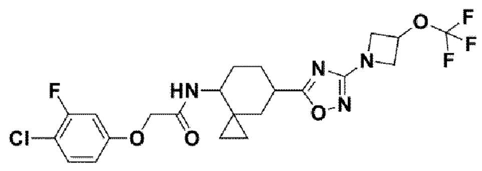
Compound IX-20

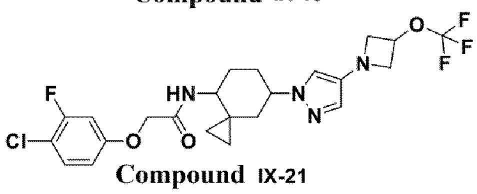
Compound IX-21

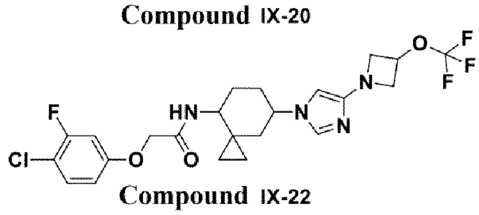
Compound IX-22

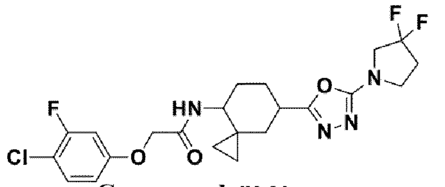

Compound IX-23

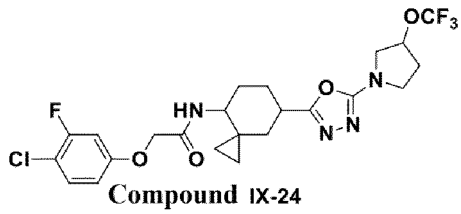

Compound IX-24

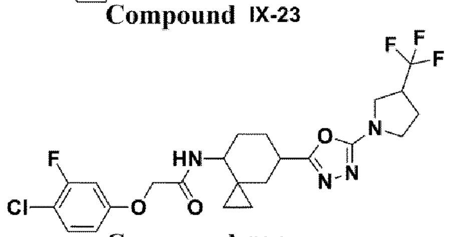

Compound IX-25

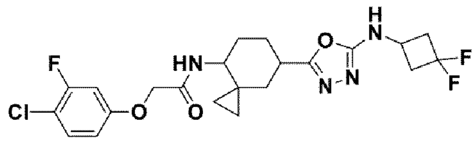

Compound IX-26

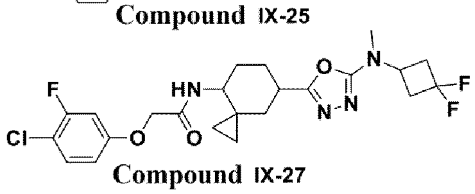

Compound IX-27

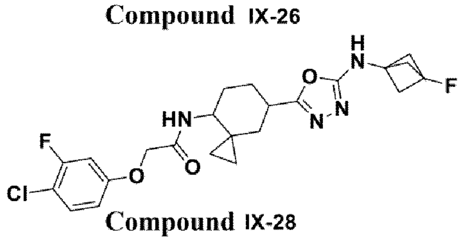

Compound IX-28

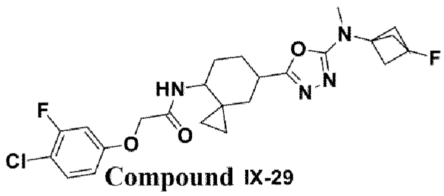

Compound IX-29

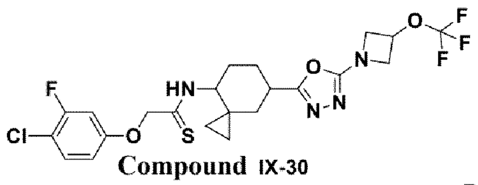

Compound IX-30

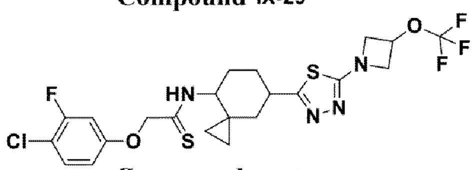

Compound IX-31

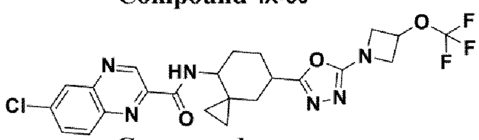

Compound IX-32

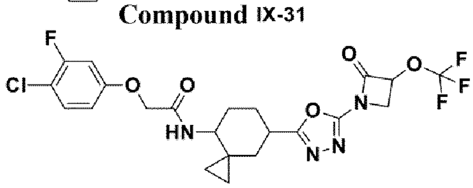

Compound IX-33

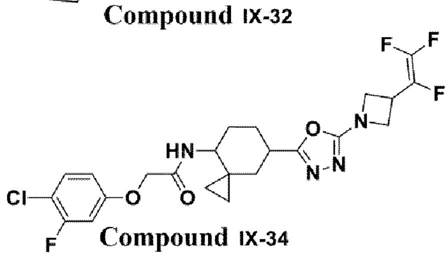

Compound IX-34

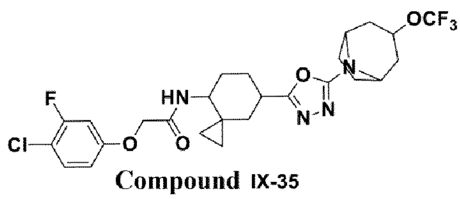

Compound IX-35

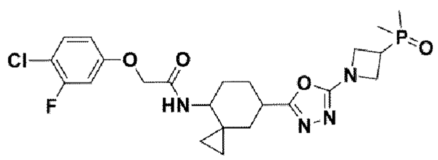

Compound IX-36

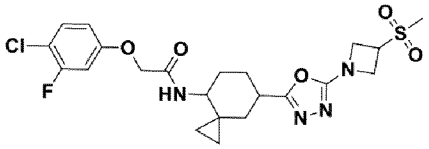

Compound IX-37

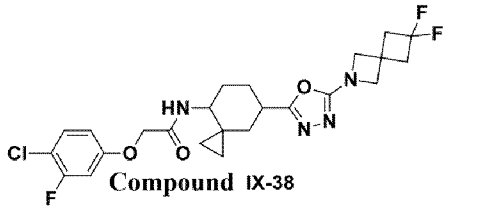

Compound IX-38

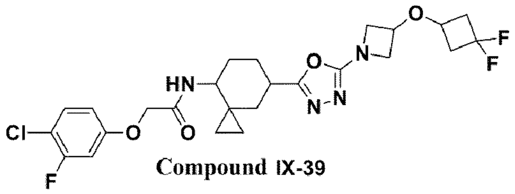

Compound IX-39

.

In some embodiments, provided is a pharmaceutical composition comprising a preparation prepared from the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof as described in any of the above.

Further, the above pharmaceutical composition further comprises a pharmaceutically acceptable carrier, excipient and vehicle.

In some embodiments, provided is the use of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof as described in any of the above, or the pharmaceutical composition as described in any of the above in the preparation of a drug for preventing and/or treating neurodegenerative diseases (such as leukodystrophy, leukoencephalopathy, dysmyelination or demyelinating disease, intellectual disability syndrome, cognitive dysfunction, glial cell dysfunction, or brain injury (such as traumatic brain injury or toxin-induced brain injury)), cancer, inflammatory diseases, autoimmune diseases, viral infections, skin diseases, fibrotic diseases, hemoglobin diseases, kidney diseases, hearing loss diseases, eye diseases, diseases with mutations causing the induction of unfolded protein response (UPR), malaria infections, musculoskeletal diseases, metabolic diseases or mitochondrial diseases.

In some embodiments, provided is the use of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof as described in any of the above, or the pharmaceutical composition as described in any of the above in the preparation of a drug for preventing and/or treating a disease or condition mediated by an integrated stress response (ISR) pathway.

In some embodiments, provided is a method for treating a disease or condition mediated by an integrated stress response (ISR) pathway in an individual in need thereof, wherein the method comprises administering a therapeutically effective amount of the compound, or the stereoisomer thereof, the pharmaceutically acceptable salt thereof, the tautomer thereof, the polymorph thereof, the solvate thereof, the hydrate thereof, the N-oxide thereof, the isotopically labeled compound thereof, the metabolite thereof, the ester thereof or the prodrug thereof as described in any of the above, or a therapeutically effective amount of the pharmaceutical composition as described in any of the above to the individual.

In some embodiments, provided is a method for treating a disease related to regulation of eIF2B activity or level, eIF2 pathway activity or level or ISR pathway activity or level, wherein the method comprises administering a therapeutically effective amount of the compound, or the stereoisomer thereof, the pharmaceutically acceptable salt thereof, the tautomer thereof, the polymorph thereof, the solvate thereof, the hydrate thereof, the N-oxide thereof, the isotopically labeled compound thereof, the metabolite thereof, the ester thereof or the prodrug thereof as described in any of the above, or a therapeutically effective amount of the pharmaceutical composition as described in any of the above to a subject.

In some embodiments, provided is a method for preventing and/or treating the above diseases, wherein the method comprises administering an effective amount of the compound, or the stereoisomer thereof or the pharmaceutically acceptable salt thereof, or the pharmaceutical composition as described in any of the above to a subject in need thereof.

In some embodiments, provided is a method for preventing and/or treating cancer, wherein the method comprises administering an effective amount of the compound, or the stereoisomer thereof or the pharmaceutically acceptable salt thereof, or the pharmaceutical composition as described in any of the above to a subject in need thereof.

In some embodiments, the neurodegenerative diseases include, but are not limited to: leukodystrophy, leukoencephalopathy, dysmyelination or demyelinating disease, intellectual disability syndrome, cognitive dysfunction, glial cell dysfunction, or brain injury (such as traumatic brain injury or toxin-induced brain injury), Alexander's disease, Alper's disease, Alzheimer's disease, amyotrophic lateral sclerosis (ALS), ataxia microvascular dilatation, Batten disease (also known as Spielmeyer-Vogt-Sjogren-Batten disease), bovine spongiform encephalopathy (BSE), Canavan disease, Cockayne syndrome, corticobasal degeneration, Creutzfeldt-Jakob disease, dystonia, frontotemporal dementia (FTD), Gerstmann-Straussler-Scheinker syndrome, Huntington's disease, HIV-related dementia, Kennedy's disease, Krabbe

disease, kuru, lewy body dementia, Machado-Joseph disease (spinocerebellar ataxia type 3), multiple system atrophy, multisystem proteinopathy, narcolepsy, neuroborreliosis, Parkinson's disease, Pelizaeus-Merzbacher disease, Pick's disease, primary lateral sclerosis, Prion disease, Refsum's disease, Sandhoff disease, Schilder's disease, Subacute combined degeneration of spinal cord secondary to pernicious anemia, schizophrenia, spinocerebellar ataxia (multiple types with different features, such as spinocerebellar ataxia type 2 or spinocerebellar ataxia type 8), spinal muscular atrophy, Steele-Richardson-Olszewski disease, progressive supranuclear palsy, corticobasal degeneration, adrenoleukodystrophy, X-linked adrenoleukodystrophy, cerebral adrenoleukodystrophy, Pelizaeus-Merzbacher disease, Krabbe disease, leukodystrophy caused by mutations in the DARS2 gene (sometimes called leukoencephalopathy with brainstem and spinal cord involvement and lactate elevation (LBSL), DARS2-related spectrum disorder, or Tabes dorsalis).

The above-mentioned cancers include but are not limited to: human cancer and carcinoma, sarcoma, adenocarcinoma, lymphoma, leukemia and melanoma, including solid cancer and lymphoid cancer, renal cancer, breast cancer, lung cancer, bladder cancer, colon cancer, ovarian cancer, prostate cancer, pancreatic cancer, stomach cancer, brain cancer, head and neck cancer, skin cancer, uterine cancer, testicular cancer, glioma, esophageal cancer, liver cancer (including hepatocarcinoma), lymphoma (including B-acute lymphoblastic lymphoma, non-Hodgkin's lymphoma (such as Burkitt's lymphoma), small cell lymphoma and large cell lymphoma), Hodgkin's lymphoma, leukemia (including AML, ALL and CML) and/or multiple myeloma. In some other instances, the "cancer" refers to lung cancer, breast cancer, ovarian cancer, leukemia, lymphoma, melanoma, pancreatic cancer, sarcoma, bladder cancer, bone cancer, brain cancer, cervical cancer, colon cancer, esophageal cancer, stomach cancer, liver cancer, head and neck cancer, renal cancer, myeloma, thyroid cancer, prostate cancer, metastatic cancer or carcinoma.

The above-mentioned leukemias include but are not limited to: acute non-lymphocytic leukemia, chronic lymphocytic leukemia, acute granulocytic leukemia, chronic granulocytic leukemia, acute promyelocytic leukemia, adult T-cell leukemia, non-leukemic leukemia, aleucemic leucemia, basophilic leukemia, blastic leukemia, bovine leukemia, chronic myelogenous leukemia, leukemia cutis, stem-cell leukemia, eosinophilic leukemia, Gross' leukemia, hairy cell leukemia, hematoblast leukemia, hemoblastic leukemia, histiocytic leukemia, stem cell leukemia, acute monocytic leukemia, leukopenic leukemia, lymphatic leukemia, lymphoblastic leukemia, lymphocytic leukemia, lymphogenic leukemia, lymphoid leukemia, lymphosarcoma cell leukemia, mast cell leukemia, megakaryocytic leukemia, small myeloblast leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia or undifferentiated cell leukemia.

The above-mentioned inflammatory diseases include but are not limited to: postoperative cognitive dysfunction, arthritis (such as rheumatoid arthritis, psoriatic arthritis and juvenile idiopathic arthritis), systemic lupus erythematosus (SLE), myasthenia gravis, juvenile-onset diabetes, type 1 diabetes, Guillain-Barre syndrome, Hashimoto's encephalitis, Hashimoto's thyroiditis, ankylosing spondylitis, psoriasis, Sjogren's syndrome, vasculitis, glomerulonephritis, autoimmune thyroiditis, Behcet's disease, Crohn's disease, ulcerative colitis, bullous pemphigoid, sarcoidosis, ichthyosis, Graves' ophthalmopathy, inflammatory bowel disease, Addison's disease, vitiligo, asthma (such as allergic asthma), acne vulgaris, celiac disease, chronic prostatitis, inflammatory bowel disease, pelvic inflammatory disease, reperfusion injury, sarcoidosis, transplant rejection, interstitial cystitis, atherosclerosis and atopic dermatitis.

The above-mentioned musculoskeletal diseases include but are not limited to: muscular dystrophy (such as Duchenne muscular dystrophy, Becker muscular dystrophy, distal muscular dystrophy, congenital muscular dystrophy, Emery-Dreifuss muscular dystrophy, facioscapulohumeral muscular dystrophy, myotonic dystrophy type 1, or myotonic dystrophy type 2), limb-girdle muscular dystrophy, multisystem proteinopathy, rhizomelic chondrodysplasia punctata, X-linked recessive chondrodysplasia punctata, Conradi-Hünermann syndrome, autosomal dominant chondrodysplasia punctata, stress-induced bone disorders (such as stress-induced osteoporosis), multiple sclerosis, amyotrophic lateral sclerosis (ALS), primary lateral sclerosis, progressive muscular atrophy, progressive bulbar palsy, pseudobulbar palsy, spinal muscular atrophy, progressive spinal and bulbar muscular atrophy, spasticity of spinal origin, spinal muscular atrophy, myasthenia gravis, neuralgia, fibromyalgia, Machado-Joseph disease, Paget disease of the bone, painful fasciculation syndrome, Friedreich ataxia, muscle wasting disorders (such as muscular dystrophy, sarcopenia and cachexia), inclusion body myopathy, motor neuron disease or paralysis.

The above-mentioned metabolic diseases include but are not limited to: non-alcoholic steatohepatitis (NASH), non-alcoholic fatty liver disease (NAFLD), liver fibrosis, obesity, heart disease, atherosclerosis, arthritis, cystinosis, diabetes (such as type 1 diabetes, type 2 diabetes, or gestational diabetes), phenylketonuria, proliferative retinopathy or Kearns-Sayre disease.

The above-mentioned mitochondrial diseases include but are not limited to: Barth syndrome, chronic progressive external ophthalmoplegia (cPEO), Kearns-Sayre syndrome (KSS), Leigh syndrome (such as MILS or maternally inherited Leigh syndrome), mitochondrial DNA deletion syndrome (MDDS, e.g., Alpers syndrome), mitochondrial encephalomyopathy (such as mitochondrial encephalomyopathy with lactic acidosis and stroke-like episodes (MELAS)), mitochondrial neurogastrointestinal encephalomyopathy (MNGIE), myoclonus epilepsy with ragged-red fibers (MERRF),

neuropathy, ataxia, retinitis pigmentosa (NARP), Leber's hereditary optic neuropathy (LHON) and Pearson syndrome.

The above-mentioned hearing loss diseases include but are not limited to: mitochondrial non-syndromic hearing loss and deafness, hair cell death, age-related hearing loss, noise-induced hearing loss, inherited or genetic hearing loss, hearing loss due to ototoxic exposure, disease-induced hearing loss and trauma-induced hearing loss. In some embodiments, the mitochondrial non-syndromic hearing loss and deafness is MT-RNR1-related hearing loss.

The above-mentioned eye diseases include but are not limited to: cataracts, glaucoma, endoplasmic reticulum (ER) stress, autophagy defects, age-related macular degeneration (AMD) or diabetic retinopathy.

The above-mentioned kidney diseases include but are not limited to: Abderhalden-Kaufmann-Lignac syndrome (Y-type cystinosis), abdominal compartment syndrome, acetaminophen-induced nephrotoxicity, acute renal failure/acute kidney injury, acute lobar nephropathy, acute phosphate nephropathy, acute tubular necrosis, adenine phosphoribosyltransferase deficiency, adenoviral nephritis, Alagille Syndrome, Alport Syndrome, amyloidosis, ANCA vasculitis related to endocarditis and other infections, angiomyolipoma, analgesic nephropathy, anorexia nervosa nephropathy, vasoconstrictor peptide antibodies and focal segmental glomerulosclerosis, antiphospholipid syndrome, glomerulonephritis related to anti-TNF-alpha therapy, APOL1 mutations, apparent mineralocorticoid excess syndrome, aristolochic acid nephropathy, Chinese herbal nephropathy, Balkan Endemic Nephropathy, urinary arteriovenous malformations and fistulas, autosomal dominant hypocalcemia, Bardet-Biedl Syndrome, Bartter Syndrome, acute kidney injury due to bath salts, beer potomania, beeturia, β-thalassemia nephropathy, bile cast nephropathy, autologous kidney BK polyomavirus nephropathy, rupture of bladder, bladder sphincter dyssynergia, bladder tamponade, border-crossers' nephropathy, Bourbon virus-induced acute kidney injury, burnt sugarcane harvesting-associated acute renal dysfunction, Byetta renal failure, Clq nephropathy, C3 glomerulopathy, C3 glomerulopathy with monoclonal gammopathy, C4 glomerulopathy, calcineurin inhibitor nephrotoxicity, Callilepsis Laureola poisoning, acute renal failure due to cannabinoid hyperemesis, cardiorenal syndrome, carfilzomib-induced renal injury, CFHR5 nephropathy, Charcot-Marie-Tooth disease with glomerular disease, Chinese herbal nephrotoxicity, acute kidney injury due to cherry concentrate, cholesterol embolism, Churg-Strauss syndrome, chyluria, ciliopathy, nephropathy related to cocaine, cold diuresis, colistininduced nephrotoxicity, collagenofibrotic glomerulalopathy, collapsing glomerulopathy, CMV-related collapsing glomerulopathy, combined antiretroviral therapy (cART)-related nephropathy, congenital anomalies of the kidney and urinary tract (CAKUT), congenital nephrotic syndrome, congestive renal failure, cone-shaped epiphysis nephrotic syndrome (Mainzer-Saldino syndrome or Saldino-Mainzer disease), contrast-induced nephropathy, copper sulfate poisoning, cortical necrosis, crizotinib-related acute kidney injury, crystal cryoglobulinemia, cryoglobulinemia, crystalline globulin-induced nephropathy, crystal-induced acute kidney injury, crystal-storing histiocytosis, acquired cystic kidney disease, cystinuria, dasatinib-induced nephrotic range proteinuria, dense deposit disease (type 2 MPGN), Dent disease (X-linked recessive nephrolithiasis), DHA crystal nephropathy, dialysis disequilibrium syndrome, diabetes and diabetic nephropathy, diabetes insipidus, dietary supplement-related renal failure, diffuse mesangial sclerosis, diuresis, Djenkol bean poisoning (Djenkolism), Down Syndrome-related nephropathy, drug abuse-related nephropathy, duplicated ureters, EAST syndrome, Ebolarelated nephropathy, ectopic kidney, ectopic ureter, edema, swelling, Erdheim-Chester Disease, Fabry's Disease, familial hypocalciuric hypercalcemia, Fanconi Syndrome, Fraser syndrome, fibronectin glomerulopathy, fibrillary glomerulonephritis and immunotactoid glomerulopathy, Fraley syndrome, fluid overload, hypervolemia, focal segmental glomerulosclerosis, focal sclerosis, focal glomerulosclerosis, Galloway Mowat syndrome, giant cell arteritis (temporal arteritis) involving the kidney, gestational hypertension, Gitelman syndrome, glomerular disease, tubulo-glomerular backflow, glycosuria, Goodpasture syndrome, green smoothie cleanse nephropathy, HANAC syndrome, Harvoni (ledipasvir and sofosbuvir)-induced kidney injury, acute kidney injury following hair dye poisoning, Hantavirus infection podocytopathy, heat stress nephropathy, hematuria (blood in urine), hemolytic uremic syndrome (HUS), atypical hemolytic uremic syndrome (aHUS), hemophagocytic syndrome, hemorrhagic cystitis, hemorrhagic fever with renal syndrome (HFRS, hantavirus nephropathy, Korean hemorrhagic fever, epidemic hemorrhagic fever, nephropathis epidemica), hemosiderosis, hemosiderosis related to paroxysmal nocturnal hemoglobinuria and hemolytic anemia, hepatic glomerular disease, hepatic veno-occlusive disease, hepatic sinusoidal obstruction syndrome, hepatitis C-related nephropathy, hepatocyte nuclear factor 1B-related nephropathy, hepatorenal syndrome, herbal supplement-related nephropathy, high altitude renal syndrome, hypertensive nephropathy, HIV-associated immune complex kidney disease (HIVICK), HIV-associated nephropathy (HIVAN), HNF1B-associated autosomal dominant tubulointerstitial nephropathy, horseshoe kidney (renal fusion), Hunner's ulcer, hydroxychloroquine-induced nephrolipidosis, hyperaldosteronism, hypercalcemia, hyperkalemia, hypermagnesemia, hypernatremia, hyperoxaluria, hyperphosphatemia, hypocalcemia, hypocomplementary urticaria vasculitis syndrome, hypopotassaemia, hypopotassaemia-induced renal dysfunction, hypokalemic periodic paralysis, hypomagnesemia, hyponatremia, hypophosphatemia, hypophosphatemia in marijuana users, hypertension, monogenic hypertension, Iced-tea nephropathy, Ifosfamideinduced nephrotoxicity, IgA nephropathy, IgG4 nephropathy, immersion diuresis, immune checkpoint therapy-related interstitial nephritis, infliximab-related nephropathy, interstitial cystitis, bladder pain syndrome (questionnaire), interstitial nephritis, megakaryocytic interstitial nephritis, Ivemark's syndrome, JC virus nephropathy, Joubert syndrome, ketamine-related bladder dysfunction, kidney stones, nephrolithiasis, kombucha tea toxicity, lead nephropathy and lead-related nephrotoxicity, lecithin cholesterol acyltransferase deficiency (LCAT deficiency), leptospirosis nephropathy, light chain deposition disease, monoclonal immunoglobulin deposition disease, light chain proximal tubulopathy, Liddle syndrome, Lightwood-Albright syndrome, lipoprotein glomerulopathy, lithium nephrotoxicity, hereditary FSGS caused by LMX1B mutations, loin pain-hematuria syndrome, lupus, systemic lupus erythematosus, lupus nephropathy, lupus nephritis, lupus nephritis with anti-neutrophil cytoplasmic antibody seropositivity, lupus podocytopathy, Lyme disease-related glomerulonephritis, lysinuria protein intolerance, lysozyme nephropathy, malarial nephropathy, malignant disease-related nephropathy, malignant hypertension, Malakoplakia, McKittrick-Wheelock syndrome, MDMA (Molly; Ecstacy; 3,4-methylenedioxymethamphetamine)-related renal failure, meatal stenosis, medullary cystic kidney disease, uromodulin-related nephropathy, Juvenile type 1 hyperuricemic nephropathy, medullary spongy kidney, megaureter, melamine-induced nephropathy, MELAS syndrome, membranoproliferative glomerulonephritis, membranous nephropathy, membranous glomerulopathy with occult IgGk deposition, MesoAmerican nephropathy, metabolic acidosis, metabolic alkalosis, methotrexate-related renal failure, microscopic polyangitis, milk-alkali syndrome, minimal change nephrosis, monoclonal gammopathy of renal significance, dysproteinemia, mouthwash toxicity, MUC1 nephropathy, multicystic renal dysplasia, multiple myeloma, myeloproliferative neoplastic glomerulopathy, nail-patella syndrome, NARP syndrome, nephrocalcinosis, nephrogenic systemic fibrosis, nephroptosis (floating kidney, renal ptosis), nephrotic syndrome, neurogenic bladder, 9/11 and kidney disease, nodular glomerulosclerosis, nongonococcal urethritis, nutcracker syndrome, oligomeganephronia, oro-facial-digital syndrome, orotic aciduria, orthostatic hypotension, orthostatic proteinuria, osmotic diuresis, osmotic nephropathy, ovarian hyperstimulation syndrome, oxalate nephropathy, Page Kidney, Papillary necrosis, Papillorenal syndrome (renal coloboma syndrome, solitary renal dysplasia), PARN-mutated nephropathy, parvovirus B19 nephropathy, peritoneo-renal syndrome, posterior urethral valve POEMS syndrome, podocyte infolding glomerulopathy, postinfectious glomerulonephritis, poststreptococcal glomerulonephritis, atypical postinfectious glomerulonephritis, postinfectious glomerulonephritis (IgA dominant), mimicking IgA nephropathy, polyarteritis nodosa, posterior urethral valve polycystic kidney disease, postobstructive diuresis, preeclampsia, propofol infusion syndrome, proliferative glomerulonephritis with monoclonal IgG deposits (Nasr Disease), propolis (bee resin)-related renal failure, proteinuria (protein in urine), pseudohyperaldosteronism, pseudohypobicarbonatemia, pseudohypoparathyroidism, pulmonary renal syndrome, pyelonephritis (kidney infection), pyonephrosis, Pyridium-induced renal failure, radiation-induced nephropathy, Ranolazine-induced nephropathy, refeeding syndrome, reflux nephropathy, rapidly progressive glomerulonephritis, renal abscess, perirenal abscess, renal hypoplasia, acute kidney injury related to renal arcuate vein microthrombosis, renal artery aneurysm, spontaneous renal artery dissection, renal artery stenosis, renal cell carcinoma, renal cyst, renal hypouricemia with exercise-induced acute renal failure, renal infarction, renal osteodystrophy, renal tubular acidosis, renin mutation and autosomal dominant tubulointerstitial nephropathy, renin-secreting tumor (juxtaglomerular cell tumor), reset osmostat, retrocaval ureter, retroperitoneal fibrosis, rhabdomyolysis, rhabdomyolysis related to bariatric surgery, nephropathy related to rheumatoid arthritis, sarcoidosis-related nephropathy, salt loss in the kidney and brain, schistosomal glomerulopathy, Schimke immunoosseous dysplasia, scleroderma renal crisis, serpentine fibula-polycystic kidney syndrome, Exner syndrome, sickle cell nephropathy, chronic kidney disease due to silica exposure, Sri lankan farmers' kidney disease, Sjogren's syndrome-related nephropathy, acute kidney injury due to synthetic cannabinoid use, post-hematopoietic cell transplant nephropathy, nephropathy related to stem cell transplantation, TAFRO syndrome, tea and toast-induced hyponatremia, tenofovir-induced nephrotoxicity, thin basement membrane disease, benign familial hematuria, ranolazine-induced nephropathy related to monoclonal gammopathy, refeeding syndrome, reflux nephropathy, rapidly progressive glomerulonephritis, renal abscess, perirenal abscess, renal hypoplasia, acute kidney injury related to renal arcuate vein microthrombosis, renal artery aneurysm, spontaneous renal artery dissection, renal artery stenosis, renal cell carcinoma, renal cyst, renal hypouricemia with exercise-induced acute renal failure, renal infarction, renal osteodystrophy, renal tubular acidosis, renin mutation and autosomal dominant tubulointerstitial nephropathy, renin-secreting tumor (juxtaglomerular cell tumor), reset osmostat, retrocaval ureter, retroperitoneal fibrosis, rhabdomyolysis, rhabdomyolysis related to bariatric surgery, nephropathy related to rheumatoid arthritis, sarcoidosis-related nephropathy, salt loss in the kidney and brain, schistosomal glomerulopathy, Schimke immunoosseous dysplasia, scleroderma renal crisis, serpentine fibula-polycystic kidney syndrome, Exner syndrome, sickle cell nephropathy, chronic kidney disease due to silica exposure, Sri lankan farmers' kidney disease, Sjogren's syndrome-related nephropathy, acute kidney injury due to synthetic cannabinoid use, post-hematopoietic cell transplant nephropathy, nephropathy related to stem cell transplantation, TAFRO syndrome, tea and toast-induced hyponatremia, tenofovir-induced nephrotoxicity, thin basement membrane disease, benign familial hematuria, monoclonal gammopathy-related thrombotic microangiopathy, trench nephritis, trigonitis, urogenital tuberculosis, tuberous sclerosis, tubular dysgenesis, immune complex tubulointerstitial nephritis due to autoantibodies to the brush border of proximal tubules, tumor lysis syndrome, uremia, uremic optic neuropathy, cystic ureteritis, ureteral hernia, urethral caruncle, urethral stricture, urinary incontinence, urinary tract infection, urinary tract obstruction, urogenital fistula, uromodulin-related nephropathy, vancomycin-related cast nephropathy, vasomotor nephropathy, vesicoenteric fistula, vesicoureteric reflux, VGEF-inhibited renal thrombotic microangiopathy, volatile anesthetic-induced acute kidney injury, Von Hippel-Lindau disease, Waldenstrom's macroglobulinemic glomerulonephritis, warfarinrelated nephropathy, acute kidney injury following wasp sting, Wegener's granulomatosis, granulomatosis with polyangiitis, West Nile virus-induced chronic kidney

disease, Wunderlich syndrome, Zellweger syndrome, or cerebrohepatorenal syndrome.

The above-mentioned skin diseases include but are not limited to: acne, alopecia areata, basal cell carcinoma, Bowen's disease, congenital erythropoietic porphyria, contact dermatitis, Darier's disease, disseminated superficial actinic po-rokeratosis, dystrophic epidermolysis bullosa, eczema (atopic eczema), extramammary Paget's disease, simplex epidermolysis bullosa, erythropoietic protoporphyria, fungal infections of the nails, Hailey-Hailey disease, herpes simplex, hidradenitis suppurativa, hirsutism, hyperhidrosis, ichthyosis, impetigo, keloid, keratosis pilaris, lichen planus, lichen sclerosus, melanoma, melanoderma, mucous membrane pemphigoid, pemphigoid, pemphigus vulgaris, pityriasis lichenoid, pityriasis rubra pilaris, plantar warts (warts), polymorphous light eruption, psoriasis, plaque psoriasis, pyoderma gangrenosum, rosacea, scabies, scleroderma, herpes zoster, squamous cell carcinoma, Sweet's syndrome, urticaria, angioedema and vitiligo.

The above-mentioned fibrotic diseases include but are not limited to: adhesive capsulitis, arterial stiffness, arthrofibrosis, atrial fibrosis, cardiac fibrosis, cirrhosis, congenital hepatic fibrosis, Crohn's disease, cystic fibrosis, Dupuytren's contracture, endomyocardial fibrosis, glial scar, hepatitis C, hypertrophic cardiomyopathy, hypersensitivity pneumonitis, idiopathic pulmonary fibrosis, idiopathic interstitial pneumonia, interstitial lung disease, keloid, mediastinal fibrosis, myelofibrosis, nephrogenic systemic fibrosis, non-alcoholic fatty liver disease, old myocardial infarction, Peyronie's disease, pneumoconiosis, pneumonia, progressive massive fibrosis, pulmonary fibrosis, radiation-induced lung injury, retroperitoneal fibrosis, scleroderma/systemic sclerosis, silicosis and ventricular remodeling.

The above-mentioned hemoglobin diseases include but are not limited to: "dominant" β-thalassemia, acquired (toxic) methemoglobinemia, carboxy hemoglobinemia, congenital Heinz-body hemolytic anemia, HbH disease, HbS/β-thalassemia, HbE/β-thalassemia, HbSC disease, homozygous α+-thalassemia (α0-thalassemia), hydrops fetalis with Hb Bart's, sickle cell anemia/disease, sickle cell trait, sickle cell β-thalassemia disease, α+-thalassemia, α0-thalassemia, α-thalassemia related to myelodysplastic syndrome, α-thalassemia with mental retardation syndrome (ATR), β0-thalassemia, β+-thalassemia, δ-thalassemia, γ-thalassemia, β-thalassemia major, β-thalassemia intermediate, δβ-thalassemia and εγδβ-thalassemia.

The above-mentioned autoimmune diseases include but are not limited to: achalasia, Addison's disease, adult Still's disease, agammaglobulinemia, alopecia areata, amyloidosis, ankylosing spondylitis, anti-GBM/anti-TBM nephritis, antiphospholipid syndrome, autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, autoimmune urticaria, axonal and neuronal neuropathy (AMAN), Baló disease, Behcet's disease, benign mucosal pemphigoid, bullous pemphigoid, Castleman disease (CD), celiac disease, Chagas disease, chronic inflammatory demyelinating polyneuropathy (CIDP), chronic recurrent multifocal osteomyelitis (CRMO), Chagas-Strauss syndrome (CSS) or eosinophilic granulomatosis with polyangiitis (EGPA), cicatricial pemphigoid, Cogan's syndrome, cold agglutinin disease, congenital heart block, coxsackiemyocarditis, CREST syndrome, Crohn's disease, dermatitis herpetiformis, dermatomyositis, Devic's disease (neuromyelitis optica), discoid lupus, Dressler's syndrome, endometriosis, eosinophilic esophagitis (EoE), eosinophilic fasciitis, erythema nodosum, essential mixed cryoglobulinemia, Evans syndrome, fibromyalgia, fibrosing alveolitis, giant cell arteritis (temporal arteritis), giant cell myocarditis, glomerulonephritis, Goodpasture Syndrome, granulomatosis with polyangiitis, Graves' disease, Guillain-Barre syndrome, Hashimoto's thyroiditis, hemolytic anemia, henoch-schonlein purpura (HSP), herpes gestation is or pemphigoid gestationis (PG), hidradenitis suppurativa (HS) (acne inversa), hypogammaglobulinemia, IgA nephropathy, IgG4-related sclerosing disease, immune thrombocytopenic purpura (ITP), inclusion body myositis (IBM), interstitial cystitis (IC), juvenile arthritis, juvenile diabetes (type 1 diabetes), juvenile myositis (JM), kawasaki disease, Lambert-Eaton syndrome, leukocytoclastic vasculitis, lichen planus, lichen sclerosus, ligneous conjunctivitis, linear IgA disease (LAD), lupus, chronic Lyme disease, Meniere's disease, microscopic polyangitis (MPA), mixed connective tissue disease (MCTD), Mooren's ulcer, Mucha-Habermann disease, multifocal motor neuropathy (MMN) or MMNCB, multiple sclerosis, myasthenia gravis, myositis, narcolepsy, neonatal lupus, neuromyelitis optica, neutropenia, ocular cicatricial pemphigoid, optic neuritis, palindromic rheumatism (PR), PANDAS, paraneoplastic cerebellar degeneration (PCD), paroxysmal nocturnal hemoglobinuria (PNH), Parry Romberg syndrome, pars planitis (peripheral uveitis), Parsonnage-Turner syndrome, pemphigus, peripheral neuropathy, perivenous encephalomyelitis, pernicious anemia (PA), POEMS syndrome, polyarteritis nodosa, polyglandular syndrome type I, polyglandular syndrome type II, polyglandular syndrome type III, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia (PRCA), pyoderma gangrenosum, Raynaud's phenomenon, reactive arthritis, reflex sympathetic dystrophy, relapsing polychondritis, restless legs syndrome (RLS), retroperitoneal fibrosis, rheumatic fever, rheumatoid arthritis, sarcoidosis, Schmidt syndrome, scleritis, scleroderma, Sjogren's syndrome, seminal and testicular autoimmune diseases, stiff-man syndrome (SPS), subacute bacterial endocarditis (SBE), Susac's syndrome, sympathetic ophthalmia (SO), Takayasu's arteritis, temporal arteritis/giant cell arteritis, Thrombotic thrombocytopenic purpura (TTP), Tolosa-Hunt syndrome (THS), transverse myelitis, type 1 diabetes, ulcerative colitis (UC), undifferentiated connective tissue disease (UCTD), uveitis, vasculitis, vitiligo, Vogt-Koyanagi-Harada Disease, and Wegener's granulomatosis (or granulomatosis with polyangiitis (GPA)).

The above-mentioned viral infections include but are not limited to: influenza, human immunodeficiency virus (HIV) and herpes.

The above-mentioned malaria infections include but are not limited to: infections caused by Plasmodium vivax, Plasmodium ovale, Plasmodium malariae and Plasmodium falciparum.

The above-mentioned diseases with mutations causing the induction of unfolded protein response (UPR) include but are not limited to: Marinesco-Sjogren syndrome, neuropathic pain, diabetic neuropathic pain, noise-induced hearing loss, nonsyndromic sensorineural hearing loss, age-related hearing loss, Wolfram syndrome, Darier White disease, Usher syndrome, collagenopathy, thin basement membrane nephropathy, Alport syndrome, skeletal chondrodysplasia, metaphyseal chondrodysplasia type Schmid and pseudoachondroplasia.

The compounds and derivatives provided in the present disclosure can be named according to the IUPAC (International Union of Pure and Applied Chemistry) or CAS (Chemical Abstracts Service, Columbus, OH) nomenclature system.

With regard to definitions of terms used in the present disclosure, the initial definitions provided for groups or terms herein are applicable to those groups or terms throughout the specification unless otherwise specified. With regard to terms not specifically defined herein, their meanings should be given in accordance with the disclosed contents and the context, which should be understandable to those skilled in the art.

"Substitution" means that a hydrogen atom in a molecule is replaced by another atom or group; or the lone pair of electrons on an atom in a molecule is replaced by another atom or group. For example, the lone pair of electrons on an S atom can be replaced by an O atom to form

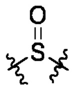 or 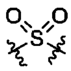.

"Optionally substituted with..." means that the "substitution" may but does not necessarily occur, encompassing both the situation where the substitution occurs and the situation where the substitution does not occur.

"Optionally" means that options can be selected but are not necessarily selected, encompassing both the situation where the options are selected and the situation where the options are not selected.

The minimum and maximum number of carbon atoms of a hydrocarbon group is indicated by a prefix, for example, the prefix in Ca-Cb alkyl indicates any alkyl group containing from "a" to "b" carbon atoms. Therefore, for example, C1-C6 alkyl refers to an alkyl group containing 1 to 6 carbon atoms.

"Alkyl" refers to a saturated hydrocarbon chain having a specified number of member atoms. Alkyl groups may be straight or branched. Representative branched alkyl groups have one, two or three branches. Alkyl groups can be optionally substituted with one or more substituents as defined herein. For example, C1-C6 alkyl includes methyl, ethyl, propyl (n-propyl and isopropyl), butyl (n-butyl, isobutyl and tert-butyl), pentyl (n-pentyl, isopentyl and neopentyl) and hexyl.

The term "C1-C10 alkyl" refers to any straight-chain or branched-chain group containing 1 to 10 carbon atoms, such as methyl, ethyl, n-propyl, isopropyl, n-butyl, isobutyl, tert-butyl, sec-butyl, n-pentyl, tert-pentyl, n-hexyl, and the following alkyl groups that are straight or branched: C7 alkyl, C8 alkyl, C9 alkyl, C10 alkyl, etc.

Furthermore, the "C1-C10 alkyl" includes straight-chain or branched-chain groups with the number of carbon atoms in the range having any two integers between 1 and 10 as endpoints. For example, the "C1-C10 alkyl" includes C1-C10 alkyl, C1-C8 alkyl, C1-C6 alkyl, C2-C10 alkyl, C2-C8 alkyl, C2-C6 alkyl, C6-C10 alkyl, etc., which are listed merely for illustration purposes and do not serve as limitations.

The term "alkoxy" and derivatives thereof refer to groups formed by any of the above-mentioned alkyl groups (such as C1-C10 alkyl and C1-C6 alkyl) being attached to the rest of a molecule via an oxygen atom (-O-).

"Alkylene" refers to a divalent saturated aliphatic hydrocarbon group having a specified number of member atoms. Ca-Cb alkylene refers to an alkylene group having a to b carbon atoms. Alkylene groups include both branched-chain and straight-chain hydrocarbon groups. For example, the term "propylene" can be exemplified by the following structure:

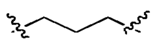.

Likewise, the term "dimethylbutylene" can be exemplified, for example, by either of the following structures:

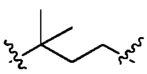 or 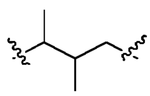.

The C1-C4 alkylene of the present disclosure may be C1 alkylene (e.g., $-CH_2-$), C2 alkylene (e.g., $-CH_2CH_2-$), C3 alkylene or C4 alkylene.

The "cycloalkyl" in the present disclosure refers to saturated cyclic alkanes with a single ring or multiple rings (fused rings, spiro rings or bridged rings) having multiple carbon atoms and no ring heteroatoms. Examples of monocarbocyclyl groups include, for example, cyclopropyl, cyclobutyl, cyclohexyl, cyclopentyl, cyclooctyl, cyclopentenyl and cyclohexenyl. Examples of fused cycloalkane systems include dicyclohexyl, dicyclopentyl, dicyclooctyl, etc. Examples of the "cycloalkyl" in the present disclosure include but are not limited to

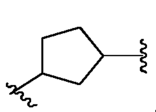, 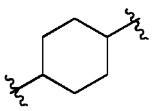,

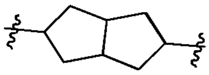 or 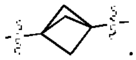.

The term "C3-C10 cycloalkyl" refers to a 3- to 10-membered all-carbon monocyclic, fused, bridged and spiro ring, which may contain 0, one or more double bonds, but does not have a completely conjugated $\pi$-electron system. Examples of C3-C10 cycloalkyl include, but are not limited to, cyclopropane, cyclobutane, cyclopentane, cyclopentene, cyclohexane, cyclohexene, cyclohexadiene, etc.

The "heterocyclyl" in the present disclosure refers to a saturated ring with a single ring or multiple rings (fused ring, bridged ring and spiro ring) having at least one heteroatom, wherein the heteroatoms refers nitrogen atoms, oxygen atoms, sulfur atoms, etc. Examples of 3- to 9-membered heterocyclyl may be oxetanyl, azetidinyl, oxocyclopentyl, oxocyclohexy, piperazinyl, piperidyl, morpholinyl, sym-trioxanyl, etc. Examples of the "heterocyclyl" in the present disclosure include but are not limited to

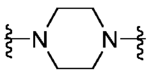, 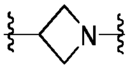, 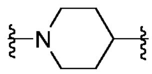,

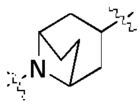

or piperidyl.

The "5- to 10-membered spiro ring" in the present disclosure refers to a saturated or non-aromatic partially saturated divalent ring formed by the spiro-fusion of two or more rings having 5 to 10 carbon atoms and no ring heteroatoms. Examples of 5- to 10-membered spiro ring systems may include: a spiro[2,5]octyl ring, a spiro[3,4]octyl ring, a spiro[3,3]heptyl ring, a spiro[3,5]nonyl ring, a spiro[4,5]decyl ring, a spiro[2,2]pentyl ring, a spiro[2,3]hexyl ring, a spiro[2,4]heptyl ring, a spiro[2,6]nonyl ring, a spiro[2,7]nonyl ring, a spiro[3,6]nonyl ring, a spiro[4,4]nonyl ring, etc.

The "5- to 10-membered spiro heterocyclic ring" in the present disclosure refers to a saturated or non-aromatic partially saturated divalent ring formed by the spiro-fusion of two or more rings formed after at least one carbon atom in the above-mentioned "5- to 10-membered spiro ring" is replaced by a heteroatom(s). Examples of 5- to 10-membered spiro heterocyclic ring systems include spiro heterocyclic rings formed by replacing at least one carbon atom in the above-mentioned spiro[2,5]octyl ring, spiro[3,4]octyl ring, spiro[3,3]heptyl ring, spiro[3,5]nonyl ring, spiro[4,5]decyl ring, spiro[2,2]pentyl ring, spiro[2,3]hexyl ring, spiro[2,4]heptyl ring, spiro[2,6]nonyl ring, spiro[2,7]nonyl ring, spiro[3,6]nonyl ring and spiro[4,4]nonyl ring with a heteroatom(s) selected from N, O, S, etc., such as

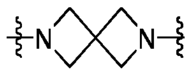, 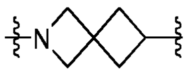,

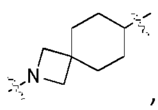, 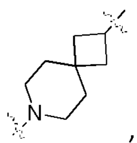, 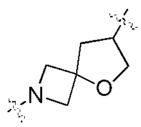 or 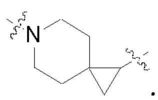.

The term "unsaturated" in the present disclosure refers to the presence of carbon-carbon double bonds, carbon-carbon triple bonds, carbon-oxygen double bonds, carbon-sulfur double bonds, carbon-nitrogen triple bonds, etc. in groups or molecules.

The "aromatic ring" in the present disclosure refers to an aromatic hydrocarbon group having multiple carbon atoms. Aryl is typically monocyclic, bicyclic or tricyclic aryl having multiple carbon atoms. Furthermore, the term "aryl" as used herein refers to an aromatic substituent containing a single aromatic ring or multiple aromatic rings that are fused together. Non-limiting examples include phenyl, naphthyl or tetrahydronaphthyl.

The "5- to 10-membered heteroaryl" in the present disclosure refers to an aromatic unsaturated ring containing at least one heteroatom, wherein the heteroatoms refers nitrogen atoms, oxygen atoms, sulfur atoms, etc., and generally refers to the aromatic monocyclic or bicyclic hydrocarbon containing a plurality of ring atoms, wherein one or more ring atoms are selected from heteroatoms of O, N, and S, and preferably containing one to three heteroatoms. For example, the 5-to 6-membered heteroaryl represents pyridyl, pyrrolyl, furyl, thienyl, pyrazolyl, imidazolyl, thiazolyl, pyranyl, thiopyranyl, piperazinyl, 1,2,3-triazolyl, 1,2,4-triazolyl, 1,2,5-triazolyl, 1,3,4-triazolyl, 1,2,3,4-tetrazolyl, 1,2,3,5-tetrazolyl, isoxazolyl, oxazolyl, 1,2,3-oxadiazolyl, 1,2,4-oxadiazolyl, 1,3,4-oxadiazolyl, furazanyl, 1,2,3,5-oxatriazolyl, 1,2,3,4-oxatriazolyl, 1,3,2-dioxazolyl, 1,2,3-dioxazolyl, 1,2,3,4-dioxadiazolyl, 1,2,3,5-dioxadiazolyl, 1,3,3,4-dioxadiazolyl, 1,3,4,5-dioxadiazolyl, 1,2,3,4-dioxadiazolyl, isothiazolyl, pyridazinyl, 1,2,3-triazinyl, 1,2,4-triazinyl, 1,3,5-triazinyl, 1,2,4,5-tetrazinyl, 1,2,4-oxazinyl, 1,2,6-oxazinyl, 1,3,2-oxazinyl, 1,3,6-oxazinyl, 1,4,2-oxazinyl, 1,2-isoxazinyl and 1,4-isoxazinyl.

The "halogen" in the present disclosure refers to fluorine, chlorine, bromine or iodine.

The "halogen-substituted alkyl" in the present disclosure refers to the alkyl having one or more hydrogen atoms substituted with halogen; for example, halogen-substituted $C_{1-4}$ alkyl refers to the alkyl containing 1 to 4 carbon atoms and having a hydrogen atom(s) substituted with one or more halogen atoms; and for example, monofluoromethyl, difluoromethyl and trifluoromethyl are included.

The "$-N(R)_2$" etc. in the present disclosure means that the R group is connected to the nitrogen atom through a single bond.

The "=O" (oxo) in the present disclosure means that an oxygen atom substitutes two hydrogen atoms in a molecule through a double bond.

In the present disclosure, it would have been obvious to those skilled in the art that for any group with a composite name, such as "6- to 10-membered aryl-D-C1-C6 alkyl", the construction of the group is based on the part from which it is derived, such as the C1-C6 alkyl, in a left-to-right manner according to the naming convention, and it should be understood that the alkyl mentioned here is divalent alkyl.

The "null" in the present disclosure means that the objects connected thereto are directly linked by a bond. For example, "$L^1$ is null" means that ring A and ring B, which are respectively connected to $L^1$, are directly linked by a bond.

In the present disclosure, the "linking group" refers to a chemical group containing two connectable bonds, such as O, S, -NH-, -N(alkyl)-, alkylene, heteroalkylene, cycloalkylene and heterocyclylene.

In the present disclosure, the "stereoisomers" refer to compounds with the same chemical constitution but different arrangements of atoms or groups in space, including enantiomers, diastereomers, conformers (rotamers), geometric isomers (cis/trans isomers), atropisomers, etc.

In the present disclosure, the "tautomers" generally refer to structural isomers of different energies which are interconvertible via a low energy barrier. For example, proton tautomers (also known as prototropic tautomers) involve interconversions through proton transfer, such as keto-enol isomerizations and imine-enamine isomerizations. Valence tautomers involve the interconversion through the reorganization of some bonding electrons.

In the present disclosure, the "geometric isomers", also called "cis-trans isomers", are isomers caused by the inability of double bonds (including double bonds in alkenes, C=N and N=N), or single bonds of ring carbon atoms to rotate freely.

In the present disclosure, the "enantiomers" refer to two isomers of a compound which are non-superimposable mirror images of each other.

In the present disclosure, the "diastereomer" refers to stereoisomers that have two or more chiral centers and whose molecules are not mirror images of each other. Diastereomers have different physical properties, such as melting points, boiling points, spectral properties and reactivity. Diastereomeric mixtures can be separated by high-resolution analytical procedures such as electrophoresis and chromatography, for example HPLC.

In the present disclosure, the "racemate", "racemic substance" or "racemic mixture" refers to an equimolar mixture of two enantiomers lacking optical activity.

The solid line (-), solid wedge ( ) or dashed wedge ( ) can be used herein to depict the chemical bonds of the compounds of the present disclosure. The use of solid lines to depict the bonds connected to asymmetric carbon atoms is intended to indicate that all possible stereoisomers at the carbon atoms (such as specific enantiomers and racemic mixtures) are included. The use of solid or dashed wedges to depict the bonds connected to asymmetric carbon atoms is intended to indicate the presence of the stereoisomers shown. When present in a racemic mixture, solid and dashed wedges are used to define the relative stereochemistry rather than the absolute stereochemistry. Unless

otherwise indicated, the compounds of the present disclosure may exist in various forms of stereoisomers, including cis and trans isomers, optical isomers (e.g., R- and S-enantiomers), diastereomers, geometric isomers, rotamers, conformers, atropisomers, and mixtures thereof. The compounds of the present disclosure may exhibit more than one type of isomerism, and consist of mixtures thereof (such as racemic mixtures and diastereomeric pairs).

In the present disclosure, the "polymorph" refers to a crystalline form of a compound (or a salt, hydrate or solvate thereof) in a specific crystal packing arrangement. All polymorphs have the same elemental composition. Different crystal forms usually have different X-ray diffraction patterns, infrared spectra, melting points, density, hardness, crystal shapes, optical and electrical properties, stability and solubility. The recrystallization solvent, crystallization rate, storage temperature, and other factors may lead to the predominance of one crystal form.

The "solvate" in the present disclosure refers to a mixture produced by dissolving a compound in a solvent.

In the present disclosure, the "N-oxide" is also called amine oxide, which is a class of organic compounds having the general formula $R_3N$+-O- (also written as $R_3N{=}O$ or $R_3N \rightarrow O$).

In the present disclosure, the "isotopically labeled compound" means that 1 or more atoms in a molecule or group are substituted with their isotope atoms; for example, hydrogen atoms are substituted with deuterium atoms, with the proportion of deuterium atoms being greater than the natural abundance of deuterium; and for example, 12C is substituted with 13C.

In the present disclosure, the "metabolites" refer to substances generated by chemical structural transformation of drug molecules under the action of the body after the drug molecules are absorbed by the body.

In the present disclosure, the "prodrug" refers to a compound obtained by chemically modifying a drug, and the "prodrug" is inactive or less active in vitro and releases active drugs through the enzymatic or non-enzymatic conversion in vivo, thereby exerting its pharmacological effects.

In the present disclosure, the "pharmaceutically acceptable" means that a carrier, vector, diluent, excipient, and/or a salt formed thereby is generally chemically or physically compatible with other ingredients constituting a pharmaceutical dosage form and is physiologically compatible with the recipient.

In the present disclosure, the "salt" and "pharmaceutically acceptable salt" refer to acidic and/or basic salts formed by the above-mentioned compounds or the stereoisomers thereof with inorganic and/or organic acids and bases, and also include zwitterionic salts (inner salts) and quaternary ammonium salts, such as alkylammonium salts. These salts can be obtained directly during the final separation and purification of the compounds. These salts can also be obtained by appropriately (for example, in equivalent amounts) mixing the above-mentioned compounds or the stereoisomers thereof with a certain amount of acids or bases. These salts may precipitate from solution and be collected by filtration, or may be recovered by evaporation of the solvent, or may be prepared by freeze-drying after reaction in an aqueous medium.

The "preventing" in the present disclosure includes suppressing and delaying the onset of a disease, and encompasses not only preventing a disease before its development, but also preventing the recurrence of a disease after treatment.

In the present disclosure, the "treating" means reversing, alleviating or eliminating the disorders or conditions to which such terms apply, or the progression of one or more symptoms of such disorders or conditions.

In certain embodiments, one or more compounds of the present disclosure may be used in combination with each other. The compounds of the present disclosure may also be used in combination with any other active agents to prepare drugs or pharmaceutical compositions for regulating cell functions or treating diseases. If a group of compounds are used, the compounds may be administered to a subject simultaneously, separately or sequentially.

Obviously, according to the above-mentioned contents of the present disclosure, in accordance with common technical knowledge and customary means in the art, and without departing from the above basic technical ideas of the present disclosure, other various forms of modifications, replacements or changes can be made.

The above contents of the present disclosure will be further described in detail in the following "Detailed Description of Embodiments" presented in the form of examples. However, this should not be construed as limiting the scope of the above-mentioned subject of the present disclosure to the following examples. All techniques achieved based on the above-mentioned contents of the present disclosure fall within the scope of the present disclosure.

Detailed Description of Embodiments

The known starting materials of the present disclosure can be synthesized by or in accordance with methods known in the art, or can be purchased from Bidepharm, Energy Chemical, Tansoole, PharmaBlock Sciences (Nanjing), Inc., Jiangsu Aikon Biopharmaceutical R&D Co., Ltd., Innochem (Beijing) Technology Co., Ltd., etc. Among them, tetrapropylammonium perruthenate is purchased from Tansoole, and 1-propylphosphonic anhydride is purchased from Innochem (Beijing) Technology Co., Ltd.

Unless otherwise specified in the examples, the reactions are carried out under nitrogen atmosphere. Unless otherwise specified in the examples, the solution refers to an aqueous solution. Unless otherwise specified in the examples, the reaction temperature is room temperature. The optimal reaction temperature is room temperature, which

ranges from 20°C-30°C. Unless otherwise specified in the examples, M represents mole per liter.

The structures of the compounds are determined by nuclear magnetic resonance (NMR) or mass spectrometry (MS). The NMR shift (δ) is given in the unit of $10^{-6}$ (ppm). NMR is determined by using a nuclear magnetic resonance spectrometer Bruker Avance III 400. The solvents for determination are deuterated dimethyl sulfoxide (DMSO-$d_6$), deuterated chloroform ($CDCl_3$), and deuterated methanol (Methanol-$d_4$), and the internal standard is tetramethylsilane (TMS). LC-MS is determined by using Shimadzu LC-MS 2020 (ESI). HPLC is determined by using a high-pressure liquid chromatograph Shimadzu LC-20A. MPLC (medium-pressure liquid chromatography) is performed using a reverse-phase preparative chromatograph Gilson GX-281. Yantai Huanghai HSGF254 or Qingdao GF254 silica gel plate is used as a thin layer chromatography silica plate, and the specification when a product is separated and purified by thin layer chromatography is 0.4 mm-0.5 mm. 200-300 mesh Yantai Huanghai silica gel is generally used as a carrier for the column chromatography.

N,N'-diisopropylethylamine (also known as diisopropylethylamine) is abbreviated as DIEA or DIPEA; 2-(7-azabenzotriazol)-N,N,N',N'-tetramethyluronium hexafluorophosphate is abbreviated as HATU; tetrapropylammonium perruthenate is abbreviated as TPAP; 1-propylphosphonic anhydride is abbreviated as T3P; N-methylmorpholine-N-oxide is abbreviated as NMO; trifluoroacetic acid is abbreviated as TFA; dimethylformamide is abbreviated as DMF; dichloromethane is abbreviated as DCM; petroleum ether is abbreviated as PE.

Example 1: Synthesis of compound I-1

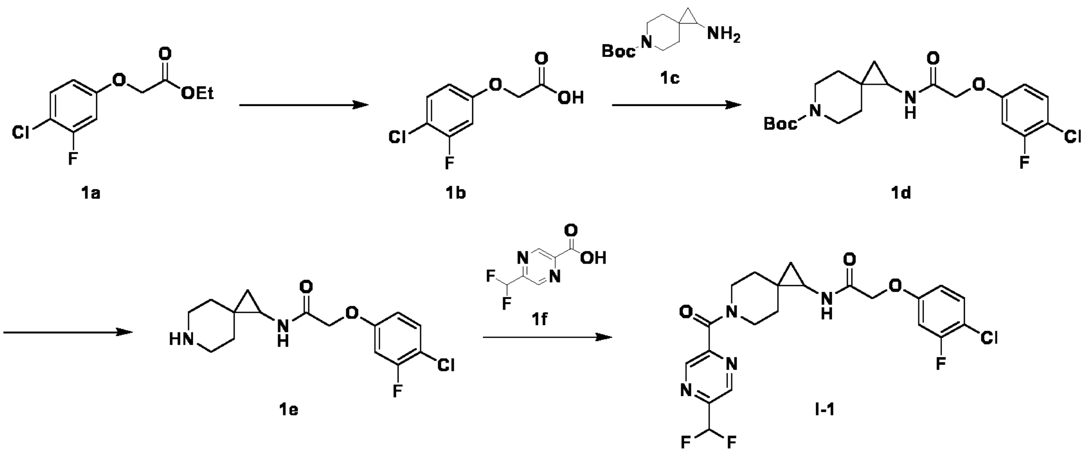

1a 1b 1c 1d 1e 1f I-1

Step 1: synthesis of intermediate 1b

To a mixed solution of tetrahydrofuran (40 mL), methanol (20 mL) and water (10 mL) were added starting material 1a (10 g, 42.98 mmol) and lithium hydroxide (5.97 mL, 214.92 mmol), and the reaction was stirred at 25°C for 18 hours. Then, the resulting reaction mixture was adjusted to pH = 7 with dilute hydrochloric acid (1 M) and extracted with ethyl acetate (100 mL x 3). The combined organic phase was washed with brine (50 mL), dried over anhydrous sodium sulfate and filtered, and the filtrate was concentrated to dryness under reduced pressure to obtain intermediate 1b.

$^1$H NMR(400 MHz, DMSO-$d_6$) δ13.21(s, 1H), 7.47(t, J=8.9 Hz, 1H), 7.07(dd, J=11.5, 2.9 Hz, 1H), 6.87-6.76(m, 1H), 4.73(s, 2H).

Step 2: synthesis of intermediate 1d

To N,N-dimethylformamide (5 mL) were added intermediate 1b (100 mg, 0.49 mmol), intermediate 1c (0.11 mL, 0.54 mmol, Bidepharm, BD215605), HATU (278.80 mg, 0.73 mmol) and DIPEA (0.24 mL, 1.47 mmol), and the reaction was stirred at 25°C for 1 hour. The resulting reaction mixture was quenched with a saturated sodium bicarbonate solution (20 mL) and extracted with ethyl acetate (30 mL x3). The combined organic phase was washed with brine (10 mL), dried over anhydrous sodium sulfate, filtered and concentrated to dryness under reduced pressure to obtain a crude. The crude was purified by preparative TLC (dichloromethane:methanol=20:1) to obtain intermediate 1d. MS m/z: 357.2[M+H-56]$^+$.

$^1$H NMR(400 MHz, $CDCl_3$) δ7.25 (t, *J*=8.6 Hz, 1H), 6.67(dd, *J*=10.3, 2.9 Hz, 1H), 6.61-6.56(m, 1H), 6.35(s, 1H), 4.40(s, 2H), 3.40-3.26(m, 4H), 2.65-2.59(m, 1H), 1.39(s, 9H), 1.31(dd, *J*=7.0, 4.3 Hz, 4H), 0.83(dd, *J*=7.9, 5.8 Hz, 1H), 0.41(d, *J*=5.8, 4.3 Hz, 1H).

Step 3: synthesis of intermediate 1e

To dichloromethane (5 mL) were added intermediate 1d (150 mg, 0.36 mmol) and trifluoroacetic acid (1 mL), and the reaction was stirred at 25°C for 1 hour. The mixture was concentrated to dryness under reduced pressure to obtain intermediate 1e.

MS m/z: 313.2 $[M+H]^+$ .

$^1H$ NMR(400MHz, $CDCl_3$) δ8.40 (s, 1H), 7.28(d, *J*=8.7 Hz, 1H), 6.76(s, 1H), 6.69(dd, *J*=10.1, 2.8 Hz, 1H), 6.62-.658(m, 1H), 4.48(s, 2H), 2.67-2.61(m, 1H), 1.93(d, *J*=11.7 Hz, 2H), 1.48(s, 2H), 1.32-1.16(m, 4H), 0.99(dd, *J*=8.0, 6.3 Hz, 1H), 0.64(t, *J*=5.5Hz, 1H).

Step 4: synthesis of compound I-1

To N,N-dimethylformamide (2 mL) were added intermediate 1e (30 mg, 0.10 mmol), starting material 1f (0.01 mL, 0.11 mmol), HATU (54.71 mg, 0.14 mmol) and DIPEA (0.05 mL, 0.29 mmol), and the reaction was stirred at 25°C for 1 hour. The resulting reaction mixture was quenched with a saturated sodium bicarbonate solution (20 mL) and extracted with ethyl acetate (30 mL x 3). The combined organic phase was washed with brine (10 mL), dried over anhydrous sodium sulfate, filtered and concentrated to dryness under reduced pressure to obtain a crude. The crude was purified by preparative TLC (eluent: petroleum ether:ethyl acetate =1:5) to obtain compound I-1.

MS m/z:469.3 $[M+H]^+$ . $^1H$ NMR(400MHz, DMSO-$d_6$) δ8.96 (d, *J*=3.9Hz, 2H), 8.23(dd, *J*=21.7, 4.1 Hz, 1H), 7.55-7.40(m, 1H), 7.34-6.97(m, 2H), 6.88-6.77(m, 1H), 4.58(d, *J*=19.6 Hz, 2H), 3.99-3.79(m, 1H), 3.54-3.39(m, 1H), 3.30-3.03(m, 2H), 2.64-2.54(m, 1H), 1.63-1.41(m, 2H), 1.27(d, *J*=43.9, 15.8 Hz, 2H), 0.83-0.74(m, 1H), 0.68-0.59(m, 1H).

Example 2: Synthesis of compound I-2

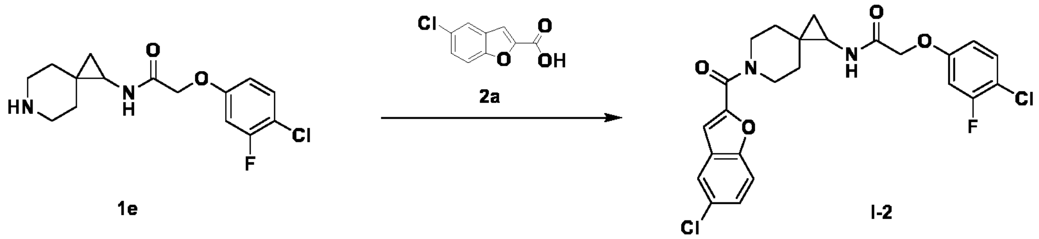


1e

I-2

Step 1: synthesis of compound I-2

To N,N-dimethylformamide (2 mL) were added intermediate 1e (30 mg, 0.10 mmol), starting material 2a (0.01 mL, 0.11 mmol), HATU (54.71 mg, 0.14 mmol) and DIPEA (0.05 mL, 0.29 mmol), and the reaction was stirred at 25°C for 2 hours. The resulting reaction mixture was quenched with a saturated sodium bicarbonate solution (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by preparative TLC (eluent: dichloromethane:methanol=20:1) to obtain compound I-2.

MS m/z: 491.3 $[M+H]^+$. $^1H$ NMR(400MHz, DMSO-$d_6$) δ8.24(d, *J*=4.0 Hz, 1H), 7.81(d, *J*=2.2 Hz, 1H), 7.71(d, *J*=8.8 Hz, 1H), 7.47(dd, *J*=8.8, 2.3 Hz, 2H), 7.31(s, 1H), 7.04(dd, *J*=11.6, 2.9 Hz, 1H), 6.84(dd, *J*=9.5, 2.6 Hz, 1H), 4.59(s, 2H), 3.95-3.43(m, 3H), 3.31(s, 1H), 2.64-2.57(m, 1H), 1.53(d, *J*=10.3 Hz, 2H), 1.30(d, *J*=12.0 Hz, 2H), 0.79(dd, *J*=8.1, 5.4 Hz, 1H), 0.65(t, *J*=5.0 Hz, 1H).

Example 3: Synthesis of compound I-3

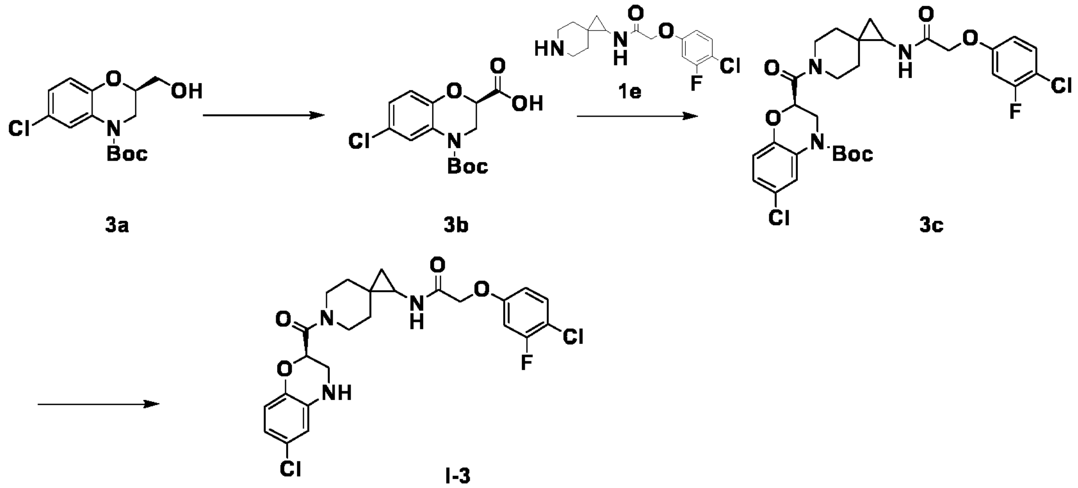


3a 3b 3c

I-3

Step 1: synthesis of intermediate 3b

To dichloromethane (5 mL) were added starting material 3a (80 mg, 0.27 mmol), tetrapropylammonium perruthenate (18.76 mg, 0.05 mmol) and NMO (187.60 mg, 1.60 mmol), and the reaction was stirred at 25°C for 2 hours. The resulting reaction mixture was concentrated to dryness under reduced pressure to obtain intermediate 3b.

MS m/z: 312.1 $[M-H]^-$ .

Step 2: synthesis of intermediate 3c

To N,N-dimethylformamide (2 mL) were added intermediate 3b (50 mg, 0.16 mmol), intermediate 1e (49.85 mg, 0.16 mmol), HATU (90.90 mg, 0.24 mmol) and DIPEA (0.08 mL, 0.48 mmol), and the reaction was stirred at 25°C for 2 hours. The resulting reaction mixture was quenched with a saturated sodium bicarbonate solution (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by preparative TLC (eluent: petroleum ether:ethyl acetate=1:5) to obtain intermediate 3c.

MS m/z :508.2 $[M+H-100]^+$ .

Step 3: synthesis of compound I-3

To dichloromethane (5 mL) were added intermediate 3c (50 mg, 0.08 mmol) and trifluoroacetic acid (1 mL), and the reaction was stirred at 25°C for 1 hour. The resulting reaction mixture was concentrated to dryness under reduced pressure to obtain a crude. Sodium bicarbonate (100 mg) was added to the crude, and the reaction was stirred at 25°C for 1 hour. The resulting mixture was filtered. The filter cake was washed with dichloromethane (20 mL x 3), and the filtrate was concentrated to dryness under reduced pressure to obtain a crude. The crude was purified by preparative TLC (eluent: dichloromethane:methanol=20:1) to obtain compound I-3.

MS m/z: 508.3 $[M+H]^+$. $^1$H NMR(400MHz, DMSO-$d_6$) δ8.21(s, 1H), 7.49(t, *J*=8.9 Hz, 1H), 7.03(d, *J*=10.4 Hz, 1H), 6.83(d, *J*=8.7 Hz, 1H), 6.67(d, *J*=9.5 Hz, 1H), 6.58(d, *J*=2.5 Hz, 1H), 6.45(dd, *J*=8.5, 2.5 Hz, 1H), 6.18(s, 1H), 4.87(d, *J*=11.3 Hz, 1H), 4.58(d, *J*=3.9 Hz, 2H), 3.87-3.52(m, 2H), 3.45-3.35(m, 2H), 2.63-2.55(m, 1H), 1.31(m, , 6H), 0.76(d, *J*=6.9 Hz, 1H), 0.61(t, *J*=5.2 Hz, 1H). Example 4: Synthesis of compound I-4

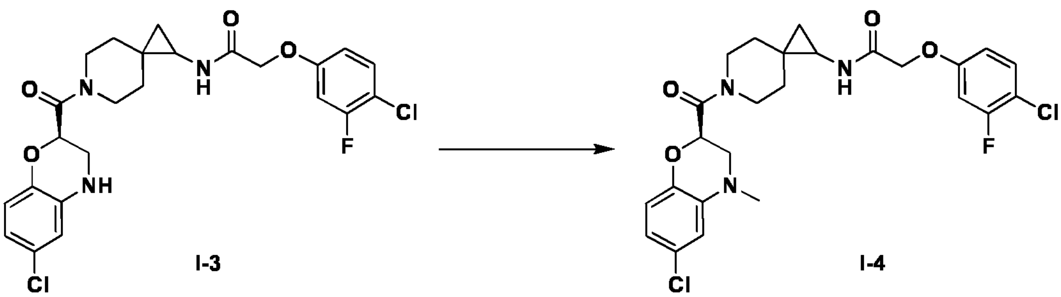

I-3 I-4

Step 1: synthesis of compound I-4

To acetonitrile (3 mL) were added compound I-3 (30 mg, 0.06 mmol), dimethyl sulfate (0.01 mL, 0.12 mmol) and potassium carbonate (24.47 mg, 0.18 mmol), and the mixture was degassed and subjected to nitrogen replacement 3 time. The reaction was stirred at 70°C for 18 hours. The resulting reaction mixture was poured into water (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by preparative TLC (eluent: dichloromethane:methanol=40:1) to obtain compound I-4.

MS m/z: 522.3 $[M+H]^+$. $^1H$ NMR(400 MHz, DMSO-$d_6$) δ8.21(s, 1H), 7.50(t, *J*=8.9 Hz, 1H), 7.03(d, *J*=9.8 Hz, 1H), 6.83(d, *J*=8.7 Hz, 1H), 6.74-6.64(m, 2H), 6.59-6.53(m, 1H), 5.08(d, *J*=12.3 Hz, 1H), 4.58(d, *J*=3.6 Hz, 2H), 3.62(m, 2H), 3.32-3.24(m, 2H), 2.86(q, *J*=1.8 Hz, 3H), 2.58(s, 1H), 1.47-1.11(m, 6H), 0.76(s, 1H), 0.62(s, 1H).

Example 5: Synthesis of compound I-5

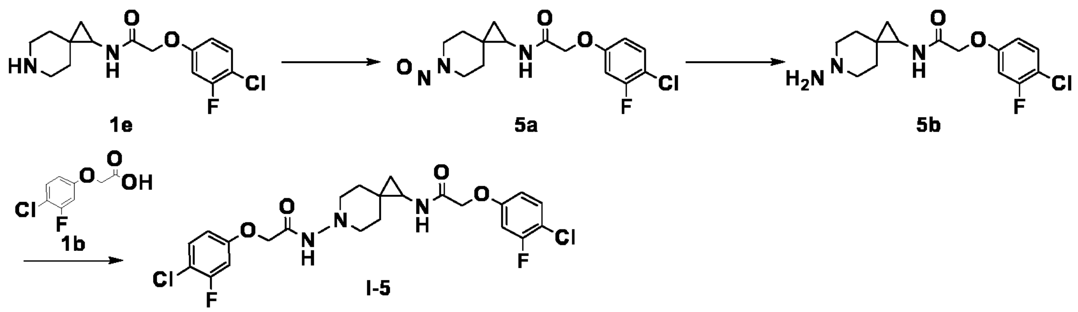

1e 5a 5b 1b I-5

Step 1: synthesis of intermediate 5a

To a mixed solution of water (3 mL) and glacial acetic acid (6 mL) were added intermediate 1e (100 mg, 0.32 mmol) and sodium nitrite (0.07 mL, 1.28 mmol), and the reaction was stirred at 25°C for 18 hours. The resulting reaction mixture was poured into water (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain intermediate 5a. MS m/z: 341.8 $[M+H]^+$.

Step 2: synthesis of intermediate 5b

To a mixed solution of tetrahydrofuran (2 mL) and water (2 mL) were added intermediate 5a (100 mg, 0.29 mmol), glacial acetic acid (0.5 mL) and zinc (97 mg, 1.48 mmol), and the reaction was stirred at 25°C for 18 hours. The reaction mixture was quenched with a saturated sodium bicarbonate solution (20 mL) and extracted with ethyl acetate (30 mL × 3) to obtain the organic phase. The combined organic phase was washed with brine (10 mL), dried over anhydrous sodium sulfate, filtered and concentrated to dryness under reduced pressure to obtain intermediate 5b. MS m/z: 312.8 $[M+H-16]^+$.

Step 3: synthesis of compound I-5

To N,N-dimethylformamide (3 mL) were added intermediate 5b (30 mg, 0.09 mmol), intermediate 1b (20.60 mg, 0.10 mmol), HATU (52.20 mg, 0.14 mmol) and DIPEA (0.05 mL, 0.27 mmol), and the reaction was stirred at 25°C for 18 hours. The mixture was poured into water (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by preparative TLC (eluent: dichloromethane:methanol=20:1) to obtain compound I-5.

MS m/z: 514.0 $[M+H]^+$. $^1H$ NMR(400 MHz, DMSO-$d_6$) δ9.01 (m, , 1H), 8.08(d, *J*=4.5 Hz, 1H), 7.53-7.41(m, 2H), 7.08-6.96(m, 2H), 6.86-6.73(m, 2H), 4.89(s, 1H), 4.55(d, *J*=7.1 Hz, 2H), 4.47(d, *J*=4.6 Hz, 1H), 3.06-2.72(m, 2H), 2.71-2.56(m, 2H), 1.90(s, 1H), 1.37(d, *J*=17.5 Hz, 2H), 1.27-0.89(m, 2H), 0.67(t, *J*=6.4 Hz, 1H), 0.56-0.46(m, 1H).

Example 6: Synthesis of compound I-6

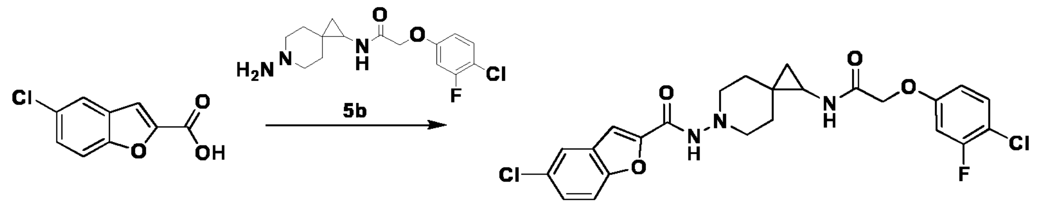


2a

I-6

Step 1: synthesis of compound I-6

To N,N-dimethylformamide (2 mL) were added intermediate 5b (30 mg, 0.09 mmol), starting material 2a (0.01 mL, 0.10 mmol), HATU (52.20 mg, 0.14 mmol) and DIPEA (0.05 mL, 0.27 mmol), and the reaction was stirred at 25°C for 18 hours. The resulting reaction mixture was poured into water (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by preparative TLC (eluent: dichloromethane:methanol=20:1) to obtain compound I-6.

MS m/z: 506.0 $[M+H]^+$. $^1$H NMR(400 MHz, DMSO-$d_6$) δ9.81(s, 1H), 8.09(d, *J*=4.6 Hz, 1H), 7.86(d, *J*=2.2 Hz, 1H), 7.69(d, *J*=8.7 Hz, 1H), 7.55-7.45(m, 3H), 7.04(dd, *J*=11.4, 2.9 Hz, 1H), 6.88-6.80(m, 1H), 4.57(s, 2H), 2.96-2.71(m, 4H), 1.45(s, 4H), 0.85(t, *J*=6.5 Hz, 1Hz), 0.70(d, *J*=8.0, 5.1 Hz, 1 Hz), 0.56(t, *J*=4.8 Hz, 1Hz).

Example 7: Synthesis of compound I-7

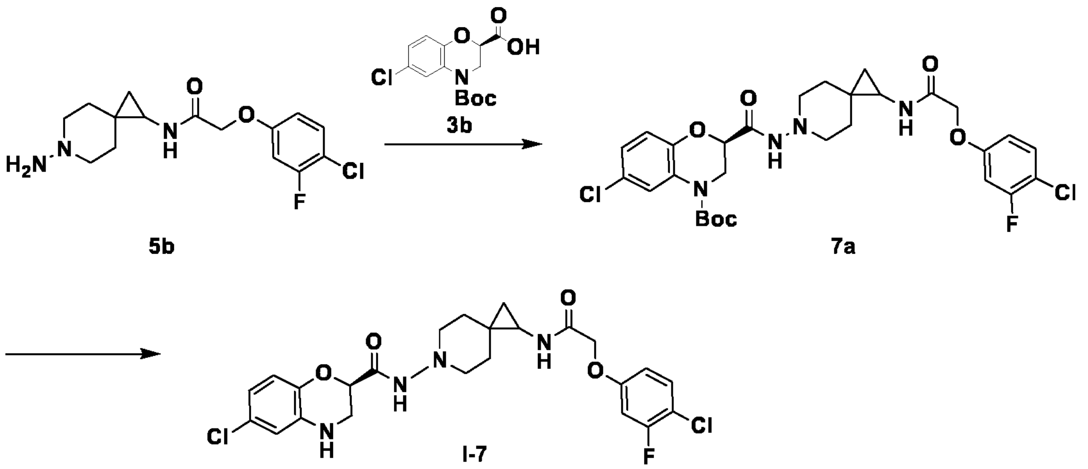


5b

7a

I-7

Step 1: synthesis of intermediate 7a

To N,N-dimethylformamide (5 mL) were added intermediate 5b (60 mg, 0.18 mmol), intermediate 3b (63.17 mg, 0.20 mmol), HATU (104.40 mg, 0.27 mmol) and DIPEA (0.09 mL, 0.55 mmol), and the reaction was stirred at 25°C for 18 hours. The resulting reaction mixture was quenched with a saturated sodium bicarbonate solution (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by preparative TLC (eluent: ethyl acetate:petroleum ether =4:1) to obtain intermediate 7a. MS m/z : 568.8 $[M+H-56]^+$ .

Step 2: synthesis of compound I-7

To a solution of dichloromethane (5 mL) were added intermediate 7a (50 mg, 0.08 mmol) and trifluoroacetic acid (1 mL), and the reaction was stirred at 25°C for 1 hour. The resulting reaction mixture was concentrated to dryness under reduced pressure to obtain a crude. The crude was dissolved in dichloromethane (5 mL), and the mixture was adjusted to pH = 7 with sodium carbonate and filtered. The filter cake was washed with dichloromethane (20 mL × 3). The filtrate was concentrated to dryness under reduced pressure to obtain compound I-7.

MS m/z: 524.4 [M+H]+. $^1$H NMR(400 MHz, DMSO-$d_6$) δ9.03 (s, 1H), 8.09(d, *J*=4.4 Hz, 1H), 7.49(td, *J*=8.9, 3.9 Hz, 1H), 7.03(dd, *J*=11.3, 2.9, 1.3 Hz, 1H), 6.85-6.80(m, 1H), 6.75(d, *J*=8.5 Hz, 1H), 6.61-6.55(m, 1H), 6.52-6.46(m, 1H), 6.15(d, *J*=33.4 Hz, 1H), 4.59-4.51(m, 2H), 4.38(dd, *J*=7.3, 2.8 Hz, 1H), 3.44-3.36(m, 1H), 3.22-3.15(m, 1H), 2.74(s, 2H), 2.69(s, 2H), 2.58(dt, *J*=8.3, 4.3 Hz, 1H), 1.39(s, 2H), 1.23(s, 2H), 0.70-0.65(m, 1H), 0.53(d, *J*=4.8 Hz, 1H).

Example 8: Synthesis of compound I-8

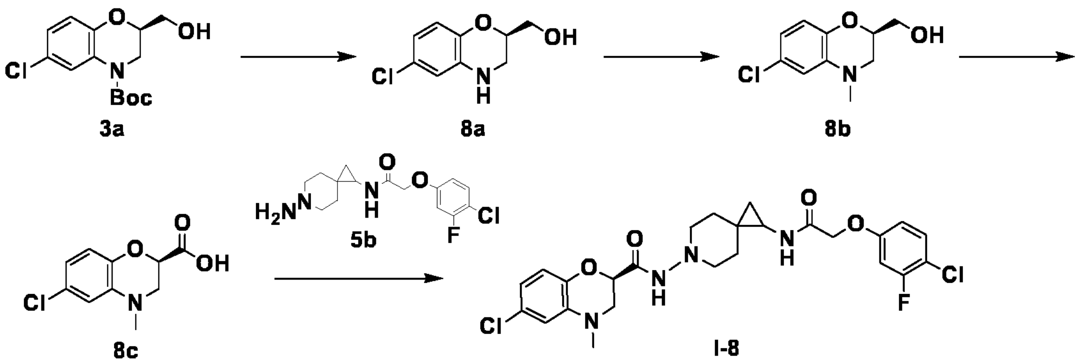

3a, 8a, 8b, 5b, 8c, I-8

Step 1: synthesis of intermediate 8a

To a solution of dichloromethane (5 mL) were added intermediate 3a (200 mg, 0.67 mmol) and trifluoroacetic acid (1 ml), and the resulting mixture was stirred at 25°C for 2 hours. The reaction solution was concentrated to obtain intermediate 8a. MS m/z: 200.6 [M+H]+.

Step 2: synthesis of intermediate 8b

To a solution of acetonitrile (10 mL) was added intermediate 8c (133 mg, 0.67 mmol). At 25°C, potassium carbonate (276.22 mg, 2.00 mmol) and dimethyl sulfate (338.03 mg, 2.68 mmol) were added. Under nitrogen atmosphere, the resulting mixture was stirred at 70°C for 18 hours. The reaction solution was filtered. The filter cake was washed with ethyl acetate (20 mL x 3), and the filtrate was concentrated to dryness under reduced pressure to obtain a crude. The crude was purified by preparative TLC (eluent: petroleum ether:ethyl acetate = 1:1) to obtain intermediate 8b. MS m/z: 214.6 [M+H]+ .

Step 3: synthesis of intermediate 8c

To a solution of dichloromethane (5 mL) was added a solution of intermediate 8b (100 mg, 0.33 mmol), tetrapropylammonium perruthenate (23.45 mg, 0.07 mmol) and NMO (234.50 mg, 2.00 mmol), and the reaction was stirred at 25°C for 2 hours. The resulting mixture was concentrated to dryness under reduced pressure to obtain intermediate 8c. MS m/z :228.6 [M+H]+ .

Step 4: synthesis of compound I-8

To a solution of N,N-dimethylformamide (3 mL) were added intermediate 5b (50 mg, 0.15 mmol), intermediate 8c (34.72 mg, 0.15 mmol), HATU (87.00 mg, 0.23 mmol) and DIPEA (0.08 mL, 0.46 mmol), and the reaction was stirred at 25°C for 2 hours. The resulting reaction mixture was poured into water (20 mL) and extracted with ethyl acetate (30 mL x 3) to obtain the organic phase. The combined organic phase was washed with brine (10 mL), dried over anhydrous sodium sulfate, filtered and concentrated to dryness under reduced pressure to obtain a crude. The crude was purified by HPLC (Phenomenex Gemini 150 mm * 25 mm * 10 um column (eluent: 30% to 60% (v / v) $CH_3CN$ and $H_2O$ and 0.025% HCOOH)) to obtain compound I-8. MS m/z: 537.4 [M+H]+.

Example 9: Synthesis of compound I-9

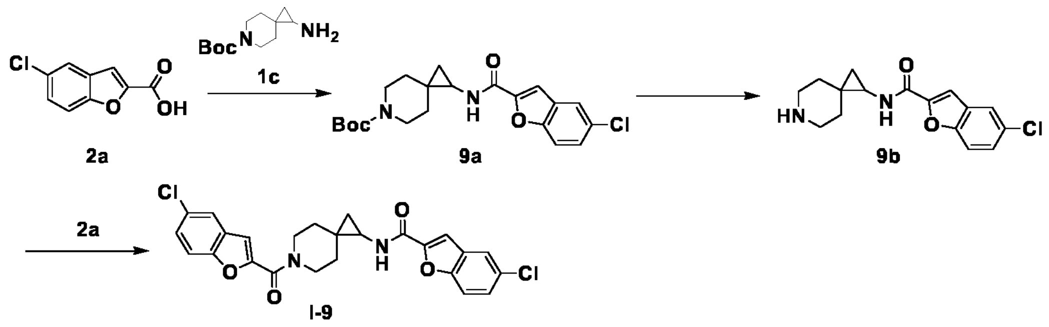


2a 9a 9b I-9

Step 1: synthesis of intermediate 9a

Starting material 2a (100 mg, 0.51 mmol), starting material 1c (115.12 mg, 0.51 mmol), DIPEA (197.24 mg, 1.53 mmol) and HATU (232.10 mg, 0.61 mmol) were added to DMF (5 mL), and the mixture was reacted at 25°C for 3 hours. The resulting mixture was washed with water (30 mL) and then extracted with ethyl acetate (10 mL x 3). Liquid separation was performed to obtain the organic phase. The organic phase was washed with saturated brine (30 mL), dried over anhydrous $Na_2SO_4$ and concentrated under reduced pressure to obtain a crude product. The crude product was separated and purified by column chromatography (mobile phase: v/v (PE/EtOAc)=20/1 to 1/1) to obtain intermediate 9a. MS m/z: 405.0 $[M+H]^+$.

Step 2: synthesis of intermediate 9b

Intermediate 9a (160 mg, 0.40 mmol) and TFA (2 mL) were added to DCM (10 mL), and the mixture was reacted at 25°C for 2 hours. The resulting reaction mixture was concentrated under reduced pressure to obtain intermediate 9b. MS m/z: 305.0 $[M+H]^+$.

Step 3: synthesis of compound I-9

Intermediate 9b (50 mg, 0.16 mmol), starting material 2a (26.88 mg, 0.14 mmol), DIPEA (53.01 mg, 0.42 mmol) and HATU (62.4 mg, 0.16 mmol) were added to DMF (1 mL), and the mixture was reacted at 25°C for 2 hours. The resulting reaction mixture was washed with water (10 mL) and then extracted with EtOAc (5 mL x 3). Liquid separation was performed to obtain the organic phase. The organic phase was washed with saturated brine (10 mL), dried over anhydrous $Na_2SO_4$ and concentrated under reduced pressure to obtain a crude product. The crude product was separated by preparative HPLC (eluent: v/v (acetonitrile/water) = 43% to 100% with 0.225% formic acid) to obtain compound I-9.

MS m/z: 483.0 $[M+H]^+$. $^1H$ NMR (400 MHz, DMSO) δ 8.91 (d, J = 3.5 Hz, 1H), 7.89 (d, J = 2.0 Hz, 1H), 7.80 (s, 1H), 7.70 (d, J = 8.8 Hz, 2H), 7.56 (s, 1H), 7.52 - 7.43 (m, 2H), 7.34 (s, 1H), 3.82 (d, J = 39.9 Hz, 2H), 2.79 (d, J = 3.8 Hz, 1H), 1.68 - 1.10 (m, 6H), 0.88 (dd, J = 11.9, 4.7 Hz, 2H).

Example 10: Synthesis of compound I-10

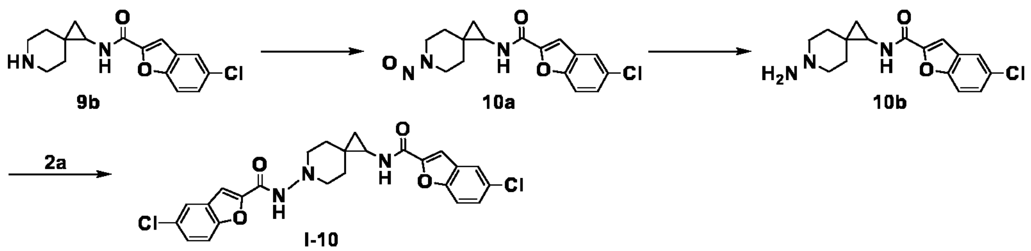


9b 10a 10b I-10

Step 1: synthesis of intermediate 10a

Intermediate 9b (70 mg, 0.23 mmol) and sodium nitrite (135.84 mg, 1.97 mmol) were added to AcOH (4 mL) and water (12 mL), and the mixture was reacted at 60°C for 18 hours. The resulting reaction mixture was washed with water (20 mL) and then extracted with DCM (5 mL x 3). Liquid separation was performed to obtain the organic phase. The organic

phase was washed with saturated brine (20 mL), dried over anhydrous $Na_2SO_4$ and concentrated under reduced pressure to obtain a crude product. The crude product was separated by TLC (PE/EtOAc (v/v)=1/1) to obtain intermediate 10a.
MS m/z: 334.0 $[M+H]^+$. $^1H$ NMR (400 MHz, DMSO) δ 8.95 (d, J = 3.9 Hz, 1H), 7.89 (dd, J = 4.2, 2.1 Hz, 1H), 7.70 (dd, J = 8.8, 5.0 Hz, 1H), 7.56 (d, J = 8.8 Hz, 1H), 7.52 - 7.47 (m, 1H), 4.17 (ddd, J = 107.1, 44.7, 36.3 Hz, 3H), 2.82 (ddd, J = 12.4, 7.9, 4.3 Hz, 1H), 1.62 (m, , 5H), 0.98 - 0.89 (m, 2H).

Step 2: synthesis of intermediate 10b

Intermediate 10a (60 mg, 0.18 mmol) and zinc powder (117.53 mg, 1.80 mmol) were added to a mixed solution of THF (2.5 mL), $H_2O$ (2.5 mL) and AcOH (0.5 mL), and the mixture was reacted at 25°C for 3 hours. The resulting reaction mixture was adjusted to pH 9 with aqueous ammonia (20%, 3 M) and then extracted with EtOAc (8 mL x 3). Liquid separation was performed to obtain the organic phase. The organic phase was washed and extracted with saturated brine (20 mL), dried over anhydrous $Na_2SO_4$ and concentrated under reduced pressure to obtain intermediate 10b. MS m/z: 320.0 $[M+H]^+$.

Step 3: synthesis of compound I-10

Intermediate 2a (33.5 mg, 0.17 mmol), intermediate 10b (59.94 mg, 0.19 mmol), DIPEA (66.07 mg, 0.51 mmol) and HATU (77.75 mg, 0.20 mmol) were added to DMF (5 mL), and the mixture was reacted at 25°C for 2 hours. The resulting reaction mixture was washed with water (10 mL) and then extracted with EtOAc (5 mL x 3). Liquid separation was performed to obtain the organic phase. The organic phase was washed and extracted with saturated brine (10 mL), dried over anhydrous $Na_2SO_4$ and concentrated under reduced pressure to obtain a crude product. The crude product was separated by preparative HPLC (eluent: 43% to 100% (v/v) $CH_3CN$ and $H_2O$ with 0.225% HCOOH) to obtain compound I-10.
MS m/z: 498.0 $[M+H]^+$.
$^1H$ NMR (400 MHz, DMSO) δ 9.87 (s, 1H), 8.78 (d, J = 4.5 Hz, 1H), 7.88 (dd, J = 9.3, 2.2 Hz, 2H), 7.70 (dd, J = 8.8, 3.5 Hz, 2H), 7.54 (d, J = 6.3 Hz, 2H), 7.50 - 7.47 (m, 2H), 2.98 - 2.81 (m, 5H), 1.56 (d, J = 15.0 Hz, 4H), 0.83 - 0.78 (m, 2H).

Example 11: Synthesis of compound II-10

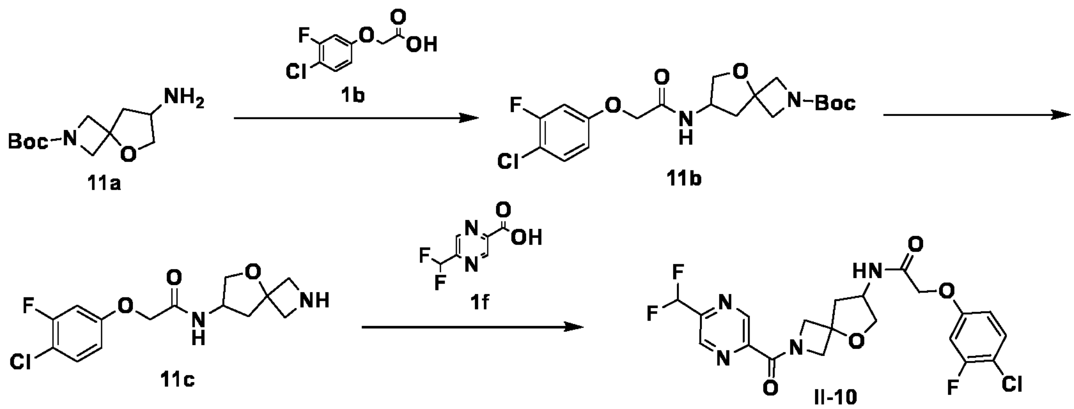
1b
11a
11b
1f
11c
II-10

Step 1: synthesis of intermediate 11b

To intermediate 1b (448.07 mg, 2.19 mmol) in DMF (10 mL) was added a solution of intermediate 11a (500 mg, 2.19 mmol), DIPEA (1.09 mL, 6.57 mmol) and HATU (999.36 mg, 2.63 mmol), and the mixture was stirred at 20°C for 1 hour. The reaction was poured into water (50 mL) and extracted with EtOAc (50 mL × 2) to obtain the organic phase. The organic phase was washed with a saturated NaCl solution (100 mL), and the organic layer was dried over $Na_2SO_4$, filtered and concentrated. The residue was purified by column chromatography (mobile phase: PE:EtOAc=1:1) and concentrated in vacuo to obtain intermediate 11b. MS m/z: 359.0 $[M+H-56]^+$. Step 2: synthesis of intermediate 11c
To intermediate 11b (100 mg, 0.24 mmol) in DCM (5 mL) was added TFA (1 mL, 13.42 mmol), and the reaction was stirred at 20°C for 3 hours and concentrated under reduced pressure to obtain intermediate 11c. MS m/z: 315.0 $[M+H]^+$.

Step 3: synthesis of compound II-10

To a solution of intermediate 11c (76 mg, 0.24 mmol) in DMF (5 mL) were added intermediate 1f (0.03 mL, 0.24 mmol), DIEA (0.12 mL, 0.72 mmol) and HATU (110.18 mg, 0.29 mmol), and the reaction was stirred at 20°C for 1 hour. The resulting reaction mixture was poured into water (20 ml) and extracted with EtOAc (20 ml × 3) to obtain the organic phase. The organic phase was combined and washed with a saturated NaCl solution (40 ml). The combined organic phase was dried over $Na_2SO_4$, filtered and concentrated. The residue was purified by preparative TLC (eluted with PE:EtOAc=1:1) and concentrated in vacuo to obtain compound II-10.

MS m/z: 471.0 $[M+H]^+$. $^1H$ NMR(400 MHz, DMSO-d6)δ9.22 (d, J=1.9Hz, 1H), 8.98(d, J=2.8 Hz, 1H), 8.34(t, J=5.9 Hz, 1H), 7.50(td, J=8.9, 6.7 Hz, 1H), 7.27(dd, J=54.0, 2.7 Hz, 1H), 7.07(dt, J=7.7, 2.9 Hz, 1H), 6.88-6.81(m, 1H), 4.66(dd, J=21.7, 11.0, 1.6 Hz, 1H), 4.57~4.51(m, 3H), 4.39-4.20(m, 2H), 4.15-4.06(m, 1H), 3.95(td, J=8.7, 6.2 Hz, 1H), 3.65(td, J=8.2, 4.5 Hz, 1H), 2.85(s, 1H), 2.69(s, 1H).

Example 12: Synthesis of compound II-11

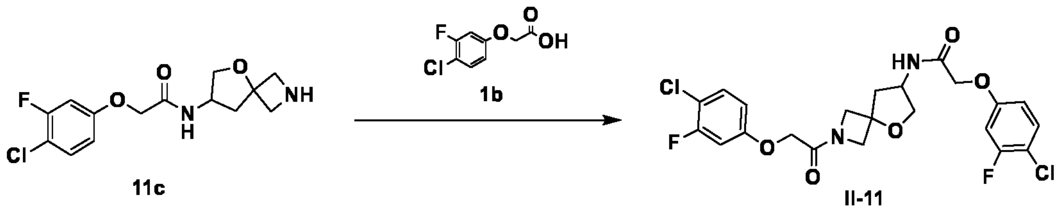

11c 1b II-11

Step 1: synthesis of compound II-11

To a solution of intermediate 11c (80 mg, 0.25 mmol), intermediate 1b (52 mg, 0.25 mmol) and DIEPA (0.17 mL, 1.02 mmol) in DMF (3 mL) was added HATU (116 mg, 0.31 mmol), and the mixture was stirred at 25°C for 2 hours. The resulting reaction mixture was poured into $H_2O$ (50 mL) and extracted with EtOAc (25 mL x 3) to obtain the organic phase. The combined organic phase was washed with saturated brine (30 mL x 2), dried over anhydrous $Na_2SO_4$, filtered and concentrated to dryness under reduced pressure to obtain a crude. The crude was purified by preparative TLC (PE:EtOAc=1:1) to obtain compound II-11.

MS m/z: 501.0 $[M+H]^+$. $^1H$ NMR (400 MHz, DMSO) δ 8.33 (d, J = 6.4 Hz, 1H), 7.49 (td, J = 8.9, 6.3 Hz, 2H), 7.07 (dt, J = 11.4, 3.3 Hz, 2H), 6.89 - 6.76 (m, 2H), 4.76 - 4.61 (m, 2H), 4.54 (s, 2H), 4.31 (dd, J = 16.9, 10.0 Hz, 2H), 4.21 (t, J = 9.0 Hz, 1H), 4.03 (dd, J = 32.2, 10.6 Hz, 1H), 3.95 - 3.81 (m, 2H), 3.63 (dd, J = 8.9, 4.4 Hz, 1H), 2.43 - 2.34 (m, 1H), 2.19 - 2.10 (m, 1H).

Example 13: Synthesis of compound II-12

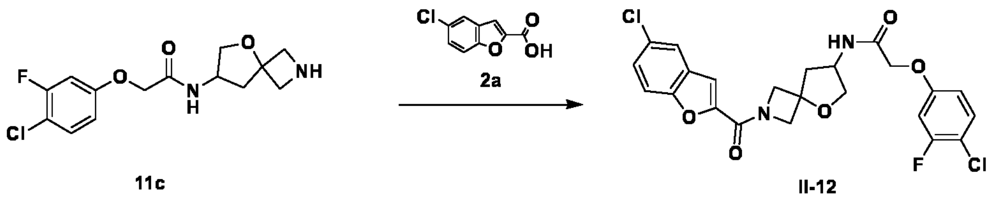

11c 2a II-12

Step 1: synthesis of compound II-12

To a solution of intermediate 11c (113 mg, 0.36 mmol), intermediate 2a (70 mg, 0.36 mmol) and DIEPA (0.24 mL, 1.44 mmol) in DMF (3 mL) was added HATU (164 mg, 0.43 mmol), and the mixture was stirred at 25°C for 2 hours. The resulting reaction mixture was poured into $H_2O$ (50 mL) and extracted with EtOAc (25 mL x 3) to obtain the organic phase. The combined organic phase was washed with brine (30 mL x 2), dried over anhydrous $Na_2SO_4$, filtered and concentrated to dryness under reduced pressure to obtain a crude. The crude was purified by preparative TLC (PE:EtOAc=7:3) to obtain compound II-12.

MS m/z: 493.0 [M+H]+.1H NMR(400 MHz, DMSO) δ8.37 (d, *J*=6.4 Hz, 1H), 7.85(s, 1H), 7.75-7.63(m, 1H), 7.59-7.43(m, 3H), 7.08(dd, *J*=11.4, 2.8 Hz, 1H), 6.85(dd, *J*=8.9, 1.9 Hz, 1H), 4.70(dd, *J*=26.2, 10.0 Hz, 1H), 4.61-4.50(m, 3H), 4.36(d, *J*=7.1 Hz, 1H), 4.22(dd, *J*=32.4, 10.6 Hz, 1H), 4.10-4.03(m, 1H), 3.97(dd, *J*=14.9, 5.9 Hz, 1H), 3.67(d, *J*=4.7 Hz, 1H), 2.44(d, *J*=12.1 Hz, 1H), 2.25-2.16(m, 1H).

Example 14: Synthesis of compound II-13

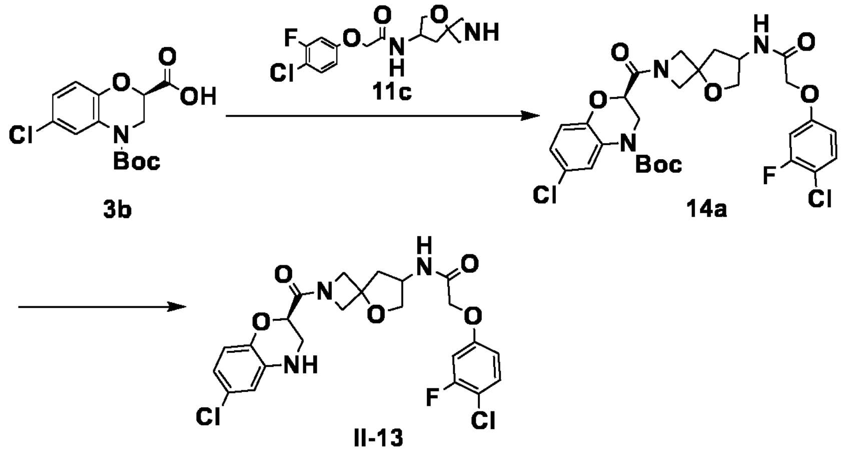

3b

11c

14a

II-13

Step 1: synthesis of intermediate 14a

To a solution of intermediate 11c (151.7 mg, 0.48 mmol), intermediate 3b (151 mg, 0.48 mmol) and HATU (219.92 mg, 0.58 mmol) in DMF (3 mL) was added DIPEA (0.32 mL, 1.93 mmol). The mixture was stirred at 25°C for 3 hours. The resulting reaction mixture was poured into water (20 mL) and then extracted with EtOAc (30 mL x 3) to obtain the organic phase. The combined organic phase was washed with saturated brine (10 mL), dried over anhydrous $Na_2SO_4$, filtered and then concentrated under reduced pressure to obtain a crude product. The crude product was purified by preparative TLC (eluent: EtOAc) to obtain intermediate 14a. MS m/z: 554.0 [M+H-56]+.

Step 2: synthesis of compound II-13

To a solution of intermediate 14a (140 mg, 0.23 mmol) in DCM (4 mL) was added $CF_3COOH$ (1 mL, 13.42 mmol) to form a mixture, and the mixture was stirred at 25°C for 20 h. The reaction was concentrated under reduced pressure to obtain a crude product, and then DCM (5 mL) and $Na_2CO_3$(30 mg) were added to the crude product. The mixture was stirred at 25°C for 30 min and filtered, and the filtrate was concentrated to obtain compound II-13.

MS m/z: 510.0 [M+H]+. 1H NMR(400 MHz, DMSO) δ8.42-8.24(m, 1H), 7.49(tdd, J=9.1, 6.2, 2.9 Hz, 1H), 7.07(dd, J=11.4, 2.7 Hz, 1H), 6.84(dd, J=9.0, 1.8 Hz, 1H), 6.78-6.68(m, 1H), 6.59(t, J=2.2 Hz, 1H), 6.52-6.42(m, 1H), 6.19(s, 1H), 4.79-4.66(m, 1H), 4.54(s, 2H), 4.52-3.72(m, 7H), 3.62(dd, J=8.9, 4.4 Hz, 1H), 3.30(d, J=12.4 Hz, 1H), 2.43-2.28(m, 1H), 2.20-2.06(m, 1H). Example 15: Synthesis of compound II-14

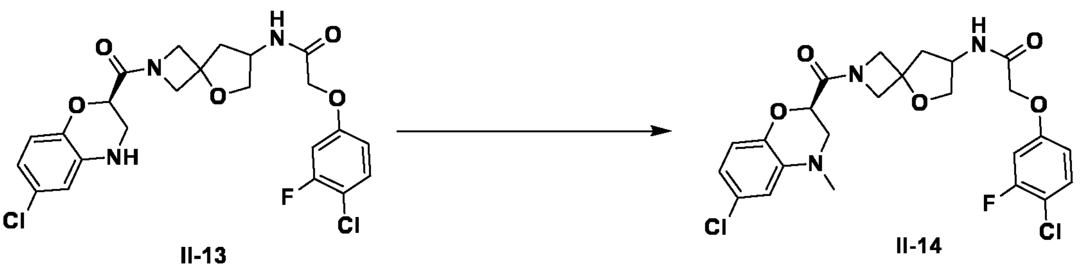

II-13

II-14

Step 1: synthesis of compound II-14

To a solution of compound II-13 in ACN (5 mL) were added dimethyl sulfate (0.02 mL, 0.20 mmol) and $K_2CO_3$

(40.62 mg, 0.29 mmol). The mixture was stirred at 70°C for 20 hours. Then, the resulting reaction mixture was concentrated under reduced pressure to obtain a crude product. The crude product was purified by preparative TLC (eluent: PE:EtOAc=2:1) to obtain compound II-14.

MS m/z: 524.0 $[M+H]^+$. $^1H$ NMR(400 MHz, DMSO) δ8.32(t, J=5.4Hz, 1H), 7.49(dd, J=11.3, 6.3 Hz, 1H), 7.07(dd, J=11.3, 2.6 Hz, 1H), 6.84(d, J=7.6 Hz, 1H), 6.74(ddd, J=16.4, 12.9, 5.1 Hz, 2H), 6.63-6.54(m, 1H), 4.89(s, 1H), 4.52(d, J=11.8 Hz, 2H), 4.51-3.70(m, 7H), 3.62(s, 1H), 3.30(d, J=9.1 Hz, 1H), 2.85(d, J=15.8 Hz, 3H), 2.43-2.33(m, 1H), 2.18-2.08(m, 1H).

Example 16: Synthesis of compound III-10

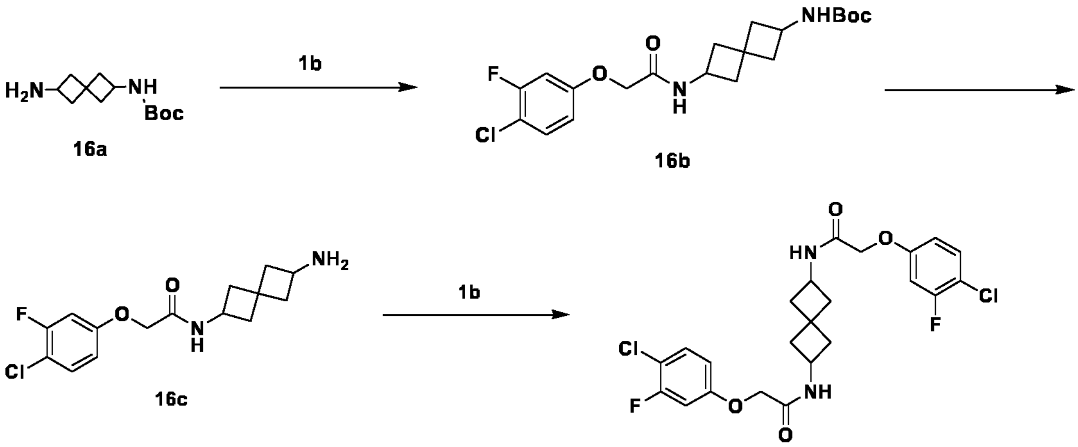


16a 16b 16c III-10

Step 1: synthesis of intermediate 16b

Intermediate 1b (100 mg, 0.49 mmol), intermediate 16a (121.69 mg, 0.54 mmol), DIPEA (189.53 mg, 1.47 mmol) and HATU (223.04 mg, 0.59 mmol) were sequentially added to DMF (1.5 mL), and the mixture was reacted at 25°C for 18 hours. The resulting reaction mixture was washed with water (30 mL) and then extracted with EtOAc (10 mL x 3). Liquid separation was performed to obtain the organic phase. The organic phase was washed and extracted with saturated brine (30 mL), dried over anhydrous $Na_2SO_4$ and concentrated under reduced pressure to obtain a crude product. The crude product was separated by column chromatography (PE/EtOAc (v/v)=1/1) to obtain intermediate 16b. MS m/z: 413.0 $[M+H]^+$.

Step 2: synthesis of intermediate 16c

Intermediate 16b (100 mg, 0.24 mmol) and TFA (1 mL) were added to DCM (5 mL), and the mixture was reacted at 25°C for 2 hours. The resulting reaction mixture was concentrated under reduced pressure to obtain intermediate 16c. MS m/z: 313.0 $[M+H]^+$.

Step 3: synthesis of compound III-10

Intermediate 16c (44 mg, 0.21 mmol), intermediate 1b (47 mg, 0.23 mmol), DIPEA (84 mg, 0.64 mmol) and HATU (102 mg, 0.26 mmol) were added to DMF (5 mL), and the mixture was reacted at 25°C for 18 hours. The resulting reaction mixture was washed with water (20 mL) and then extracted with EtOAc (5 mL x 3). Liquid separation was performed to obtain the organic phase. The organic phase was washed and extracted with saturated brine (20 mL), dried over anhydrous $Na_2SO_4$ and concentrated under reduced pressure to obtain a crude product. The crude product was separated by preparative TLC (eluent: petroleum ether:EtOAc=1:1) to obtain compound III-10.

MS m/z: 499.0 $[M+H]^+$. $^1H$ NMR(400 MHz, DMSO) δ8.29(d, *J*=7.8 Hz, 2H), 7.56-7.43(m, 2H), 7.06(dt, *J*=9.5, 4.7 Hz, 2H), 6.91-6.78(m, 2H), 4.49(d, *J*=14.7 Hz, 4H), 4.17(dt, *J*=16.1, 8.2 Hz, 2H), 2.40-2.31(m, 2H), 2.16(dd, *J*=7.8, 3.1Hz, 2H), 2.06-1.94(m, 4H).

Example 17: Synthesis of compound IV-11

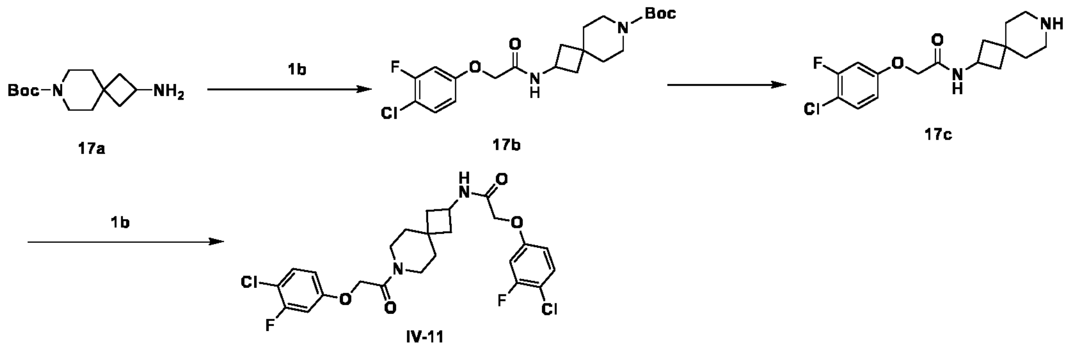


17a 17b 17c IV-11

Step 1: synthesis of intermediate 17b

To a solution of intermediate 1b (85.12 mg, 0.42 mmol) in DMF (5 mL) were added intermediate 17a (100 mg, 0.42 mmol), DIPEA (0.21 mL, 1.25 mmol) and HATU (189.84 mg, 0.50 mmol), and the mixture was stirred and reacted at 20°C for 1 hour. The resulting reaction mixture was poured into water (50 ml) and extracted with EtOAc (50 ml × 2) to obtain the organic phase. The resulting organic phase was washed with a saturated NaCl solution (100 ml), and the EtOAc layer (100 ml) was dried over $Na_2SO_4$, filtered and concentrated. The residue was separated and purified by silica gel column chromatography (mobile phase: PE/EtOAc (V/V)=1/1) and concentrated in vacuo to obtain intermediate 17b. MS m/z: 371.0 $[M+H-56]^+$.

Step 2: synthesis of intermediate 17c

To a solution of intermediate 17b (140 mg, 0.33 mmol) in DCM (10 mL) was added TFA (2 mL, 26.84 mmol), and the mixture was stirred and reacted at 20°C for 2 hours. After the reaction was completed, the reaction solution was concentrated under reduced pressure to obtain intermediate 17c. MS m/z: 327.0 $[M+H]^+$.

Step 3: synthesis of compound IV-11

To a solution of intermediate 1b (66.36 mg, 0.32 mmol) in DMF (5 mL) were added intermediate 17c (106 mg, 0.32 mmol), DIEA (0.16 mL, 0.97 mmol) and HATU (148.00 mg, 0.39 mmol), and the mixture was stirred and reacted at 20°C for 1 hour. The resulting reaction mixture was poured into water (50 ml) and extracted with EtOAc (50 ml × 2) to obtain the organic phase. The resulting organic phase was washed with a saturated NaCl solution (100 ml), and the EtOAc layer was dried over $Na_2SO_4$, filtered and concentrated. The resulting residue was separated and purified by silica gel column chromatography (mobile phase: PE/EtOAc (V/V)=1/1) and concentrated in vacuo to obtain compound IV-11.

MS m/z: 513.0 $[M+H]^+$. $^1H$ NMR(400 MHz, DMSO-$d_6$) δ8.35(t, *J*=7.4 Hz, 1H), 7.48(dt, *J*=20.2, 8.8 Hz, 2H), 7.12-7.02(m, 2H), 6.89-6.77(m, 2H), 4.87(d, *J*=7.7 Hz, 2H), 4.50(s, 2H), 4.29(p, *J*=7.9 Hz, 1H), 3.35(s, 4H), 2.16(t, *J*=9.7 Hz, 2H), 1.80(d, *J*=12.8 Hz, 2Hz), 1.55(dt, *J*=40.7, 15.8 Hz, 4Hz).

Example 18: Synthesis of compound V-10

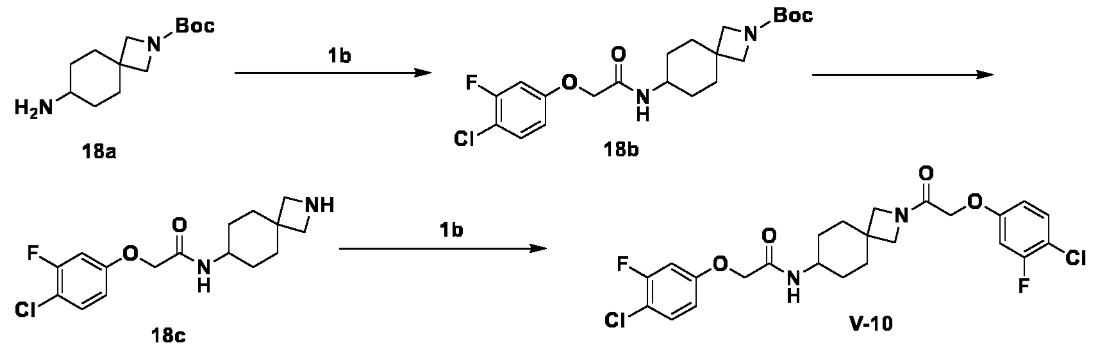


18a 18b 18c V-10

Step 1: synthesis of intermediate 18b

To a solution of intermediate 1b (85.12 mg, 0.42 mmol) in DMF (5 mL) were added 18a (100 mg, 0.42 mmol), DIPEA (0.21 mL, 1.25 mmol) and HATU (189.84 mg, 0.50 mmol), and the mixture was stirred and reacted at 20°C for 1 hour. The resulting reaction mixture was poured into water (50 ml) and extracted with EtOAc (50 ml × 2) to obtain the organic phase. The resulting organic phase was washed with a saturated NaCl solution (100 ml), and the EtOAc layer was dried over $Na_2SO_4$, filtered and concentrated. The resulting residue was separated and purified by silica gel column chromatography (mobile phase: PE/EtOAc (V/V)=1/1) and concentrated under reduced pressure to obtain intermediate 18b. MS m/z: 371.0 $[M+H-56]^+$. Step 2: synthesis of intermediate 18c

To a solution of intermediate 18b (90 mg, 0.21 mmol) in DCM (5 mL) was added TFA (1 mL, 13.42 mmol), and the mixture was stirred and reacted at 20°C for 1 hour. After the reaction was completed, the resulting reaction mixture was concentrated under reduced pressure to obtain intermediate 18c. MS m/z: 327.0 $[M+H]^+$.

Step 3: synthesis of compound V-10

To a solution of intermediate 1b (42.96 mg, 0.21 mmol) in DMF (5 mL) were added intermediate 18c (68.63 mg, 0.21 mmol), DIPEA (0.10 mL, 0.63 mmol) and HATU (95.82 mg, 0.25 mmol), and the mixture was stirred and reacted at 20°C for 1 hour. The resulting reaction mixture was poured into water (50 ml) and extracted with EtOAc (50 ml × 2) to obtain the organic phase. The resulting organic phase was washed with a saturated NaCl solution (100 ml), and the EtOAc layer (100 ml) was dried over $Na_2SO_4$, filtered and concentrated. The resulting residue was separated and purified by silica gel column chromatography (mobile phase: PE/EtOAc (V/V)=1/1) and concentrated in vacuo to obtain compound V-10. MS m/z: 513.0 $[M+H]^+$. $^1H$ NMR(400 MHz, DMSO-$d_6$) δ7.93(t, *J*=7.1Hz, 1H), 7.53-7.45(m, 2H), 7.07(dd, *J*=11.4, 3.9, 2.7 Hz, 2H), 6.87-6.79(m, 2H), 4.67(d, *J*=7.0 Hz, 2H), 4.50(d, *J*=1.9 Hz, 2H), 3.88(d, *J*=24.1 Hz, 2H), 3.61(s, 2H), 3.54(s, 1H), 1.85(d, *J*=12.9 Hz, 2Hz), 1.65(s, 2H), 1.50(t, *J*=13.0 Hz, 2H), 1.32-1.22(m, 2H).

Example 19: Synthesis of compound III-11

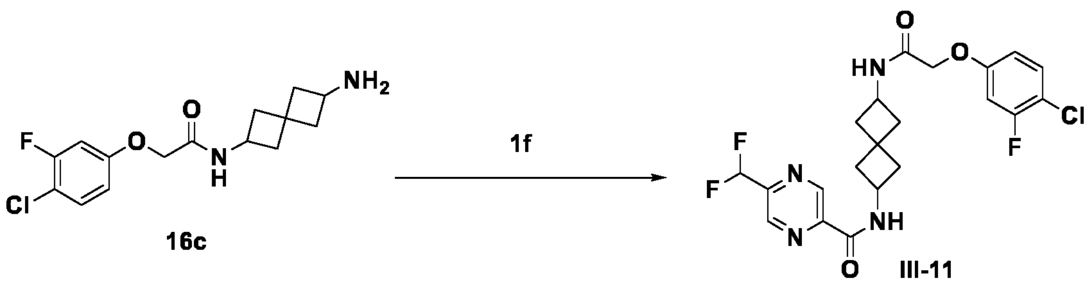


16c III-11

Step 1: synthesis of compound III-11

Intermediate 16c (100 mg, 0.32 mmol), intermediate 1f (50.61 mg, 0.29 mmol), DIPEA (112.70 mg, 0.87 mmol) and HATU (132.62 mg, 0.35 mmol) were added to DMF (5 mL), and the mixture was reacted at 25°C for 2 hours. To the resulting reaction mixture was added water (30 mL), and then the mixture was extracted with EtOAc (10 mL x 3). Liquid separation was performed to obtain the organic phase. The organic phase was washed with saturated brine (30 mL), dried

over anhydrous $Na_2SO_4$ and concentrated under reduced pressure to obtain a crude product. The crude product was separated and purified by column chromatography (PE/EtOAc (v/v)=1/1) to obtain compound III-11.

MS m/z: 469.0 $[M+H]^+$. $^1H$ NMR(400 MHz, DMSO) δ9.42-9.11(m, 2H), 9.01(s, 1H), 8.30(d, *J*=7.8 Hz, 1H), 7.49(d, *J*=8.9 Hz, 1H), 7.36-7.05(m, 2H), 6.85(dd, *J*=8.9, 2.7 Hz, 1H), 4.48(s, 2H), 4.37(dd, *J*=16.4, 8.2 Hz, 1H), 4.18(dd, *J*=16.3, 8.2 Hz, 1H), 2.40(s, 2H), 2.22(d, *J*=8.4 Hz, 4H), 2.04(s, 2H).

Example 20: Synthesis of compound III-13

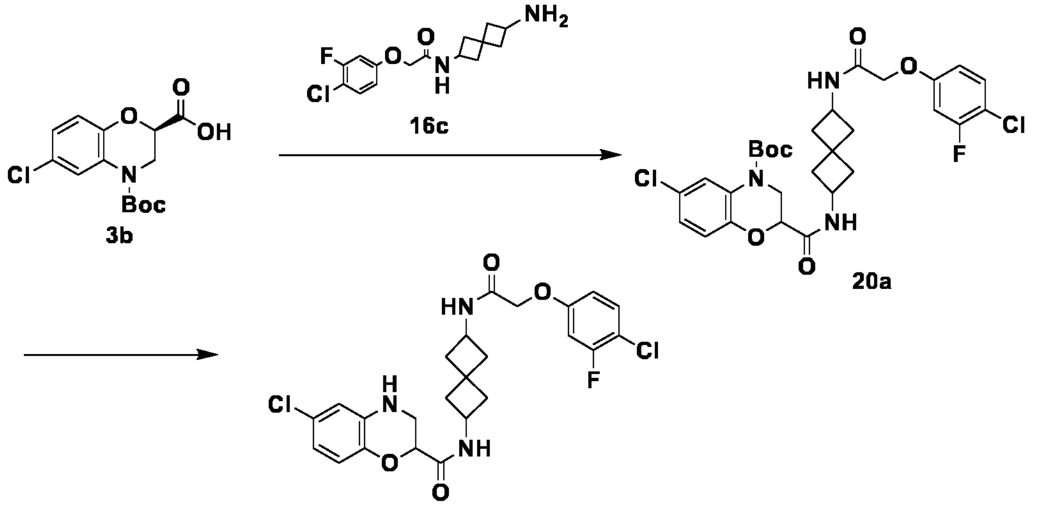

3b

16c

20a

III-13

Step 1: synthesis of intermediate 20a

To N,N-dimethylformamide (5 mL) were added intermediate 16c (90 mg, 0.29 mmol), intermediate 3b (99.30 mg, 0.32 mmmol), HATU (164.12 mg, 0.43 mmol) and DIPEA (ethyldiisopropylamine) (111.58 mg, 0.86 mmol), and the reaction was stirred at 25°C for 2 hours. The resulting reaction mixture was poured into water (20 mL) and extracted with ethyl acetate (30 mL x 3) to obtain the organic phase. The combined organic phase was washed with saturated brine (10 mL), dried over anhydrous sodium sulfate, filtered and concentrated to dryness under reduced pressure to obtain a crude. The crude was purified by preparative TLC (eluent: petroleum ether:ethyl acetate=10:1) to obtain intermediate 20a. MS m/z: 608.5$[M+H]^+$. Step 2: synthesis of compound III-13

To dichloromethane (5 mL) were added 20a (120 mg, 0.20 mmol) and trifluoroacetic acid (1 mL), and the reaction was stirred at 25°C for 1 hour. The resulting reaction mixture was concentrated to dryness under reduced pressure to obtain a crude. The crude was purified by preparative TLC (eluent: petroleum ether:ethyl acetate =5:1) to obtain compound III-13.

MS m/z: 508.4 $[M+H]^+$. $^1H$ NMR(400 MHz, DMSO-$d_6$) δ8.28(d, *J*=7.8 Hz, 1H), 8.18(d, *J*=7.8 Hz, 1H), 7.50(t, *J*=8.9 Hz, 1H), 7.07(dd, *J*=11.4, 2.8 Hz, 1H), 6.89-6.83(m, 1H), 6.78(d, *J*=8.5Hz, 1H), 6.59(dd, *J*=2.5, 1.0 Hz, 1H), 6.53-6.48(m, 1H), 6.18(s, 1H), 4.47(s, 2H), 4.41(dt, *J*=7.5, 2.8 Hz, 1H), 4.14(p, *J*=8.2 Hz, 2H), 3.42(d, *J*=12.2 Hz, 1H), 3.16(dd, *J*=11.9, 7.4 Hz, 1H), 2.38-2.28(m, 2H), 2.15(s, 2H), 2.08-1.95(m, 4H).

Example 21: Synthesis of compound IV-12

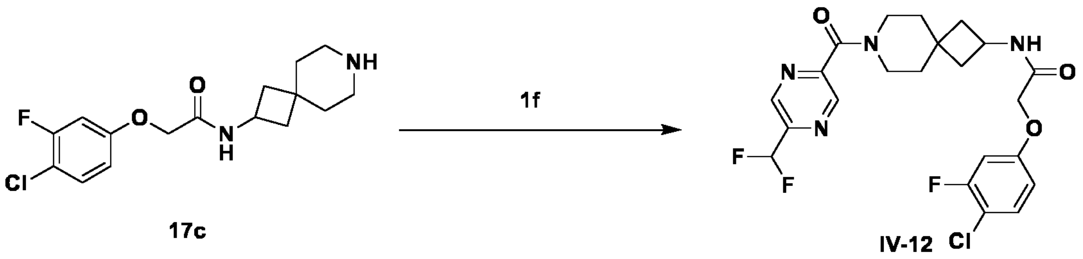


17c IV-12

Step 1: synthesis of compound IV-12

To a solution of intermediate 17c (53 mg, 0.16 mmol) in DMF (5 mL) were added intermediate 1f (28.24 mg, 0.16 mmol), DIPEA (0.08 mL, 0.48 mmol) and HATU (73.01 mg, 0.19 mmol), and the mixture was stirred and reacted at 20°C for 1 hour. The resulting reaction mixture was poured into water (20 ml) and extracted with EtOAc (20 ml × 2) to obtain the organic phase. The resulting organic phase was washed with a saturated NaCl solution (40 ml), and the EtOAc layer was dried over $Na_2SO_4$, filtered and concentrated. The resulting residue was separated and purified by preparative TLC (eluent: DCM/MeOH=20/1) and concentrated under reduced pressure to obtain compound IV-12.

MS m/z: 483.0 $[M+H]^+$. $^1$H NMR(400 MHz, DMSO-$d_6$)δ8.98(s, 1H), 8.94(s, 1H), 8.35(dd, *J*=11.8, 7.7 Hz, 1H), 7.50(td, *J*=8.9, 4.8 Hz, 1H), 7.08(dt, *J*=11.4, 3.0 Hz, 1H), 6.90-6.82(m, 1H), 4.50(d, *J*=4.0 Hz, 2H), 4.28(dq, J=16.4, 8.2 Hz, 1H), 3.67-3.51(m, 2H), 3.33-3.19(m, 3H), 2.19(q, *J*=10.0 Hz, 2H), 1.80(q, *J*=10.6 Hz, 2H), 1.58(dt, *J*=31.9, 26.1, 6.0 Hz, 4H).

Example 22: Synthesis of compound IV-13

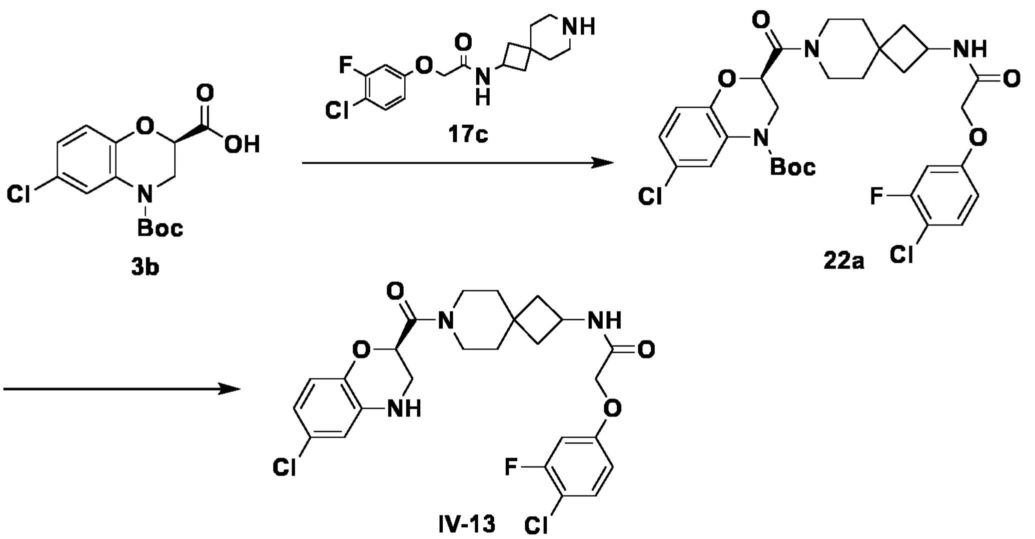


3b 22a IV-13

Step 1: synthesis of intermediate 22a

To a solution of intermediate 17c (53 mg, 0.16 mmol) in DMF (5 mL) were added intermediate 3b (50.88 mg, 0.16 mmol), DIPEA (0.08 mL, 0.48 mmol) and HATU (73.01 mg, 0.19 mmol), and the mixture was stirred and reacted at 20°C for 1 hour. The resulting reaction mixture was poured into water (50 ml) and extracted with EtOAc (50 ml × 2) to obtain the organic phase. The resulting organic phase was washed with a saturated NaCl solution (100 ml), and the EtOAc layer was dried over $Na_2SO_4$, filtered and concentrated. The resulting residue was separated and purified by silica gel column chromatography (mobile phase: PE/EtOAc (V/V)=1/1) and concentrated in vacuo to obtain intermediate 22a. MS m/z: 522.2 $[M+H-100]^+$.

Step 2: synthesis of compound IV-13

To a solution of intermediate 22a (90 mg, 0.14 mmol) in DCM (5 mL) was added TFA (0.5 mL, 6.71 mmol), and the

mixture was stirred and reacted at 20°C for 1 hour. The resulting reaction mixture was concentrated under reduced pressure, and the residue was separated and purified by preparative TLC (mobile phase: DCM/MeOH(V/V)=20/1) and concentrated in vacuo to obtain compound IV-13.

MS m/z: 522.0 $[M+H]^+$. $^1H$ NMR(400 MHz, DMSO-$d_6$) δ8.35(d, *J*=7.2 Hz, 1H), 7.51(t, J=8.9 Hz, 1H), 7.08(dd, *J*=11.4, 2.9 Hz, 1H), 6.86(dd, *J*=9.0, 2.9, 1.2 Hz, 1H), 6.68(d, *J*=8.5 Hz, 1H), 6.58(d, *J*=2.5 Hz, 1H), 6.46(dd, *J*=8.5, 2.6 Hz, 1H), 6.18(t, *J*=2.6 Hz, 1H), 4.90(d, *J*=8.5 Hz, 1H), 4.50(s, 2H), 4.28(q, *J*=8.2 Hz, 1H), 3.58-3.35(m, 3H), 3.26(dd, *J*=12.5, 7.0, 2.1 Hz, 1H), 2.16(q, *J*=12.9, 11.3 Hz, 2H), 1.79(t, *J*=9.9 Hz, 2H), 1.54(dd, *J*=43.5, 24.0 Hz, 4H).

Example 23: Synthesis of compound V-11

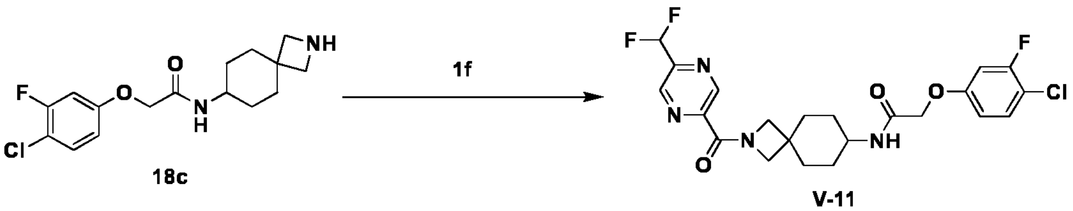


18c V-11

Step 1: synthesis of compound V-11

To a solution of intermediate 18c (23 mg, 0.07 mmol) in DMF (2 mL) were added intermediate 1f (0.01 mL, 0.07 mmol), DIPEA (0.03 mL, 0.21 mmol) and HATU (32.11 mg, 0.08 mmol), and the mixture was stirred and reacted at 20°C for 1 hour. The resulting reaction mixture was poured into water (20 ml) and extracted with EtOAc (20 ml × 2) to obtain the organic phase. The resulting organic phase was washed with a saturated NaCl solution (40 ml), and the EtOAc layer was dried over anhydrous $Na_2SO_4$, filtered and concentrated. The residue was separated and purified by preparative TLC (mobile phase: PE/EtOAc (V/V)=1/1) and concentrated in vacuo to obtain compound V-11. MS m/z: 483.0 $[M+H]^+$. $^1H$ NMR(400 MHz, DMSO-$d_6$) δ9.20(d, *J*=5.1Hz, 1H), 8.99(s, 1H), 7.92(dd, *J*=26.8, 8.0Hz, 1H), 7.50(td, *J*=8.8, 1.6 Hz, 1H), 7.27(dd, *J*=53.9, 3.3 Hz, 1H), 7.10-7.04(m, 1H), 6.85(d, *J*=9.0 Hz, 1H), 4.50(d, *J*=5.2Hz, 2H), 4.25(d, *J*=28.7 Hz, 2H), 3.80(d, *J*=26.3 Hz, 2H), 3.63(s, 1H), 1.91(d, *J*=12.9 Hz, 2H), 1.66(d, *J*=12.7 Hz, 2H), 1.54(t, *J*=12.6 Hz, 2H), 1.38-1.27(m, 2H).

Example 24: Synthesis of compound V-12

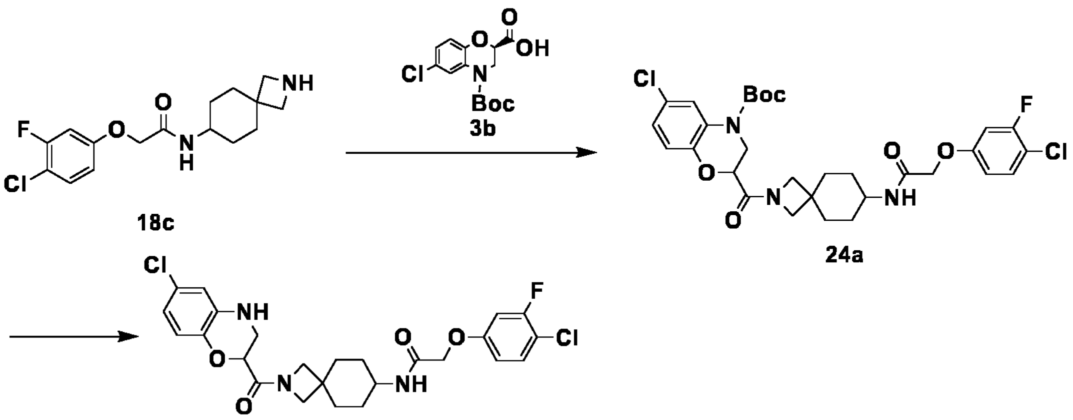

3b 18c 24a V-12

Step 1: synthesis of intermediate 24a

To a solution of intermediate 18c (38 mg, 0.12 mmol) in DMF (3 mL) were added intermediate 3b (36.48 mg, 0.12 mmol), DIPEA (0.06 mL, 0.35 mmol) and HATU (53.06 mg, 0.14 mmol), and the mixture was stirred and reacted at 20°C for 1 hour. The resulting reaction mixture was poured into water (50 ml) and extracted with EtOAc (50 ml × 2) to obtain the

organic phase. The resulting organic phase was washed with a saturated NaCl solution (100 ml), and the EtOAc layer (100 ml) was dried over $Na_2SO_4$, filtered and concentrated. The resulting residue was separated and purified by silica gel column chromatography (mobile phase: PE/EtOAc (V/V)=1/1) and concentrated in vacuo to obtain intermediate 24a. MS m/z: 566.2 $[M+H-56]^+$.

Step 2: synthesis of compound V-12

To a solution of intermediate 24a (48 mg, 0.08 mmol) in DCM (5 mL) was added TFA (0.5 mL, 6.71 mmol), and the mixture was stirred and reacted at 20°C for 1 hour. The reaction solution was concentrated under reduced pressure to obtain a crude. The crude was separated and purified by silica gel column chromatography (mobile phase: DCM/MeOH(V/V)=20/1) and concentrated in vacuo to obtain the target compound V-12. MS m/z: 522.0 $[M+H]^+$.

$^1H$ NMR(400 MHz, DMSO-$d_6$) δ7.92(dd, *J*=8.1, 3.6 Hz, 1H), 7.50(t, *J*=8.8 Hz, 1H), 7.06(dd, *J*=11.4, 2.8 Hz, 1H), 6.84(dd, *J*=8.9, 2.9, 1.2 Hz, 1H), 6.73(d, *J*=8.5 Hz, 1H), 6.59(dd, *J*=3.7, 2.5 Hz, 1H), 6.49(dd, *J*=8.3, 5.5, 2.5 Hz, 1H), 6.18(d, *J*=7.1 Hz, 1H), 4.70(dt, *J*=6.0, 3.0 Hz, 1H), 4.49(d, *J*=1.1 Hz, 2H), 3.97(dd, *J*=33.6, 28.2, 8.9 Hz, 2H), 3.64-3.55(m, 2H), 3.55-3.48(m, 1H), 3.42-3.36(m, 1H), 3.30(d, *J*=13.0 Hz, 1H), 1.81(d, *J*=18.3 Hz, 2H), 1.64(d, *J*=12.0 Hz, 2H), 1.47(d, *J*=13.2 Hz, 2H), 1.27(d, *J*=15.5 Hz, 2H).

Example 25: Synthesis of compound IV-15

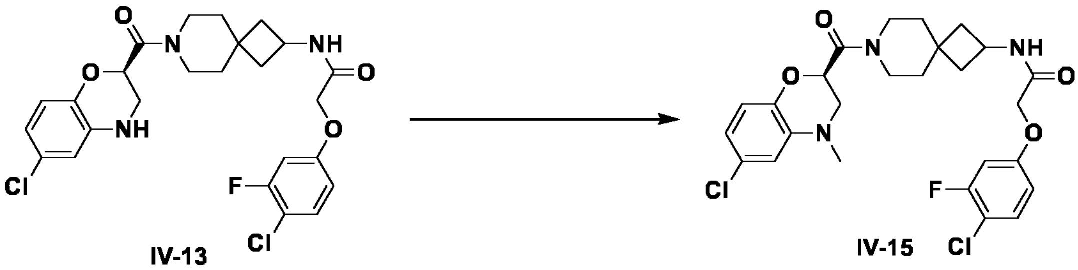

IV-13 IV-15

Step 1: synthesis of compound IV-15

To a solution of compound 22 (40 mg, 0.08 mmol) in $CH_3CN$ (5 ml) were added $K_2CO_3$ (31.75 mg, 0.23 mmol) and dimethyl sulfate (0.03 ml, 0.31 mmol), and the mixture was stirred and reacted at 70°C for 18 hours. The resulting reaction mixture was separated and purified by preparative TLC (mobile phase: DCM/MeOH (V/V)=20/1) to obtain compound IV-15.

MS m/z: 536.0 $[M+H]^+$. $^1H$ NMR(400 MHz, DMSO-$d_6$) δ8.35(d, *J*=7.8 Hz, 1H), 7.51(t, *J*=8.9Hz, 1H), 7.08(dd, *J*=11.3, 2.9 Hz, 1H), 6.86(dd, *J*=9.2, 2.9 Hz, 1H), 6.73-6.66(m, 2H), 6.56(dd, *J*=8.4, 2.4 Hz, 1H), 5.09(s, 1H), 4.50(s, 2H), 4.29(d, *J*=8.8 Hz, 1H), 3.58-3.36(m, 5H), 3.27(dd, *J*=12.3, 6.8Hz, 1H), 2.85(s, 3H), 2.15(d, *J*=21.8 Hz, 2H), 1.86-1.74(m, 2H), 1.55(dd, *J*=47.1, 22.9 Hz, 3H), 1.35(d, *J*=1.9 Hz, 1H).

Example 26: Synthesis of compound III-12

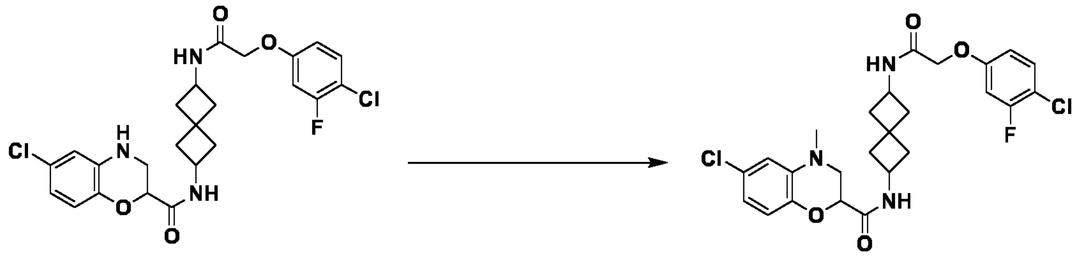

III-13 III-12

Step 1: synthesis of compound III-12

To acetonitrile (5 mL) were added compound III-13 (60 mg, 0.12 mmol), dimethyl sulfate (0.01 mL, 0.12 mmol) and potassium carbonate (16.31 mg, 0.12 mmol), and the mixture was degassed and subjected to nitrogen replacement 3 time. The reaction was stirred at 70°C for 18 hours. The resulting reaction mixture was poured into water (20 mL) and extracted with ethyl acetate (30 mL x 3) to obtain the organic phase. The combined organic phase was washed with saturated brine (10 mL), dried over anhydrous sodium sulfate, filtered and concentrated to dryness under reduced pressure to obtain a crude. The crude was separated and purified by preparative TLC (mobile phase: PE/EtOAc (V/V)=5/1) to obtain compound III-12.

MS m/z: 522.0 $[M+H]^+$. $^1H$ NMR(400 MHz, DMSO-$d_6$) $\delta$8.28(d, *J*=7.8Hz, 1H), 8.21(d, *J*=7.8 Hz, 1H), 7.50(t, *J*=8.8 Hz, 1H), 7.07(dd, *J*=11.4, 2.9 Hz, 1H), 6.87-6.79(m, 2H), 6.69(d, *J*=2.5 Hz, 1H), 6.62(dd, *J*=8.4, 2.5 Hz, 1H), 4.60(dt, *J*=7.3, 2.4 Hz, 1H), 4.47(s, 2H), 4.13(q, *J*=8.0Hz, 2H), 3.38(dd, *J*=12.0, 3.0 Hz, 1H), 3.17(dd, *J*=11.9, 7.2 Hz, 1H), 2.82(s, 3H), 2.37-2.32(m, 2H), 2.15(s, 2H), 2.08-1.95(m, 4H).

Example 27: Synthesis of compound IV-14

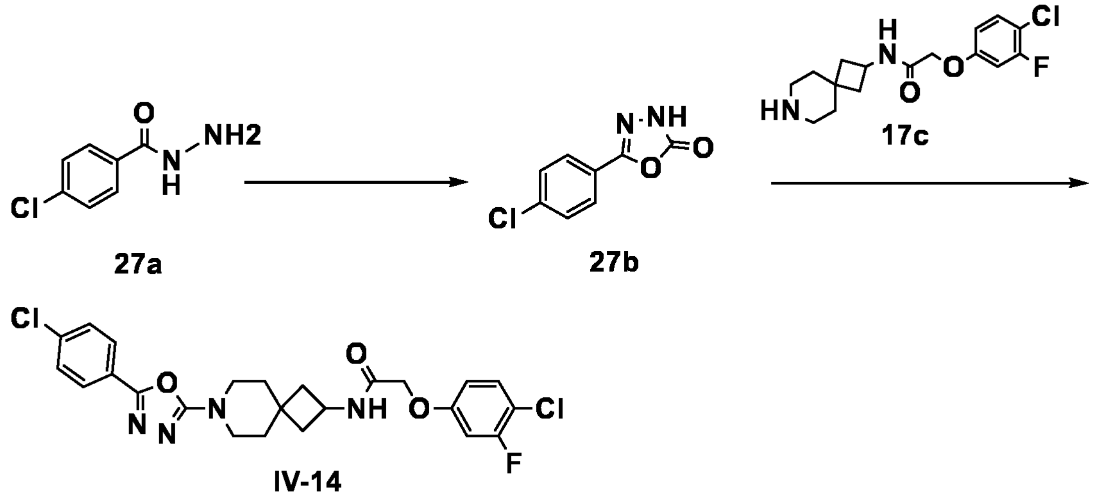

27a 27b 17c IV-14

Step 1: synthesis of intermediate 27b

At room temperature, to a 100 mL single-necked flask were added compound 27a (1.0 g, 5.86 mmol) and 1,2-dichloroethane (50.0 mL). Under stirring, the compound N,N'-carbonyldiimidazole (1.3 g, 8.21 mmol) was added. The mixture was reacted at 25°C for 16 hours. To the resulting reaction solution was added 25 mL of water. Liquid separation was performed. The resulting organic phase was washed with 100 mL of saturated brine, dried over anhydrous sodium sulfate and filtered, and the filtrate was concentrated under reduced pressure to obtain a crude. The crude was separated and purified by silica gel column chromatography to obtain intermediate 27b.

Step 2: synthesis of compound IV-14

At room temperature, to a 50 mL single-necked flask were added intermediate 17c (100 mg, 0.31 mmol) and N'N-dimethylformamide (5.0 mL). Under stirring, intermediate 27b (84 mg, 0.43 mmol), DIEA (159 mg, 1.22 mmol) and benzotriazol-1-yloxytris(dimethylamino)phosphonium hexafluorophosphate (BOP) (28 mg, 0.45 mmol) were sequentially added. The mixture was reacted at 25°C for 16 hours. To the resulting reaction solution was added 25 mL of water. Liquid separation was performed. The organic phase was washed with 100 mL of saturated brine, dried over anhydrous sodium sulfate and filtered, and the filtrate was concentrated under reduced pressure to obtain a crude. The crude was separated and purified by HPLC (ammonium bicarbonate/acetonitrile/water system) to obtain compound IV-14.

MS m/z: 505.0 $[M+H]^+$. $^1H$ NMR (400 MHz, $CDCl_3$-d) $\delta$ 7.86 - 7.83(m, 2H), 7.46 - 7.36 (m, 2H), 7.34 - 7.26(m, 1H), 6.80- 6.77 (m, 1H), 6.71 - 6.70 (m, 1H), 6.69- 6.60 (m, 1H), 4.56 - 4.49 (m, 1H), 4.44 (s, 2H), 3.65 - 3.60 (m, 4H), 2.47- 2.45 (m, 2H), 1.84 - 1.82 (m, 4H), 1.79 - 1.76 (m, 2H).

Example 28: Synthesis of compound IV-18

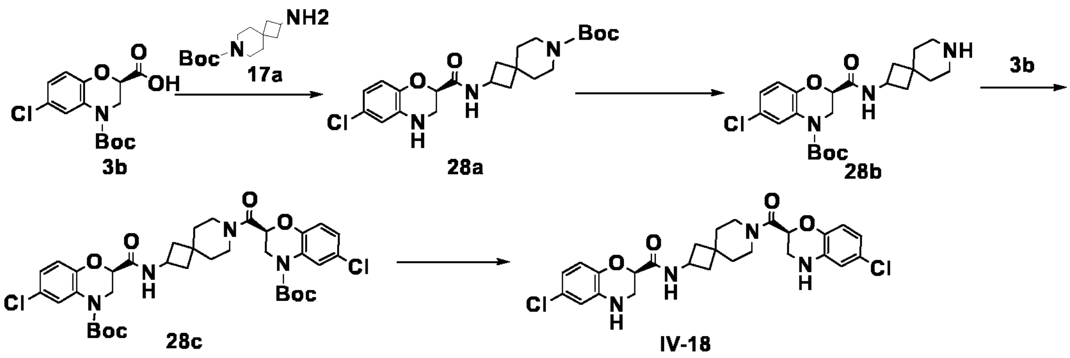


Step 1: synthesis of intermediate 28a

At room temperature, to a 50 mL single-necked flask were added intermediate 3b (85 mg, 0.27 mmol) and N'N-dimethylformamide (5 mL). Under stirring, intermediate 17a (65 mg, 0.27 mmol), 2-(7-azobenzotriazole)-N,N,N',N'-tetramethyluronium hexafluorophosphate (154 mg, 0.41 mmol) and DIEA (130 mg, 1.08 mmol) were sequentially added. The mixture was reacted at 25°C for 12 hours. To the resulting reaction solution was added 50 mL of water. The organic phase was washed with 100 mL of saturated brine, dried over anhydrous sodium sulfate and filtered, and the filtrate was concentrated under reduced pressure to obtain a crude. The crude was separated and purified by silica gel column chromatography to obtain intermediate 28a. MS m/z: 436.2 $[M+H]^+$.

Step 2: synthesis of intermediate 28b

At 25°C, to a 50 mL single-necked flask were added intermediate 28a (101 mg, 0.19 mmol) and dichloromethane (3 mL). Under stirring, trifluoroacetic acid (1 mL) was added. The mixture was reacted at 25°C for 30 minutes. The resulting reaction solution was concentrated under reduced pressure to obtain intermediate 28b. MS m/z: 336.0 $[M+H-100]^+$.

Step 3: synthesis of intermediate 28c

At room temperature, to a 50 mL single-necked flask were added intermediate 28b (76 mg, 0.23 mmol) and N'N-dimethylformamide (5 mL). Under stirring, intermediate 3b (92 mg, 0.29 mmol), 2-(7-azobenzotriazole)-N,N,N',N'-tetramethyluronium hexafluorophosphate (128 mg, 0.34 mmol) and DIEA (108 mg, 0.90 mmol) were sequentially added. The mixture was reacted at 25°C for 12 hours. To the resulting reaction solution was added 50 mL of water. The organic phase was washed with 100 mL of saturated brine, dried over anhydrous sodium sulfate and filtered, and the filtrate was concentrated under reduced pressure to obtain a crude. The crude was separated and purified by silica gel column chromatography to obtain intermediate 28c. MS m/z: 629.2$[M-H-100]^+$.

Step 4: synthesis of compound IV-18

At 25°C, to a 50 mL single-necked flask were added intermediate 28c (106 mg, 0.17 mmol) and dichloromethane (3 mL). Under stirring, trifluoroacetic acid (1 mL) was added. The mixture was reacted at 25°C for 30 minutes. The resulting reaction solution was concentrated under reduced pressure to obtain a crude. The crude was separated and purified by HPLC (ammonium bicarbonate/acetonitrile/water system) to obtain compound IV-18.

MS m/z: 531.0 $[M+H]^+$. $^1H$ NMR (400 MHz, DMSO-d6) $\delta$ 8.20 - 8.18 (m, 1H), 6.74 - 6.72 (m, 1H), 6.62 - 6.60(m, 1H), 6.53 - 6.50 (m, 2H), 6.43 - 6.41(m, 1H), 6.40 - 6.38 (m, 1H), 6.13 - 6.11 (m, 2H), 4.83 - 4.80 (m, 1H), 4.35 - 4.32 (m, 1H), 4.20 - 4.18 (m, 1H), 3.52 - 3.35 (m, 4H), 3.20- 3.18(m, 4H), 2.20 - 1.99 (m, 2H), 1.85 - 1.65 (m, 2H), 1.65 - 1.28 (m, 4H).

Example 29: Synthesis of compound IV-19

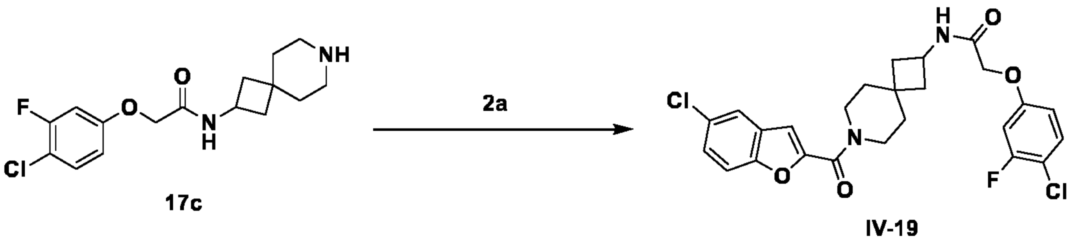


17c

IV-19

Step 1: synthesis of compound IV-19

To a solution of intermediate 17c (170 mg, 0.39 mmol) and intermediate 2a (85 mg, 0.43 mmol) in dichloromethane (6 mL) were added DIEA (102 mg, 0.79 mmol) and 1-propylphosphonic anhydride (493 mg, 0.77 mmol) to obtain a mixture. The mixture was stirred and reacted at 25°C for 12 h. The resulting reaction mixture was poured into $H_2O$ (15 mL) and extracted with EtOAc (10 mLx3) to obtain the organic phase. The combined organic layer was washed with saturated brine (20 mL), dried over anhydrous $Na_2SO_4$, filtered and concentrated. The resulting crude was separated and purified by preparative TLC (eluent: PE/EtOAc (V/V)=3/1) to obtain compound IV-19.

MS m/z: 505.1 $[M+H]^+$. $^1$H NMR (DMSO-d6 ) $\delta$: 8.35 (d, J=7.6 Hz, 1H), 7.81 (d, J=2.1 Hz, 1H), 7.71 (d, J=8.8 Hz, 1H), 7.44-7.53 (m, 2H), 7.31 (s, 1H), 7.05-7.11 (m, 1H), 6.86 (dd, J=8.9, 1.8 Hz, 1H), 4.50 (s, 2H), 4.24-4.35 (m, 1H), 3.62 (s, 2H), 3.54 (s, 2H), 2.16-2.24 (m, 2H), 1.76-1.87 (m, 2H), 1.61-1.69 (m, 2H), 1.55-1.61 (m, 2H).

Example 30: Synthesis of compound IV-20

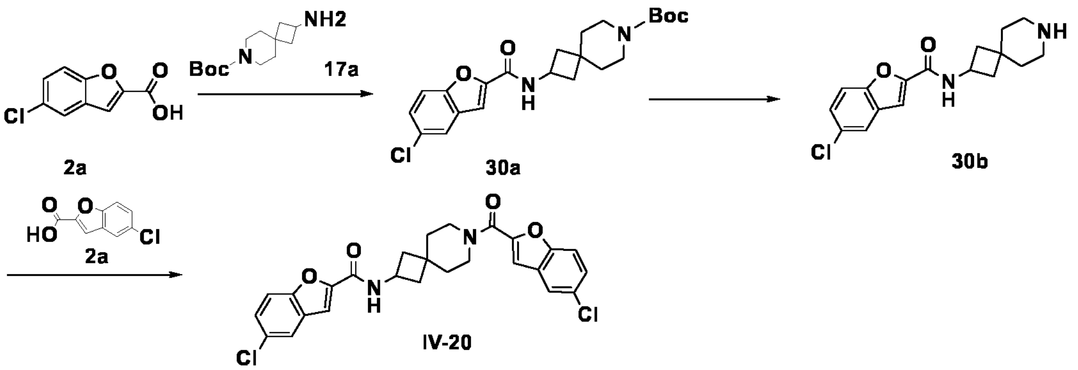


2a 30a 30b IV-20

Step 1: synthesis of intermediate 30a

At 25°C, to a 100 mL single-necked flask were added intermediate 2a (150 mg, 0.76 mmol) and DMF (5.0 mL). Under stirring, intermediate 17a (220 mg, 0.92 mmol), 1-propylphosphonic anhydride (970 mg, 1.53 mmol) and DIEA (395 mg, 3.04 mmol) were sequentially added. The mixture was reacted at 25°C for 16 h. The resulting reaction solution was poured into 50 mL of water and then extracted with EtOAc (50 mL x 3) to obtain the organic phase. The organic phase was washed with 100 mL of saturated brine, dried over anhydrous sodium sulfate and filtered, and the filtrate was concentrated under reduced pressure to obtain a crude. The crude was separated and purified by silica gel column chromatography to obtain intermediate 30a. MS m/z: 363.0 $[M+H-56]^+$.

Step 2: synthesis of intermediate 30b

At 25°C, to a 50 mL single-necked flask were added intermediate 30a (120 mg, 0.29 mmol) and dichloromethane (3 mL). Under stirring, trifluoroacetic acid (1 mL) was added. The mixture was reacted at 25°C for 30 minutes. The resulting reaction solution was concentrated under reduced pressure to obtain intermediate 30b. MS m/z: 319.0 $[M+H]^+$.

Step 3: synthesis of compound IV-20

At 25°C, to a 50 mL single-necked flask were added intermediate 30b (89 mg, 0.28 mmol) and DMF (5 mL). Under stirring, intermediate 2a (65 mg, 0.33 mmol), 1-propylphosphonic anhydride (355 mg, 0.56 mmol, 50% in dimethylformamide) and DIEA (134 mg, 1.12 mmol) were sequentially added. The mixture was reacted at 25°C for 16 h. The resulting reaction solution was poured into 50 mL of water and then extracted with EtOAc (50 mL x 3) to obtain the organic phase. The organic phase was washed with 100 mL of saturated brine, dried over anhydrous sodium sulfate and filtered, and the filtrate was concentrated under reduced pressure to obtain a crude. The crude was purified by HPLC (formic acid/acetonitrile /water system) to obtain compound IV-20.

MS m/z: 497.0 $[M+H]^+$. $^1H$ NMR (400 MHz, DMSO-d6) δ 9.05 (d, J = 7.7 Hz, 1H), 7.88 (d, J = 2.3 Hz, 1H), 7.83 (d, J = 2.2 Hz, 1H), 7.74- 7.70 (m, 2H), 7.53 (s, 1H) 7.51 - 7.46 (m, 2H), 7.33(s, 1H), 4.50- 4.44 (m, 1H), 3.61 (d, J = 31.6 Hz, 4H), 2.30 - 2.24 (m, 2H), 2.00- 1.94 (m, 2H), 1.70 - 1.62 (m, 4H).

Example 31: Synthesis of compound IV-16

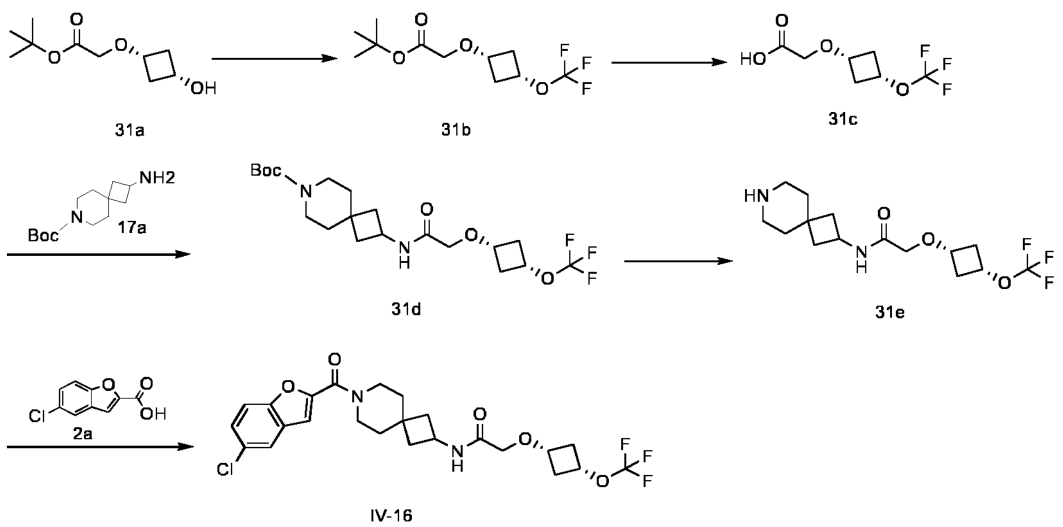


Step 1: synthesis of intermediate 31b

Under ice bath, to a 100 mL three-necked flask were added intermediate 31a (300 mg, 1.61 mmol) and ethyl acetate (20 mL). Under stirring in the dark, silver trifluoromethanesulfonate (1.41 g, 4.83 mmol), 1-chloromethyl-4-fluoro-1,4-diazoniabicyclo[2.2.2]octane bis(tetrafluoroborate) (855 mg, 2.42 mmol), potassium fluoride (374 mg, 6.44 mmol), 2-fluoropyridine (469 mg, 4.83 mmol) and (trifluoromethyl)trimethylsilane (686 mg, 4.83 mmol) were added. The mixture was reacted at 25°C for 16 h. The resulting reaction solution was filtered, and the filtrate was concentrated under reduced pressure to obtain a crude. The crude was separated and purified by silica gel column chromatography to obtain intermediate 31b. $^1H$ NMR (400 MHz, DMSO-d6) δ 4.62 - 4.35 (m, 1H), 3.92 (s, 2H), 3.73 (t, J = 6.9 Hz, 1H), 2.87 - 2.68 (m, 2H), 2.12 - 2.10 (m, 2H), 1.42 (s, 9H).

Step 2: synthesis of intermediate 31c

At 25°C, to a 50 mL single-necked flask were added intermediate 31b (246 mg, 0.97 mmol) and dichloromethane (3 mL). Under stirring, trifluoroacetic acid (1 mL) was added. The mixture was reacted at 25°C for 30 minutes. The resulting reaction solution was concentrated under reduced pressure to obtain intermediate 31c.

Step 3: synthesis of intermediate 31d

At 25°C, to a 50 mL single-necked flask were added intermediate 31c (230 mg, 1.07 mmol) and DMF (5 mL). Under stirring, intermediate 17a (283 mg, 1.18 mmol), 2-(7-azobenzotriazole)-*N*,*N*,*N'*,*N'*-tetramethyluronium hexafluorophosphate (610 mg, 1.60 mmol) and DIEA (556 mg, 4.28 mmol) were sequentially added. The mixture was reacted at 25°C for 12 h. To the resulting reaction solution was added 50 mL of water. The resulting organic phase was separated, washed with 100 mL of saturated brine, dried over anhydrous sodium sulfate and filtered, and the filtrate was concentrated under reduced pressure to obtain a crude. The crude was separated and purified by silica gel column chromatography to obtain intermediate 31d. MS m/z: 381.0 $[M+H-56]^+$.

Step 4: synthesis of intermediate 31e

At 25°C, to a 50 mL single-necked flask were added intermediate 31d (140 mg, 0.32 mmol) and dichloromethane (3 mL). Under stirring, trifluoroacetic acid (1 mL) was added. The mixture was reacted at 25°C for 30 minutes. The reaction solution was concentrated under reduced pressure to obtain intermediate 31e. MS m/z: 337.0 $[M+H]^+$.

Step 5: synthesis of compound IV-16

At 25°C, to a 50 mL single-necked flask were added intermediate 31e (66 mg, 0.21 mmol) and DMF (5 mL). Under stirring, intermediate 2a (50 mg, 0.25 mmol), 2-(7-azobenzotriazole)-*N*,*N*,*N*',*N*'-tetramethyluronium hexafluorophosphate (120 mg, 0.32 mmol) and DIEA (109 mg, 0.84 mmol) were sequentially added. The mixture was reacted at 25°C for 12 h. To the resulting reaction solution was added 50 mL of water. The resulting organic phase was separated, washed with 100 mL of saturated brine, dried over anhydrous sodium sulfate and filtered, and the filtrate was concentrated under reduced pressure to obtain a crude. The crude was separated and purified by HPLC (ammonium bicarbonate/acetonitrile/water system) to obtain compound IV-16.

MS m/z: 515.2 $[M+H]^+$. $^1H$ NMR (400 MHz, DMSO-d6) δ 8.04 (d, J = 8.0 Hz, 1H), 7.82 (d, J = 2.2 Hz, 1H), 7.73 - 7.71(m, 1H), 7.48 - 7.44 (m, 1H), 7.32 (s, 1H), 4.54 - 4.46 (m, 1H), 4.32 - 4.26 (m, 1H), 3.76 (s, 2H), 3.75 - 3.68 (m, 1H), 3.64 - 3.62 (m, 2H), 3.60 - 3.54 (m, 2H), 2.78 - 2.74(m, 2H), 2.20 - 2.14 (m, 4H), 1.86 - 1.80 (m, 2H), 1.66 - 1.58(m, 4H).

Example 32: Synthesis of compound IV-17

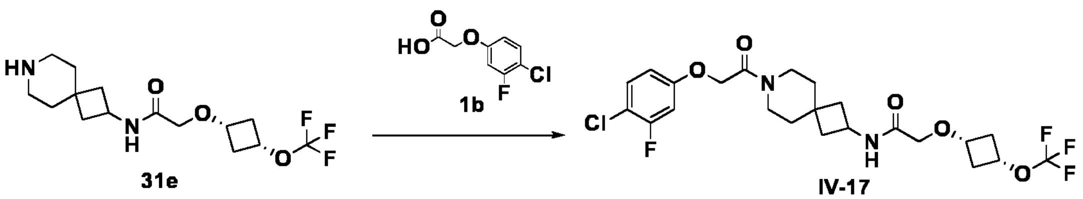


31e

IV-17

Step 1: synthesis of compound IV-17

At 25°C, to a 50 mL single-necked flask were added intermediate 31e (106 mg, 0.31 mmol) and DMF (5 mL). Under stirring, intermediate 1b (74 mg, 0.35 mmol), 2-(7-azobenzotriazole)-*N*,*N*,*N*',*N*'-tetramethyluronium hexafluorophosphate (78 mg, 0.21 mmol) and DIEA (161 mg, 1.24 mmol) were sequentially added. The mixture was reacted at 25°C for 12 h. To the resulting reaction solution was added 50 mL of water. The resulting organic phase was separated, washed with 100 mL of saturated brine, dried over anhydrous sodium sulfate and filtered, and the filtrate was concentrated under reduced pressure to obtain a crude. The crude was separated and purified by HPLC (formic acid/acetonitrile/water system) to obtain compound IV-17.

MS m/z: 523.2 $[M+H]^+$. $^1H$ NMR (400 MHz, DMSO-d6) δ 8.00 (t, J = 8.8 Hz, 1H), 7.46 (t, J = 8.9 Hz, 1H), 7.05 (ddd, J = 11.4, 2.8, 1.4 Hz, 1H), 6.88 - 6.73 (m, 1H), 4.86 (d, J = 7.9 Hz, 2H), 4.49 (p, J = 7.1 Hz, 1H), 4.35 - 4.20 (m, 1H), 3.73 (d, J = 18.1 Hz, 3H), 3.46 - 3.39 (m, 3H), 3.25 (d, J = 6.0 Hz, 1H), 2.75 (dtd, J = 9.7, 6.7, 3.3 Hz, 2H), 2.23 - 2.06 (m, 4H), 1.87 - 1.74 (m, 2H), 1.65 - 1.41 (m, 4H).

Example 33: Synthesis of compound VIII-1

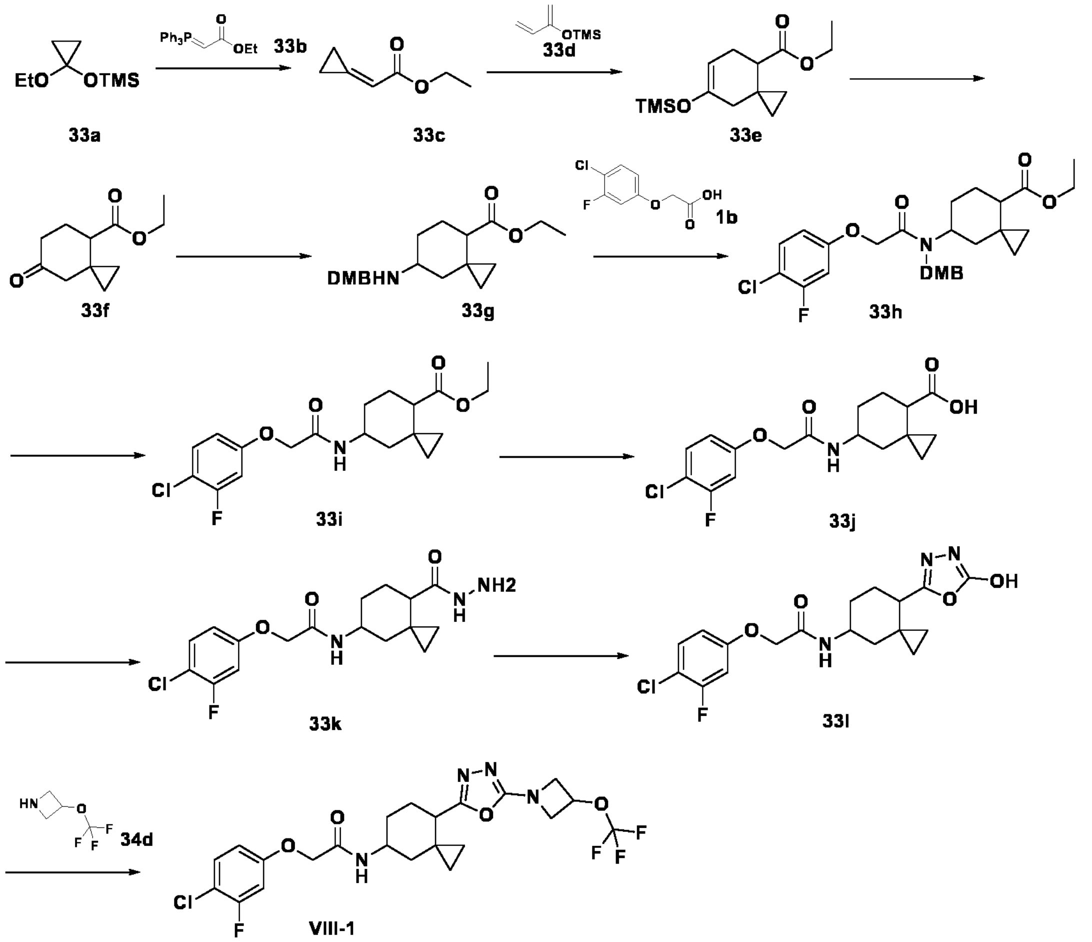


33a 33c 33e 33f 33g 33h 33i 33j 33k 33l VIII-1

Step 1: synthesis of compound 33c

At room temperature, to a single-necked flask were added 33a (11 g, 63.15 mmol) and dichloromethane (36 mL). Under stirring, 33b (20 g, 57.41 mmol), acetic acid (1.93 g, 32.15 mmol) and tetraethylene glycol dimethyl ether (40 mL) were added. The mixture was reacted at 100°C for 3 hours. The reaction solution was concentrated under reduced pressure. The crude was purified by flash column chromatography (petroleum ether:ethyl acetate =100:1-10:1) to obtain intermediate 33c. $^1$H NMR (400 MHz, DMSO) δ 6.22 (t, J = 1.9 Hz, 1H), 4.13 (m, 2H), 1.41 (m, 2H), 1.30 - 1.26 (m, 2H), 1.22 (t, J = 7.1 Hz, 3H).

Step 2: synthesis of compound 33e

At room temperature, to a single-necked flask were added 33c (2.3 g, 17.57 mmol) and toluene (10 mL). Under stirring, 33d (1.0 g, 1.03 mmol) was added. The mixture was reacted at 130°C for 18 hours. The reaction solution was concentrated under reduced pressure. The crude was purified by flash column chromatography (petroleum ether:ethyl acetate =100:1-7:1) to obtain intermediate 33e. LC-MS: m/z : 269.0 $(M+H)^+$.

Step 3: synthesis of compound 33f

At room temperature, to a single-necked flask were added 33e (2.4 g, 5.36 mmol), potassium fluoride (0.36 g, 8.05 mmol) and methanol (10 mL). The mixture was stirred, and reacted under nitrogen atmosphere for 2 hours. The reaction solution was concentrated under reduced pressure. The crude was purified by flash column chromatography (petroleum ether:ethyl acetate =100:1-5:1) to obtain intermediate 33f. LC-MS: m/z : 197.0 $(M+H)^+$.

Step 4: synthesis of compound 33g

At room temperature, to a single-necked flask were added 33f (877 mg, 1.45 mmol) and methanol (30 mL). Under stirring, acetic acid (80.0 mg, 0.44 mmol) and 2,4-dimethoxybenzylamine (1.76 g, 10.26 mmol) were added. At room

temperature, the mixture was reacted for two hours, and then cyano sodiumborohydride (258.03 mg, 6.6 mmol) was added. At room temperature, the resulting mixture was reacted for 16 hours. The reaction solution was concentrated under reduced pressure to obtain a crude. The crude was purified by flash column chromatography (petroleum ether:ethyl acetate =100:1-5:1) to obtain intermediate 33g. LC-MS: m/z : 348.0 $(M+H)^+$.

Step 5: synthesis of compound 33h

Under ice bath, to a single-necked flask were added 1b (1 g, 0.4 mmol) and dichloromethane (30 mL). Under stirring, oxalyl chloride (600 mg, 0.4 mmol) and three drops of N,N-dimethylformamide were added. At room temperature, the mixture was reacted for 30 minutes, and then the reaction solution was concentrated under reduced pressure to obtain a crude. The crude was dissolved in dichloromethane (20 mL), and then under ice bath, to the reaction solution were added triethylamine (4 mL) and 33g (1.45 g, 4.17 mmol). The mixture was reacted under ice bath for 2 hours. The reaction solution was concentrated under reduced pressure to obtain a crude. The crude was purified by flash column chromatography (petroleum ether:ethyl acetate =100:1-2:1) to obtain intermediate 33h. LC-MS: m/z : 534.1 $(M+H)^+$.

Step 6: synthesis of compound 33i

At room temperature, to a single-necked flask were added 33h (1.2 g, 2.24 mmol) and dichloromethane (30 mL). Under stirring, TFA (15 mL) was added. The mixture was reacted at room temperature for 2 hours. The reaction solution was concentrated under reduced pressure to obtain a crude. The crude was purified by flash column chromatography (petroleum ether:ethyl acetate =100:1-1:1) to obtain intermediate 33i (800 mg). LC-MS: m/z : 384.1 $(M+H)^+$. Step 7: synthesis of compound 33j

At room temperature, to a single-necked flask were added 33i (800 mg, 2.09 mmol), ethanol (20 mL) and water (7 mL). Under stirring, lithium hydroxide (160 mg, 4.10 mmol) was added. The mixture was reacted at room temperature for 3 hours. The reaction solution was adjusted to pH = 6 with hydrochloric acid (1 N) and extracted with ethyl acetate (20 mL x 3). The combined organic phase was washed with 50 mL of saturated brine, dried over sodium sulfate and filtered, and then the filtrate was concentrated under reduced pressure to obtain intermediate 33j. LC-MS: m/z : 356.1 $(M+H)^+$.

Step 8: synthesis of compound 33k

At room temperature, to a single-necked flask were added 33j (532 mg, 1.50 mmol) and tetrahydrofuran (20 mL). Under stirring, N,N'-carbonyldiimidazole (2.43 g, 15 mmol) and 80% hydrazine hydrate (1.0 mL) were added. The mixture was reacted at 60°C for 16 hours. The reaction solution was concentrated under reduced pressure to obtain a crude. The crude was purified by flash column chromatography (petroleum ether:ethyl acetate =100:1-0:1) to obtain intermediate 33k. LC-MS: m/z :370.1 $(M+H)^+$.

Step 9: synthesis of compound 33l

At room temperature, to a single-necked flask were added 33k (407 mg, 1.1 mmol) and 1,2-dichloroethane (10 mL). Under stirring, N,N'-carbonyldiimidazole (891 mg, 5.5 mmol) was added. The mixture was reacted at 80°C for 16 hours. The reaction solution was concentrated under reduced pressure to obtain a crude. The crude was purified by flash column chromatography (petroleum ether:ethyl acetate =100:1-0:1) to obtain compound 33l. LC-MS: m/z :396.0 $(M+H)^+$.

Step 10: synthesis of compound VIII-1

At room temperature, to a single-necked flask were added 33K (356 mg, 0.9 mmol) and N,N-dimethylformamide (3.0 mL). Under stirring, D (132 mg, 0.94 mmol), N,N-diisopropylethylamine (0.19 mL, 1.12 mmol) and benzotriazol-1-yloxytris(dimethylamino)phosphonium hexafluorophosphate (166 mg, 0.37 mmol) were added. The mixture was reacted at room temperature for 18 hours. The reaction solution was concentrated under reduced pressure to obtain a crude. The crude was purified by high performance liquid chromatography (ammonium bicarbonate/acetonitrile/water system) to obtain compound VIII-1. $^1H$ NMR (400 MHz, MeOD) δ 7.38 (t, J = 8.7 Hz, 1H), 6.94 (dd, J = 11.0, 2.7 Hz, 1H), 6.85 - 6.79 (m, 1H), 5.32 - 5.18 (m, 1H), 4.51 (s, 2H), 4.50 - 4.46 (m, 2H), 4.25 (dd, J = 9.5, 4.1 Hz, 2H), 4.04 (t, J = 10.2 Hz, 1H), 3.06 (d, J = 7.7 Hz, 1H), 2.14 - 1.90 (m, 3H), 1.85 - 1.73 (m, 1H), 1.56 (dd, J = 19.2, 10.5 Hz, 1H), 1.39 (d, J = 12.8 Hz, 1H), 0.50 - 0.27 (m, 4H), LC-MS: m/z : 519.1 $(M+H)^+$.

Example 34: Synthesis of compound III-1

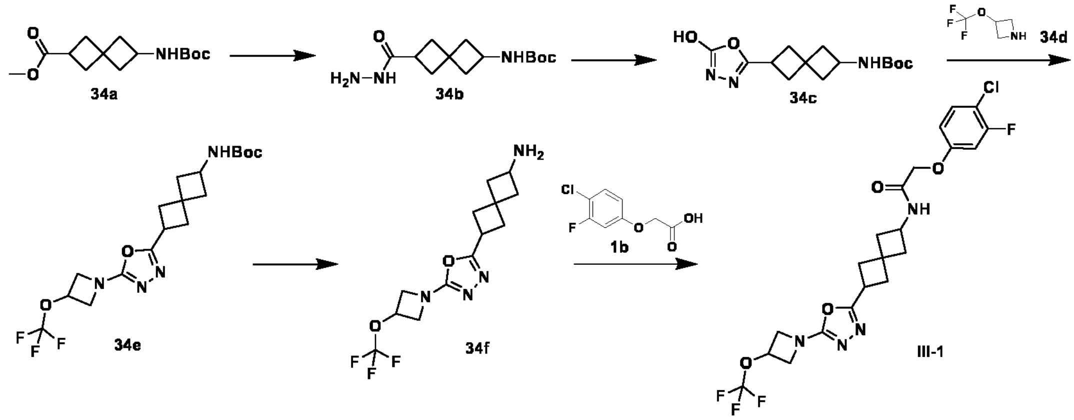

Step 1: synthesis of compound 34b

At room temperature, to a 100 mL single-necked flask were added 34a (2.5g, 9.28 mmol) and ethanol (20 mL). Under stirring, 50% hydrazine hydrate (9.83 g, 139.2 mmol) was added. The mixture was reacted at 80°C overnight. The reaction solution was concentrated under reduced pressure to obtain a crude. The crude was purified by recrystallization using an acetonitrile solvent, so as to obtain intermediate 34b (2.1 g, 7.02 mmol). LC-MS: m/z :214.0 $(M+H-56)^+$.

Step 2: synthesis of compound 34c

At room temperature, to a 100 mL single-necked flask were added 34b (2.1 g, 7.80 mmol) and 1,2-dichloroethane (25 mL). Under stirring, the compound N,N'-carbonyldiimidazole (1.9 g, 11.69 mmol) was sequentially added. The mixture was reacted at room temperature for 18 hours. To the reaction solution was added 50 mL of water, and the mixture was extracted with ethyl acetate. The organic phase was washed with 100 mL of saturated brine, dried over sodium sulfate and filtered, and then the filtrate was concentrated under reduced pressure to obtain a crude. The crude was purified by a column chromatography machine to obtain intermediate 34c (850 mg, 2.59 mmol). LC-MS: m/z : 240 $(M+H-56)^+$.

Step 3: synthesis of compound 34e

Under ice bath, to a 50 mL single-necked flask were added 34d (611 mg, 3.46 mmol) and dimethylformamide (10.0 mL). Under stirring, 2-(7-azobenzotriazole)-N,N,N',N'-tetramethyluronium hexafluorophosphate (1.93 g, 4.61 mmol), diisopropylethylamine (1.87 mg, 14.38 mmol) and compound 34c (850 mg, 2.88 mmol) were sequentially added. At room temperature, the mixture was reacted overnight. To the reaction solution was added 25 mL of water, and the mixture was extracted with ethyl acetate. The organic phase was washed with 50 mL of saturated brine, dried over sodium sulfate and filtered, and then the filtrate was concentrated under reduced pressure to obtain a crude. The crude was purified by a column chromatography machine to obtain intermediate 34e as a colorless oil. LC-MS: m/z : 419 $(M+H)^+$.

Step 4: synthesis of compound 34f

At room temperature, to a 100 mL single-necked flask were added 34e (220 mg, 0.53 mmol) and dichloromethane (8 mL). Under stirring, trifluoroacetic acid (3.0 mL) was added. The mixture was reacted at room temperature for 1 hour and concentrated under reduced pressure to obtain the crude 34f. LC-MS: m/z: 319 $(M+H)^+$.

Step 4: synthesis of compound III-1

Under ice bath, to a 50 mL single-necked flask were added 1b (115 mg, 0.56 mmol) and dimethylformamide (5.0 mL). Under stirring, 2-(7-azobenzotriazole)-N,N,N',N'-tetramethyluronium hexafluorophosphate (268 mg, 0.71 mmol) and diisopropylethylamine (245 mg, 1.88 mmol) were sequentially added. The mixture was reacted at room temperature for half an hour, and then compound 34f (150 mg, 0.47 mmol) was added to the reaction solution. At room temperature, the mixture was reacted overnight. To the reaction solution was added 25 mL of water, and the mixture was extracted with ethyl acetate. The organic phase was washed with 100 mL of saturated brine, dried over sodium sulfate and filtered, and then the filtrate was concentrated under reduced pressure to obtain a crude. The crude was purified by high performance liquid chromatography (ammonium bicarbonate/acetonitrile/water system) to obtain compound III-1.

$^1H$ NMR (400 MHz, DMSO-d6) δ 8.30 - 8.28 (m, 1H), 7.52 - 7.47(m, 1H), 7.09 - 7.05(m, 1H), 6.86 - 6.83(m, 1H),

5.33 - 5.30 (m, 1H), 4.48 - 4.44 (m, 4H), 4.21 - 4.13 (m, 3H), 3.48 - 3.44 (m, 1H), 2.51 - 2.43 (m, 2H), 2.33- 2.23 (m, 4H), 2.07 - 2.01 (m, 2H), LC-MS: m/z : 505.0 (M+H)+. Example 35: Synthesis of compound VI-1

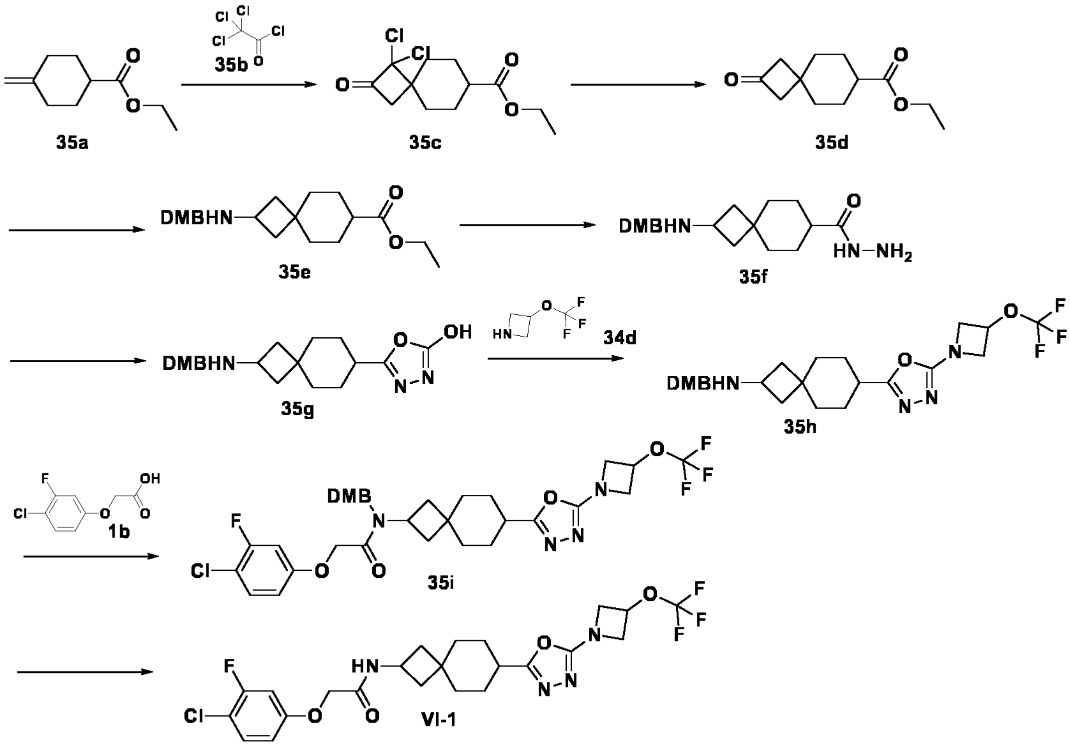


Step 1: synthesis of compound 35c

Under ice bath, to a single-necked flask were added 35a (3 g, 17.83 mmol), a zinc copper reagent (4.6 g, 35.66 mmol) and tetrahydrofuran (50 mL). Under stirring, 35b (6.04 mL, 53.50 mmol) was slowly added dropwise for 20 minutes. The mixture was reacted at 35°C for 16 hours. The reaction solution was filtered, and the filtrate was concentrated under reduced pressure to obtain a crude. The crude was separated by flash column chromatography (petroleum ether:ethyl acetate = 100:1 - 5:1) to obtain intermediate 35c.

Step 2: synthesis of compound 35d

At room temperature, to a single-necked flask were added 35c (4.7 g, 16.84 mmol) and acetic acid (10 mL). Under stirring, zinc powder (4.4 g, 67.34 mmol) was added. The mixture was reacted at 100°C for 16 hours. The reaction solution was filtered, and the filtrate was concentrated under reduced pressure to obtain a crude. The crude was separated by flash column chromatography (petroleum ether:ethyl acetate = 100:1 - 4:1) to obtain intermediate 35d. LC-MS: m/z : 211.0 (M+H)+.

Step 3: synthesis of compound 35e

At room temperature, to a single-necked flask were added 35d (1 g, 4.76 mmol) and methanol (35 mL). Under stirring, 2,4-dimethoxybenzylamine (0.86 mL, 5.71 mmol) was added. At room temperature, the mixture was reacted for 16 hours, and then cyano sodiumborohydride (597 mg, 9.51 mmol) was added to the reaction solution. At room temperature, the mixture was reacted for 6 hours. The reaction solution was concentrated under reduced pressure to obtain a crude. The crude was separated by flash column chromatography (dichloromethane:methanol=100:1-10:1) to obtain intermediate 35e. LC-MS: m/z : 362.2 (M+H)+.

Step 4: synthesis of compound 35f

At room temperature, to a single-necked flask were added 35e (400 mg, 1.11 mmol) and ethanol (5 mL). Under stirring, 80% hydrazine hydrate (5 mL) was added. The mixture was reacted under reflux for 16 hours. The reaction solution was concentrated under reduced pressure to obtain a crude. The crude was separated by high performance liquid

chromatography (aqueous ammonia/acetonitrile/water system) to obtain intermediate 35f (360 mg). LC-MS: m/z : 348.2 $(M+H)^+$.

Step 5: synthesis of compound 35g

At room temperature, to a single-necked flask were added 35f (360 mg, 1.04 mmol) and 1,2-dichloroethane (10 mL). Under stirring, N,N'-carbonyldiimidazole (252 mg, 1.55 mmol) was added. The mixture was reacted at room temperature for 16 hours. The reaction solution was poured into 20 mL of water and extracted with dichloromethane (10 mL x 3). The organic phase was washed with 10 mL of saturated brine, dried over sodium sulfate and filtered, and then the filtrate was concentrated under reduced pressure to obtain a crude. The crude was separated by flash column chromatography (dichloromethane:methanol =100:1-10:1) to obtain intermediate 35g. LC-MS: m/z : 374.1 $(M+H)^+$.

Step 6: synthesis of compound 35h

At room temperature, to a single-necked flask were added 35g (70 mg, 0.19 mmol) and N,N-dimethylformamide (3.0 mL). Under stirring, 34d (132 mg, 0.94 mmol), N,N-diisopropylethylamine (0.19 mL, 1.12 mmol) and benzotriazol-1-yloxytris(dimethylamino)phosphonium hexafluorophosphate (166 mg, 0.37 mmol) were added. The mixture was reacted at room temperature for 18 hours. The reaction solution was poured into 20 mL of water and extracted with ethyl acetate (20 mL x 3). The organic phase was washed with 30 mL of saturated brine, dried over sodium sulfate and filtered, and then the filtrate was concentrated under reduced pressure to obtain a crude. The crude was separated by high performance liquid chromatography (ammonium bicarbonate/acetonitrile /water system) to obtain intermediate 35h. LC-MS: m/z : 497.2 $(M+H)^+$.

Step 7: synthesis of compound 35i

Under ice bath, to a single-necked flask were added 1b (10 mg, 0.04 mmol) and dichloromethane (3.0 mL). Under stirring, oxalyl chloride (6 mg, 0.04 mmol) and a drop of N,N-dimethylformamide were added. At room temperature, the mixture was reacted for 30 minutes, and then triethylamine (12 mg, 0.12 mmol) and 35h (20 mg, 0.04 mmol) were added to the reaction solution. At room temperature, the mixture was reacted for one hour. The reaction solution was diluted with 20 mL of saturated aqueous ammonium chloride solution and extracted with dichloromethane (20 mL x 3). The organic phase was washed with 100 mL of saturated brine, dried over sodium sulfate and filtered, and then the filtrate was concentrated under reduced pressure to obtain the crude intermediate 35i. The crude was used directly in the next step without purification. LC-MS: m/z : 683.2 $(M+H)^+$.

Step 8: synthesis of compound VI-1

Under ice bath, to a single-necked flask were added 35i (30 mg, 0.04 mmol) and dichloromethane (3.0 mL). Under stirring, trifluoroacetic acid (1.5 mL) was added. At room temperature, the mixture was reacted for 10 minutes. To the reaction solution was added 25 mL of water, and the mixture was extracted with dichloromethane (20 mL x 1). The organic phase was washed with 100 mL of saturated brine, dried over sodium sulfate and filtered, and then the filtrate was concentrated under reduced pressure to obtain a crude. The crude was purified by high performance liquid chromatography (trifluoroacetic acid/acetonitrile/water system) to obtain compound VI-1. $^1$H NMR (400 MHz, DMSO) $\delta$ 8.31 (d, J = 7.7 Hz, 1H), 7.50 (t, J = 8.9 Hz, 1H), 7.08 (dd, J = 11.4, 2.8 Hz, 1H), 6.85 (ddd, J = 9.0, 2.8, 1.0 Hz, 1H), 5.36 - 5.27 (m, 1H), 4.49 (s, 2H), 4.45 (m, 2H), 4.23 (m, 1H), 4.18 (m, 2H), 2.76 - 2.66 (m, 1H), 2.20 - 2.11 (m, 1H), 2.07 - 1.96 (m, 1H), 1.84 (m, 1H), 1.76 (m, 3H), 1.71 - 1.60 (m, 2H), 1.57 - 1.36 (m, 4H), LC-MS: m/z : 533.0 $(M+H)^+$.

Example 36: Synthesis of compound IV-1

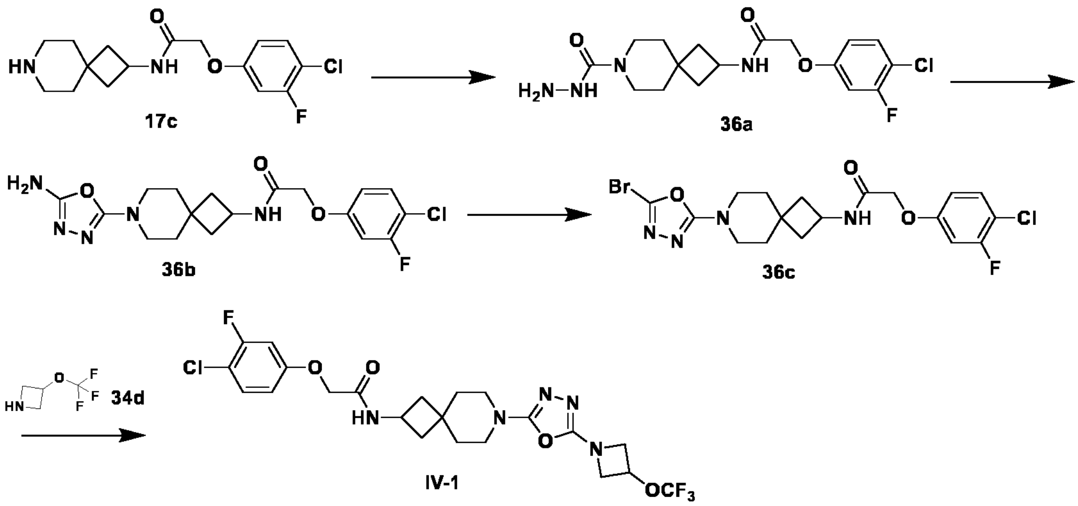


Step 1: synthesis of compound 36a

At room temperature, to a single-necked flask were added 17c (1.3 g, 3.98 mmol) and tetrahydrofuran (15.0 mL). Under stirring, the compound N,N'-carbonyldiimidazole (1.29 g, 7.96 mmol) was added. The mixture was reacted at room temperature for 2 hours, and then diisopropylethylamine (2.59 g, 19.89 mmol) and 80% hydrazine hydrate (1.59 g, 15.91 mmol) were sequentially added. The mixture was reacted at 45°C overnight. To the reaction solution was added 50 mL of water, and the mixture was extracted with ethyl acetate (50 mL x 2). The organic phase was washed with 100 mL of saturated brine, dried over sodium sulfate and filtered, and then the filtrate was concentrated under reduced pressure to obtain a crude. The crude was slurried by using an acetonitrile solvent to obtain intermediate 36a. LC-MS: m/z :385.2 $(M+H)^+$.

Step 2: synthesis of compound 36b

Under ice bath, to a single-necked flask were added 36a (200 mg, 0.52 mmol), 1,4-dioxane (5.0 mL) and water (0.5 mL). Under stirring, sodium bicarbonate (65 mg, 1.49 mmol) and bromoacetonitrile (60 mg, 0.57 mmol) were sequentially added. The mixture was warmed to room temperature and reacted overnight. To the reaction solution was added 25 mL of water, and the mixture was extracted with ethyl acetate (20 mL x 2). The organic phase was washed with 30 mL of saturated brine, dried over sodium sulfate and filtered, and then the filtrate was concentrated under reduced pressure to obtain the crude compound 36b, which was used directly in the next step without purification. LC-MS: m/z : 410.2 $(M+H)^+$.

Step 3: synthesis of compound 36c

At room temperature, to a single-necked flask were added 36b (180 mg, 0.44 mmol) and anhydrous acetonitrile (5 mL). Under stirring, cuprous bromide (126.0 mg, 0.88 mmol) was added. After 5 minutes, tert-butyl nitrite (25 mg, 0.24 mmol) was added. The mixture was reacted at room temperature overnight. The reaction solution was concentrated under reduced pressure to obtain a crude. The crude was purified by flash column chromatography (petroleum ether:ethyl acetate =100:1-3:1) to obtain intermediate 36c. LC-MS: m/z :473.1 $(M+H)^+$.

Step 4: synthesis of compound VI-1

Under ice bath, to a single-necked flask were added 36c (50 mg, 0.11 mmol) and tetrahydrofuran (5.0 mL). Under stirring, potassium carbonate (15 mg, 0.11 mmol) and compound B (22 mg, 0.16 mmol) were sequentially added. The mixture was reacted at 80°C overnight. To the reaction solution was added 25 mL of water, and the mixture was extracted once with 25 mL of ethyl acetate. The organic phase was washed with 25 mL of saturated brine, dried over sodium sulfate and filtered, and then the filtrate was concentrated under reduced pressure to obtain a crude. The crude was purified by high performance liquid chromatography (ammonium bicarbonate/acetonitrile/water system) to obtain compound IV-1. $^1$HNMR (400 MHz, MeOD) $\delta$ = 7.38 (t, J = 8.7 Hz, 1H), 7.00 - 6.92 (m, 1H), 6.87 - 6.79 (m, 1H), 4.67 - 4.56 (m, 2H), 4.50 (s, 2H), 4.46 - 4.39 (m, 2H), 4.18 (dd, J = 4.4, 9.5 Hz, 2H), 3.39 - 3.34 (m, 2H), 3.30 - 3.25 (m, 2H), 2.31 (br t, J = 10.5 Hz, 2H), 1.87 (br t, J = 10.2 Hz, 2H), 1.78 - 1.53 (m, 4H), LC-MS: m/z : 534.2 $(M+H)^+$.

It should be noted that other compounds in the present application can be prepared by methods similar to those in the above examples (if necessary, appropriate adjustments such as replacing reaction raw materials can be made).

Biological test

The ATF4 luciferase reporter plasmid consists of two parts, i.e., the 5' untranslated region sequence of the ATF4 gene and the luciferase coding sequence. Specifically, the ATF4 5' untranslated region sequence (NCBI database number BC022088.2) containing two upstream open reading frames (uORFs), and the firefly luciferase encoding gene were cloned into the pLVX-Puro vector (YouBio, VT1465). The packaging plasmids of lentivirus were psPAX2 (YouBio, VT1444) and pMD2.G (YouBio, VT1443). 650,000 HEK293T cells were seeded per well of a 6-well plate and cultured overnight in a 5% $CO_2$ incubator. The cells were transfected with the packaged lentivirus for 48 hours and then centrifuged, and the supernatant was discarded. The medium was replaced and changed to the DMEM complete medium containing 0.8 μg/ml puromycin, and the cells were resuspended and then cultured. After one week of cell screening with puromycin, the surviving cells were HEK293T-ATF4 uORF-Luc-Puro polyclonal cells. 200 polyclonal cells were taken and subjected to limiting gradient dilution by using a 96-well plate, so as to obtain monoclonal cells.

The specific method for screening the monoclonal cells involves: placing the 200 polyclonal cells in well A1 of the 96-well plate; step one: performing gradient dilution from columns A1 to H1 at a ratio of 1:2; step two: performing gradient dilution from rows A to H, with each row from wells 1 to 12 at a ratio of 1:2; marking the wells with a theoretical cell number less than 0.5 and observing one by one under a microscope; marking the wells with only one cell; and culturing the cells for two weeks to obtain the monoclonal cells.

By using this cell line and the cold fluorescence readings, the translational regulation of ATF4 can be detected, and the activity of eIF2B activated by the compounds can be tested.

The specific experimental process is as follows: 6000 HEK293T/17-ATF4 uORF-Luc-Puro monoclonal cells were plated in a 384-well plate and allowed to adhere overnight. The test compounds were dissolved in DMSO and diluted to 10 different concentrations, which were respectively added together with 50 nM thapsigargin to the cell culture medium and incubated for 6 hours, wherein the thapsigargin was used to cause cellular stress and upregulate the protein translation of ATF4. Six hours after the addition, the cells were lysed using the One-Glo Luciferase Assay Kit (Promega #E6120), and then the cold fluorescence values were read using the LUM program of the EnVision 2104 plate reader.

$EC_{50}$ was calculated based on the fluorescence values at 9 concentrations (0.1 nM, 0.3 nM, 1.0 nM, 3.0 nM, 10.0 nM, 30.0 nM, 100.0 nM, 300.0 nM and 1000.0 nM) of each compound and recorded in Table 1.

The ATF4 reporter expression was calculated as follows:

$$\text{ATF4 reporter expression} = (\text{ave_sample}-\text{ave_vc})/(\text{ave_pc}-\text{ave_vc})$$

where ave_vc represents the average signal value of negative controls, ave_pc represents the average signal value of positive controls, and ave_sample represents the average signal value of samples.

The dose-response curves were fitted, and the $EC_{50}$ values were calculated.

The nonlinear regression log(inhibitor) vs. response -- variable slope (four parameters) method of GraphPad 9 software was used to fit the corresponding relationship between the ATF4 reporter expression and the compound concentration.

The X-axis represents the log value of the compound concentration; and the Y-axis represents the ATF4 reporter expression.

$$\text{Formula: } Y=\text{Bottom} + (\text{Top}-\text{Bottom})/(1+10\text{^}((\text{LogEC}_{50}-X)*\text{HillSlope}))$$

Table 1 $EC_{50}$ values of exemplary compounds of the present disclosure in ATF4-Luc assay

| Compound structure | Compound No. | ATF4-luc $EC_{50}$ (nM)[a] |
|---|---|---|
| 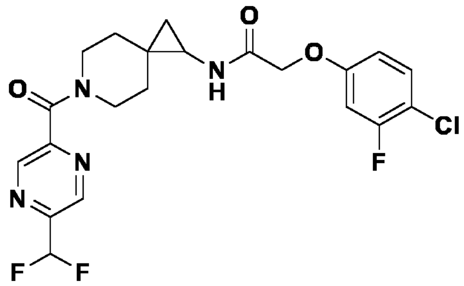 | I-1 | + |
| 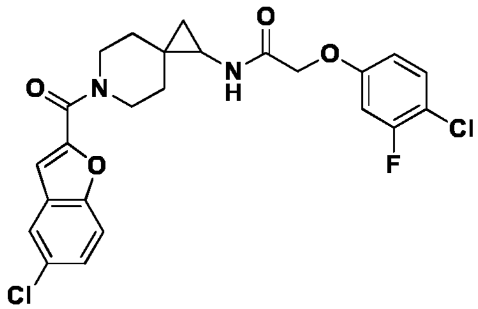 | I-2 | + |
| 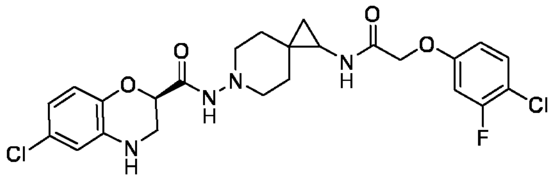 | I-3 | + |
| 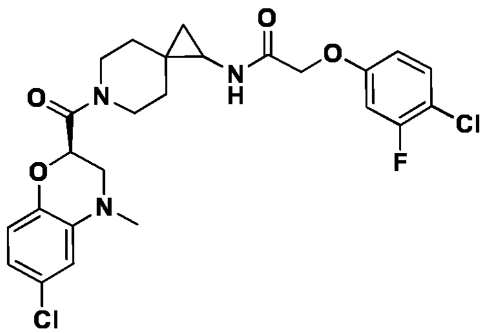 | I-4 | + |
| 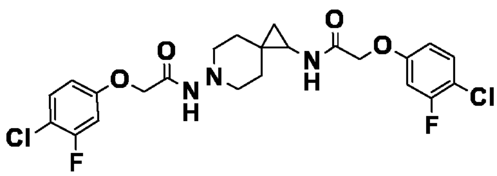 | I-5 | + |
| 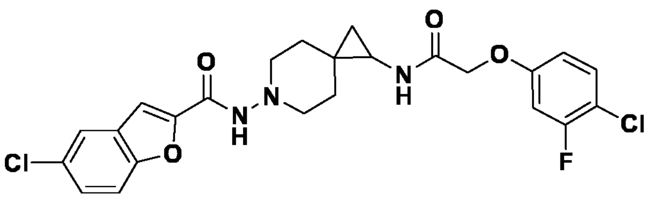 | I-6 | + |

(continued)

| Compound structure | Compound No. | ATF4-luc $EC_{50}$ (nM)[a] |
|---|---|---|
| 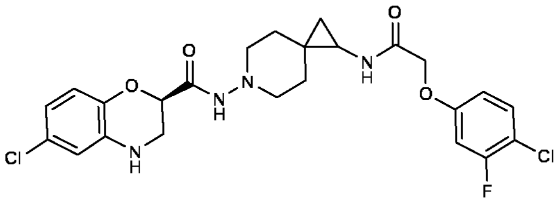 | I-7 | + |
| 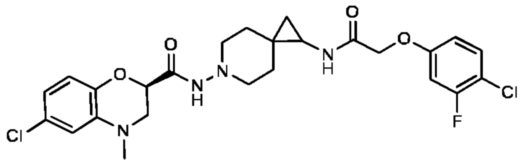 | I-8 | + |
| 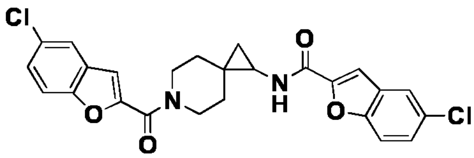 | I-9 | + |
| 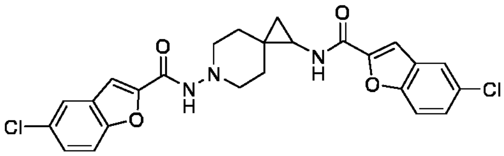 | I-10 | ++ |
| 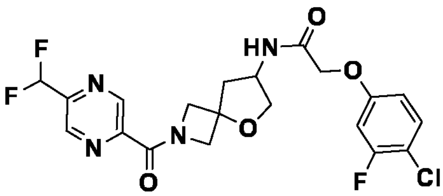 | II-10 | + |
| 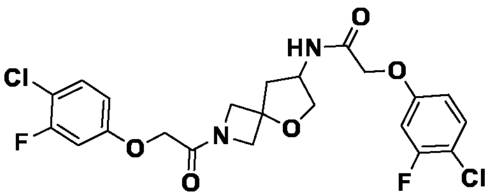 | II-11 | + |
| 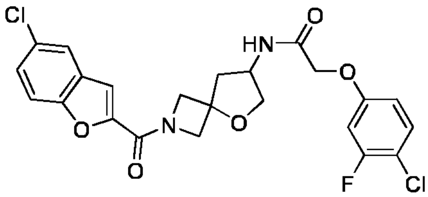 | II-12 | ++ |
| 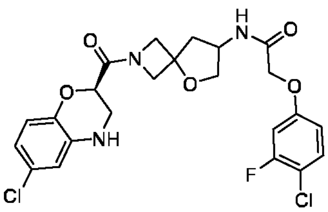 | II-13 | + |

(continued)

| Compound structure | Compound No. | ATF4-luc $EC_{50}$ (nM)[a] |
|---|---|---|
| 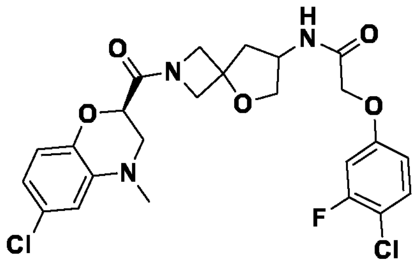 | II-14 | + |
| 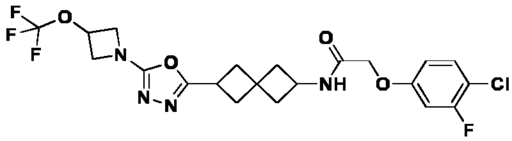 | III-1 | +++ |
| 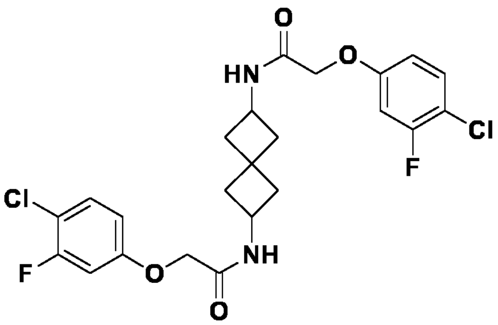 | III-10 | + |
| 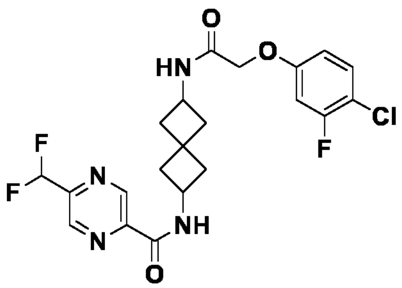 | III-11 | + |
| 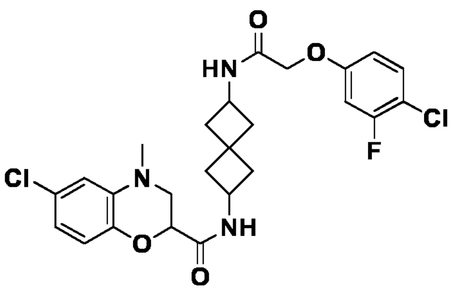 | III-12 | + |
| 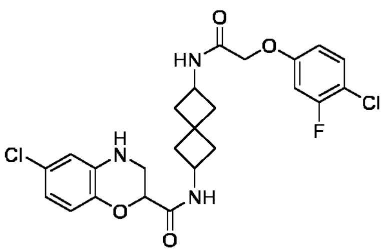 | III-13 | ++ |

(continued)

| Compound structure | Compound No. | ATF4-luc $EC_{50}$ (nM)[a] |
|---|---|---|
| 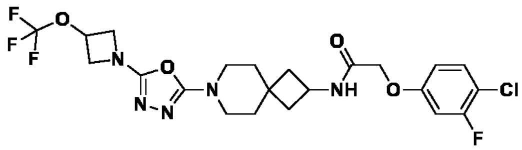 | IV-1 | ++ |
| 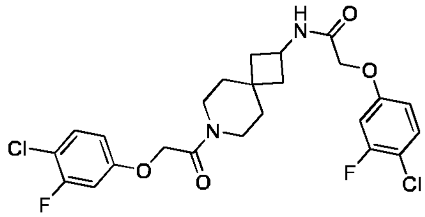 | IV-11 | ++ |
| 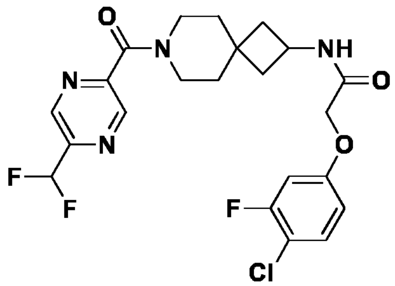 | IV-12 | + |
| 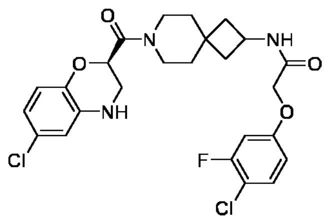 | IV-13 | +++ |
| 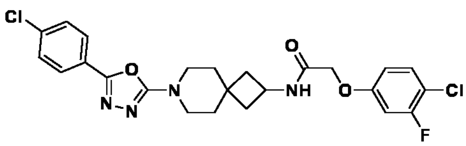 | IV-14 | ++ |
| 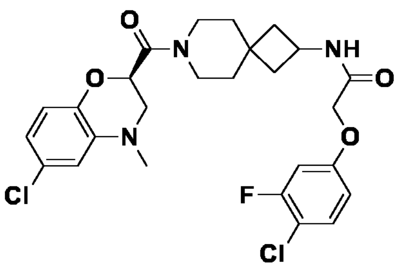 | IV-15 | + |
| 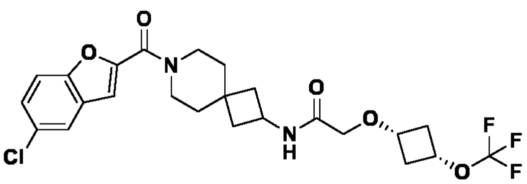 | IV-16 | + |

(continued)

| Compound structure | Compound No. | ATF4-luc $EC_{50}$ (nM)[a] |
|---|---|---|
| 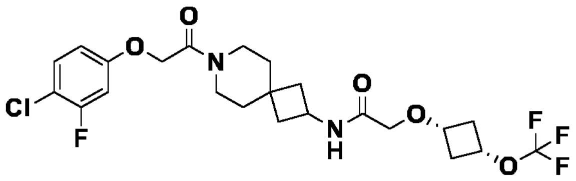 | IV-17 | + |
| 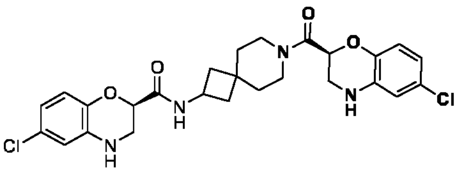 | IV-18 | ++ |
| 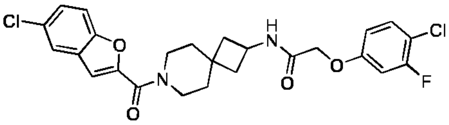 | IV-19 | ++ |
| 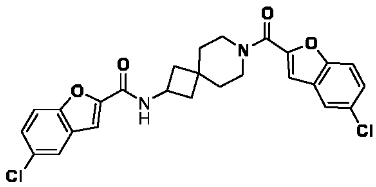 | IV-20 | +++ |
| 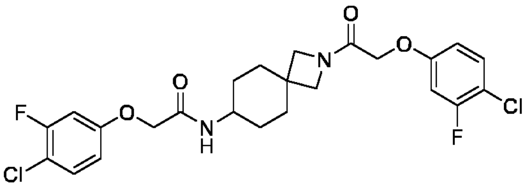 | V-10 | ++ |
| 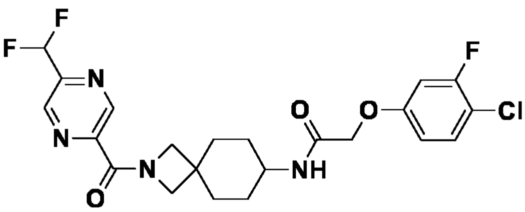 | V-11 | + |
| 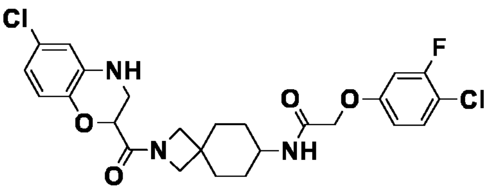 | V-12 | + |
| 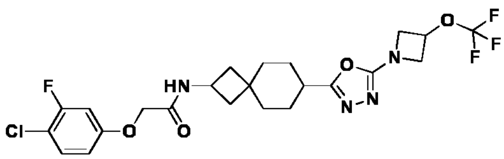 | VI-1 | ++ |

(continued)

| Compound structure | Compound No. | ATF4-luc $EC_{50}$ (nM)[a] |
|---|---|---|
| 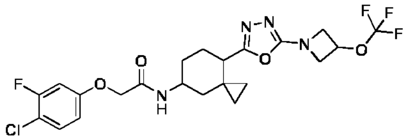 | VIII-1 | +++ |
| "+" indicates the ATF4-luc $EC_{50}$ being greater than 100 nM; "++" indicates the ATF4-luc $EC_{50}$ ranging from 10 nM and 100 nM; "+++" indicates the ATF4-luc $EC_{50}$ being less than 10 nM. | | |

The experimental results (partially not shown) show that the compounds of the present application can enhance the activity of eIF2B and reduce the expression of ATF4, thereby weakening the fluorescence intensity. It is indicated that the compounds of the present application can significantly alleviate the cellular stress caused by thapsigargin and weaken the integrated stress response of cells, so that proteins in the cells tend to be synthesized normally.

2. Kinetic solubility evaluation

The test compounds were dissolved in DMSO to prepare 10 mM stock solutions. 8.71 g of $K_2HPO_4$ was added to 500 mL of deionized water to prepare a 100 mM $K_2HPO_4$ solution. 2.05 g of potassium dihydrogen phosphate was added to 150 mL of deionized water to prepare a 100 mM potassium dihydrogen phosphate solution. 405 mL of 100 mM $K_2HPO_4$ and 95 mL of 100 mM $KH_2PO_4$ were mixed, and the mixed solution was adjusted to pH 7.4 by a 100 mM $K_2HPO_4/KH_2PO_4$ solution. 10.41 g of FaSSIF buffer concentrate was added to 240.3 g of deionized water to prepare a buffer solution (fasted-state simulated intestinal fluid, pH = 6.5). 4.071 g of FeSSIF buffer concentrate was added to 45.97 g of deionized water to prepare a buffer solution (fasted-state simulated intestinal fluid, pH = 5.0).

16 μL of the 10 mM stock solution of the compound was added to 784 μL of different buffer solutions (n = 3) in a 96-well plate, and the plate was sealed and shaken at 1000 rpm for 1.5 h at 25°C (for PBS) or 37°C (for others). After the incubation, the solution was transferred to a filter plate. All samples were filtered. 5 μL of the filtrate was added to 5 μL of DMSO, and 490 μL of an aqueous acetonitrile solution containing the internal standard (1:1), and the mixture was mixed evenly. Based on the properties of the compound and its response in mass spectrometry, dilution with an aqueous acetonitrile solution containing the internal standard (1:1) was performed. The dilution factor was varied according to the solubility value and UPLC-MS/MS signal response.

The experiments show that at least some of the compounds of the present application have good solubility in the above-mentioned different simulated environments.

3. Evaluation of cell membrane permeability

The test compounds were diluted from 10 mM stock solutions to a concentration of 10 μM using the transport buffer (HBSS + BSA) and applied to the apical or basolateral side of the cell monolayer. After the incubation at 37°C, 5% $CO_2$ and 95% relative humidity for 120 minutes, the permeability of the test compounds in the A to B direction or the B to A direction was dually determined. In addition, the efflux ratio (Papp B to A / Papp A to B) of each compound was determined. The test substances and reference substances were quantitatively analyzed by the LC-MS/MS method based on the analyte/IS peak area ratio.

Table 2 Cell membrane permeability of exemplary compounds

| Compound No. | Papp ($10^{-6}$ cm/s) | | Efflux Ratio |
|---|---|---|---|
| | A to B | B to A | |
| Atenolol | 0.41 | 0.61 | |
| Propranolol | 32.43 | 20.14 | |
| Digoxin | 0.25 | 11.17 | 44.68 |
| III-1 | 17.72 | 18.63 | 1.05 |

The experimental results show that at least some of the compounds of the present application (such as III-1) have good cell membrane permeability and are not P-glycoprotein substrates.

4. Pharmacokinetic evaluation in mice

The test compounds were dissolved in vehicles to prepare clear solutions or homogeneous suspensions. Three mice were placed in each group and received an intravenous (IV) administration via the tail vein at 1 mg/kg and an oral (PO) administration at 30 mg/kg. Blood samples were collected at 0.083 h, 0.25 h, 0.5 h, 1 h, 2 h, 4 h, 8 h, and 24 h after the intravenous administration, and at 0.25 h, 0.5 h, 1 h, 2 h, 4 h, 6 h, 8 h, and 24 h after the oral administration. Plasma samples were centrifuged, and the supernatant was collected to prepare samples, which were then quantitatively analyzed by LC/MS/MS.

Table 3 PK properties of exemplary compounds in mice

| | | |
|---|---|---|
| mouse IV (1 mg/kg) | $C_0$ (ng/mL) | 1344 |
| | $T_{1/2}$ (h) | 2.07 |
| | Cl (mL/h/kg) | 450 |
| | $V_{ss}$ (L/kg) | 1.18 |
| | $AUC_{(0-inf)}$ (ng*h/ml) | 2230.64 |
| mouse PO (30 mg/kg) | $C_{max}$ (ng/mL) | 21541.07 |
| | $T_{1/2}$ (h) | 1.93 |
| | $T_{max}$ (h) | 1.33 |
| | $AUC_{(0-inf)}$ (ng*h/ml) | 110833.28 |
| | F% | 177.04 |

The experimental results show that at least some of the compounds of the present application (such as III-1) have excellent pharmacokinetic properties in mice (including but not limited to $C_0$ (initial drug concentration), CL (clearance), AUC, t1/2 (half-life), $C_{max}$ (peak concentration), $T_{max}$ (time to reach peak concentration), AUC (area under the plasma drug concentration-time curve), F (bioavailability) and Vss (apparent volume of distribution at steady state)).

The technical solutions of the present disclosure are not limited to the above-mentioned specific examples. All technical variations made according to the technical solutions of the present disclosure fall within the protection scope of the present disclosure.

## Claims

1. A compound of formula 0, or a stereoisomer thereof, a tautomer thereof, a geometric isomer thereof, an enantiomer thereof, a diastereomer thereof, a racemate thereof, a polymorph thereof, a solvate thereof, a hydrate thereof, an N-oxide thereof, an isotopically labeled compound thereof, a metabolite thereof, an ester thereof, a prodrug thereof or a pharmaceutically acceptable salt thereof:

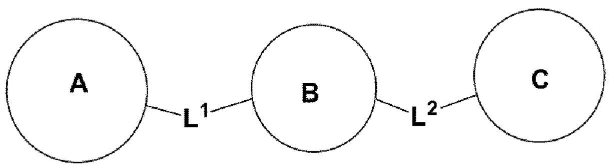

Formula 0

wherein,

ring A and ring C are each independently C1-C6 alkyl, C3-C9 cycloalkyl, 3- to 9-membered heterocyclyl, 6- to 10-membered aryl, 5- to 10-membered heteroaryl, 6- to 10-membered aryl-D-C1-C6 alkyl, 5- to 10-membered heteroaryl-D-C1-C6 alkyl, C3-C9 cycloalkyl-D-C1-C6 alkyl or 3-to 9-membered heterocyclyl-D-C1-C6 alkyl, wherein the alkyl is optionally substituted with deuterium, halogen and C1-C6 alkyl; the cycloalkyl, heterocyclyl, aryl and heteroaryl can each be optionally substituted with 1-5 $R^1$;

each of the $R^1$ is independently selected from the group consisting of hydrogen, deuterium, halogen, hydroxyl,

phenyl, cyano, C1-C10 alkyl, hydroxyl-substituted C1-C6 alkyl, halogen-substituted C1-C6 alkyl, C1-C6 alkoxy, halogen-substituted C1-C6 alkoxy, halogen-substituted C1-C6 alkoxy-C1-C6 alkenyl, amino-substituted C1-C6 alkyl, cyano-substituted C1-C6 alkyl, C1-C3 alkoxy-C1-C6 alkyl, C3-C6 cycloalkyl, C1-C3 alkoxy-C3-C6 cycloalkyl, C1-C3 alkoxy-3- to 6-membered heterocyclyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-SR^{1E}$, $-S(O)R^{1D}$, $-S(O)_2R^{1D}$, $-G^1$, $-O-G^1$ and $-NR^{1B}-G^1$;
or 2 $R^1$ groups on adjacent atoms taken together with the atoms to which they are attached can form C3-C7 cycloalkyl, 3- to 7-membered heterocyclyl, 6- to 10-membered aryl or 5- to 6-membered heteroaryl; the C3-C7 cycloalkyl, 3- to 7-membered heterocyclyl, 6- to 10-membered aryl or 5- to 6-membered heteroaryl is each optionally substituted with 1-5 $R^{1F}$; with regard to options for the $R^1$, the phenyl, C1-C10 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen;
each of the $R^{1A}$, $R^{1B}$, $R^{1C}$, $R^{1D}$ and $R^{1E}$ is independently selected from the group consisting of hydrogen, deuterium, C1-C6 alkyl, C3-C9 cycloalkyl, 3- to 9-membered heterocyclyl, halogen-substituted C1-C6 alkyl, halogen-substituted C3-C9 cycloalkyl, halogen-substituted 3- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;
each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F}$;
$L^1$ is null, -NH-,

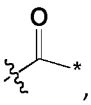, 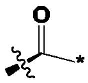, 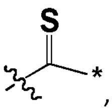, 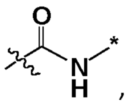, 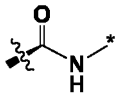, 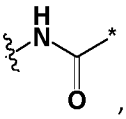, 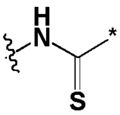

or

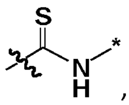,

wherein the end is connected to ring A, and the * end is connected to ring B;
preferably, the $L^1$ is null, -NH-,

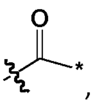, 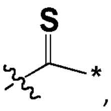, 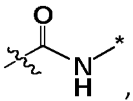, 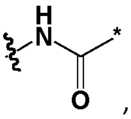, 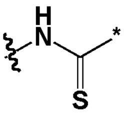 or 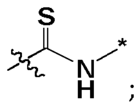;

preferably, the $L^1$ is null,

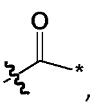, 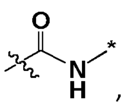, 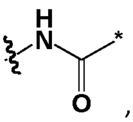, 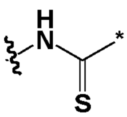 or 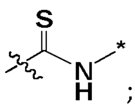;

preferably, the $L^1$ is null,

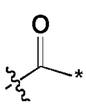, 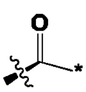, 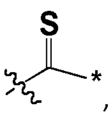, 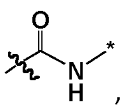, 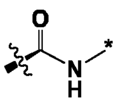, or 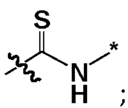;

preferably, the $L^1$ is null,

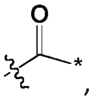, 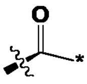, 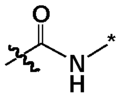 or 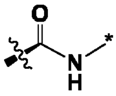;

preferably, the $L^1$ is null,

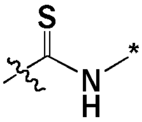 or 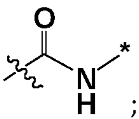;

ring B is a 5- to 10-membered spiro ring or a 5- to 10-membered spiro heterocyclic ring, wherein the 5- to 10-membered spiro ring and the 5- to 10-membered spiro heterocyclic ring are optionally substituted with 1-5 $R^{1F}$; each of the $R^{1F}$ is independently selected from the group consisting of hydrogen, deuterium, halogen, hydroxyl, amino, cyano, carboxyl, C1-C6 alkyl, C3-C9 cycloalkyl, hydroxyl-substituted C1-C6 alkyl, halogen-substituted C1-C6 alkyl, halogen-substituted C1-C6 alkoxy, halogen-substituted C3-C9 cycloalkyl, halogen-substituted C1-C6 alkenyl, halogen-substituted C1-C6 alkoxy-C1-C6 alkenyl, amino-substituted C1-C6 alkyl, cyano-substituted C1-C6 alkyl, oxo, $-OR^{1A}$, - $NR^{1B}R^{1C}$, $-NR^{1B}C(O)R^{1D}$, $-C(O)NR^{1B}R^{1C}$, $-C(O)R^{1D}$, $-COOR^{1D}$, -P(O) $R^{1B}R^{1C}$, $-SR^{1E}$, $-S(O)R^{1D}$ and - $S(O)_2R^{1D}$;

$L^2$ is null, -NH-,

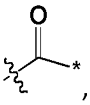, 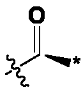, 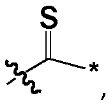, 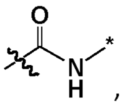, 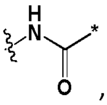, 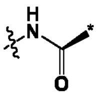, 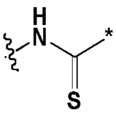 or 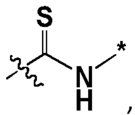,

wherein the end is connected to ring B, and the * end is connected to ring C;

preferably, the $L^2$ is null, -NH-,

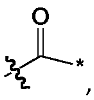, 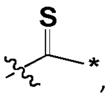, 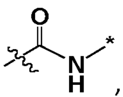, 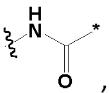, 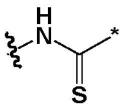 or 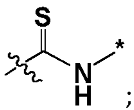;

preferably, the $L^2$ is null,

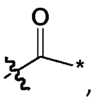, 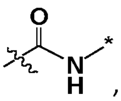, 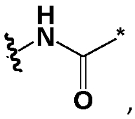, 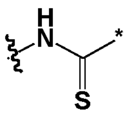 or 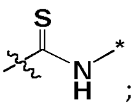;

preferably, the $L^2$ is null,

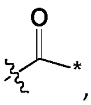, 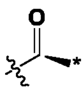, 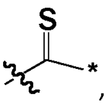, 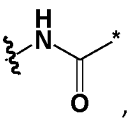, 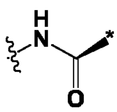 or 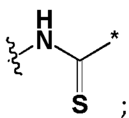;

preferably, the $L^2$ is null,

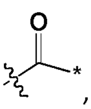, 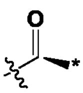, 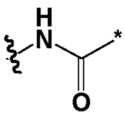 or 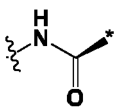;

preferably, the $L^2$ is null or

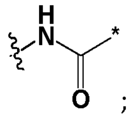

;

each of the D is independently a linking group containing a heteroatom; preferably, each of the D is independently O or $NR^d$ or S, wherein each $R^d$ is independently selected from hydrogen, deuterium, C1-C6 alkyl and halogen-substituted C1-C6 alkyl; more preferably, each of the D is independently O or NH.

**2.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to claim 1, wherein

the ring A is C1-C6 alkyl, C3-C9 cycloalkyl, 3- to 9-membered heterocyclyl, 6- to 10-membered aryl, 5- to 10-membered heteroaryl, 6- to 10-membered aryl-D-C1-C6 alkyl, 5- to 10-membered heteroaryl-D-C1-C6 alkyl or 3- to 9-membered heterocyclyl-D-C1-C6 alkyl, wherein the alkyl is optionally substituted with halogen or C1-C6 alkyl; the cycloalkyl, heterocyclyl, aryl and heteroaryl can each be optionally substituted with 1-5 $R^1$;
each of the $R^1$ is independently selected from the group consisting of hydrogen, halogen, cyano, C1-C10 alkyl, hydroxyl-substituted C1-C6 alkyl, halogen-substituted C1-C6 alkyl, C1-C6 alkoxy, halogen-substituted C1-C6 alkoxy, halogen-substituted C1-C6 alkoxy-C1-C6 alkenyl, amino-substituted C1-C6 alkyl, cyano-substituted C1-C6 alkyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-SR^{1E}$, $-S(O)R^{1D}$, $-S(O)_2R^{1D}$, $-G^1$, $-O-G^1$ and $-NR^{1B}-G^1$; or 2 $R^1$ groups on adjacent atoms taken together with the atoms to which they are attached can form C3-C7 cycloalkyl, 3- to 7-membered heterocyclyl, 6- to 10-membered aryl or 5- to 6-membered heteroaryl; the C3-C7 cycloalkyl, 3- to 7-membered heterocyclyl, 6- to 10-membered aryl or 5-to 6-membered heteroaryl is each optionally substituted with 1-5 $R^{1F}$;
each of the $R^{1A}$, $R^{1B}$, $R^{1C}$, $R^{1D}$ and $R^{1E}$ is independently selected from the group consisting of hydrogen, C1-C6 alkyl, C3-C9 cycloalkyl, 3- to 9-membered heterocyclyl, halogen-substituted C1-C6 alkyl, halogen-substituted C3-C9 cycloalkyl, halogen-substituted 3- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;
each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F}$;
each of the $R^{1F}$ is independently selected from the group consisting of hydrogen, halogen, hydroxyl, amino, cyano, carboxyl, C1-C6 alkyl, C3-C9 cycloalkyl, hydroxyl-substituted C1-C6 alkyl, halogen-substituted C1-C6 alkyl, halogen-substituted C1-C6 alkoxy, halogen-substituted C3-C9 cycloalkyl, halogen-substituted C1-C6 alkenyl, halogen-substituted C1-C6 alkoxy-C1-C6 alkenyl, amino-substituted C1-C6 alkyl, cyano-substituted C1-C6 alkyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-COOR^{1D}$, $-P(O)R^{1B}R^{1C}$, $-SR^{1E}$, $-S(O)R^{1D}$ and $-S(O)_2R^{1D}$;
the ring C is 6- to 10-membered aryl-E-C1-C6 alkyl, C3-C9 cycloalkyl-E-C1-C6 alkyl, 3- to 9-membered heterocyclyl-E-C1-C6 alkyl, 3- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, deuterium, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen; or 2 $R^2$ groups on adjacent atoms taken together with the atoms to which they are attached can form C3-C7 cycloalkyl, 3- to 7-membered heterocyclyl, 5- to 6-membered aryl (such as phenyl) or 5- to 6-membered heteroaryl; the C3-C7 cycloalkyl, 3- to 7-membered heterocyclyl, 5- to 6-membered aryl (such as phenyl) or 5- to 6-membered heteroaryl is each optionally substituted with 1-5 $R^{1F}$;
each of the D or E is independently a linking group containing a heteroatom; preferably, each of the D or E is independently O or $NR^d$ or S, wherein each $R^d$ is independently selected from hydrogen, deuterium and C1-C3 alkyl; more preferably, each of the D or E is independently O or NH.

**3.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the

prodrug thereof or the pharmaceutically acceptable salt thereof according to claim 1 or 2, wherein the ring A is 3- to 9-membered heterocyclyl, 6- to 10-membered aryl, 5- to 10-membered heteroaryl, 6- to 10-membered aryl-D-C1-C6 alkyl or 3- to 9-membered heterocyclyl-D-C1-C6 alkyl, wherein the heterocyclyl, aryl and heteroaryl can each be optionally substituted with 1-5 $R^1$;

each of the $R^1$ is independently selected from the group consisting of hydrogen, halogen, halogen-substituted C1-C6 alkyl, halogen-substituted C1-C6 alkoxy, $-OR^{1A}$, $-NR^{1B}R^{1C}$, $-NR^{1B}C(O)R^{1D}$, $-C(O)NR^{1B}R^{1C}$, $-C(O)R^{1D}$, $-G^1$, $-O-G^1$ and $-NR^{1B}-G^1$; or 2 $R^1$ groups on adjacent atoms taken together with the atoms to which they are attached can form 6- to 10-membered aryl or 5- to 6-membered heteroaryl; the 6- to 10-membered aryl or 5- to 6-membered heteroaryl is each optionally substituted with 1-5 $R^{1F}$;
each of the $R^{1A}$, $R^{1B}$, $R^{1C}$ and $R^{1D}$ is independently selected from the group consisting of hydrogen, C1-C6 alkyl, C3-C9 cycloalkyl, 3- to 9-membered heterocyclyl, halogen-substituted C1-C6 alkyl, halogen-substituted C3-C9 cycloalkyl, halogen-substituted 3- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;
each of the $G^1$ is independently selected from C3-C9 cycloalkyl, 3- to 9-membered heterocyclyl and 6- to 10-membered aryl; each of the $G^1$ is optionally substituted with 1-3 $R^{1F}$; when $G^1$ is phenyl, the phenyl and 3- to 9-membered heterocyclyl or 5- to 10-membered heteroaryl optionally share two carbon atoms to form a fused ring;
each of the $R^{1F}$ is independently selected from the group consisting of hydrogen, halogen, C1-C6 alkyl, C3-C9 cycloalkyl, halogen-substituted C1-C6 alkyl, halogen-substituted C1-C6 alkoxy, halogen-substituted C3-C9 cycloalkyl, halogen-substituted C1-C6 alkenyl, oxo, $-OR^{1A}$, $-NR^{1B}R^{1C}$, $-NR^{1B}C(O)R^{1D}$, $-C(O)NR^{1B}R^{1C}$, $-C(O)R^{1D}$, $-P(O)R^{1B}R^{1C}$ and $-S(O)_2R^{1D}$.

**4.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-3, wherein

ring A is 4- to 9-membered heterocyclyl, 5- to 7-membered heteroaryl, phenyl-D-C1-C6 alkyl or 4- to 9-membered heterocyclyl-D-C1-C6 alkyl, wherein the heterocyclyl, heteroaryl and phenyl can each be optionally substituted with 1-5 $R^1$;
each of the $R^1$ is independently selected from the group consisting of hydrogen, halogen, halogen-substituted C1-C6 alkyl, halogen-substituted C1-C6 alkoxy, $-OR^{1A}$, $-NR^{1B}R^{1C}$, $-NR^{1B}C(O)R^{1D}$, $-C(O)NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C6 alkyl, C4-C9 cycloalkyl, 4- to 9-membered heterocyclyl, halogen-substituted C1-C6 alkyl, halogen-substituted C4-C9 cycloalkyl, halogen-substituted 4- to 9-membered heterocyclyl and halogen-substituted C1-C6 alkoxy; $R^{1B}$ and $R^{1C}$ taken together with the atom to which they are attached can form 3- to 7-membered heterocyclyl, wherein the 3- to 7-membered heterocyclyl is optionally substituted with 1-3 $R^{1F}$;
each of the $G^1$ is independently selected from C3-C9 cycloalkyl, 3- to 9-membered heterocyclyl and phenyl; each of the $G^1$ is optionally substituted with 1-3 $R^{1F}$; when $G^1$ is phenyl, the phenyl and 3- to 9-membered heterocyclyl or 5- to 7-membered heteroaryl optionally share two carbon atoms to form a fused ring;
each of the $R^{1F}$ is independently selected from the group consisting of hydrogen, halogen, C1-C6 alkyl, C3-C9 cycloalkyl, halogen-substituted C1-C6 alkyl, halogen-substituted C1-C6 alkoxy, halogen-substituted C3-C9 cycloalkyl, halogen-substituted C1-C6 alkenyl, oxo, $-OR^{1A}$, $-NR^{1B}R^{1C}$, $-P(O)R^{1B}R^{1C}$ and $-S(O)_2R^{1D}$.

**5.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-4, wherein

the ring A is 4- to 8-membered heterocyclyl, 5- to 6-membered heteroaryl, phenyl-D-C1-C3 alkyl or 4- to 8-membered heterocyclyl-D-C1-C3 alkyl, wherein the heterocyclyl, heteroaryl and phenyl can each be optionally substituted with 1-2 $R^1$;
each of the $R^1$ is independently selected from the group consisting of hydrogen, halogen, halogen-substituted C1-C3 alkyl, halogen-substituted C1-C3 alkoxy, $-OR^{1A}$, $-NR^{1B}R^{1C}$, $-NR^{B}C(O)R^{1D}$, $-C(O)NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C3 alkyl,

C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl, wherein preferably, the C3-C6 cycloalkyl is cyclobutyl or cyclopentyl, and the 3- to 6-membered heterocyclyl is oxetanyl, azetidinyl, oxocyclopentyl or azacyclopentyl; each of the $G^1$ is optionally substituted with 1-3 $R^{1F}$; when $G^1$ is phenyl, the phenyl and 5- to 6-membered heterocyclyl or 5- to 7-membered heteroaryl optionally share two carbon atoms to form a fused ring;

each of the $R^{1F}$ is independently selected from the group consisting of hydrogen, halogen, C1-C3 alkyl, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, - P(O) $R^{1B}R^{1C}$ and $-S(O)_2R^{1D}$.

**6.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-5, wherein

the ring A is 5- to 6-membered heterocyclyl, preferably

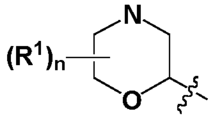,

or preferably

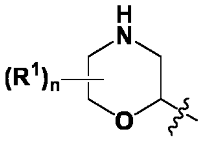 or 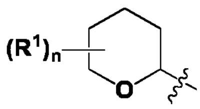;

preferably, the number n of $R^1$ is 1, 2 or 3;

preferably, each of the $R^1$ is independently selected from the group consisting of hydrogen, halogen, hydroxyl, C1-C3 alkyl and phenyl; and when $R^1$ is phenyl, the phenyl and the 3- to 6-membered heterocyclyl share two carbon atoms to form a fused ring, preferably the phenyl and

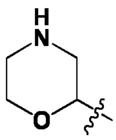 or 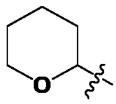

share two carbon atoms to form a fused ring, and the phenyl is optionally substituted with halogen, C1-C3 alkyl, C1-C3 haloalkyl or C1-C3 haloalkoxy;

further preferably, the fusion mode of the phenyl and

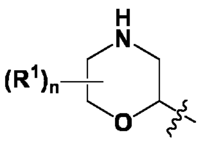 or 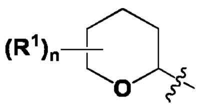 is

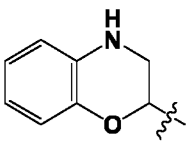 or 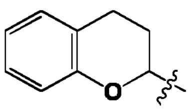,

and the phenyl is optionally substituted with halogen;
or the ring A is 5- to 6-membered heteroaryl, preferably any one of the following groups:

(1) 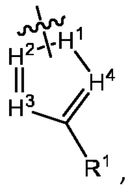,

, wherein $H^1$, $H^2$, $H^3$ and $H^4$ are each independently C or a heteroatom, with at least one being a heteroatom, and preferably, the heteroatom is selected from any one of N, O and S; preferably, the $R^1$ is selected from the group consisting of hydrogen, $-OR^{1A}$, $-NR^{1B}R^{1C}$, $-G^1$, $-O-G^1$ and $-NR^{1B}-G^1$, wherein each of the $R^{1A}$ , $R^{1B}$ and $R^{1C}$ is independently selected from the group consisting of hydrogen, C1-C3 alkyl, C3-C6 cycloalkyl, 4- to 6-membered heterocyclyl, halogen-substituted C1-C3 alkyl, halogen-substituted C3-C6 cycloalkyl, halogen-substituted 4- to 6-membered heterocyclyl and halogen-substituted C1-C3 alkoxy;
each of the $G^1$ is independently selected from 3- to 6-membered cycloalkyl, 3- to 6-membered heterocyclyl and phenyl; each of the $G^1$ is optionally substituted with 1-3 $R^{1F}$;
each of the $R^{1F}$ is independently selected from the group consisting of hydrogen, halogen, C1-C3 alkyl, C3-C6 cycloalkyl, C1-C3 alkoxy, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O) R^{1B}R^{1C}$ and $-S(O)_2R^{1D}$, wherein the $R^{1D}$ is selected from the group consisting of hydrogen, C1-C3 alkyl, halogen-substituted C1-C3 alkyl and halogen-substituted C1-C3 alkoxy;
or

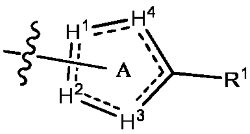,

wherein the ring A is 5-membered heteroaryl, $H^1$, $H^2$, $H^3$ and $H^4$ are each independently selected from CH, N, O and S, and - - - - - - is a single bond or a double bond; each of the $R^1$ is independently selected from the group consisting of $-OR^{1A}$, $-NR^{1B}R^{1C}$, $-G^1$, $-O-G^1$ and $-NR^{1B}-G^1$; each of the $R^{1A}$, $R^{1B}$ and $R^{1C}$ is independently selected from the group consisting of hydrogen, C1-C3 alkyl, C1-C3 alkoxy-C1-C6 alkyl, C3-C6 cycloalkyl, 4- to 6-membered heterocyclyl, halogen-substituted C1-C3 alkyl, halogen-substituted C1-C3 alkoxy-C1-C6 alkyl, halogen-substituted C3-C6 cycloalkyl, halogen-substituted 4- to 6-membered heterocyclyl and halogen-substituted C1-C3 alkoxy; each of the $G^1$ is independently selected from C4-C6 cycloalkyl and 4- to 9-membered heterocyclyl; each of the $G^1$ is optionally substituted with 1-3 $R^{1F}$; each of the $R^{1F}$ is independently selected from the group consisting of hydrogen, halogen, C1-C3 alkyl, C3-C6 cycloalkyl, C1-C3 alkoxy, halogen-substituted C1-C3 alkyl, halogen-substituted C1-C3 alkoxy, halogen-substituted C1-C3 alkenyl, oxo, $-OR^{1A}$, $-NR^{1B}R^{1C}$, $-P(O)R^{1B}R^{1C}$ and $-S(O)_2R^{1D}$, wherein the $R^{1D}$ is selected from the group consisting of hydrogen, C1-C3 alkyl, halogen-substituted C1-C3 alkyl and halogen-substituted C1-C3 alkoxy;
preferably, the ring A is

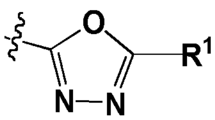, 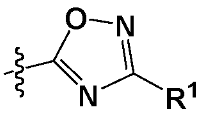, 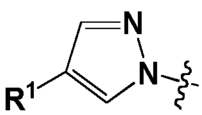,

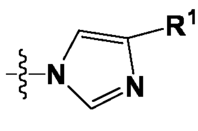 or 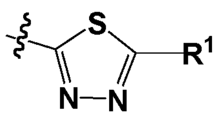 ;

further preferably, the $L^1$ is null;
preferably, the $R^1$ is selected from $-OR^{1A}$, $-G^1$, $-O-G^1$ and $-NR^{1B}-G^1$; $R^{1A}$ is selected from halogen-substituted C1-C3 alkoxy-C1-C3 alkyl; $R^{1B}$ is selected from H and C1-C3 alkyl; each of the $R^{1F}$ is independently selected from the

group consisting of halogen, C1-C3 alkyl, C3-C6 cycloalkyl, C1-C3 alkoxy, halogen-substituted C1-C3 alkyl, halogen-substituted C1-C3 alkoxy, halogen-substituted C1-C3 alkenyl, oxo, $-P(O)R^{1B}R^{1C}$ and $-S(O)_2R^{1D}$; $R^{1B}$, $R^{1C}$ and $R^{1D}$ are each independently selected from the group consisting of hydrogen and C1-C3 alkyl;

(2) 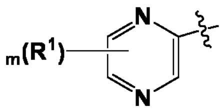 or 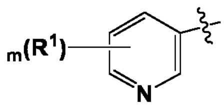 ;

preferably, each $R^1$ is independently selected from any one of the following groups: hydrogen, halogen, C1-C3 alkyl, C1-C3 haloalkyl, C1-C3 haloalkoxy and phenyl, and when $R^1$ is phenyl, the phenyl and

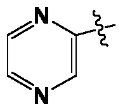 or 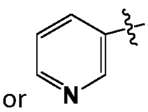

share two carbon atoms to form a fused ring, and the phenyl is optionally substituted with halogen, C1-C3 alkyl, C1-C3 haloalkyl and C1-C3 haloalkoxy; further preferably, the phenyl is optionally substituted with halogen; the number m of $R^1$ is 1 or 2;

(3) 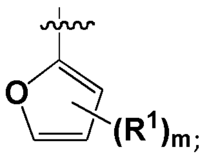 ;

preferably, the $R^1$ is hydrogen, halogen or phenyl, wherein the phenyl is optionally substituted with 1 or more halogens, and the number m of $R^1$ is 1 or 2; when $R^1$ is phenyl, the phenyl and

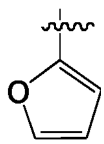

optionally share two carbon atoms to form a fused ring;
further preferably, the $R^1$ is phenyl, m is 1, the fusion mode of the phenyl and

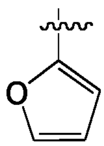

is

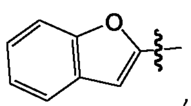 ,

and the phenyl is optionally substituted with 1 or more halogens;
or the ring A is phenyl-O-C1-C3 alkyl, wherein the phenyl is optionally substituted with 1 or 2 $R^1$; each of the $R^1$ is independently selected from the group consisting of hydrogen, halogen, C1-C3 alkyl, halogen-substituted C1-C3 alkyl and halogen-substituted C1-C3 alkoxy;
preferably, the ring A is phenyl-O-methyl, and $R^1$ is H or halogen;
or the ring A is 4- to 8-membered heterocyclyl-O-C1-C3 alkyl, wherein the heterocyclyl is optionally substituted with 1-3 $R^1$; each of the $R^1$ is independently selected from the group consisting of hydrogen, halogen, C1-C3 alkyl, halogen-substituted C1-C3 alkyl and halogen-substituted C1-C3 alkoxy;

preferably, the ring A is 4- to 7-membered heterocyclyl-O-methyl, and $R^1$ is halogen-substituted C1-C3 alkyl or halogen-substituted C1-C3 alkoxy;

or the ring A is C3-C7 cycloalkyl-O-C1-C3 alkyl, wherein the C3-C7 cycloalkyl is optionally substituted with 1-3 $R^1$; each of the $R^1$ is independently selected from the group consisting of hydrogen, halogen, C1-C3 alkyl, halogen-substituted C1-C3 alkyl and halogen-substituted C1-C3 alkoxy;

preferably, the ring A is C4-C6 cycloalkyl-O-methyl, and $R^1$ is halogen-substituted C1-C3 alkyl or halogen-substituted C1-C3 alkoxy;

or the ring A is phenyl, wherein the phenyl is substituted with a group selected from halogen, C1-C6 alkyl, C1-C6 alkoxy, C1-C6 haloalkyl and C1-C6 haloalkoxy;

preferably, the phenyl is substituted with a group selected from halogen, C1-C3 alkyl, C1-C3 alkoxy, C1-C3 haloalkyl and C1-C3 haloalkoxy;

further preferably, the phenyl is substituted with C1-C3 haloalkyl;

preferably, the ring A is selected from any one of the following groups:

(1) 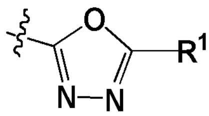 ;

preferably, the $R^1$ is selected from the group consisting of -$G^1$, O-$G^1$ and -$NR^{1B}$-$G^1$, preferably each of the $G^1$ is optionally substituted with 1 or 2 $R^{1F}$; the $R^{1B}$ is selected from the group consisting of hydrogen and C1-C3 alkyl; each of the $G^1$ is independently selected from C3-C6 cycloalkyl and 4- to 9-membered heterocyclyl;

further preferably, the C3-C6 cycloalkyl is cyclobutyl or cyclopentyl; the 4- to 9-membered heterocyclyl is preferably oxocycloalkyl or azacycloalkyl, and further preferably oxetanyl, azetidinyl, oxocyclopentyl, azacyclopentyl,

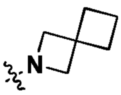 or 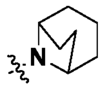 ,

further preferably oxetanyl, azetidinyl, oxocyclopentyl or azacyclopentyl, or further preferably

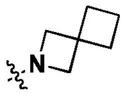 or 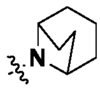 ;

each of the $R^{1F}$ is independently selected from the group consisting of oxo, C1-C3 alkyl, C1-C3 alkoxy, halogen-substituted C1-C3 alkyl and halogen-substituted C1-C3 alkoxy; moreover, the $L^1$ is preferably null;

(2) 4- to 8-membered heterocyclyl-O-C1-C3 alkyl, wherein the heterocyclyl is optionally substituted with 1 or 2 $R^1$; each of the $R^1$ is independently selected from the group consisting of halogen-substituted C1-C3 alkyl and halogen-substituted C1-C3 alkoxy;

(3) C4-C6 cycloalkyl-O-C1-C3 alkyl, wherein the C4-C6 cycloalkyl is optionally substituted with 1 or 2 $R^1$; each of the $R^1$ is independently selected from halogen-substituted C1-C3 alkyl and halogen-substituted C1-C3 alkoxy.

**7.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-6, wherein the ring A is selected from any one of the following groups:

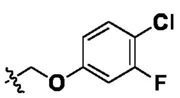 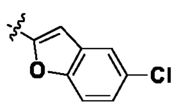 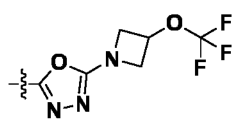 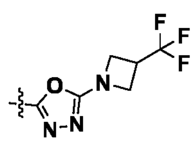

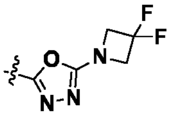
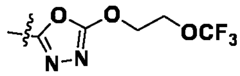
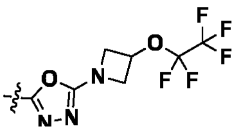
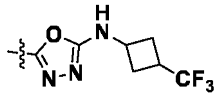

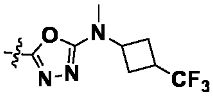
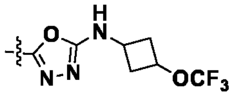
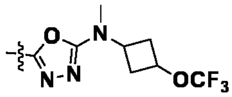
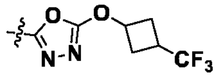

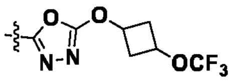
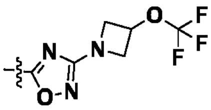
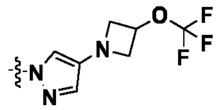
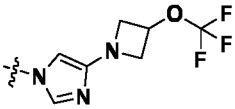

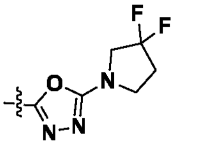
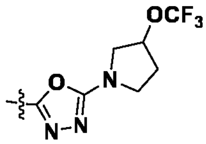
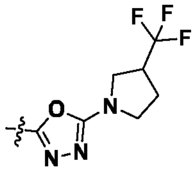
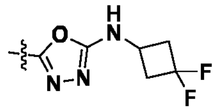

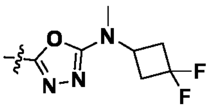
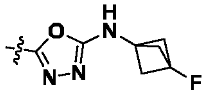
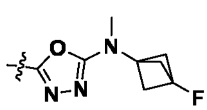
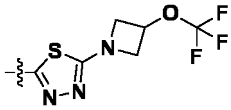

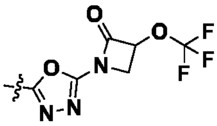
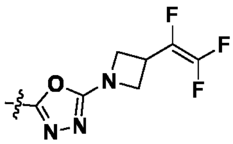
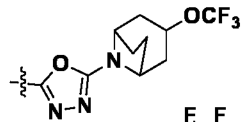
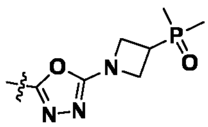

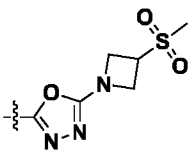
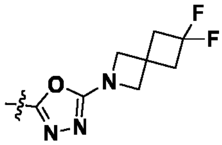
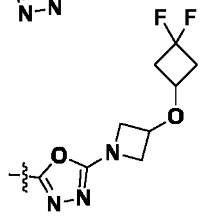
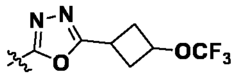

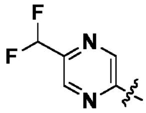
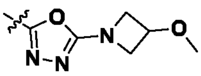
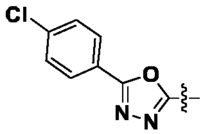
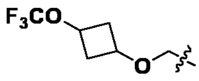

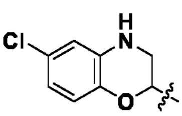
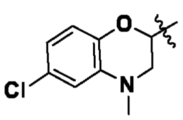
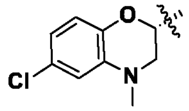
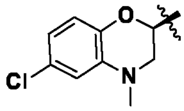

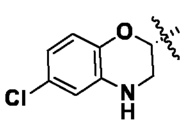
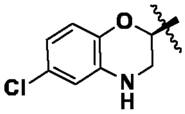
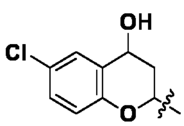
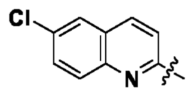

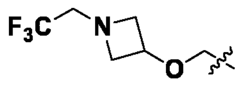 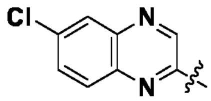 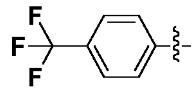 ;

preferably, ring A is selected from any one of the following groups:

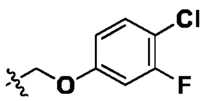 , 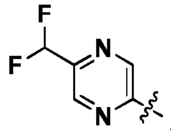 , 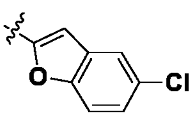 , 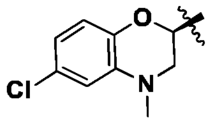 , 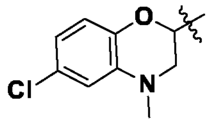 ,

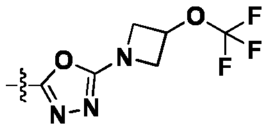 , 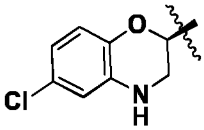 , 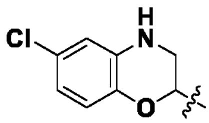 , 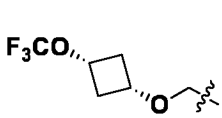 ,

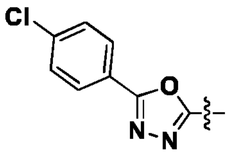 ;

preferably, ring A is selected from any one of the following groups:

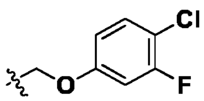 , 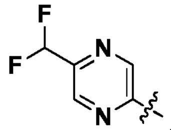 , 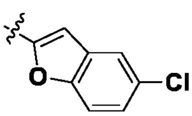 , 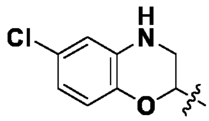 , 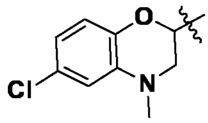 ,

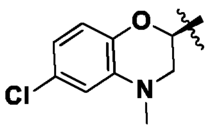 , 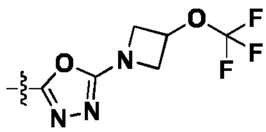 , 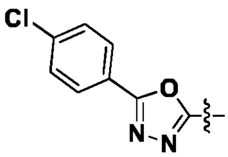 , 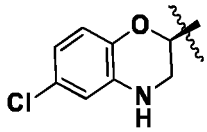 ;

preferably, ring A is selected from any one of the following groups:

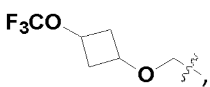 , 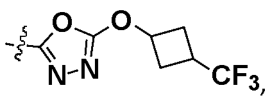 , 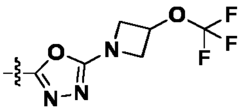 , 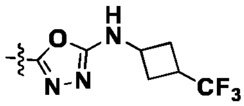 ,

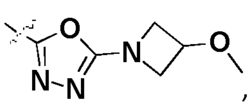 , 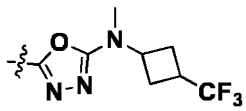 .

**8.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-7, wherein the spiro ring is selected from any one of the following groups: a spiro[2,5]octyl ring, a spiro[3,4]octyl ring, a spiro[3,3]heptyl ring, a spiro[3,5]nonyl ring, a spiro[4,5]decyl ring, a spiro[2,2]pentyl ring, a spiro[2,3]hexyl ring, a spiro[2,4]heptyl ring, a spiro[2,6]nonyl ring, a spiro[2,7]nonyl ring, a spiro[3,6]nonyl ring and a spiro[4,4]nonyl ring; and the spiro heterocyclic

ring is a group formed by substituting any one or more carbon atoms in the spiro ring with a heteroatom(s), wherein the number of heteroatoms in the spiro heterocyclic ring is 1, 2 or 3; preferably, the heteroatom in the spiro heterocyclic ring is nitrogen, or a combination of nitrogen and oxygen;

preferably, the substituent $R^{1F}$ of the spiro ring or the spiro heterocyclic ring is hydrogen, halogen, hydroxyl, amino, cyano, C1-C6 alkyl, C3-C9 cycloalkyl, hydroxyl-substituted C1-C6 alkyl, halogen-substituted C1-C6 alkyl or halogen-substituted C1-C6 alkoxy;
more preferably, the substituent $R^{1F}$ of the spiro ring or the spiro heterocyclic ring is halogen, hydroxyl or amino;
most preferably, the substituent $R^{1F}$ of the spiro ring or the spiro heterocyclic ring is hydroxyl.

**9.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to claim 8, wherein

the ring B is a 7- to 9-membered spiro ring or a 7- to 9-membered spiro heterocyclic ring; preferably, the spiro ring is selected from any one of the following groups: a spiro[2,5]octyl ring, a spiro[3,4]octyl ring, a spiro[3,3]heptyl ring and a spiro[3,5]nonyl ring; the 7- to 9-membered spiro heterocyclic ring is a group formed by substituting any one or more carbon atoms in the 7-to 9-membered spiro ring with a heteroatom(s), wherein the number of heteroatoms in the spiro heterocyclic ring is 1, 2 or 3;
preferably, the heteroatom in the spiro heterocyclic ring is nitrogen, or a combination of nitrogen and oxygen;
preferably, the ring B is selected from any one of the following groups:

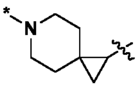 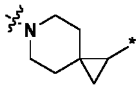 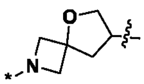 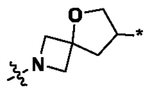 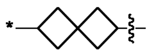

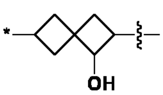 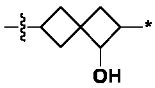 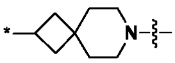 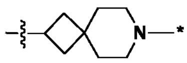 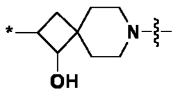

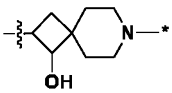 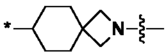 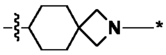 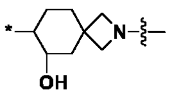 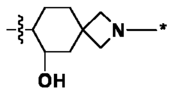

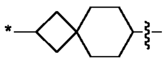 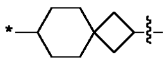 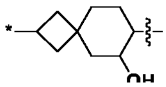 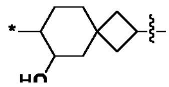 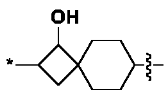

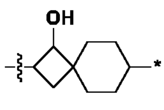 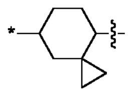 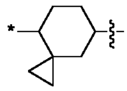

wherein the end is connected to $L^1$, and the * end is connected to $L^2$;
preferably, the ring B is selected from any one of the following groups:

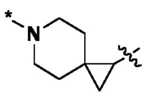 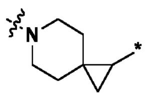 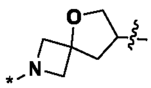 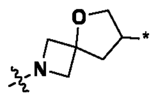 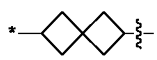

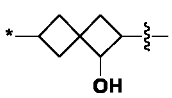 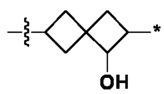 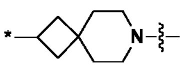 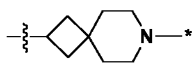 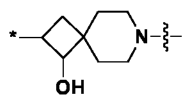

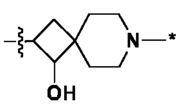 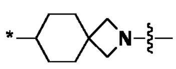 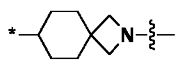 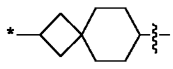 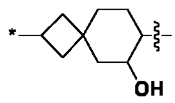

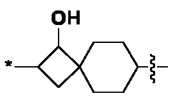 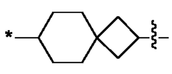 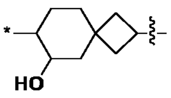 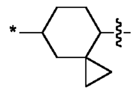 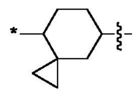 ,

further preferably, the ring B is selected from any one of the following groups:

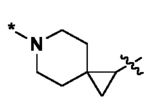 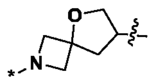 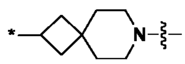 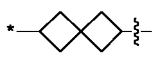 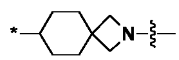

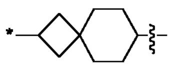 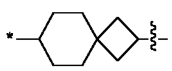 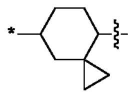 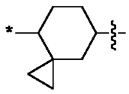

,

further preferably, the ring B is selected from any one of the following groups:

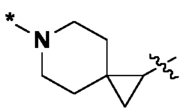 , 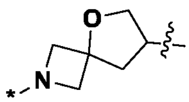 , 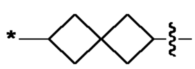 , 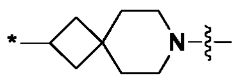 , 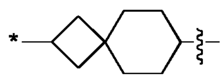 ,

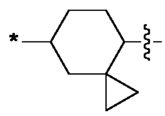 .

**10.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-9, wherein

the ring C is 6- to 10-membered aryl-E-C1-C3 alkyl, C4-C9 cycloalkyl-E-C1-C3 alkyl, 4- to 9-membered heterocyclyl-E-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5-to 6-membered heteroaryl; the C3-C7 cycloalkyl, 3- to 7-membered heterocyclyl, phenyl or 5- to 6-membered heteroaryl is each optionally substituted with 1-5 $R^{1F}$; when $R^2$ is phenyl, the phenyl and 4- to 9-membered heterocyclyl or 5- to 10-membered heteroaryl optionally share two carbon atoms to form a fused ring;
each of the E is independently a linking group containing a heteroatom;
preferably, each of the E is independently O or $NR^d$ or S, wherein each $R^d$ is independently selected from hydrogen, deuterium and C1-C3 alkyl;
more preferably, each of the E is independently O or NH.

**11.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-10, wherein

the ring C is phenyl-E-C1-C3 alkyl, wherein the phenyl is substituted with 1, 2 or 3 $R^2$, and the E is a linking group containing a heteroatom(s);
preferably, the E is O or $NR^d$ or S, and $R^d$ is selected from hydrogen, deuterium and C1-C3 alkyl; preferably, the E is O or NH;
preferably, each of the $R^2$ is independently halogen;
preferably, the ring C is phenyl-O-C1-C3 alkyl;
or the ring C is C4-C9 cycloalkyl-E-C1-C3 alkyl or 4- to 9-membered heterocyclyl-E-C1-C3 alkyl, wherein the cycloalkyl or heterocyclyl is substituted with $R^2$; the E is O or $NR^d$ or S, and $R^d$ is selected from hydrogen, deuterium and C1-C3 alkyl; the $R^2$ is hydrogen, halogen, C1-C3 alkyl, C1-C3 haloalkyl, C1-C3 alkoxy or C1-C3 haloalkoxy;
preferably, the cycloalkyl is cyclopropyl, cyclobutyl or cyclopentyl;
preferably, the heterocyclyl is oxiranyl, oxetanyl, oxocyclopentyl, aziridinyl, azetidinyl or azacyclopentyl;
preferably, the E is O or NH;
or the ring C is 4- to 9-membered heterocyclyl, preferably 5- to 6-membered heterocyclyl;
preferably, the ring C is

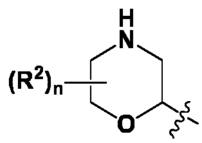 or 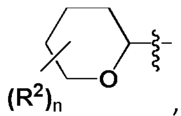 ,

wherein the number n of the $R^2$ is 1 or 2 or 3;
preferably, each of the $R^2$ is independently selected from the group consisting of hydrogen, halogen, C1-C3 alkyl, phenyl, halophenyl and hydroxyl; when the $R^2$ is phenyl or halophenyl, the phenyl or halophenyl and

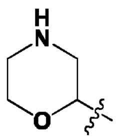 or 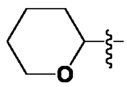

optionally share two carbon atoms to form a fused ring, wherein the fusion mode is preferably

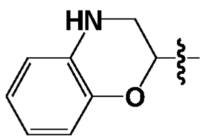 or 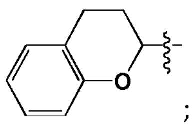 ;

preferably, the ring C is

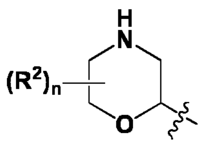 ,

wherein the number n of the $R^2$ is 1 or 2 or 3;
preferably, each of the $R^2$ is independently selected from the group consisting of hydrogen, halogen, C1-C3 alkyl, phenyl and halophenyl; when the $R^2$ is a substituent on an N atom, the $R^2$ is selected from the group consisting of hydrogen, halogen and C1-C3 alkyl; when the $R^2$ is phenyl or halophenyl, the phenyl or the halophenyl and morpholinyl optionally share two carbon atoms
to form a fused ring, wherein the fusion mode is preferably

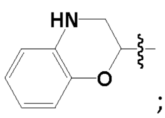 ;

preferably, the ring C is

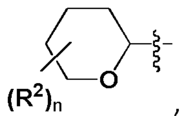,

wherein the number n of the $R^2$ is 1 or 2 or 3;
preferably, each of the $R^2$ is independently selected from the group consisting of halogen, phenyl, halophenyl and hydroxyl; when the $R^2$ is phenyl or halophenyl, the phenyl or the halophenyl and

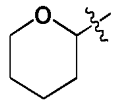

optionally share two carbon atoms to form a fused ring, wherein the fusion mode is preferably

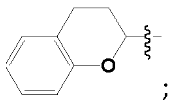;

or the ring C is 5- to 10-membered heteroaryl, and preferably, the ring C is any one of the following 5- to 10-membered heteroaryl groups:

(1) 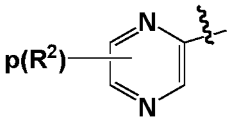 or 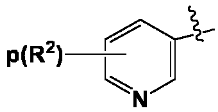;

preferably, each $R^2$ is independently selected from any one of the following groups: hydrogen, halogen, C1-C3 alkyl, C1-C3 haloalkyl, phenyl and halophenyl, wherein the number p of the $R^2$ is 1 or 2; and when the $R^2$ is phenyl or halophenyl, the phenyl or halophenyl and

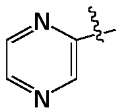

or

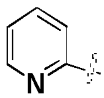

share two carbon atoms to form a fused ring, wherein the fusion mode is preferably

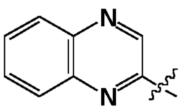 or 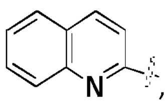,

respectively;

(2) 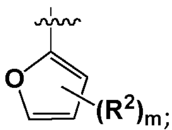;

preferably, the $R^2$ is selected from the group consisting of hydrogen, halogen, phenyl, C1-C3 alkyl and C1-C3 alkoxy-C3-C6 cycloalkyl; the phenyl, the alkyl and the alkoxy are unsubstituted or substituted with halogen; when the $R^2$ is phenyl, the phenyl and

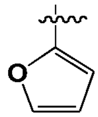

optionally share two carbon atoms to form a fused ring, wherein the fusion mode is preferably

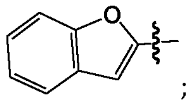;

or the ring C is phenyl; the phenyl is substituted with $R^2$; preferably, the $R^2$ is selected from the group consisting of C1-C3 haloalkyl and C1-C3 haloalkoxy.

**12.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-11, wherein the ring C is selected from any one of the following groups:

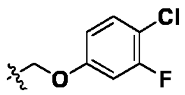 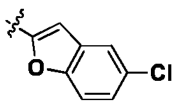 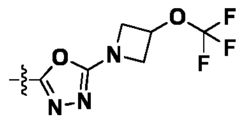 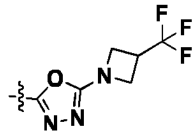

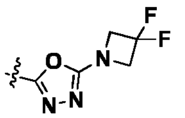 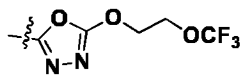 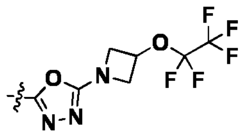 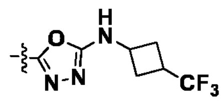

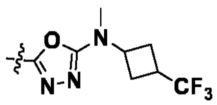 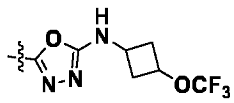 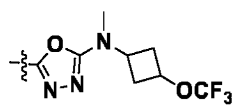 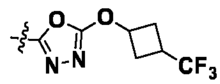

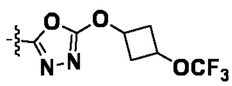 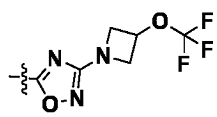 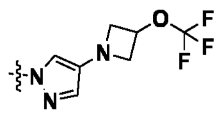 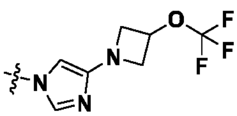

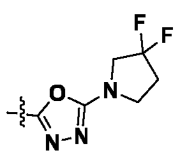 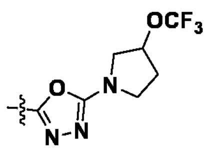 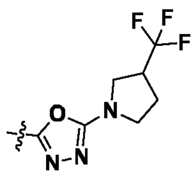 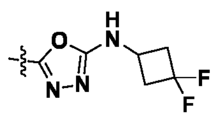

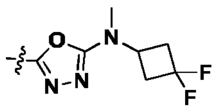 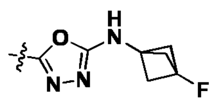 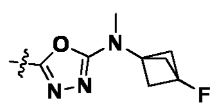 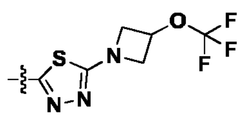

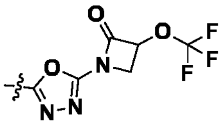 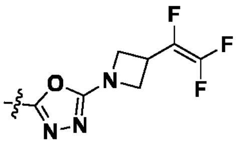 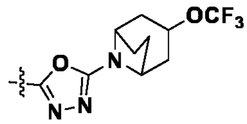 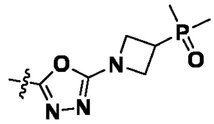

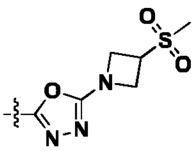 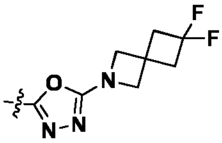 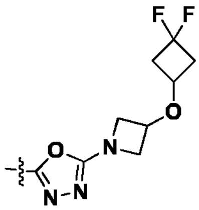 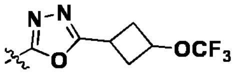

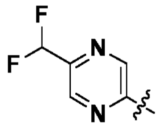 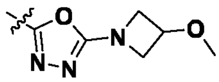 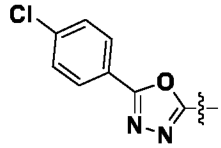 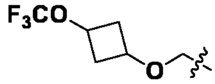

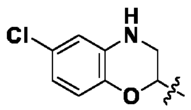 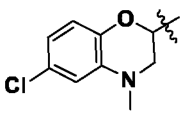 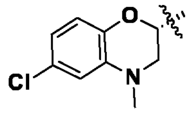 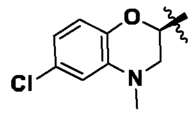

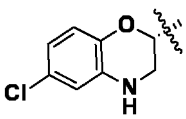 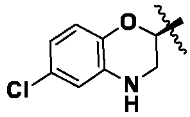 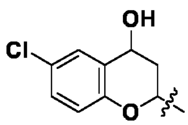 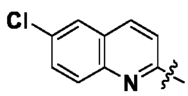

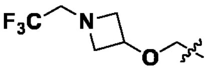 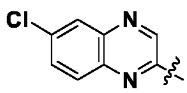 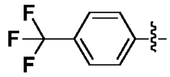 ;

preferably, the ring C is selected from any one of the following groups:

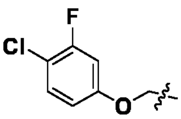 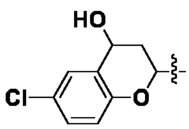 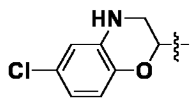 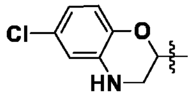

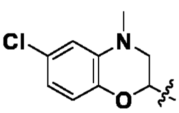 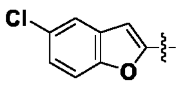 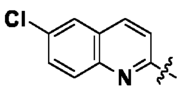 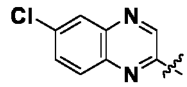

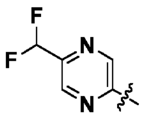 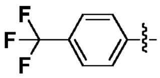 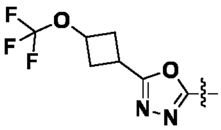 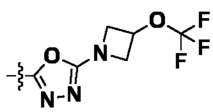

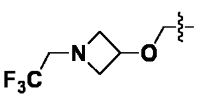 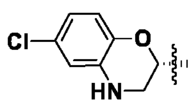 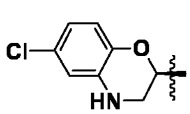

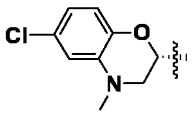 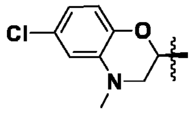 ,

preferably, the ring C is selected from any one of the following groups:

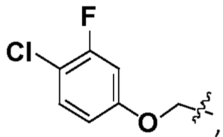, 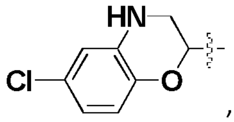, 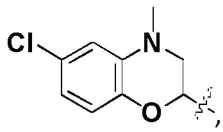, 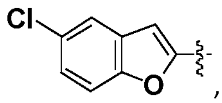,

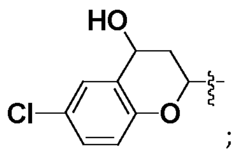 ;

preferably, the ring C is selected from any one of the following groups:

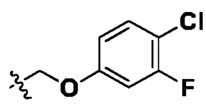, 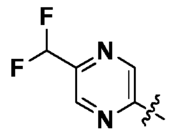, 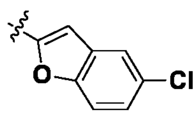, 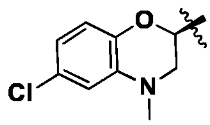, 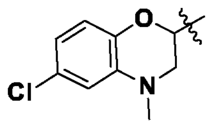,

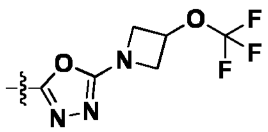, 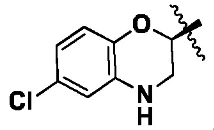, 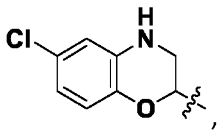, 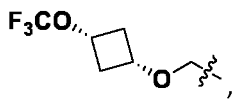,

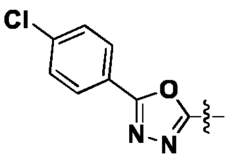 ;

preferably, the ring C is selected from any one of the following groups:

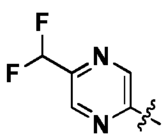, 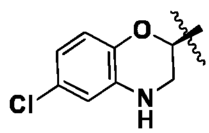, 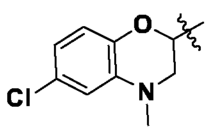, 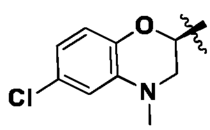, 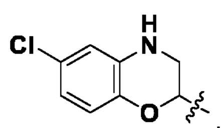,

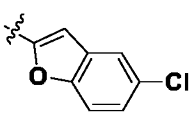, 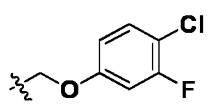, 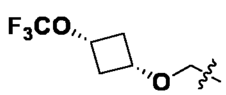, 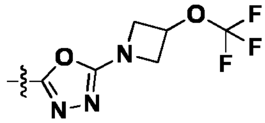 .

**13.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-12, wherein the compound has a structure as shown in formula I:

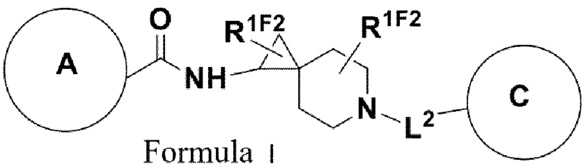

Formula I

.

preferably,

ring A is 3- to 9-membered heterocyclyl, 5- to 10-membered heteroaryl, 6- to 10-membered aryl-D-C1-C6 alkyl or 5- to 10-membered heteroaryl-D-C1-C6 alkyl, wherein the heterocyclyl, aryl and heteroaryl can each be optionally substituted with 1-5 $R^1$;

each of the $R^1$ is independently selected from the group consisting of halogen, C1-C10 alkyl, hydroxyl-substituted C1-C6 alkyl, halogen-substituted C1-C6 alkyl, halogen-substituted C1-C6 alkoxy, -$G^1$ and -O-$G^1$; or 2 $R^1$ groups on adjacent atoms taken together with the atoms to which they are attached can form C3-C7 cycloalkyl, 3- to 7-membered heterocyclyl, 6- to 10-membered aryl or 5- to 6-membered heteroaryl; the C3-C7 cycloalkyl, 3- to 7-membered heterocyclyl, 6- to 10-membered aryl or 5- to 6-membered heteroaryl is each optionally substituted with 1-5 $R^{1F1}$; each of the $G^1$ is independently selected from C3-C9 cycloalkyl, 3- to 9-membered heterocyclyl, 6- to 10-membered aryl and 5- to 10-membered heteroaryl; each of the $G^1$ is optionally substituted with 1-3 $R^{1F1}$;

each of the $R^{1F1}$ and $R^{1F2}$ is independently selected from the group consisting of hydrogen, halogen, hydroxyl, amino, cyano, carboxyl, C1-C6 alkyl, C3-C9 cycloalkyl, hydroxyl-substituted C1-C6 alkyl, halogen-substituted C1-C6 alkyl and halogen-substituted C1-C6 alkoxy; preferably, each of the $R^{1F2}$ is independently selected from hydrogen, halogen, hydroxyl and amino;

$L^2$ is null, -NH-,

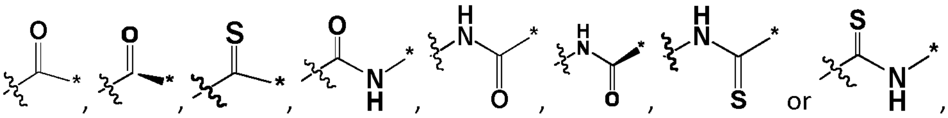

, , , , , , , or ,

wherein the … end is connected to ring B, the * end is connected to ring C, and preferably, the : end is connected to the N atom in

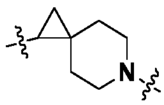

in formula I;

preferably, the $L^2$ is null, -NH-,

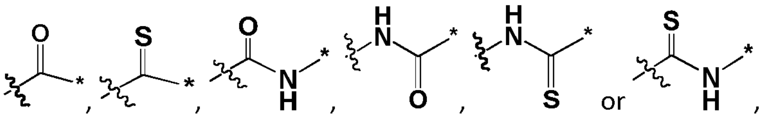

, , , , or ,

preferably, the $L^2$ is null,

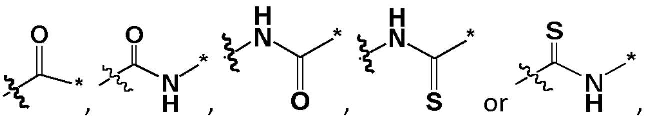

, , , or ,

preferably, the $L^2$ is null,

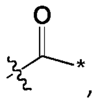, 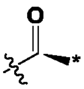, 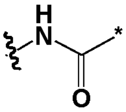 or 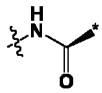;

preferably, the $L^2$ is

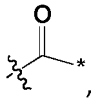, 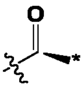, 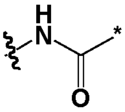 or 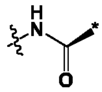;

preferably, the $L^2$ is null,

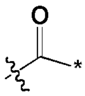 or 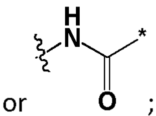;

ring C is 6- to 10-membered aryl-E-C1-C6 alkyl, 3- to 9-membered heterocyclyl, 5- to 10-membered heteroaryl or 6- to 10-membered aryl; the aryl, heteroaryl and heterocyclyl can each be optionally substituted with 1-3 $R^2$; each of the $R^2$ is independently selected from the group consisting of halogen, hydroxyl, phenyl, C1-C6 alkyl, C1-C6 alkoxy and C1-C3 alkoxy-C1-C6 alkyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl and C1-C3 alkoxy in the C1-C3 alkoxy-C3-C6 cycloalkyl are each optionally substituted with halogen; when $R^2$ is phenyl, the phenyl and 3- to 9-membered heterocyclyl or 6- to 10-membered heteroaryl optionally share two carbon atoms to form a fused ring; each of the D or E is independently a linking group containing a heteroatom; preferably, each of the D or E is independently O or $NR^d$ or S, wherein each $R^d$ is independently selected from hydrogen, deuterium, C1-C6 alkyl and halogen-substituted C1-C6 alkyl; more preferably, each of the D or E is independently O or NH.

**14.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-12, wherein the compound has a structure as shown in formula II or formula III:

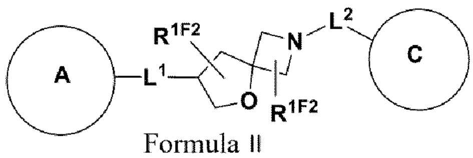

Formula II

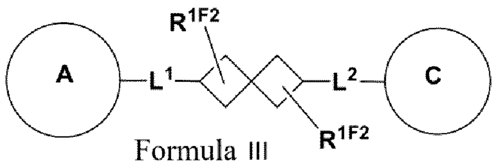

Formula III

preferably,

each of the ring A is independently 5- to 7-membered heteroaryl, phenyl-D-C1-C6 alkyl or 3- to 9-membered heterocyclyl, wherein the heteroaryl and phenyl can each be optionally substituted with 1 or 2 $R^1$;
each of the $R^1$ is independently selected from the group consisting of halogen, halogen-substituted C1-C6 alkyl, halogen-substituted C1-C6 alkoxy, $-OR^{1A}$, $-NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C6 alkyl, C4-C9 cycloalkyl, 4- to 9-membered heterocyclyl, halogen-substituted C1-C6 alkyl, halogen-substituted C4-C9 cycloalkyl, halogen-substituted 4- to 9-membered heterocyclyl and halogen-substituted C1-C6 alkoxy;

each of the $G^1$ is independently selected from C3-C9 cycloalkyl, 3- to 9-membered heterocyclyl and phenyl; each of the $G^1$ is optionally substituted with 1-3 $R^{1F1}$; when $G^1$ is phenyl, the phenyl and 3- to 9-membered heterocyclyl or 5- to 7-membered heteroaryl optionally share two carbon atoms to form a fused ring;
each of the $R^{1F1}$ is independently selected from the group consisting of halogen, C1-C6 alkyl, C3-C9 cycloalkyl, halogen-substituted C1-C6 alkyl, halogen-substituted C3-C9 cycloalkyl, halogen-substituted C1-C3 alkoxy, halogen-substituted C1-C6 alkenyl, oxo, $-OR^{1A}$, $-NR^{1B}R^{1C}$, $-P(O)\ R^{1B}R^{1C}$ and $-S(O)_2R^{1D}$;
$L^1$ is null, -NH-,

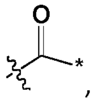, 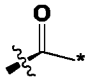, 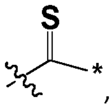, 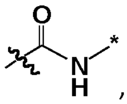, 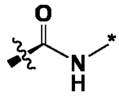, 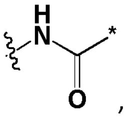, 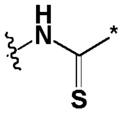

or

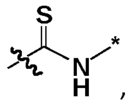,

wherein the end is connected to ring A, and the * end is connected to ring B;
preferably, the * end is connected to the &C atom in

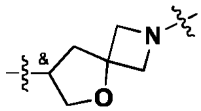

in formula II or the &C atom in

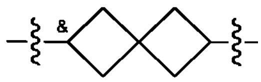

in formula III;
preferably, the $L^1$ is null, -NH-,

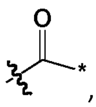, 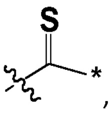, 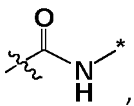, 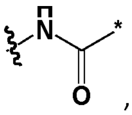, 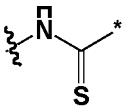 or 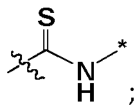;

preferably, the $L^1$ is null,

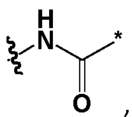, 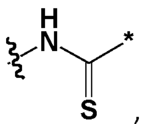, 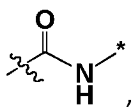, 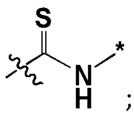;

preferably, the $L^1$ is null,

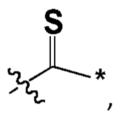, 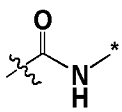 or 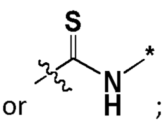;

further preferably, the $L^1$ is null or

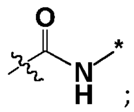;

$L^2$ is null, -NH-,

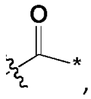, 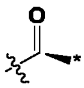, 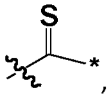, 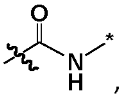, 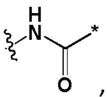, 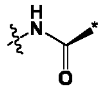, 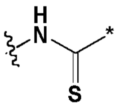 or 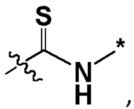,

wherein the end is connected to ring B, and the * end is connected to ring C; preferably, the end is connected to the N atom in

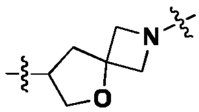

in formula II or the @C atom in

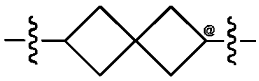

in formula III;
preferably, the $L^2$ is null, -NH-,

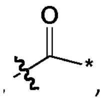, 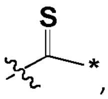, 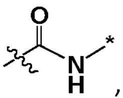, 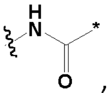, 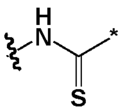 or 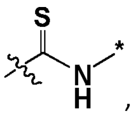,

preferably, the $L^2$ is null,

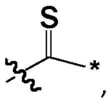, 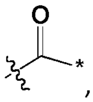, 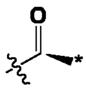, 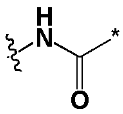 or 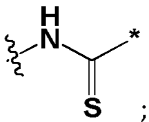;

preferably, in the formula II, the $L^2$ is

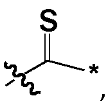, 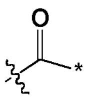 or 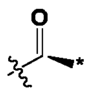,

, more preferably

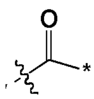

or

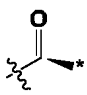;

preferably, in the formula III, the $L^2$ is null,

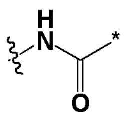 or 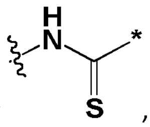,

more preferably is null or

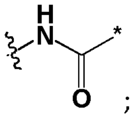;

each of the $R^{1F2}$ is independently selected from hydrogen, halogen, hydroxyl and amino;

preferably, each of the $R^{1F2}$ is independently selected from hydrogen and hydroxyl;

the ring C is selected from 6- to 10-membered aryl-E-C1-C3 alkyl, C4-C9 cycloalkyl-E-C1-C3 alkyl, 4- to 9-membered heterocyclyl-E-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F}$;

when $R^2$ is phenyl, the phenyl and 4- to 9-membered heterocyclyl or 5- to 10-membered heteroaryl optionally share two carbon atoms to form a fused ring;

each of the E is independently a linking group containing a heteroatom; preferably, each of the E is independently O or $NR^d$ or S, wherein each $R^d$ is independently selected from hydrogen, deuterium and C1-C3 alkyl; more preferably, each of the E is independently O or NH.

**15.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-12, wherein the compound has a structure as shown in formula IV:

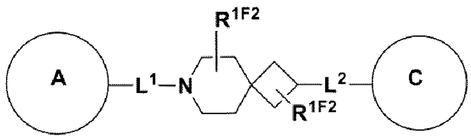

Formula IV

;

preferably,

the ring A is 4- to 8-membered heterocyclyl, 5- to 7-membered heteroaryl, phenyl-O-C1-C6 alkyl or C4-C8 cycloalkyl-O-C1-C6 alkyl, wherein the heterocyclyl, heteroaryl, phenyl and cycloalkyl can each be optionally substituted with 1-2 $R^1$;

each of the $R^1$ is independently selected from the group consisting of halogen, halogen-substituted C1-C3 alkyl, halogen-substituted C1-C3 alkoxy, $-OR^{1A}$, $-NR^{1B}R^{1C}$, $-G^1$, $O-G^1$ and $-NR^{1B}-G^1$;

each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C6 alkyl, halogen-substituted C3-C6 cycloalkyl, halogen-substituted 4- to 6-membered heterocyclyl and halogen-substituted C1-C3 alkoxy;

each of the $G^1$ is independently selected from C3-C6 cycloalkyl, 3- to 6-membered heterocyclyl and phenyl; each of the $G^1$ is optionally substituted with 1-3 $R^{1F1}$; when $G^1$ is phenyl, the phenyl and 5- to 6-membered heterocyclyl or 5- to 7-membered heteroaryl optionally share two carbon atoms to form a fused ring;

each of the $R^{1F1}$ is independently selected from the group consisting of halogen, C1-C3 alkyl, C1-C3 alkoxy, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, - P(O) $R^{1B}R^{1C}$ and $-S(O)_2R^{1D}$;
each of the $R^{1F2}$ is independently selected from hydrogen, halogen, hydroxyl and amino;
preferably, each of the $R^{1F2}$ is independently selected from hydrogen and hydroxyl;
$L^1$ is null, -NH-,

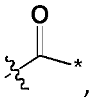, 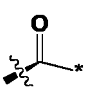, 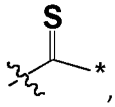, 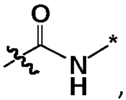, 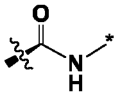, 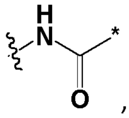, 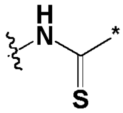 or

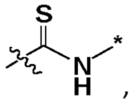,

wherein the end is connected to ring A, and the * end is connected to ring B;
preferably, the * end is connected to the N atom in

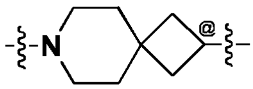

in formula IV;
preferably, the $L^1$ is null, -NH-,

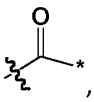, 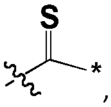, 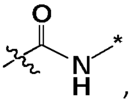, 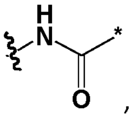, 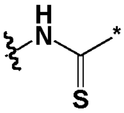 or 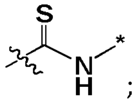;

preferably, the $L^1$ is null,

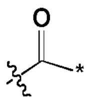, 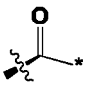, 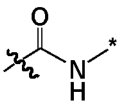 or 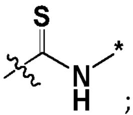;

preferably, the $L^1$ is null,

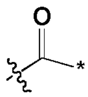 or 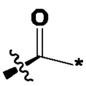;

$L^2$ is null, -NH-,

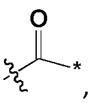, 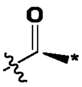, 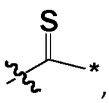, 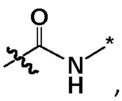, 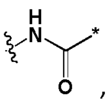, 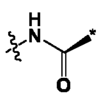, 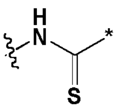 or 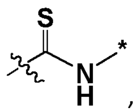,

wherein the end is connected to ring B, and the * end is connected to ring C; preferably, the end is connected to the @C atom in

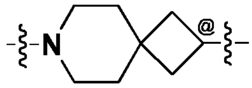

in formula IV;

preferably, the $L^2$ is null, -NH-,

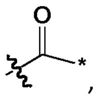, 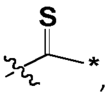, 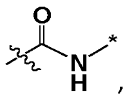, 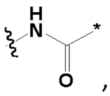, 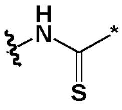 or 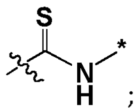;

preferably, the $L^2$ is null,

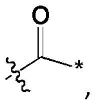, 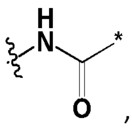, 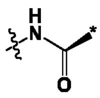 or 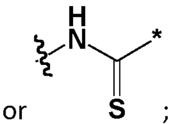;

further preferably, the $L^2$ is

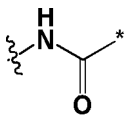 or 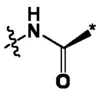;

the ring C is 6- to 10-membered aryl-O-C1-C3 alkyl, C4-C9 cycloalkyl-O-C1-C3 alkyl, 4- to 9-membered heterocyclyl-O-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F1}$; when $R^2$ is phenyl, the phenyl and 4- to 9-membered heterocyclyl or 5- to 10-membered heteroaryl optionally share two carbon atoms to form a fused ring.

**16.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-12, wherein the compound has a structure as shown in formula V:

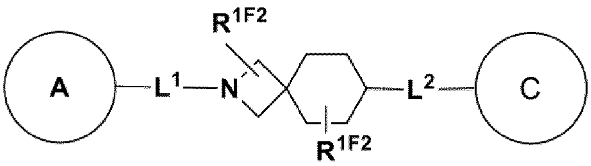

Formula V

preferably,

the ring A is 4- to 8-membered heterocyclyl, 5- to 7-membered heteroaryl or phenyl-O-C1-C6 alkyl, wherein the heterocyclyl, heteroaryl and phenyl can each be optionally substituted with 1-2 $R^1$;
each of the $R^1$ is independently selected from the group consisting of halogen, halogen-substituted C1-C3 alkyl, halogen-substituted C1-C3 alkoxy, $-OR^{1A}$, $-NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C6 alkyl, halogen-substituted C3-C6 cycloalkyl, halogen-substituted 4- to 6-membered heterocyclyl and halogen-substituted C1-C3 alkoxy;
each of the $G^1$ is independently selected from C3-C6 cycloalkyl, 3- to 6-membered heterocyclyl and phenyl; each of the $G^1$ is optionally substituted with 1-3 $R^{1F1}$; when $G^1$ is phenyl, the phenyl and 5- to 6-membered heterocyclyl or 5- to 7-membered heteroaryl optionally share two carbon atoms to form a fused ring;

each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, -P(O) $R^{1B}R^{1C}$ and $-S(O)_2R^{1D}$;

$L^1$ is null, -NH-,

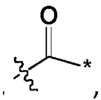, 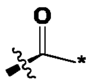, 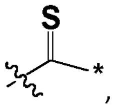, 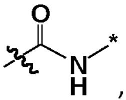, 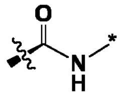, 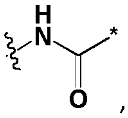, 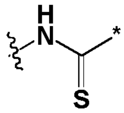 or

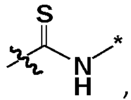,

wherein the end is connected to ring A, and the * end is connected to ring B;

preferably, the * end is connected to the N atom in

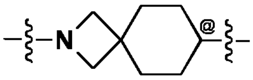

in formula V;

preferably, the $L^1$ is null, -NH-,

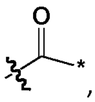, 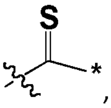, 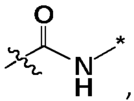, 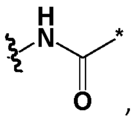, 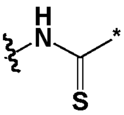 or 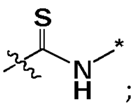;

preferably, the $L^1$ is null,

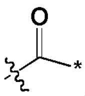, 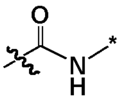 or 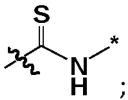;

preferably, the $L^1$ is

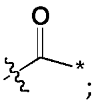;

each $R^{1F2}$ is independently selected from hydrogen, halogen, hydroxyl and amino;

preferably, each of the $R^{1F2}$ is independently selected from hydrogen and hydroxyl;

$L^2$ is null, -NH-,

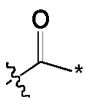, 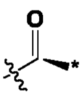, 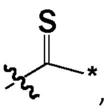, 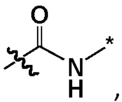, 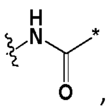, 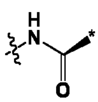, 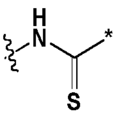 or 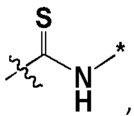,

wherein the end is connected to ring B, and the * end is connected to ring C; preferably, the end is connected to the @C atom in

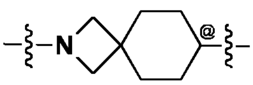

in formula V;

preferably, the $L^2$ is null, -NH-,

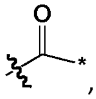, 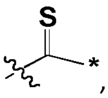, 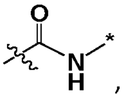, 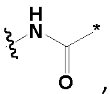, 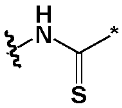 or 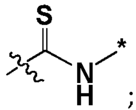;

preferably, the $L^2$ is null,

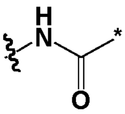 or 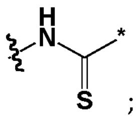;

preferably, the $L^2$ is

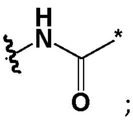;

the ring C is 6- to 10-membered aryl-O-C1-C3 alkyl, 4- to 9-membered heterocyclyl-O-C1-C3 alkyl, 4- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of halogen, hydroxyl, phenyl, C1-C3 alkyl and C1-C3 alkoxy, wherein with regard to options for the $R^2$, the phenyl, C1-C3 alkyl and C1-C3 alkoxy are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F1}$; when $R^2$ is phenyl, the phenyl and 4- to 9-membered heterocyclyl or 5- to 10-membered heteroaryl optionally share two carbon atoms to form a fused ring.

**17.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-12, wherein the compound has a structure as shown in formula VI, formula VII, formula VIII or formula IX:

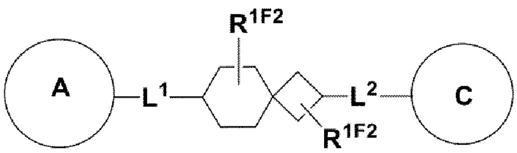

Formula VI

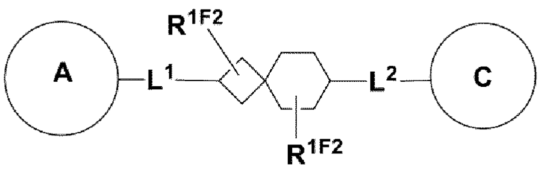

Formula VII

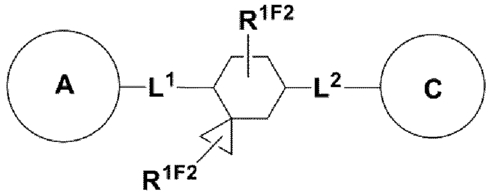

Formula VIII

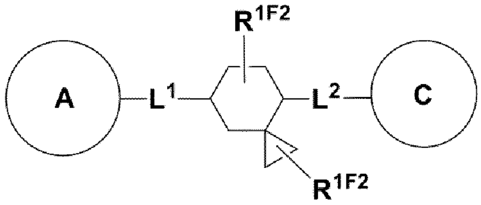

Formula IX

preferably,

the ring A is 3- to 9-membered heterocyclyl, 5- to 10-membered heteroaryl, 6- to 10-membered aryl-D-C1-C6 alkyl, 5- to 10-membered heteroaryl-D-C1-C6 alkyl or 3- to 9-membered heterocyclyl-D-C1-C6 alkyl, wherein the heterocyclyl, heteroaryl and aryl can be optionally substituted with 1-5 $R^1$;
each of the $R^1$ is independently selected from the group consisting of halogen, halogen-substituted C1-C3 alkyl, halogen-substituted C1-C3 alkoxy, $-OR^{1A}$, $-NR^{1B}R^{1C}$, $-G^1$, $O-G^1$ and $-NR^{1B}-G^1$;
each of the $R^{1A}$, $R^{1B}$, $R^{1C}$ and $R^{1D}$ is independently selected from the group consisting of hydrogen, C1-C3 alkyl, C3-C6 cycloalkyl, 4- to 6-membered heterocyclyl, halogen-substituted C1-C6 alkyl, halogen-substituted C3-C6 cycloalkyl, halogen-substituted 4- to 6-membered heterocyclyl and halogen-substituted C1-C3 alkoxy;
each of the $G^1$ is independently selected from C3-C6 cycloalkyl, 3- to 6-membered heterocyclyl and phenyl; each of the $G^1$ is optionally substituted with 1-3 $R^{1F1}$; when $G^1$ is phenyl, the phenyl and 5- to 6-membered heterocyclyl or 5- to 7-membered heteroaryl optionally share two carbon atoms to form a fused ring;
each of the $R^{1F1}$ is independently selected from the group consisting of halogen, C1-C3 alkyl, C3-C6 cycloalkyl, halogen-substituted C1-C3 alkyl, halogen-substituted C1-C3 alkoxy, halogen-substituted C3-C6 cycloalkyl, halogen-substituted C1-C3 alkenyl, oxo, $-OR^{1A}$, $-NR^{1B}R^{1C}$, $-P(O)\ R^{1B}R^{1C}$ and $-S(O)_2R^{1D}$;
further preferably, the ring A is 5- to 7-membered heteroaryl, wherein the heteroaryl can be substituted with 1-2 $R^1$;
$L^1$ is null, -NH-,

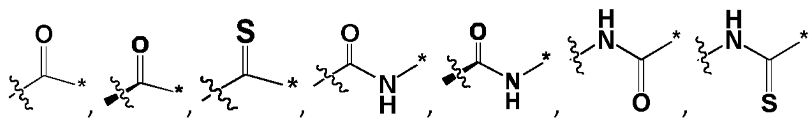

or

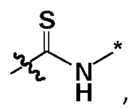,

wherein the end is connected to ring A, and the * end is connected to ring B;
preferably, the * end is connected to the &C atom in

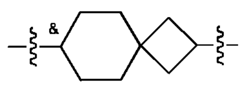

in formula VI, the &C atom in

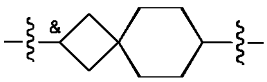

in formula VII, the &C atom in

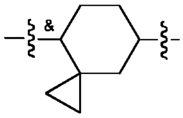

in formula VIII or the &C atom in

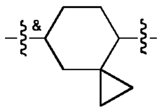

in formula IX;
preferably, the $L^1$ is null, -NH-,

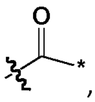, 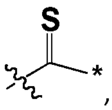, 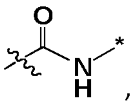, 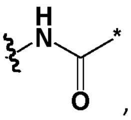, 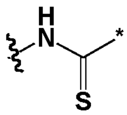 or 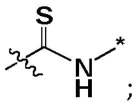;

preferably, the $L^1$ is null,

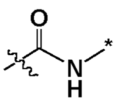 or 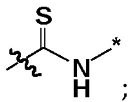;

$L^2$ is null, -NH-,

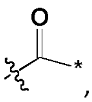, 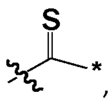, 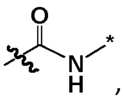, 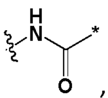, 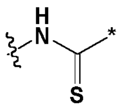 or 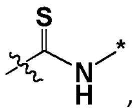,

wherein the end is connected to ring B, and the * end is connected to ring C; preferably, the end is connected to the @C atom in

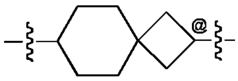

in formula VI, the @C atom in

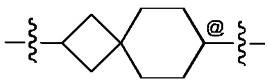

in formula VII, the @C atom in

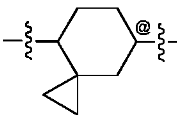

in formula VIII or the @C atom in

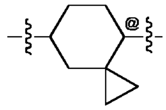

in formula IX;
preferably, the $L^2$ is null, -NH-

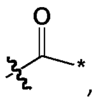, 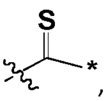, 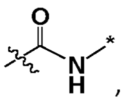, 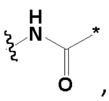, 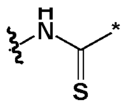 or 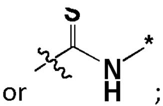;

preferably, the $L^2$ is null,

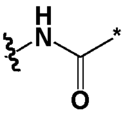 or 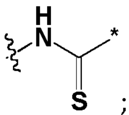;

each of the $R^{1F2}$ is independently selected from hydrogen, halogen, hydroxyl, amino and carboxyl;
preferably, each of the $R^{1F2}$ is independently selected from hydrogen and hydroxyl;
the ring C is 6- to 10-membered aryl-E-C1-C6 alkyl, C3-C9 cycloalkyl-E-C1-C6 alkyl, 3- to 9-membered heterocyclyl-E-C1-C6 alkyl, 3- to 9-membered heterocyclyl, 5- to 10-membered heteroaryl or 6- to 10-membered aryl, wherein the aryl, heteroaryl, cycloalkyl and heterocyclyl can each be optionally substituted with 1-3 $R^2$; each of the $R^2$ is independently selected from the group consisting of hydrogen, deuterium, halogen, hydroxyl, phenyl, C1-C6 alkyl, C1-C6 alkoxy, C1-C3 alkoxy-C1-C6 alkyl, C3-C6 cycloalkyl, C1-C3 alkoxy-C3-C6 cycloalkyl and C1-C3 alkoxy-3- to 6-membered heterocyclyl, wherein with regard to options for the $R^2$, the phenyl, C1-C6 alkyl, C1-C6 alkoxy, C1-C3 alkoxy in the C1-C3 alkoxy-C1-C6 alkyl, C3-C6 cycloalkyl, C1-C3 alkoxy in the C1-C3 alkoxy-C3-C6 cycloalkyl and C1-C3 alkoxy in the C1-C3 alkoxy-3- to 6-membered heterocyclyl are each optionally substituted with halogen; or 2 $R^2$ groups on adjacent atoms taken together with the atoms to which they are attached can form C3-C7 cycloalkyl, 3- to 7-membered heterocyclyl, phenyl or 5- to 6-membered heteroaryl; the C3-C7 cycloalkyl, 3- to 7-membered heterocyclyl, phenyl or 5- to 6-membered heteroaryl is each optionally substituted with 1-5 $R^{1F}$; each of the D or E is independently a linking group containing a heteroatom; preferably, each of the D or E is independently O or $NR^d$ or S, wherein each $R^d$ is independently selected from hydrogen, deuterium and C1-C3 alkyl; more preferably, each of the D or E is independently O or NH.

**18.** The compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-17, wherein the compound is specifically:

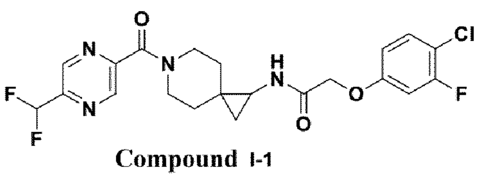

Compound I-1

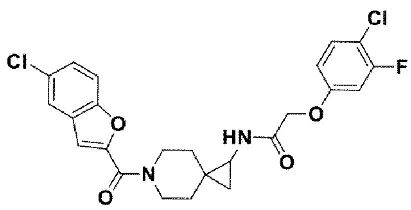

Compound I-2

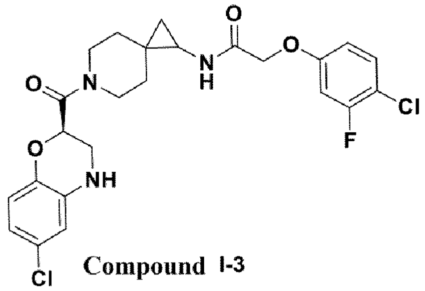

Compound I-3

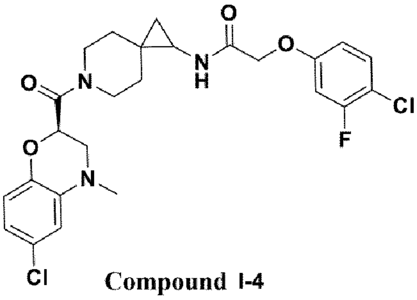

Compound I-4

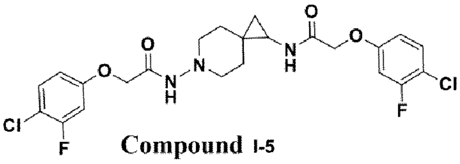

Compound I-5

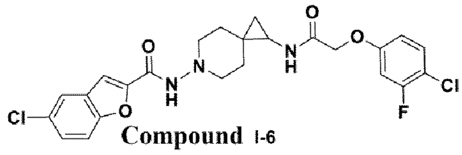

Compound I-6

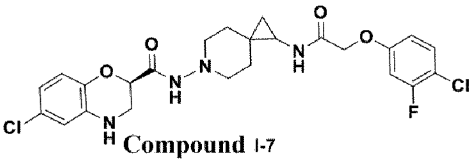

Compound I-7

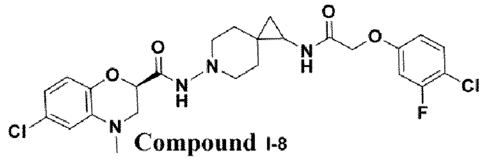

Compound I-8

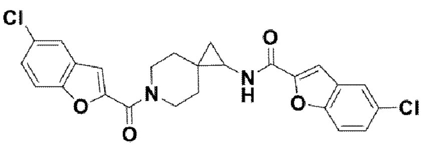

Compound I-9

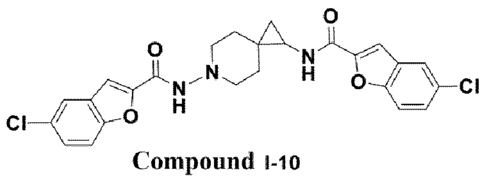

Compound I-10

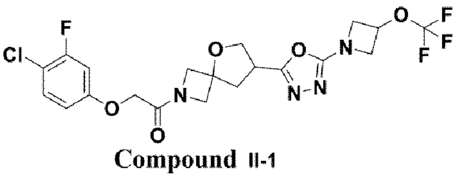

Compound II-1

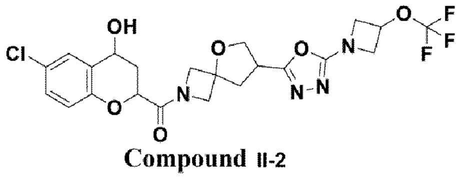

Compound II-2

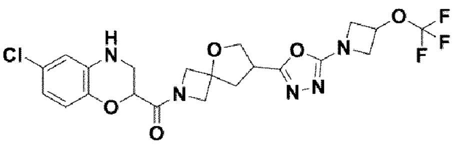

Compound II-3

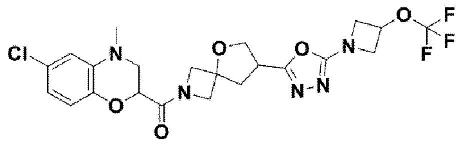

Compound II-4

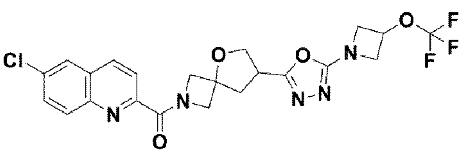

Compound II-5

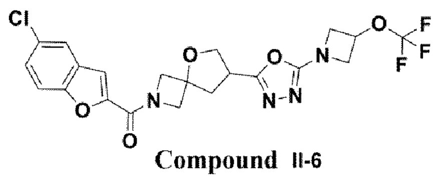

Compound II-6

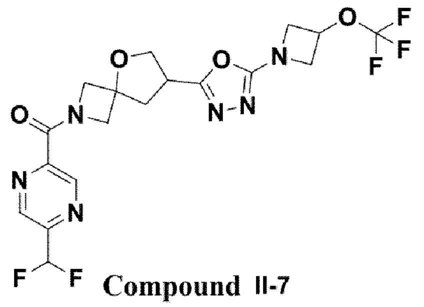

Compound II-7

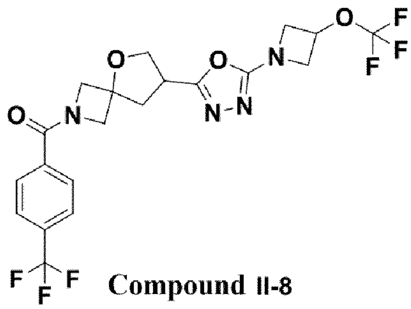

Compound II-8

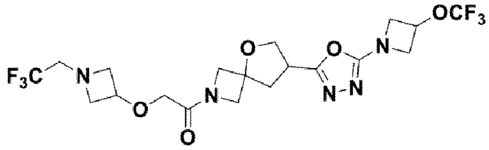

Compound II-9

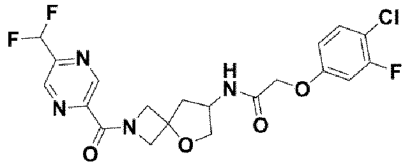

Compound II-10

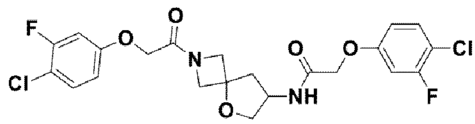

Compound II-11

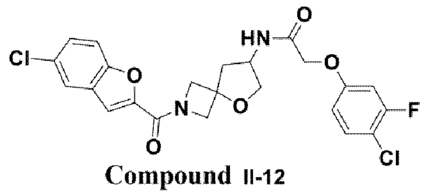

Compound II-12

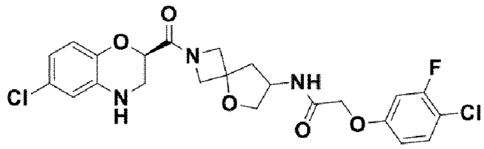

Compound II-13

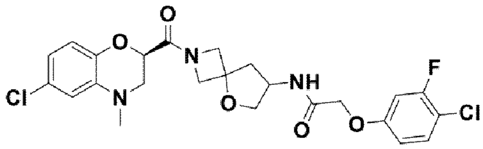

Compound II-14

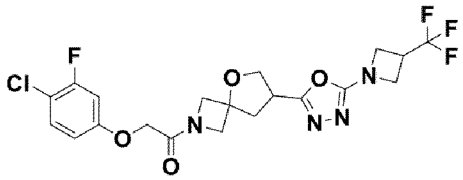

Compound II-15

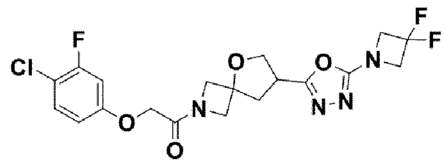

Compound II-16

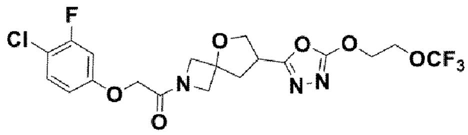

Compound II-17

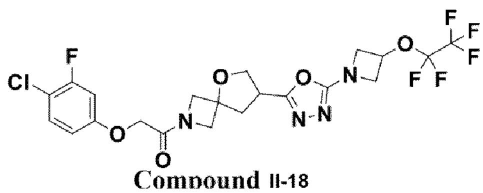

Compound II-18

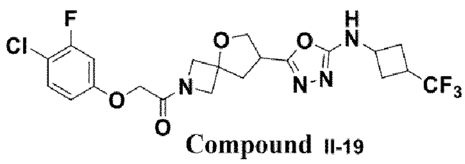

Compound II-19

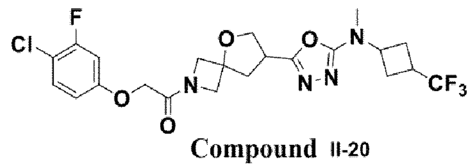

Compound II-20

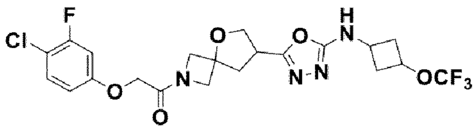

Compound II-21

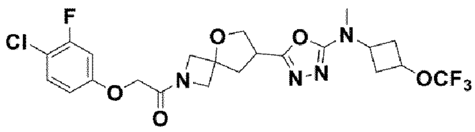

Compound II-22

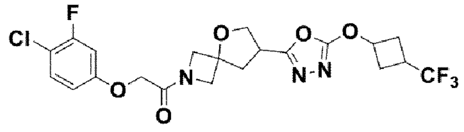

Compound II-23

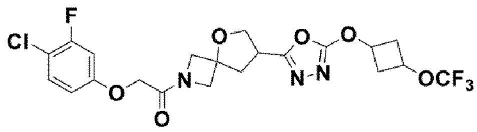

Compound II-24

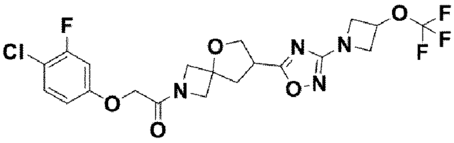

Compound II-25

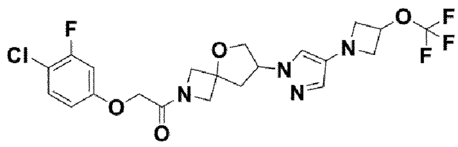

Compound II-26

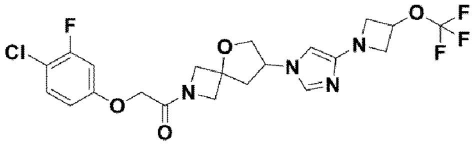

Compound II-27

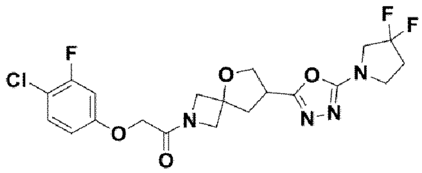

Compound II-28

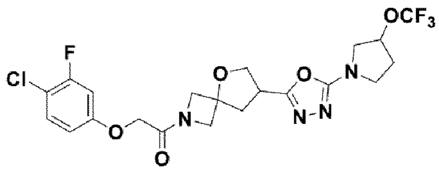

Compound II-29

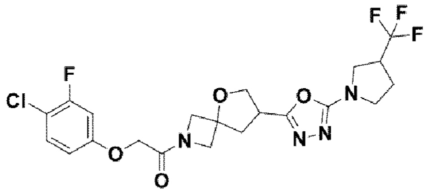

Compound II-30

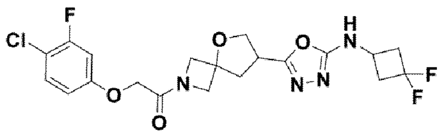

Compound II-31

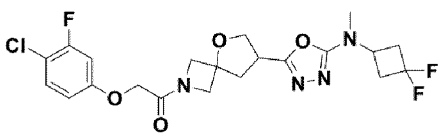

Compound II-32

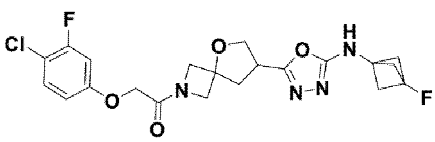

Compound II-33

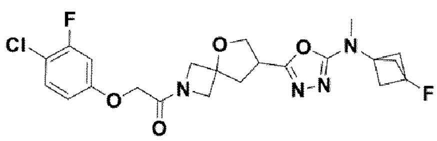

Compound II-34

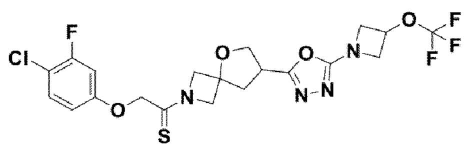

Compound II-35

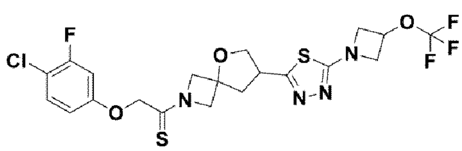

Compound II-36

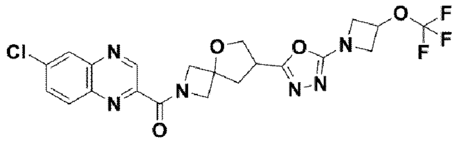

Compound II-37

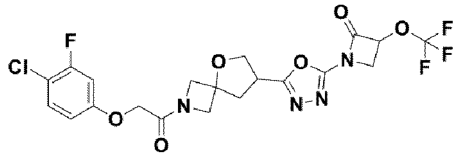

Compound II-38

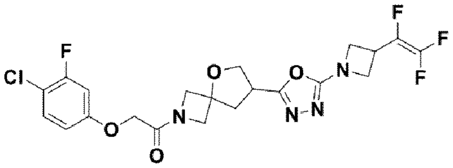

Compound II-39

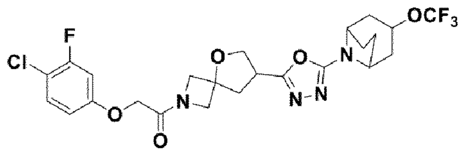

Compound II-40

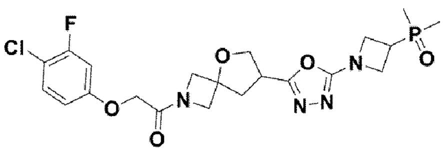

Compound II-41

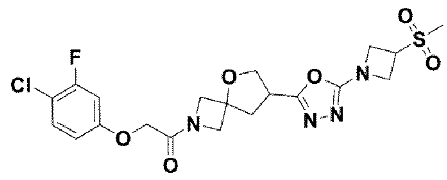

Compound II-42

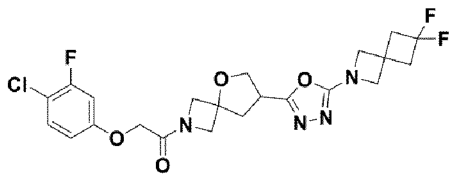

Compound II-43

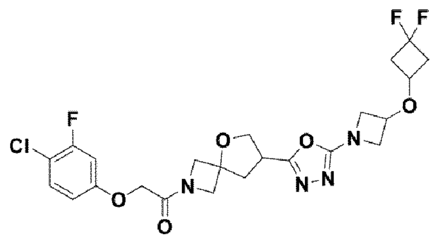

Compound II-44

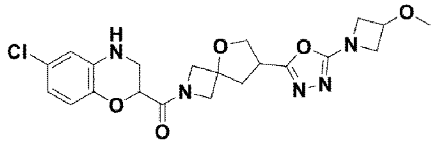

Compound II-45

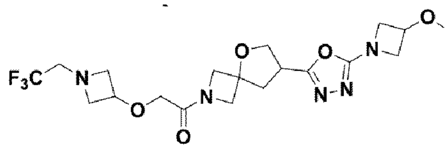

Compound II-46

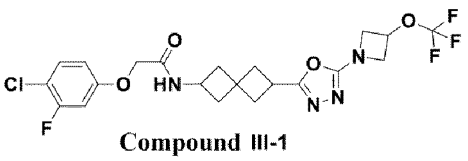

Compound III-1

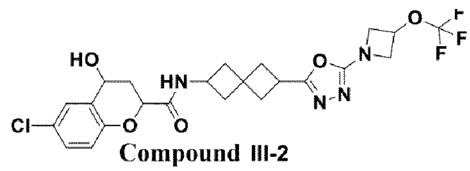

Compound III-2

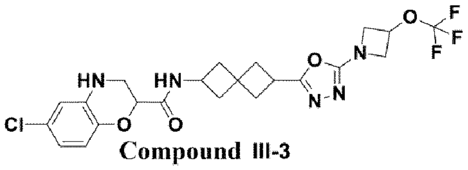

Compound III-3

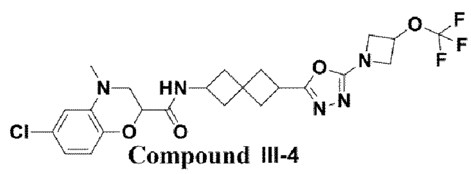

Compound III-4

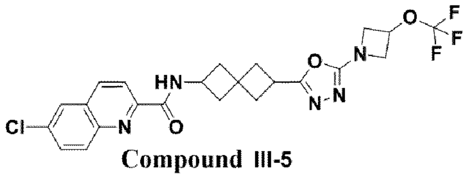

Compound III-5

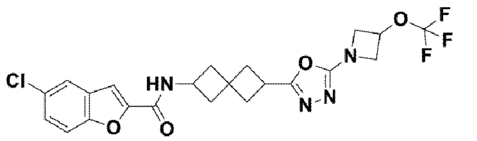

Compound III-6

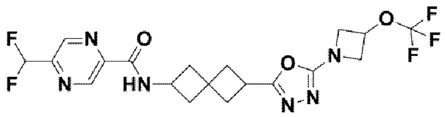

Compound III-7

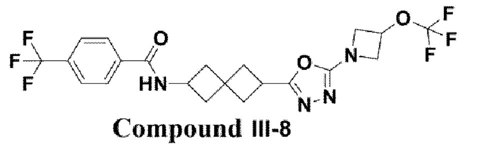

Compound III-8

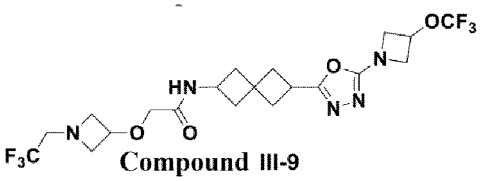

Compound III-9

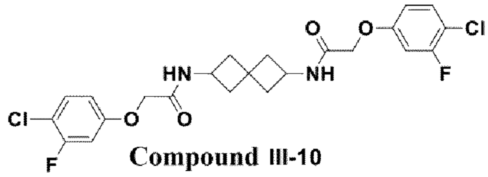

Compound III-10

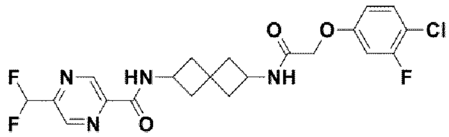

Compound III-11

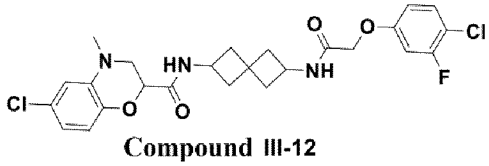

Compound III-12

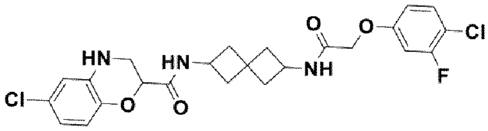

Compound III-13

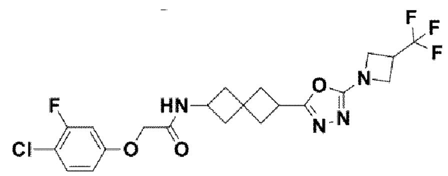

Compound III-14

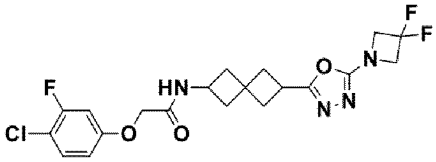

Compound III-15

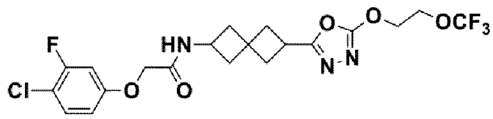

Compound III-16

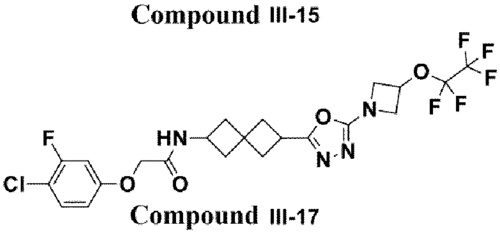

Compound III-17

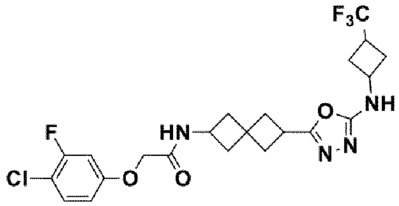

Compound III-18

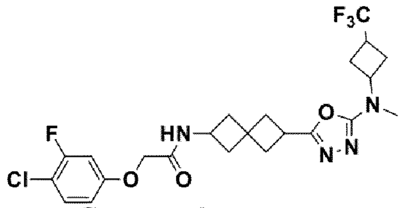

Compound III-19

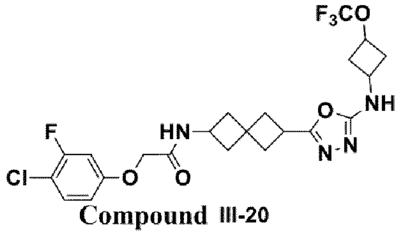

Compound III-20

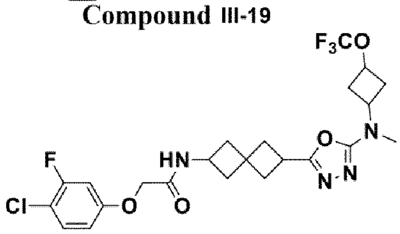

Compound III-21

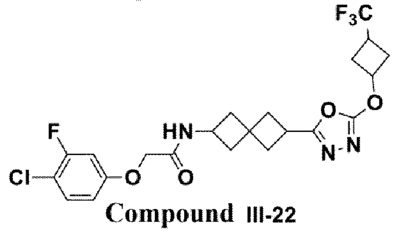

Compound III-22

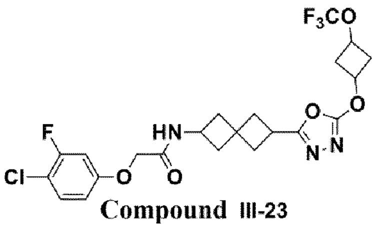

Compound III-23

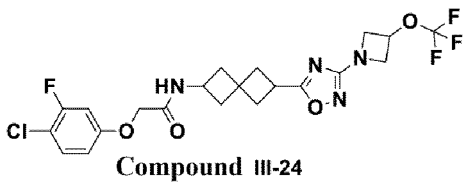

Compound III-24

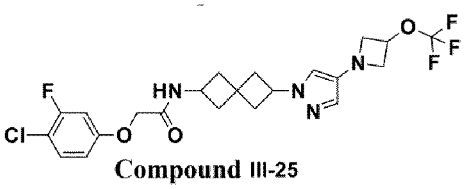

Compound III-25

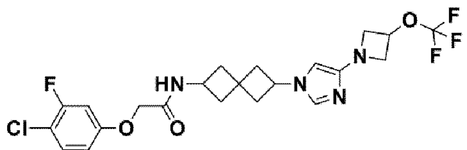

Compound III-26

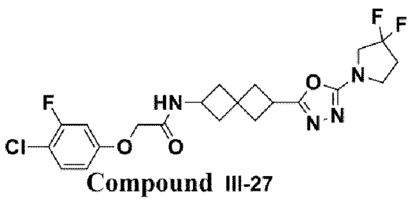

Compound III-27

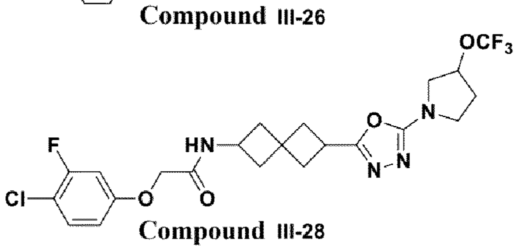

Compound III-28

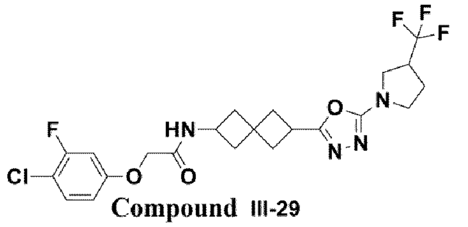

Compound III-29

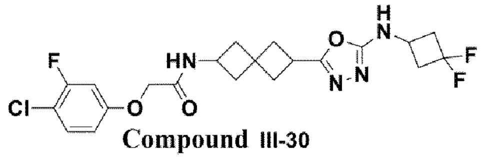

Compound III-30

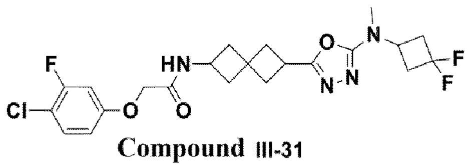

Compound III-31

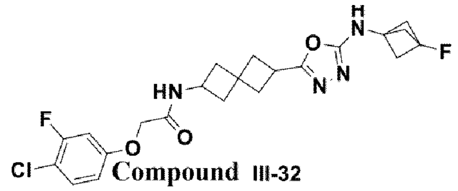

Compound III-32

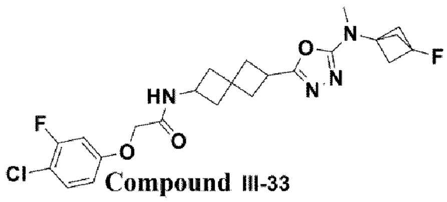

Compound III-33

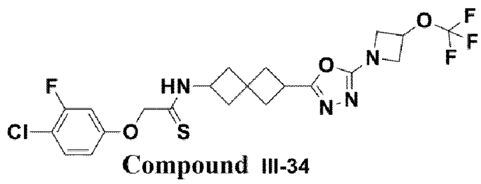

Compound III-34

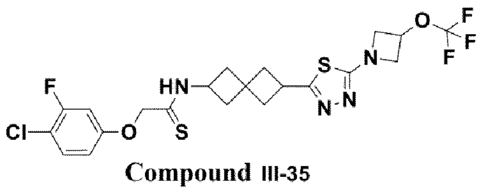

Compound III-35

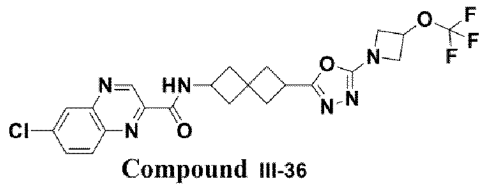

Compound III-36

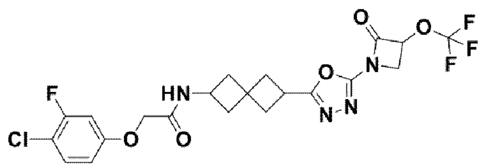

Compound III-37

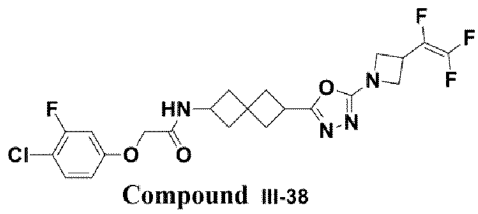

Compound III-38

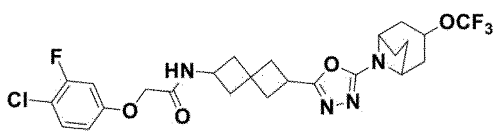

Compound III-39

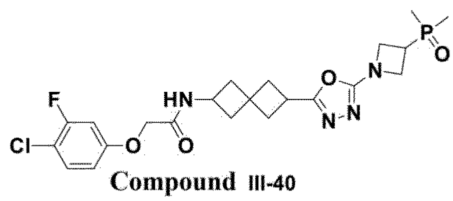

Compound III-40

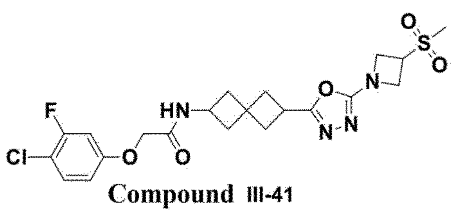

Compound III-41

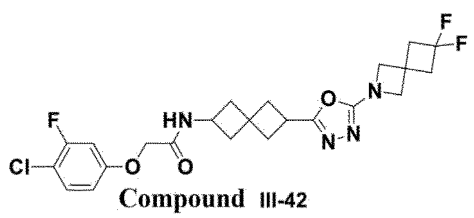

Compound III-42

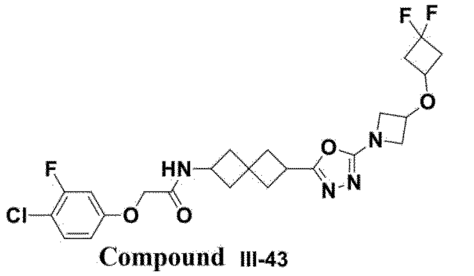

Compound III-43

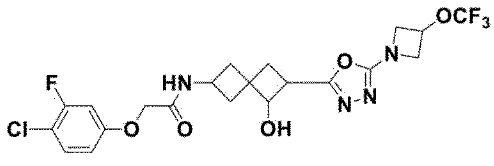

Compound III-44

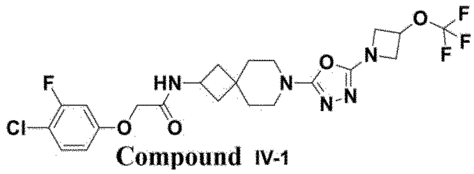

Compound IV-1

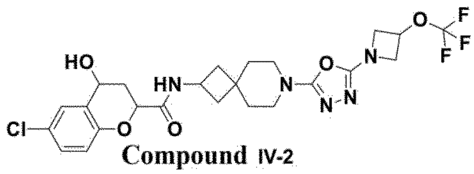

Compound IV-2

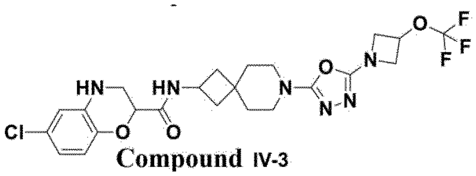

Compound IV-3

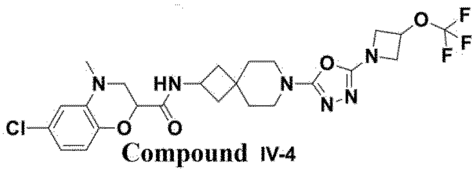

Compound IV-4

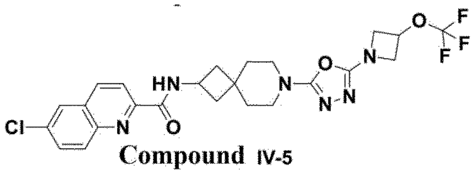

Compound IV-5

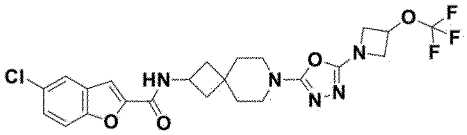

Compound IV-6

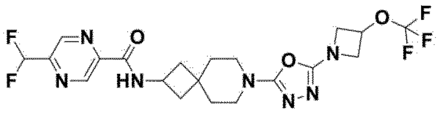

Compound IV-7

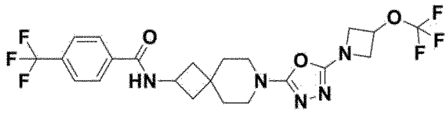

Compound IV-8

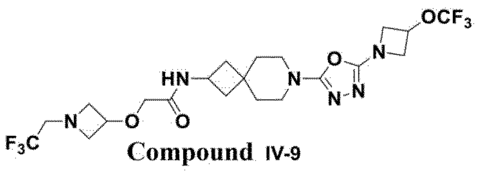

Compound IV-9

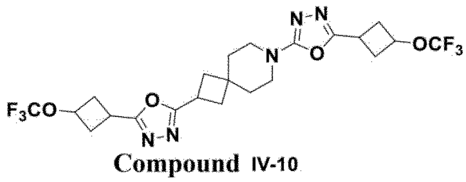

Compound IV-10

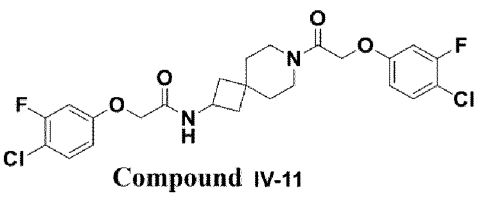

Compound IV-11

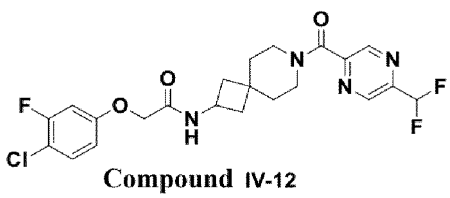

Compound IV-12

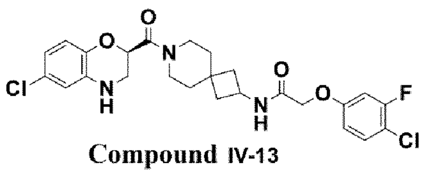

Compound IV-13

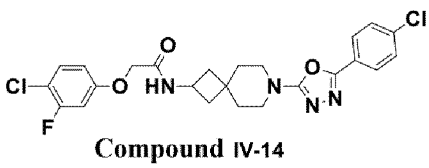

Compound IV-14

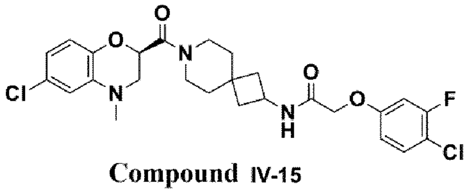

Compound IV-15

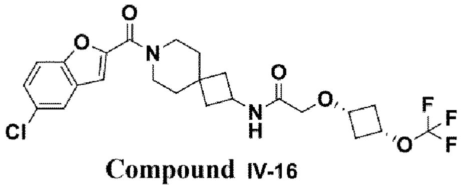

Compound IV-16

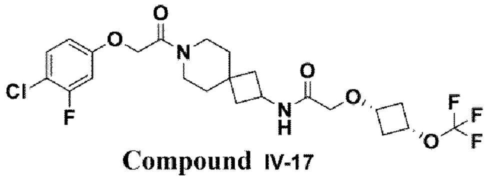

Compound IV-17

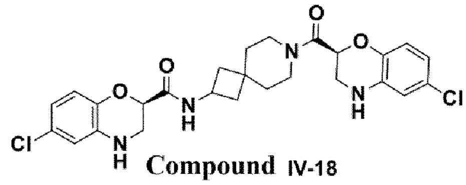

Compound IV-18

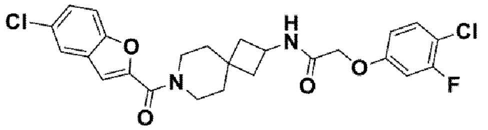

Compound IV-19

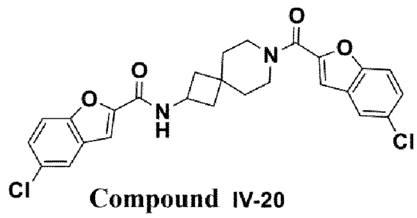

Compound IV-20

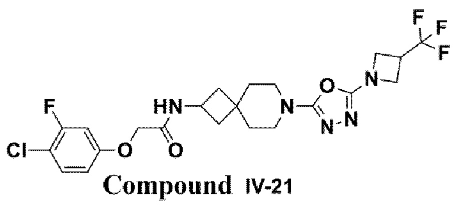

Compound IV-21

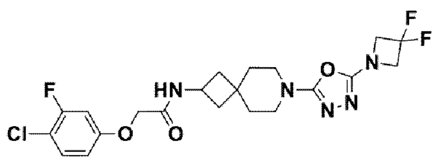

Compound IV-22

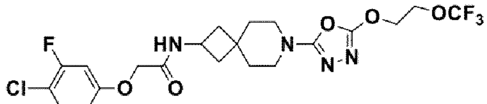

Compound IV-23

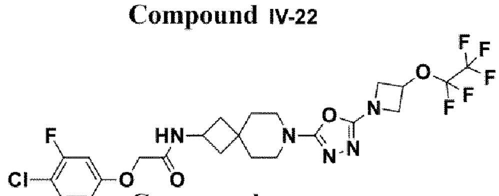

Compound IV-24

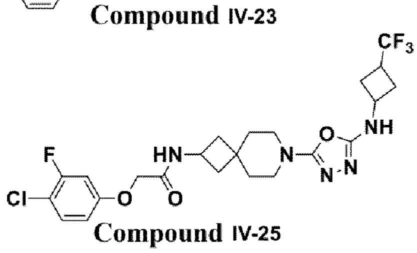

Compound IV-25

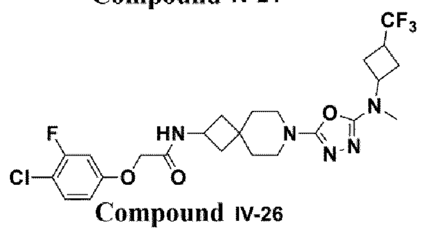

Compound IV-26

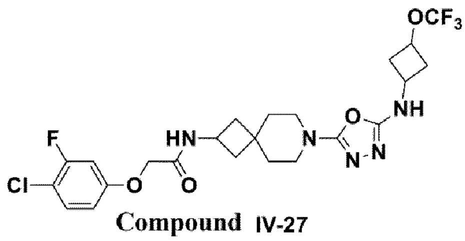

Compound IV-27

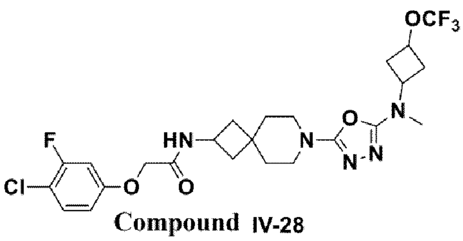

Compound IV-28

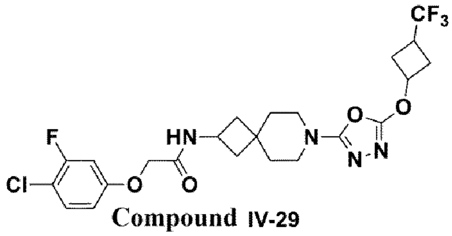

Compound IV-29

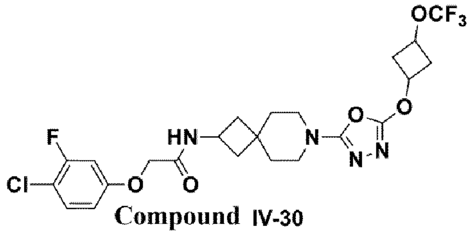

Compound IV-30

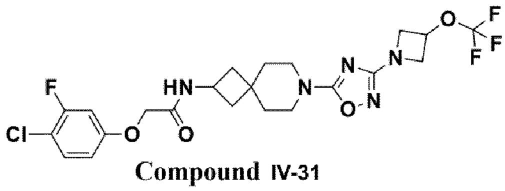

Compound IV-31

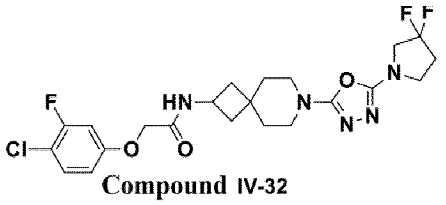

Compound IV-32

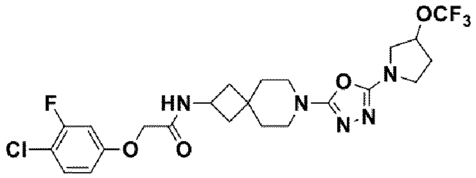

Compound IV-33

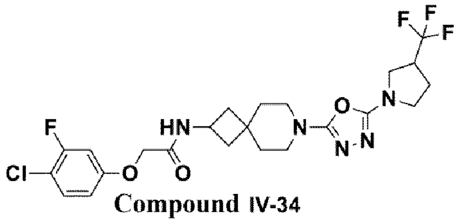

Compound IV-34

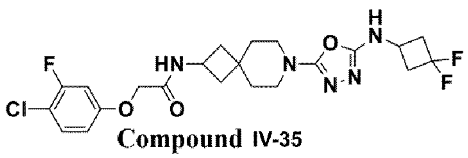

Compound IV-35

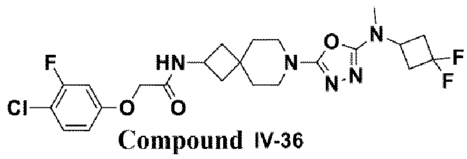

Compound IV-36

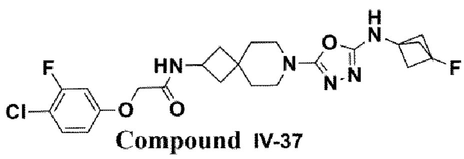

Compound IV-37

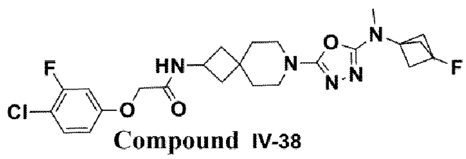

Compound IV-38

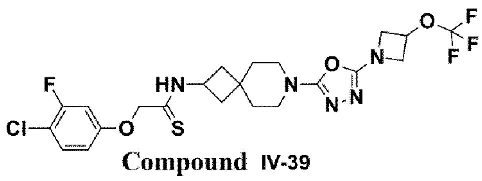

Compound IV-39

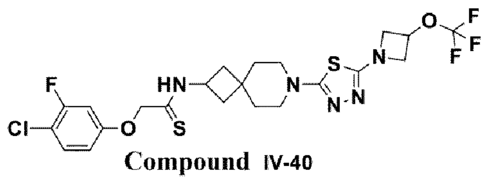

Compound IV-40

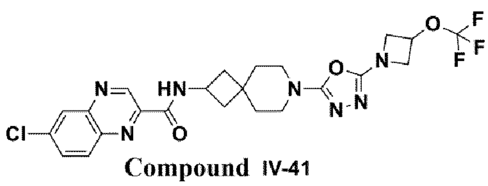

Compound IV-41

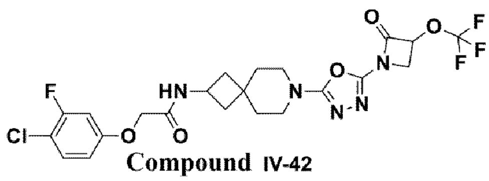

Compound IV-42

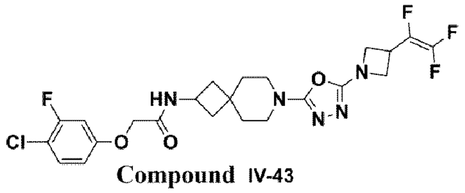
Compound IV-43

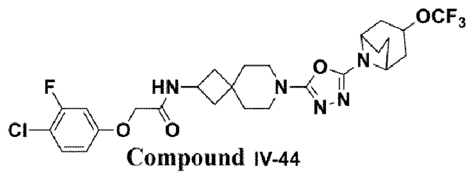
Compound IV-44

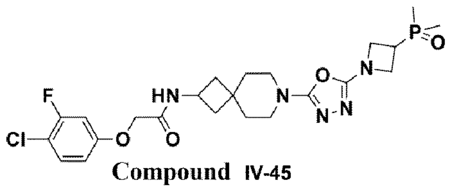
Compound IV-45

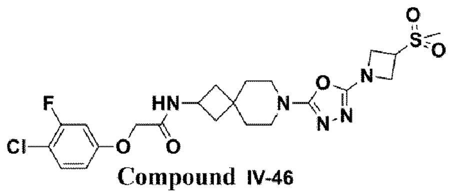
Compound IV-46

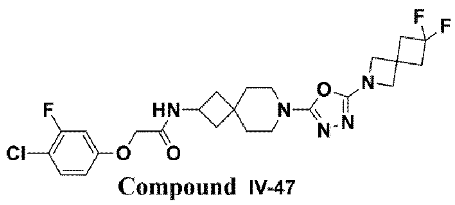
Compound IV-47

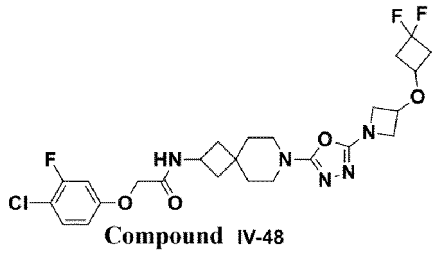
Compound IV-48

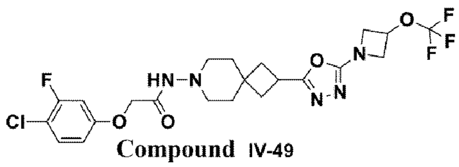
Compound IV-49

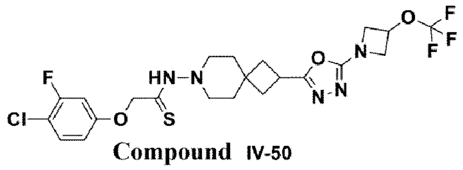
Compound IV-50

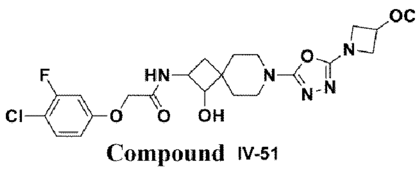
Compound IV-51

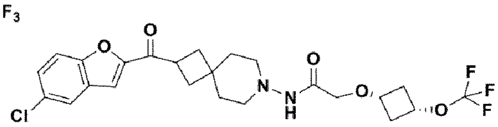
Compound IV-52

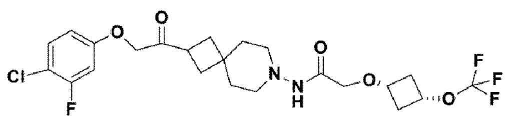
Compound IV-53

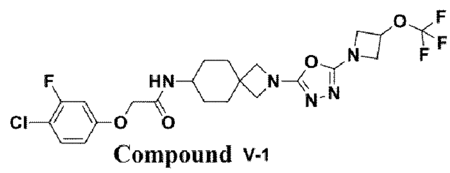
Compound V-1

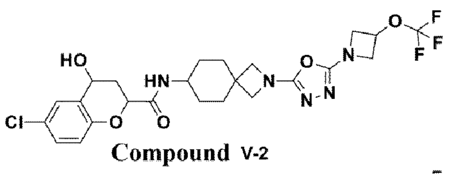
Compound V-2

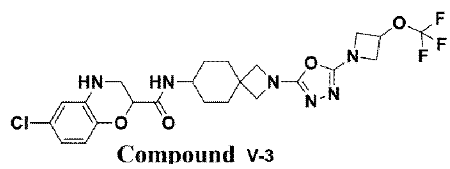
Compound V-3

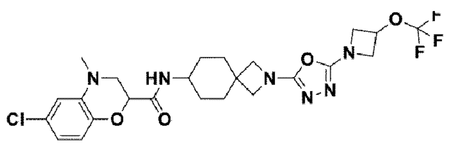
Compound V-4

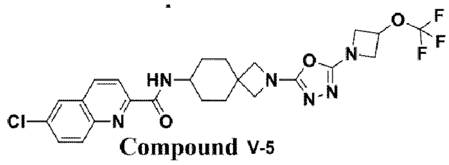
Compound V-5

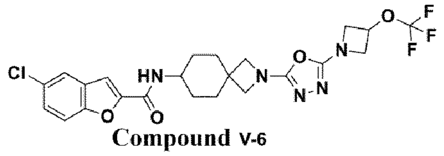
Compound V-6

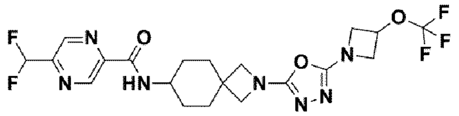
Compound V-7

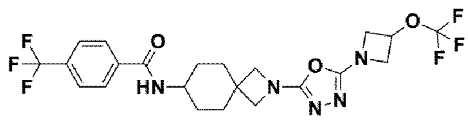
Compound V-8

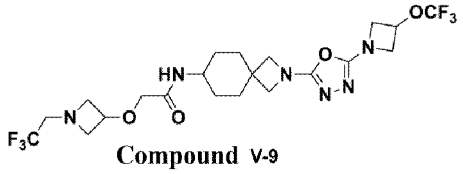
Compound V-9

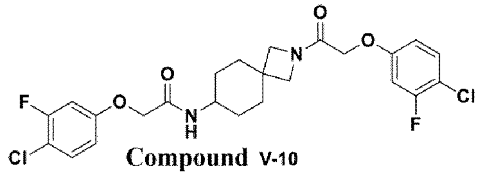
Compound V-10

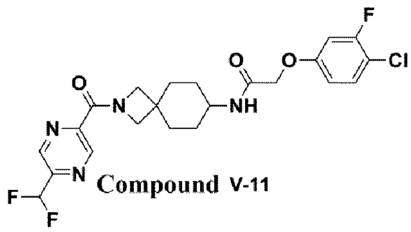
Compound V-11

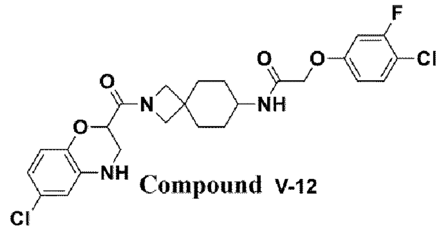
Compound V-12

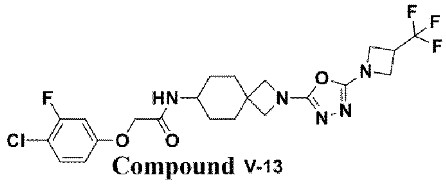
Compound V-13

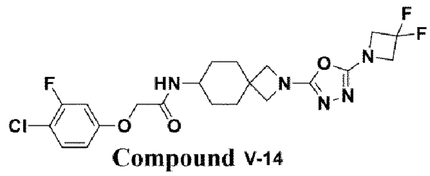
Compound V-14

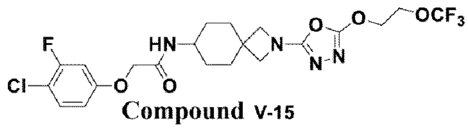
Compound V-15

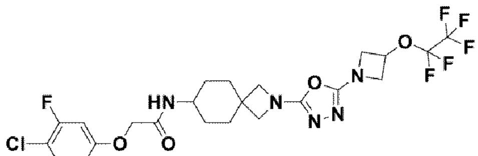
Compound V-16

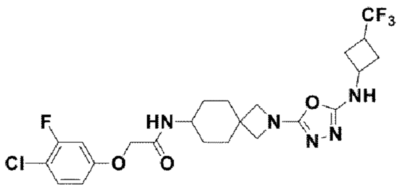
Compound V-17

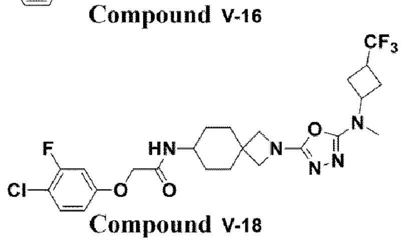
Compound V-18

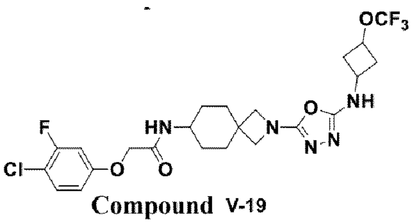
Compound V-19

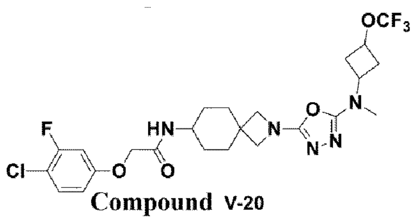
Compound V-20

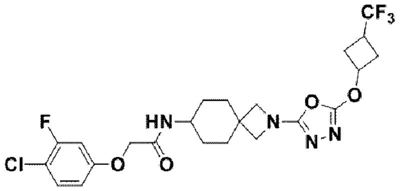

Compound V-21

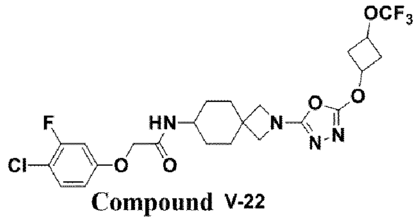

Compound V-22

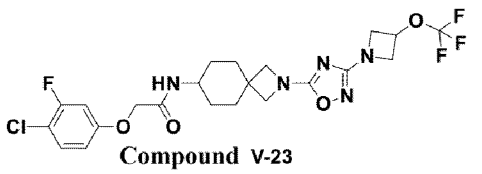

Compound V-23

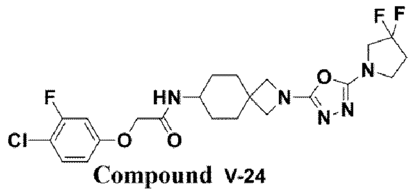

Compound V-24

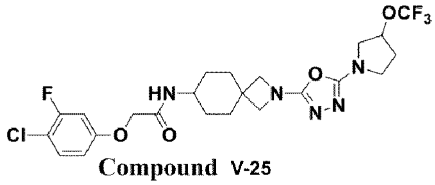

Compound V-25

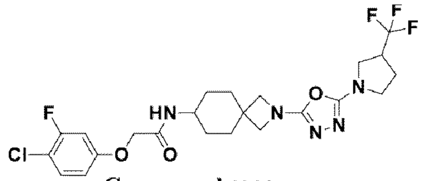

Compound V-26

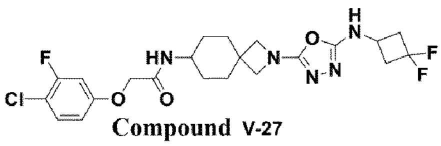

Compound V-27

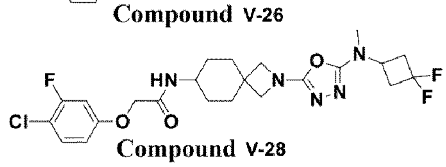

Compound V-28

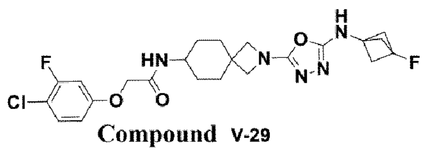

Compound V-29

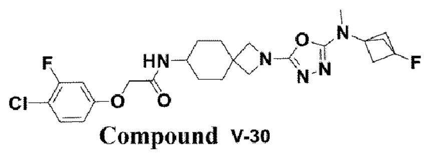

Compound V-30

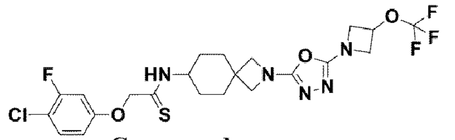
Compound V-31

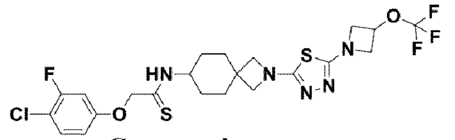
Compound V-32

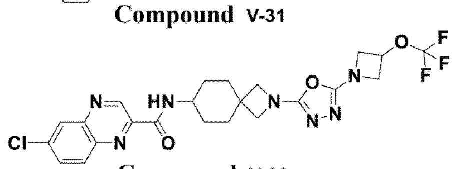
Compound V-33

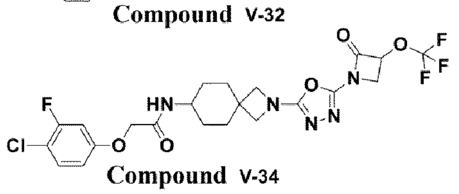
Compound V-34

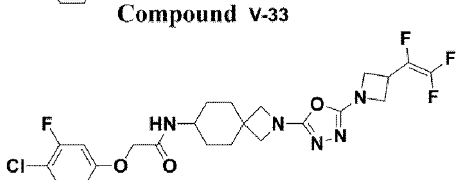
Compound V-35

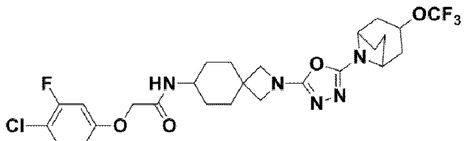
Compound V-36

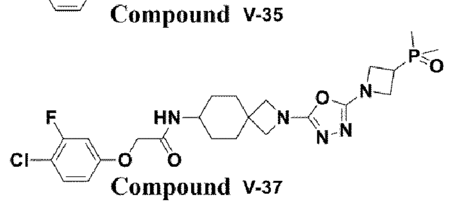
Compound V-37

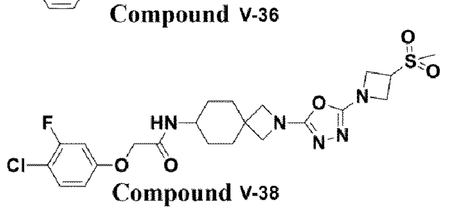
Compound V-38

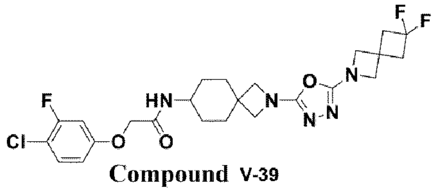
Compound V-39

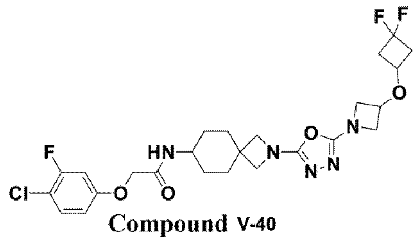
Compound V-40

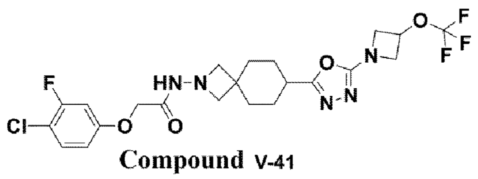
Compound V-41

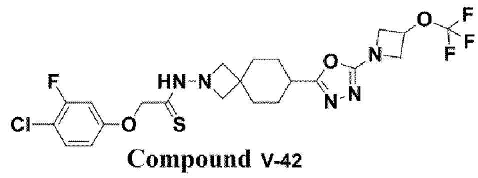
Compound V-42

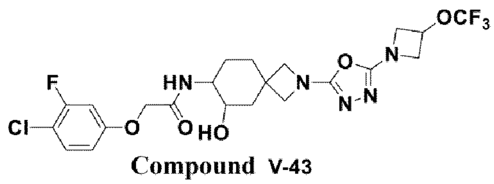
Compound V-43

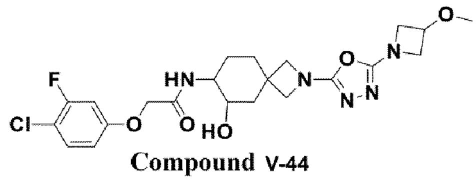
Compound V-44

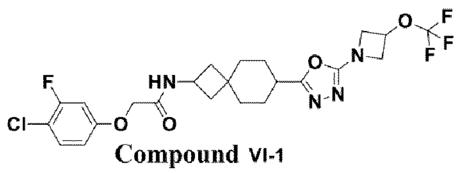
Compound VI-1

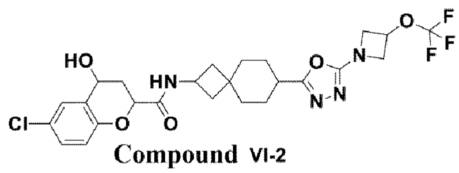
Compound VI-2

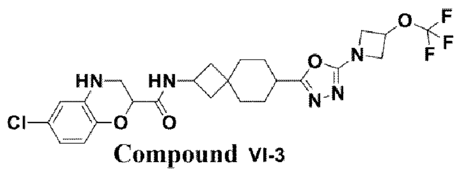
Compound VI-3

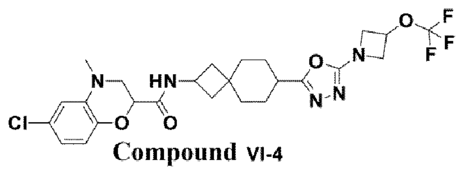
Compound VI-4

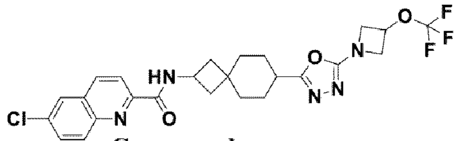

Compound VI-5

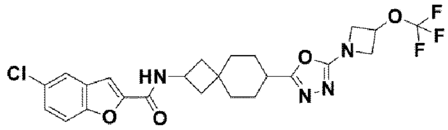

Compound VI-6

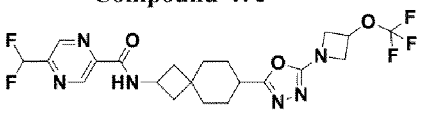

Compound VI-7

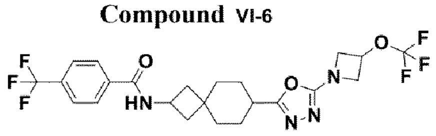

Compound VI-8

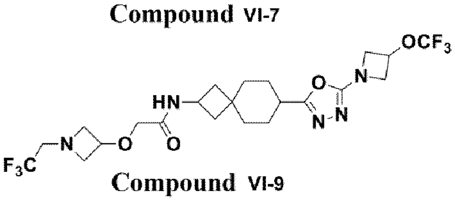

Compound VI-9

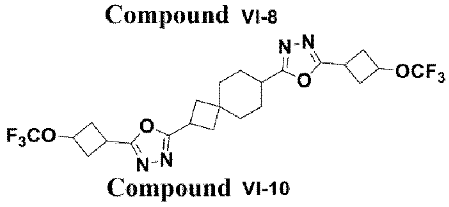

Compound VI-10

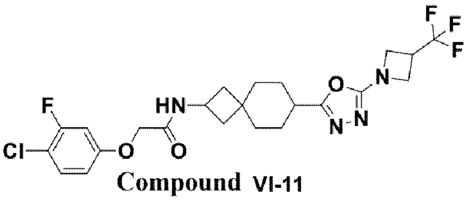

Compound VI-11

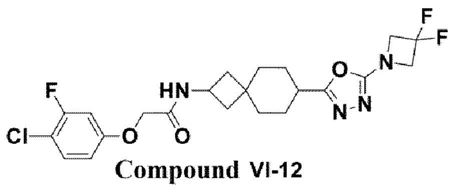

Compound VI-12

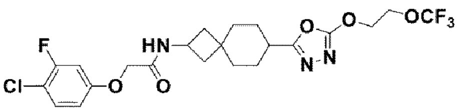

Compound VI-13

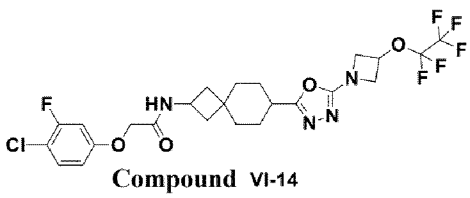

Compound VI-14

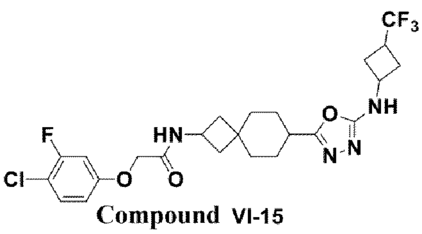

Compound VI-15

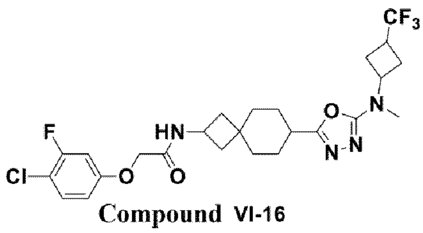

Compound VI-16

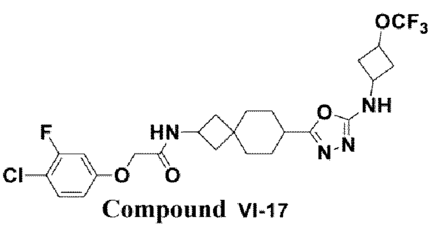

Compound VI-17

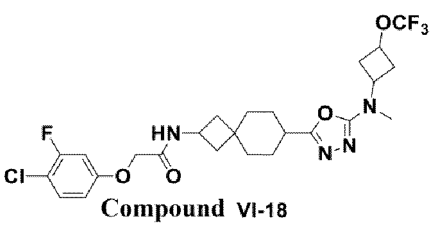

Compound VI-18

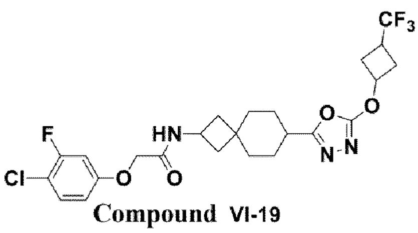

Compound VI-19

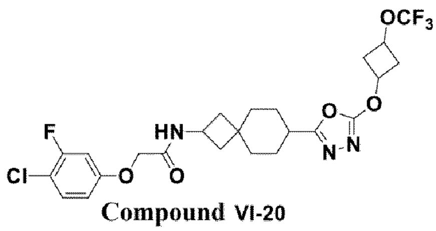

Compound VI-20

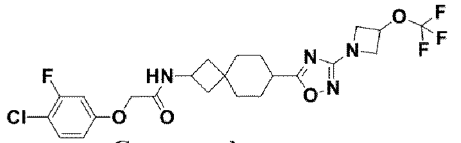
Compound VI-21

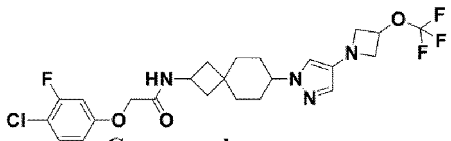
Compound VI-22

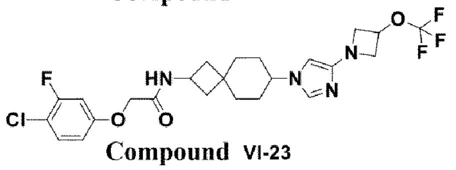
Compound VI-23

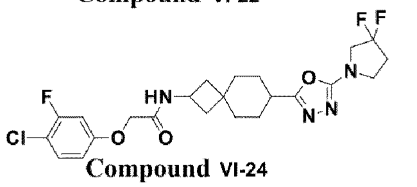
Compound VI-24

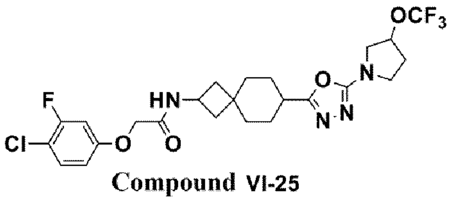
Compound VI-25

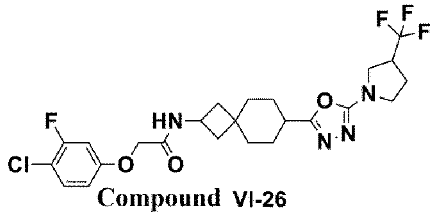
Compound VI-26

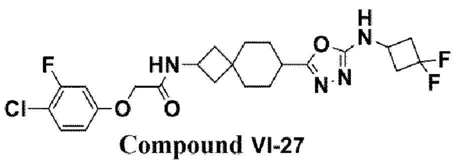
Compound VI-27

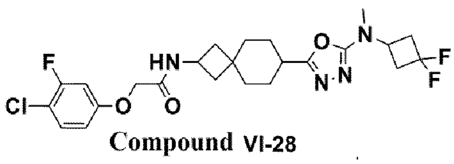
Compound VI-28

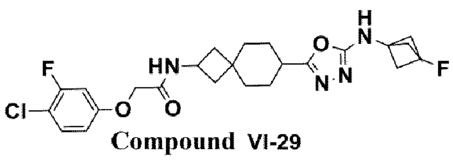
Compound VI-29

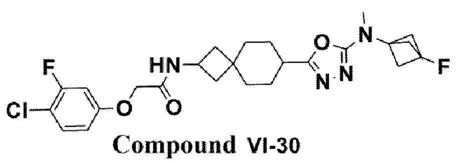
Compound VI-30

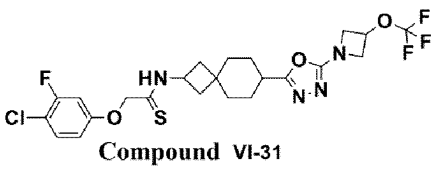
Compound VI-31

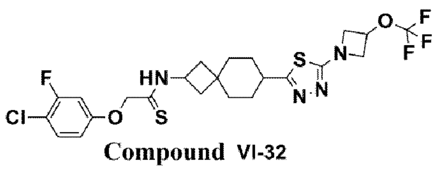
Compound VI-32

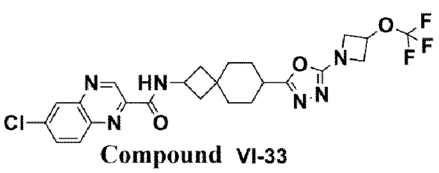
Compound VI-33

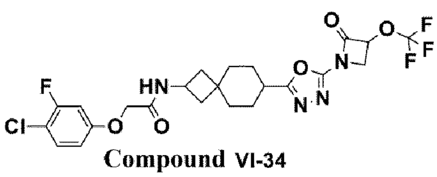
Compound VI-34

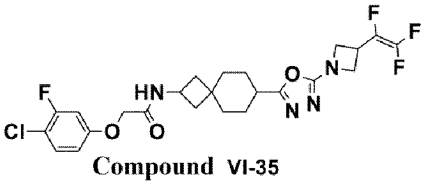
Compound VI-35

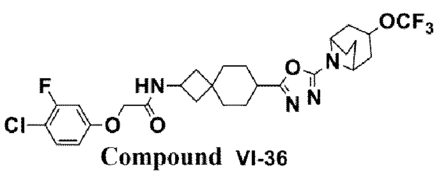
Compound VI-36

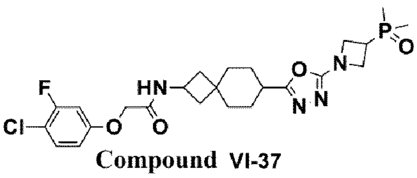
Compound VI-37

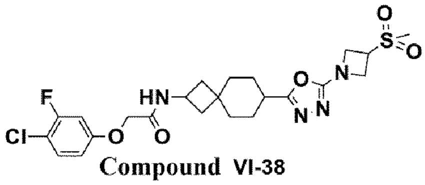
Compound VI-38

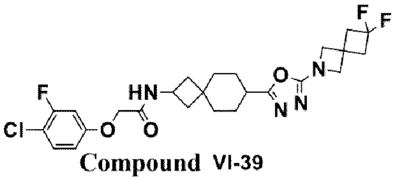
Compound VI-39

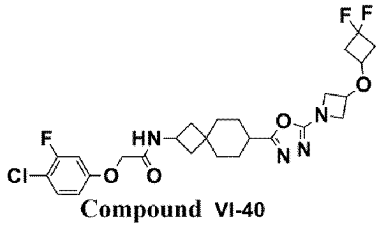
Compound VI-40

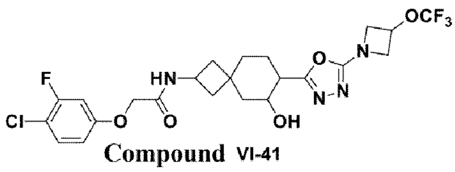
Compound VI-41

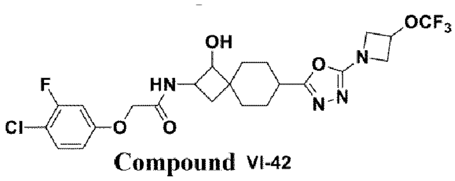
Compound VI-42

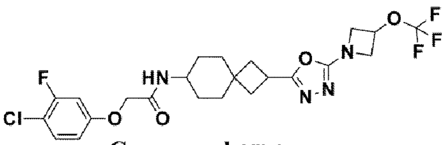
Compound VII-1

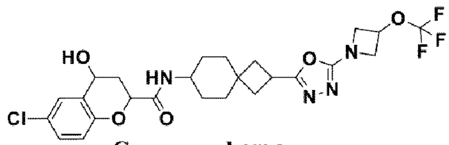
Compound VII-2

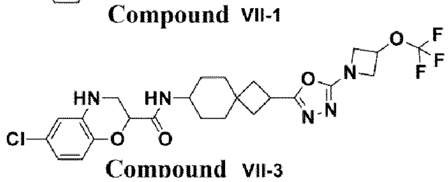
Compound VII-3

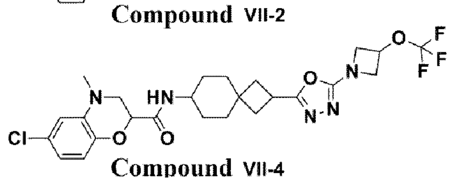
Compound VII-4

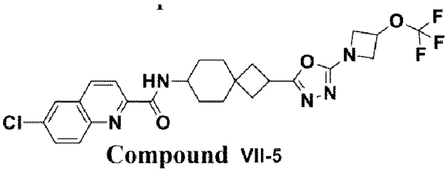
Compound VII-5

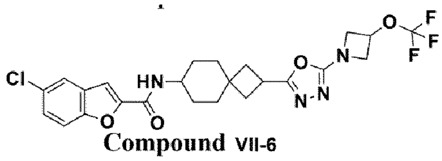
Compound VII-6

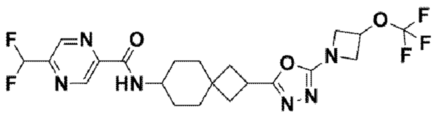
Compound VII-7

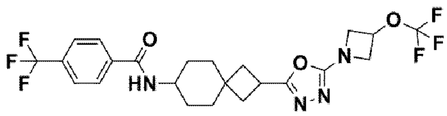
Compound VII-8

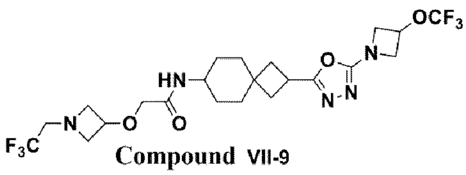
Compound VII-9

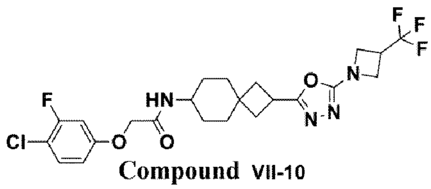
Compound VII-10

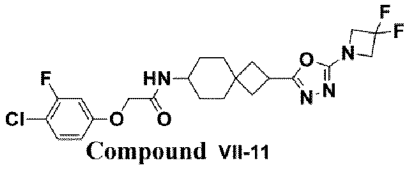

Compound VII-11

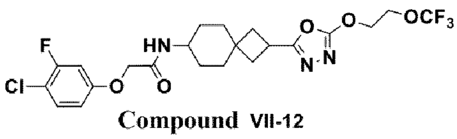

Compound VII-12

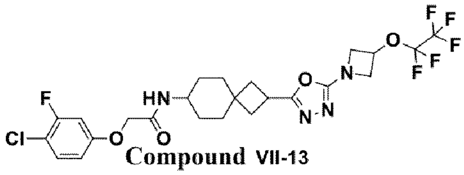

Compound VII-13

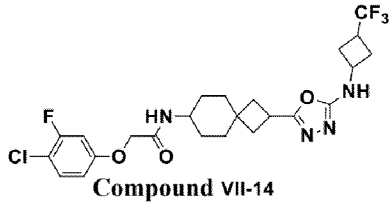

Compound VII-14

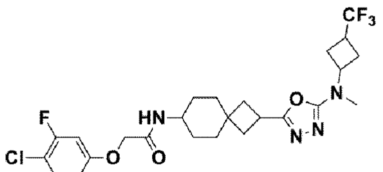

Compound VII-15

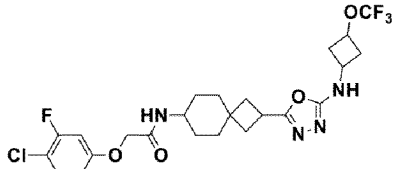

Compound VII-16

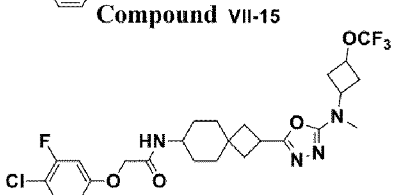

Compound VII-17

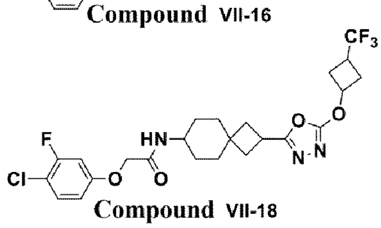

Compound VII-18

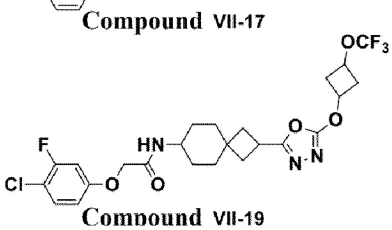

Compound VII-19

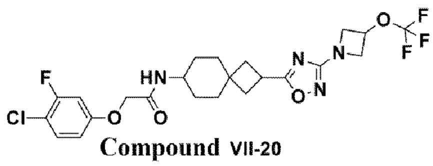

Compound VII-20

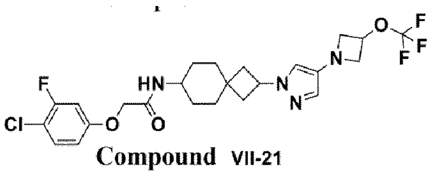

Compound VII-21

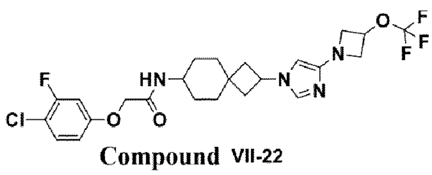

Compound VII-22

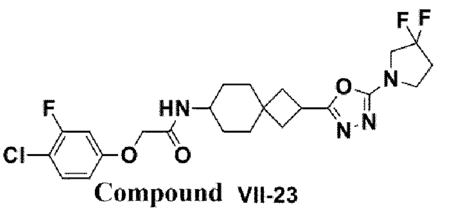

Compound VII-23

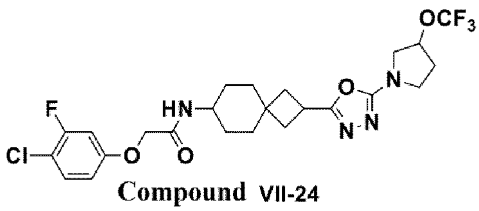

Compound VII-24

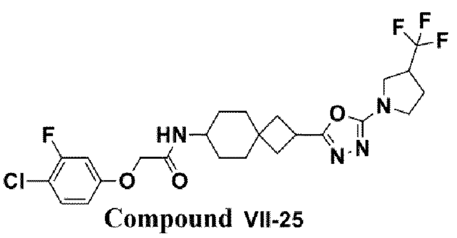

Compound VII-25

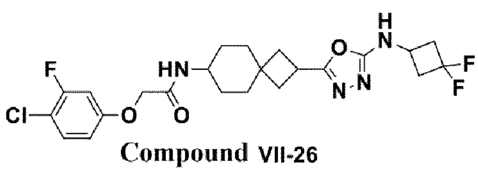

Compound VII-26

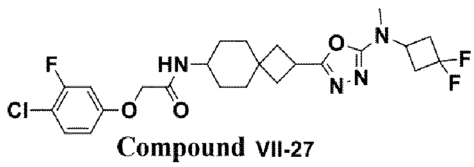

Compound VII-27

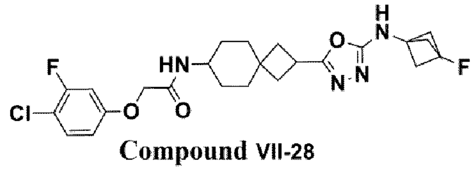

Compound VII-28

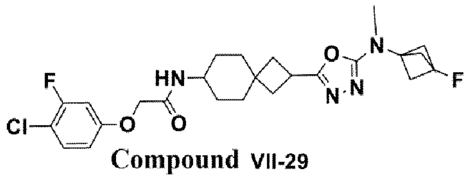

Compound VII-29

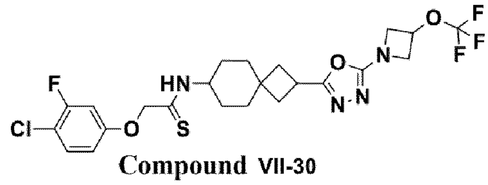

Compound VII-30

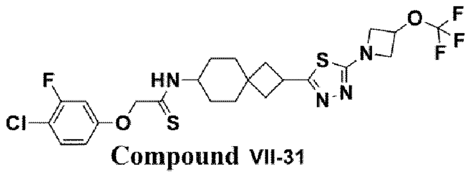

Compound VII-31

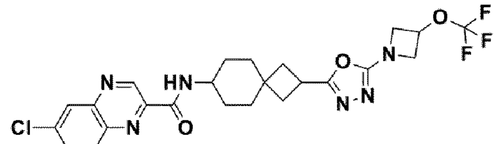

Compound VII-32

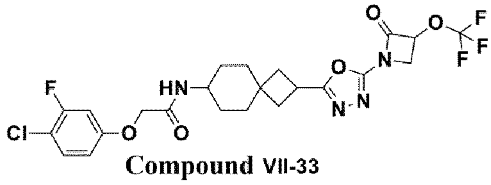

Compound VII-33

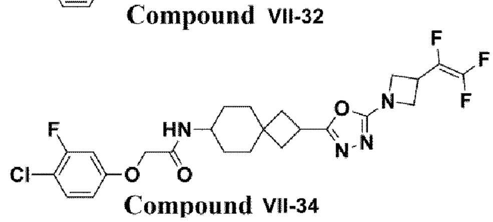

Compound VII-34

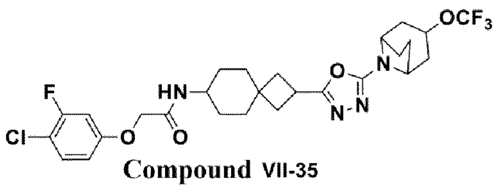

Compound VII-35

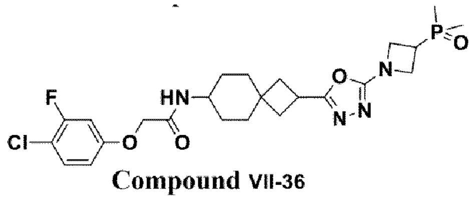

Compound VII-36

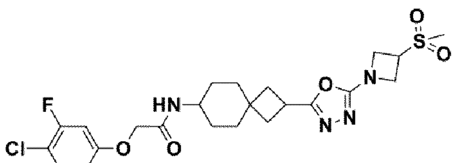

Compound VII-37

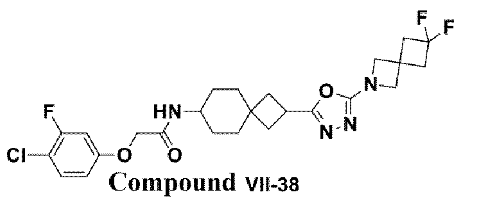

Compound VII-38

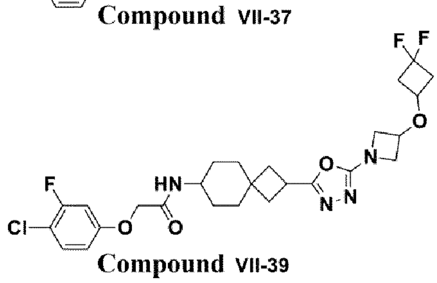

Compound VII-39

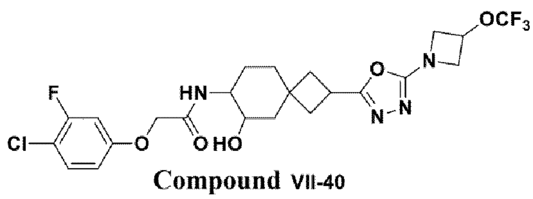

Compound VII-40

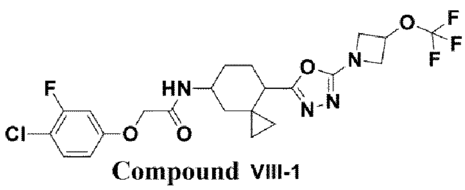

Compound VIII-1

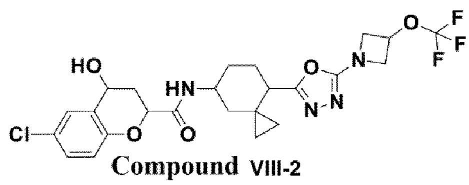

Compound VIII-2

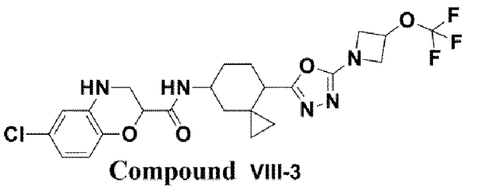

Compound VIII-3

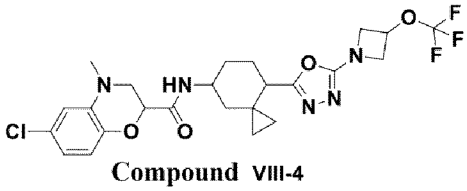

Compound VIII-4

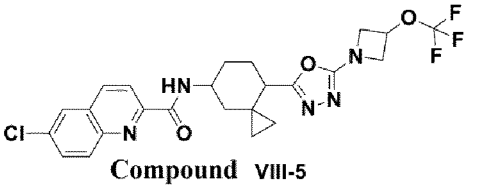

Compound VIII-5

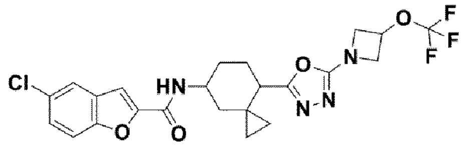

Compound VIII-6

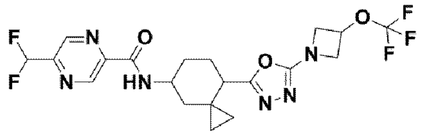

Compound VIII-7

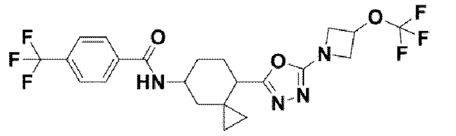

Compound VIII-8

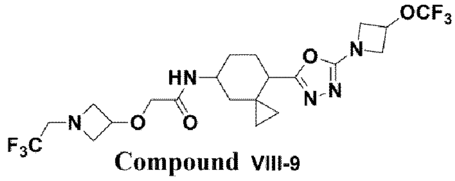

Compound VIII-9

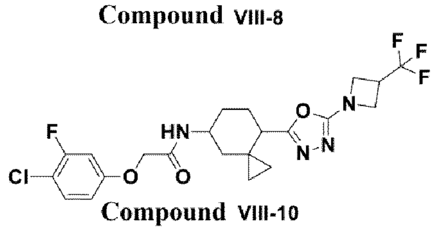

Compound VIII-10

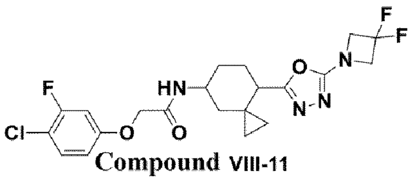

Compound VIII-11

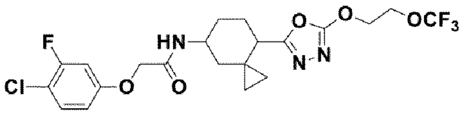

Compound VIII-12

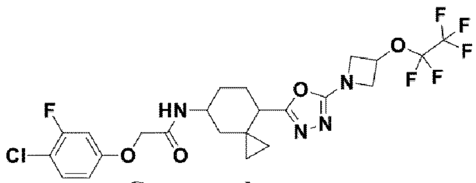

Compound VIII-13

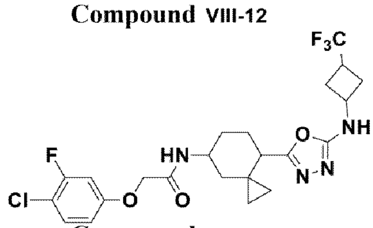

Compound VIII-14

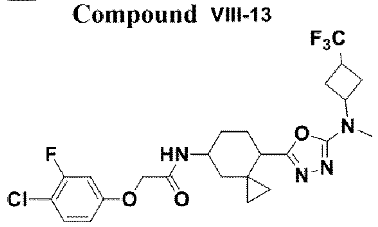

Compound VIII-15

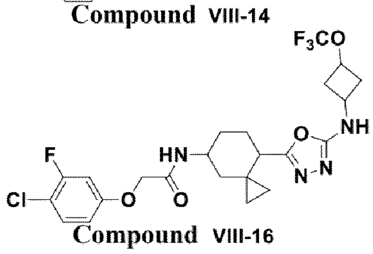

Compound VIII-16

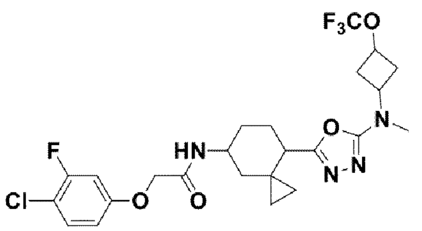

Compound VIII-17

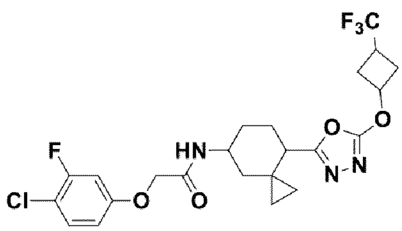

Compound VIII-18

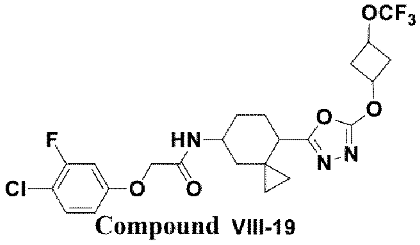

Compound VIII-19

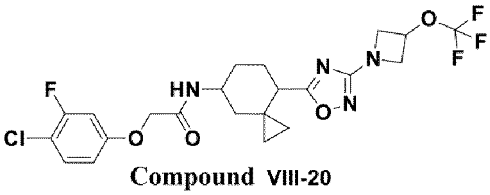

Compound VIII-20

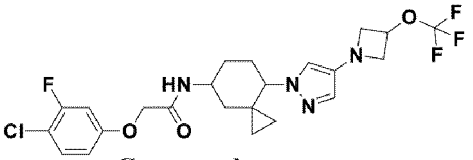

Compound VIII-21

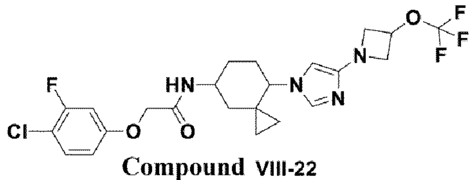

Compound VIII-22

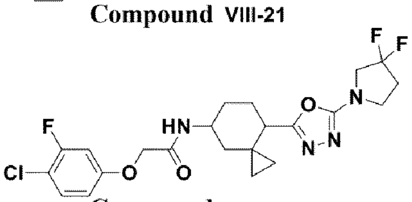

Compound VIII-23

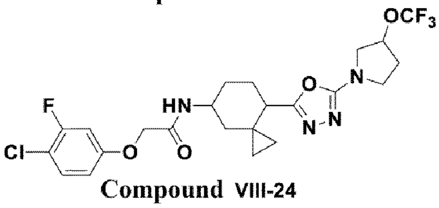

Compound VIII-24

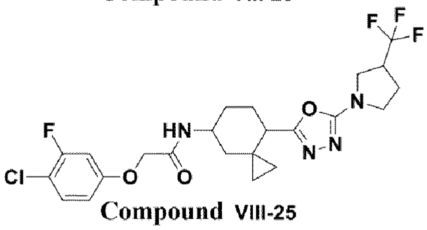

Compound VIII-25

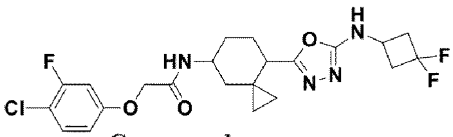

Compound VIII-26

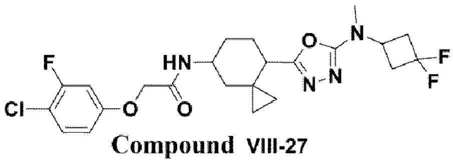

Compound VIII-27

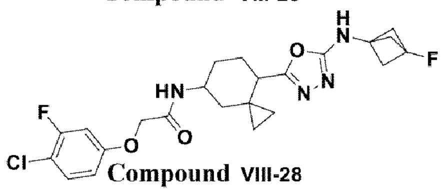

Compound VIII-28

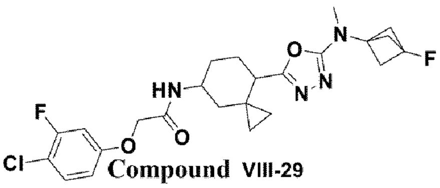

Compound VIII-29

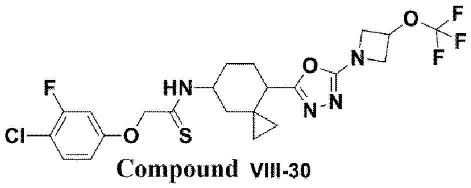

Compound VIII-30

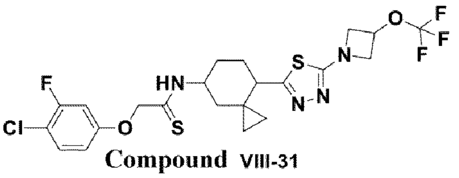

Compound VIII-31

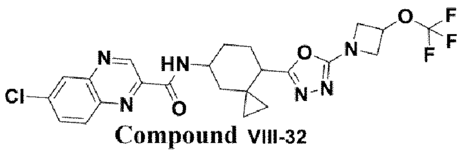

Compound VIII-32

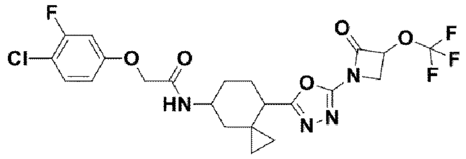
Compound VIII-33

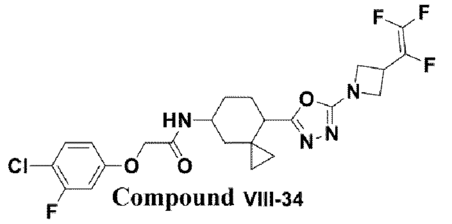
Compound VIII-34

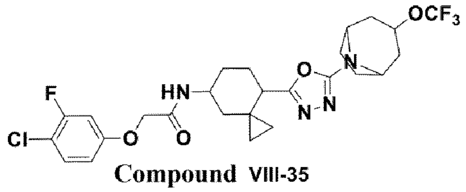
Compound VIII-35

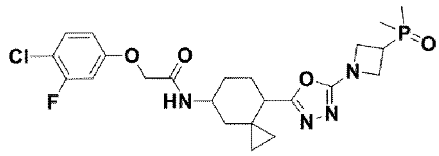
Compound VIII-36

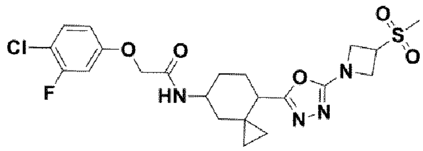
Compound VIII-37

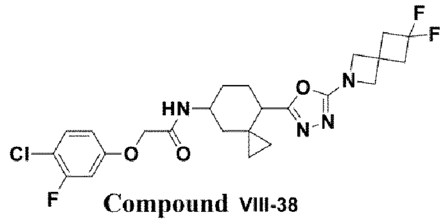
Compound VIII-38

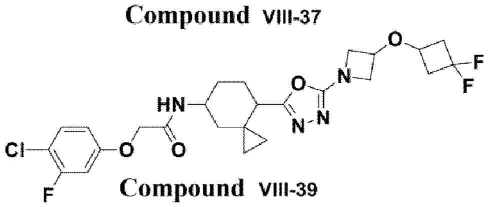
Compound VIII-39

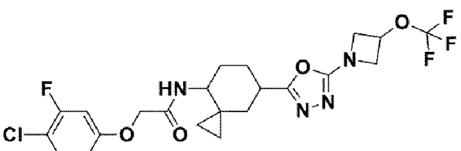
Compound IX-1

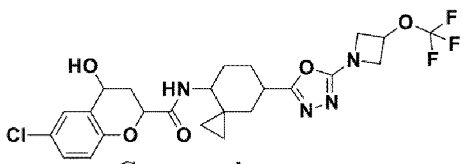
Compound IX-2

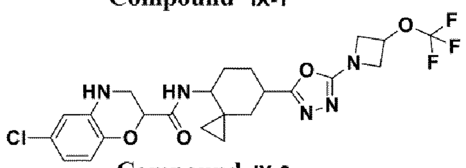
Compound IX-3

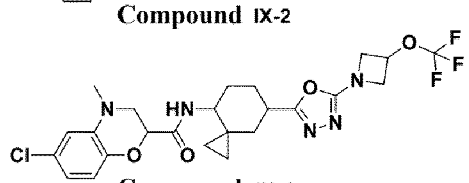
Compound IX-4

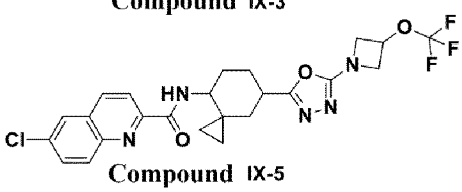
Compound IX-5

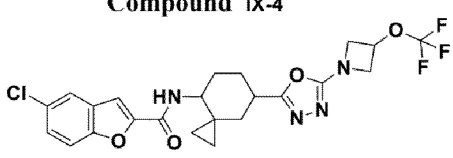
Compound IX-6

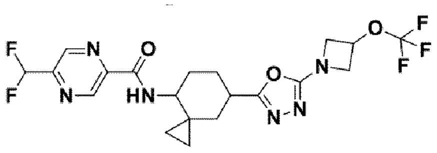
Compound IX-7

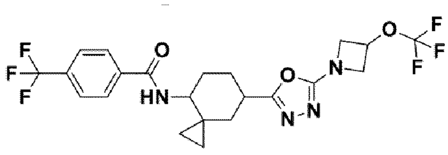
Compound IX-8

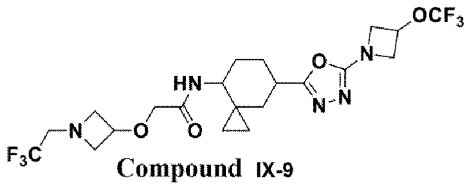

Compound IX-9

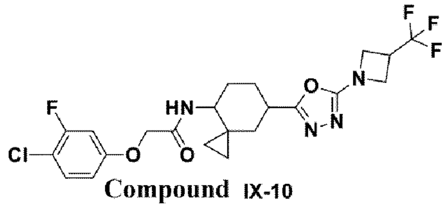

Compound IX-10

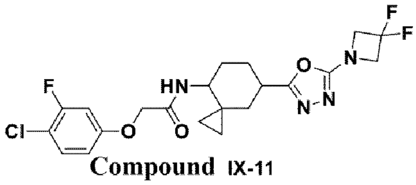

Compound IX-11

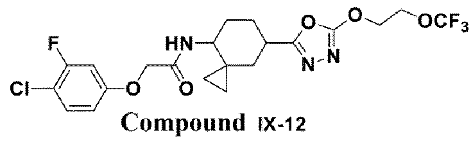

Compound IX-12

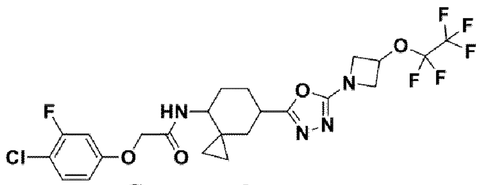

Compound IX-13

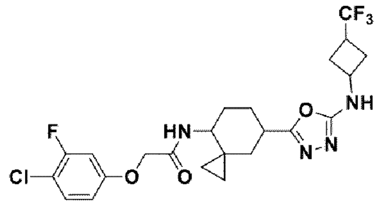

Compound IX-14

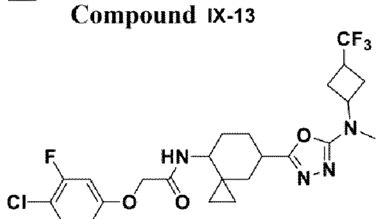

Compound IX-15

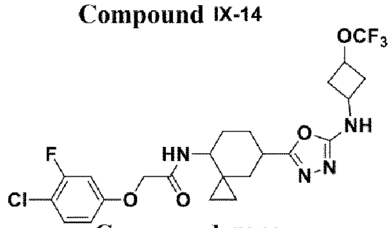

Compound IX-16

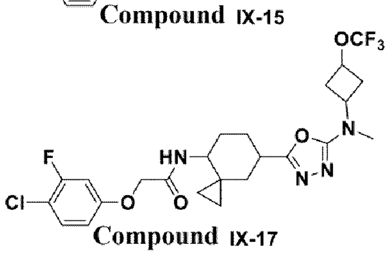

Compound IX-17

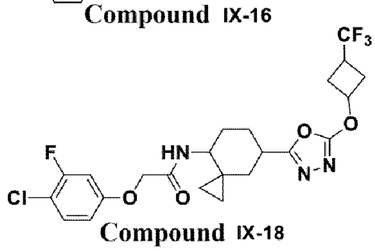

Compound IX-18

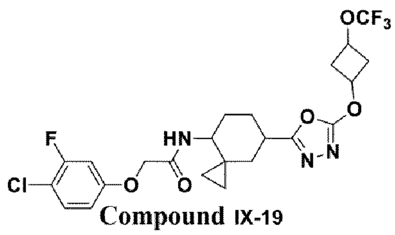

Compound IX-19

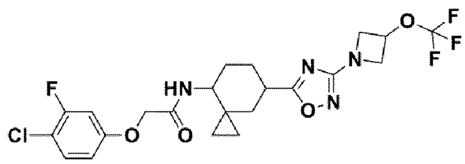

Compound IX-20

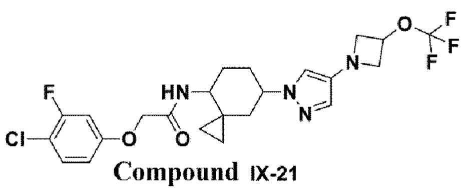

Compound IX-21

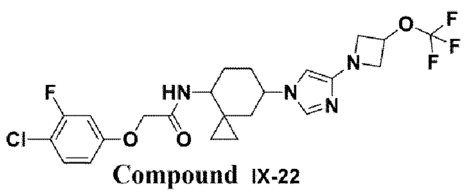

Compound IX-22

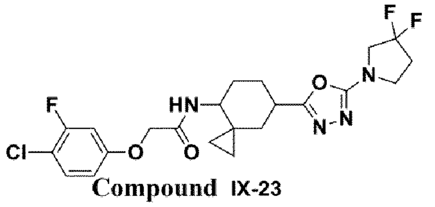

Compound IX-23

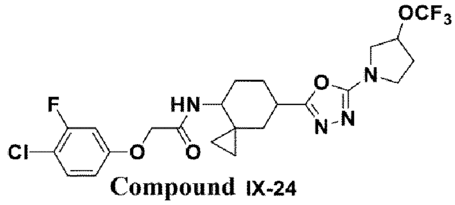

Compound IX-24

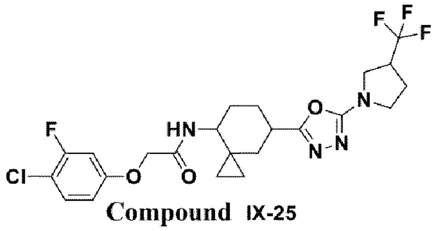

Compound IX-25

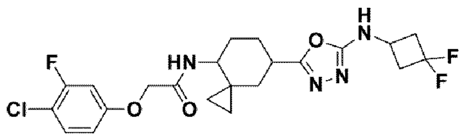

Compound IX-26

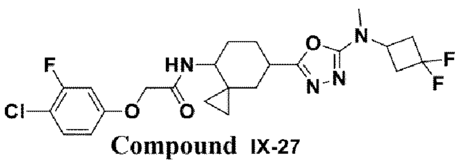

Compound IX-27

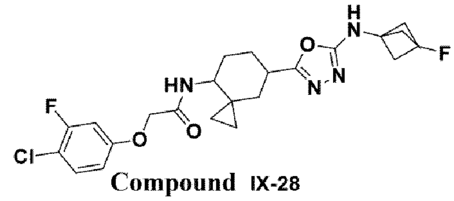

Compound IX-28

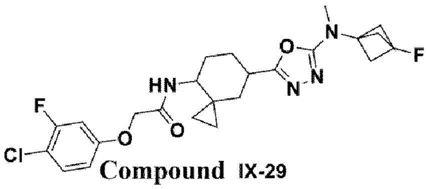

Compound IX-29

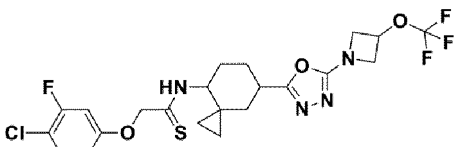

Compound IX-30

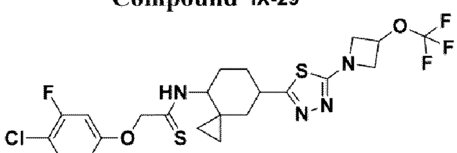

Compound IX-31

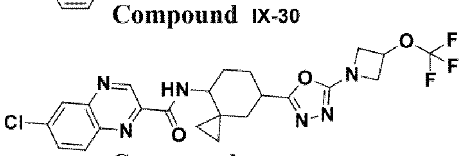

Compound IX-32

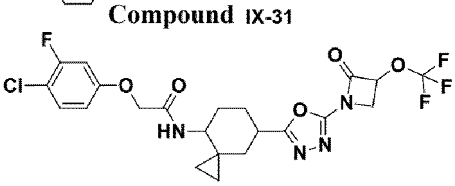

Compound IX-33

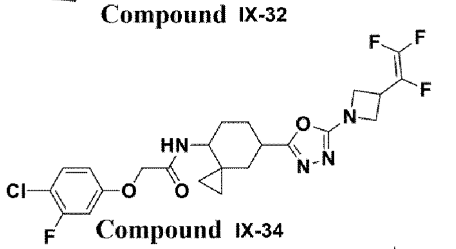

Compound IX-34

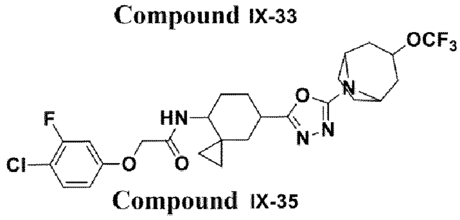

Compound IX-35

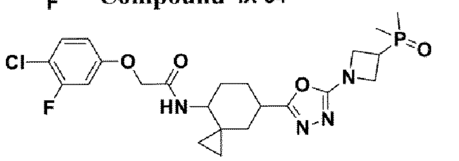

Compound IX-36

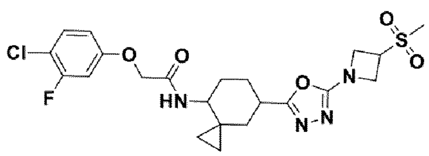

Compound IX-37

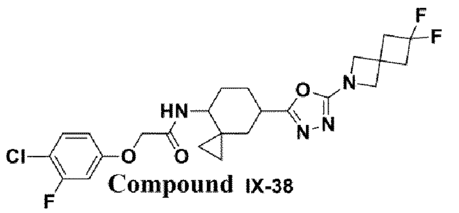

Compound IX-38

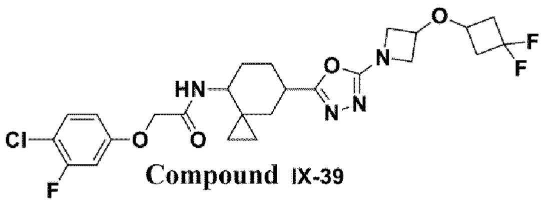

Compound IX-39

**19.** A pharmaceutical composition comprising a preparation prepared from the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18.

**20.** The pharmaceutical composition according to claim 19, further comprising a pharmaceutically acceptable carrier, excipient and vehicle.

**21.** Use of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18, or the pharmaceutical composition according to claim 19 or 20 in the preparation of a drug for preventing and/or treating neurodegenerative diseases, cancer, inflammatory diseases, autoimmune diseases, viral infections, skin diseases, fibrotic diseases, hemoglobin diseases, kidney diseases, hearing loss diseases, eye diseases, diseases with mutations causing the induction of unfolded protein response (UPR), malaria infections, musculoskeletal diseases, metabolic diseases or mitochondrial diseases.

**22.** Use of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18, or the pharmaceutical composition according to claim 19 or 20 in the preparation of a drug for preventing and/or treating a disease or condition mediated by an integrated stress response (ISR) pathway.

**23.** A method for treating a disease or condition mediated by an integrated stress response (ISR) pathway in an individual in need thereof, wherein the method comprises administering a therapeutically effective amount of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18, or the pharmaceutical composition according to claim 19 or 20 to the individual.

**24.** A method for treating a disease related to regulation of eIF2B activity or level, eIF2 pathway activity or level or ISR pathway activity or level, wherein the method comprises administering a therapeutically effective amount of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18, or the pharmaceutical composition according to claim 19 or 20 to a subject.

**25.** A method for preventing and/or treating a disease or condition mediated by an integrated stress response (ISR) pathway, wherein the method comprises administering an effective amount of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18, or the pharmaceutical composition according to claim 19 or 20 to a subject in need thereof.

**26.** A method for preventing and/or treating cancer, wherein the method comprises administering an effective amount of the compound, or the stereoisomer thereof, the tautomer thereof, the geometric isomer thereof, the enantiomer thereof, the diastereomer thereof, the racemate thereof, the polymorph thereof, the solvate thereof, the hydrate thereof, the N-oxide thereof, the isotopically labeled compound thereof, the metabolite thereof, the ester thereof, the prodrug thereof or the pharmaceutically acceptable salt thereof according to any one of claims 1-18, or the pharmaceutical composition according to claim 19 or 20 to a subject in need thereof.

**INTERNATIONAL SEARCH REPORT**

International application No.
**PCT/CN2023/111229**

**A. CLASSIFICATION OF SUBJECT MATTER**

C07D417/14(2006.01)i; C07D413/14(2006.01)i; C07D271/113(2006.01)i; A61K31/4178(2006.01)i; A61K31/445(2006.01)i; A61P25/28(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS, CNTXT, DWPI, WPABS, CAPLUS(STN), REGISTRY(STN), MARPAT(STN), CNKI: 结构式检索, structural formula search, 螺环, 螺杂环, 整合应激反应, 渐冻症, amyotrophic lateral sclerosis, motor neurone disease, ISR, eIF2B

**C. DOCUMENTS CONSIDERED TO BE RELEVANT**

| Category* | Citation of document, with indication, where appropriate, of the relevant passages | Relevant to claim No. |
|---|---|---|
| X | ACS. "CAS registry number 2755298-21-4, 2755297-90-4, etc." *STN Registry,* 05 January 2022 (2022-01-05), pp. 1-13 | 13 |
| X | ACS. "CAS registry number 2700327-46-2, 2700183-17-9, etc." *STN Registry,* 26 September 2021 (2021-09-26), pp. 1-16 | 14 |
| X | CN 113518618 A (DENALI THERAPEUTICS INC.) 19 October 2021 (2021-10-19) claims 1-7, and description, table 1, paragraph 45, and biological example 1 | 13-26 |
| X | US 2021130308 A1 (DENALI THERAPEUTICS INC.) 06 May 2021 (2021-05-06) description, pages 1, 19-20, 22, 35 and 38-40 | 13-26 |
| X | WO 2019078968 A2 (ANGEX PHARMACEUTICAL, INC.) 25 April 2019 (2019-04-25) claim 49, and description, pages 74 and 80 | 15, 17 |
| X | US 2010130477 A1 (ASTRAZENECA AB) 27 May 2010 (2010-05-27) embodiments 1-3, 8-26, 34-36, 42-47, 53-55, 57 and 64-67 | 15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 November 2023** | **21 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/111229**

**C. DOCUMENTS CONSIDERED TO BE RELEVANT**

| Category* | Citation of document, with indication, where appropriate, of the relevant passages | Relevant to claim No. |
|---|---|---|
| X | ACS. "CAS registry number 2637340-72-6, 2637340-51-1, etc." *STN Registry,* 23 April 2021 (2021-04-23), pp. 1-18 | 15 |
| X | ACS. "CAS registry number 2637340-37-3, 2637135-51-2, etc." *STN Registry,* 23 April 2021 (2021-04-23), pp. 1-4 | 16 |
| X | CN 111263752 A (GLAXOSMITHKLINE INTELLECTUAL PROPERTY DEVELOPMENT LIMITED) 09 June 2020 (2020-06-09) abstract, and description, embodiments 3-4 | 17 |

Form PCT/ISA/210 (second sheet) (July 2022)

**INTERNATIONAL SEARCH REPORT**

International application No.
**PCT/CN2023/111229**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **23-26**
because they relate to subject matter not required to be searched by this Authority, namely:

The subject matter of claims 23-26 falls within methods for treatment of a disease in the human body (PCT Rule 39.1(iv)). The search is carried out on the basis of the use of a compound, a stereoisomer thereof, etc. in the preparation of a drug for treating and/or preventing a corresponding disease.

2. ☑ Claims Nos.: **1-12 (all), 13-17 (in part), 19-26 (in part)**
because they relate to parts of the international application that do not comply with the prescribed requirements to such an extent that no meaningful international search can be carried out, specifically:

Claims 1-12 relate to a compound of formula 0, claims 13-17 relate to compounds of formulae I-IX, and claims 19-26 relate to the use of compounds. The formula 0 is composed of five variables in total, i.e., fragments A, B and C and linking group fragments $L^1$ and $L^2$. Claims 1-12 define these variables broadly. Similarly, claims 13-17 also broadly define fragments such as ring A, ring C, linking group $L^1$ and/or linking group $L^2$ in formulae I-IX. Therefore, formula 0 and formulae I-IX cover a large number of compounds, which include a large number of compounds known in the prior art. Moreover, a person skilled in the art would not have been able to expect that all the compounds within formulae 0 and I-IX defined in claims 1-17 can achieve the purpose of the present application. Therefore, the search for the claims is made on the basis of a reasonable range, i.e., the technical solutions when relating to $L^2$ in formula I of claim 13 being or , $L^1$ in formulae II-III of claim 14 being empty or , and $L^2$ being or , $L^1$ in formulae IV-VII of claims 15-17 being empty or , and $L^2$ being empty, or , $L^1$ and $L^2$ in formulae VIII-IX of claim 17 being empty, or , all the solutions of claim 18, and some of the technical solutions of claims 19-26. No search is performed on claims 1-12, some of the technical solutions of claims 13-17, and some of the technical solutions of claims 19-26.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/111229**

| Patent document cited in search report | Publication date (day/month/year) | Patent family member(s) | Publication date (day/month/year) |
|---|---|---|---|
| CN 113518618 A | 19 October 2021 | MA 54953 A | 22 December 2021 |
| | | EP 3923935 A1 | 22 December 2021 |
| | | EP 3923935 A4 | 26 October 2022 |
| | | SG 11202108552 QA | 29 September 2021 |
| | | AU 2020223228 A1 | 26 August 2021 |
| | | CL 2021002104 A1 | 08 April 2022 |
| | | MX 2021009669 A | 13 October 2021 |
| | | JP 2022520236 A | 29 March 2022 |
| | | WO 2020168011 A1 | 20 August 2020 |
| | | CO 2021010490 A2 | 30 August 2021 |
| | | US 2023114472 A1 | 13 April 2023 |
| | | CA 3129609 A1 | 20 August 2020 |
| | | CR 20210426 A | 30 September 2021 |
| | | PE 20212023 A1 | 18 October 2021 |
| | | KR 20210126036 A | 19 October 2021 |
| | | US 2021292311 A1 | 23 September 2021 |
| | | US 11306077 B2 | 19 April 2022 |
| | | TW 202045164 A | 16 December 2020 |
| | | IL 285302 A | 30 September 2021 |
| US 2021130308 A1 | 06 May 2021 | WO 2019183589 A1 | 26 September 2019 |
| | | EP 3768660 A1 | 27 January 2021 |
| | | US 2023250072 A1 | 10 August 2023 |
| WO 2019078968 A2 | 25 April 2019 | WO 2019078968 A3 | 02 April 2020 |
| | | US 2020239464 A1 | 30 July 2020 |
| | | US 11180497 B2 | 23 November 2021 |
| US 2010130477 A1 | 27 May 2010 | TW 201024276 A | 01 July 2010 |
| | | WO 2010062245 A1 | 03 June 2010 |
| | | UY 32258 A | 30 June 2010 |
| | | AR 074213 A1 | 29 December 2010 |
| CN 111263752 A | 09 June 2020 | BR 112020008490 A2 | 06 October 2020 |
| | | CA 3080100 A1 | 09 May 2019 |
| | | JP 2021501164 A | 14 January 2021 |
| | | US 2020239420 A1 | 30 July 2020 |
| | | WO 2019087028 A1 | 09 May 2019 |
| | | EP 3704095 A1 | 09 September 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210945768
- CN 202310729419X